# EUROPEAN PATENT SPECIFICATION

(11) **EP 3 790 616 B1**
(45) Date of publication and mention of the grant of the patent: **31.12.2025**
(21) Application number: 19800701.5
(22) Date of filing: 09.05.2019
(51) Int. Cl.: A61M 16/16, C08J 7/12, C08J 7/06, C08J 5/12, H05K 1/00, C08K 3/34, C08K 5/1539, A61M 13/00, A61M 16/00, A61M 16/08, A61M 16/10, A61M 16/12, A61M 16/14, H05B 3/34, A61M 16/06

(54) **MEDICAL COMPONENTS WITH THERMOPLASTIC MOLDINGS BONDED TO SUBSTRATES**
MEDIZINISCHE KOMPONENTEN MIT AN SUBSTRATE GEBUNDENEN THERMOPLASTISCHEN FORMTEILEN
COMPOSANTS MÉDICAUX AVEC MOULAGES THERMOPLASTIQUES LIÉS À DES SUBSTRATS

(30) Priority: 09.05.2018 US 201862669321 P
(43) Date of publication of application: 17.03.2021
(73) Proprietor: Fisher & Paykel Healthcare Limited, East Tamaki Auckland 2013 (NZ)
(72) Inventor: MASTERTON, Benjamin James Trace, 2013 Auckland (NZ); PRICE, John David, 2013 Auckland (NZ); SITTHIRACHA, Manatchanok, 2013 Auckland (NZ); CHAI, Maurice Wen-Bin, 2013 Auckland (NZ); PEACOCK, Mathew Ian, 2013 Auckland (NZ); CHEN, Jeffrey, 2013 Auckland (NZ); POWELL, Kevin Blake, 2013 Auckland (NZ); KLENNER, Jason Allan, 2013 Auckland (NZ); GIERKE, Timothy Dee, 2013 Auckland (NZ); LESCHER, Peter Edward, deceased (NZ)
(74) Representative: Dehns
(86) International application number: PCT/NZ2019/050050
(87) International publication number: WO 2019/216774

(56) References cited:
- WO-A1-2006/059845
- WO-A1-2012/171072
- WO-A1-2014/088430
- WO-A1-2016/036260
- US-A- 5 700 581
- US-A1- 2008 105 257
- US-A1- 2015 115 483
- US-A1- 2016 128 182
- US-B1- 6 215 011
- US-B2- 6 548 175

## Description

### BACKGROUND

### Field

This disclosure relates generally to components suitable for medical use and more specifically to components that are suitable for providing humidified gases to and/or removing humidified gases from a patient.

### Description of the Related Art

In medical circuits, various components transport naturally or artificially humidified gases to and from patients. For example, a respiratory system can include a respiratory device that can humidify and heat gases passing through the respiratory device to improve patient comfort and/or improve the prognosis of the patient's respiratory illness. Some respiratory devices can include a water reservoir and a heating element for heating the water in the reservoir. As the water heats up, water vapor is formed that can join the stream of gases passing through the respiratory device. Some respiratory devices can include a liquid flow controller for providing a controlled flow of liquid, and a heating system including a heating surface configured to be located in a gases passage way and provide humidification to gases passing through the passage way.

Respiratory humidification systems and arrangements are disclosed in documents WO2014/088430, US2015/115483, US2008/105257 and WO2012/171072.

### SUMMARY

The invention is defined by the appended claims.

Some embodiments provide a molded member with a robust substrate-molding material interface, which can prevent or delay delamination of the molding material from the substrates under elevated humidity and/or elevated temperature environment.

Some embodiments disclosed herein pertain to a humidification apparatus (e.g., for respiratory humidification, insufflation, etc.). In some embodiments, the humidification apparatus comprises a gas flow passage. The gas flow passage may have an internal area and an inlet configured to receive gases into the internal area. The gas flow passage may have an outlet configured to allow the passage of gases out of the internal area of the gas flow passage. In some embodiments, the apparatus comprises a heater having a heating surface. The heater may be disposed between the inlet and the outlet of the gas flow passage. In some implementations, the heater is configured to heat a humidification liquid received by the heating surface to humidify gases flowing through the gas flow passage. In certain embodiments, the heater comprises a printed circuit board (PCB). The PCB may have heating tracks. The PCB may have at least one electrical contact. In some embodiments, the electrical contact is configured to receive and/or send an electrical signal (e.g., from an electricity source, etc.). In some embodiments, the electrical contact is configured to receive electricity from an electricity source (e.g., through an electrical conduit connected to the electrical contact) and to provide power to the PCB. In some embodiments, the electrical contact is in electrical communication with the heating tracks. In some implementations, the heater comprises a molding material disposed over at least a portion of the PCB. The molding material and a portion of the PCB may be adhered by a bonding layer comprising a silicon-containing linker. In some embodiments, the bonding layer couples at least a portion of the molding material to the PCB. In some implementations, the respiratory apparatus is configured to provide to humidified gases to a patient.

In some embodiments, the PCB may span a portion of the gas flow passage from the internal area to an area that is external to the gas flow passage. A portion of the PCB may be exposed to the external area of the gas flow passage while a different portion of the PCB may be in thermal communication with the internal area of the gas flow passage. In some embodiments, the PCB is in thermal communication with the gas flow passage. For example, in some embodiments, the PCB can receive temperature information from the gas flow passage (e.g., from gases in the gas flow chamber), transmit thermal energy to gas flow passage (e.g., to heat the gas(es) in the gas flow passage), or both.

In certain implementations, the PCB comprises one or more electrical components configured to receive, transmit, and/or process information about conditions of the internal area of the respiratory apparatus. For example, in some embodiments, the PCB may receive, transmit, and/or process information (e.g. temperature, pressure, and humidity data) regarding one or more gases in the apparatus. In certain implementations, the PCB comprises one or more electrical components configured to receive, transmit, and/or process information about conditions of the internal area of the gas flow passage. The PCB may comprise one or more sensors. In some embodiments, the PCB may comprise one or more temperature sensors in thermal communication with the internal area of the gas flow passage. The PCB may comprise two or more temperature sensors, wherein at least one of the temperature sensors is configured to measure a temperature of the heating surface. The PCB may comprise two or more temperature sensors, wherein at least one of the temperature sensors is configured to measure a surface temperature of the molding material. In some embodiments, the PCB comprises a temperature sensor configured to detect whether the heating surface is wetted by the humidification liquid in at least one region. The temperature sensor or sensors may be located at, on, adjacent, or proximal to the heating surface. The temperature sensor or sensors may be located at, on, adjacent, or proximal to the molding material. In some embodiments, the PCB comprise one or more of a pressure sensor, a flow sensor, a humidity sensor, and/or a fluid level sensor.

In certain embodiments, the heating tracks of the PCB are in thermal communication with the internal area of the gas flow passage. The heating tracks of the PCB may be covered by the molding material and at the same time in thermal communication with the internal area of the gas flow passage through the molding material. For instance, the heating surface may comprises a portion of the molding material that is covering the heating tracks. In other embodiments, the heating surface comprises the heating tracks and the heating tracks (or a portion thereof) are directly exposed to the internal area of the gas flow passage.

In some embodiments, the molding material covers one or more of the electrical components and/or sensors of the PCB. The molding material may separate the one or more of the electrical components and/or sensors from direct contact to the internal area of the gas flow passage way (e.g., through the molding material). The molding material may be configured to allow monitoring of the internal area of the gas passage way (e.g., the temperature of the gas(es) inside, pressure, humidity, the relative ratios of different gases where more than one is present, etc.) by the electrical components and/or sensors of the PCB through the molding material.

In some embodiments, the humidification apparatus comprises a humidification liquid inlet for delivering the humidification liquid from a reservoir to the heater. In some embodiments, the humidification apparatus comprises a humidification liquid inlet for delivering the humidification liquid from a reservoir to a heating surface of the heater. In some embodiments, the inlet is configured to drip a humidification liquid (e.g., water) on the heating surface. In some embodiments, the inlet is configured to deliver a humidification liquid on to a surface of the apparatus (e.g., a wall of the apparatus, a trough, a ramp, etc.) that guides the humidification liquid to the heating surface. In some embodiments, the humidification apparatus comprises a humidification liquid pre-heater. In some embodiments, the respiratory humidification apparatus comprises a humidification liquid flow controller. In some embodiments, the liquid flow controller comprises a metering system and/or a pump. In some embodiments, the humidification apparatus comprises a gas pre-heater.

In certain implementations, the molding material comprises a thermoplastic material and/or a thermosetting material. In certain implementations, the molding material comprises a thermoplastic material. In certain implementations, the molding material comprises a thermosetting material. In some embodiments, the molding material comprises an injection molded thermoplastic material. In some embodiments, the molding material comprises an overmolded thermoplastic material. In some embodiments, the heater is formed by overmolding the thermoplastic material around the PCB. In some embodiments, the heater is formed by injection molding the thermoplastic material around the PCB. In some embodiments, the heater is formed by insert molding the thermoplastic material around the PCB. In some embodiments, the thermoplastic material is selected from the group consisting of ethylene/methacrylic acid copolymer, propylene/methacrylic acid copolymer, ethylene/methacrylic acid/acrylate terpolymer, propylene/methacrylic acid/acrylate terpolymer, ethylene/acrylate copolymer, propylene/acrylate copolymer, succinic anhydride grafted polypropylene, succinic anhydride grafted polyethylene, polyurethane, polyamide, ethylene/butyl acrylate/glycidyl methacrylate terpolymer, propylene/butyl acrylate/glycidyl methacrylate terpolymer, ethylene/glycidyl methacrylate copolymer, propylene/glycidyl methacrylate copolymer, crosslinkable polyethylene, crosslinkable polypropylene, crosslinkable polyolefin, and combinations thereof. In some embodiments, the molding material comprises a thermosetting material. In some embodiments, the thermosetting material comprises one or more of silicone rubber, epoxy, and polyurethane.

In some embodiments, the molding material encapsulates at least a portion of the PCB. In some embodiments, the portion of the PCB encapsulated by the molding material comprises a portion of the heating tracks. In some embodiments, the PCB is encapsulated so that the PCB is not exposed to the internal area of the gas flow passage. In some embodiments, the molding material physically insulates the PCB from the internal area of the gas flow passage while allowing the PCB to collect and/or distribute information regarding the internal area of the passage (temperature, humidity, flow rate of gas, etc.). In some embodiments, the encapsulated PCB has a portion that is not covered by the molding material (e.g., the portion of the PCB that is external to the gas flow passage) so that contact may be made with the PCB (e.g., with electrical wires/conduits, etc.). In some embodiments, the molding material provides a barrier between the PCB and the gas flow passage. In some embodiments, the molding material provides complete separation of the PCB from the internal area of the gas flow passage. In some embodiments, at least one electrical contact of the PCB is not encapsulated and/or covered by the molding material.

In some embodiments, a portion of the molding material comprises micro-channels and/or surface structures. In some embodiments, the micro-channels and/or surface structures are configured to receive, distribute, and/or hold the humidification liquid so it can be heated by the heating tracks. In some embodiments, a portion of the molding material is configured to receive the humidification liquid and retain it so that it can be heated by the heating tracks. In some embodiments, the portion of molding material that is configured to receive the humidification liquid is coupled to the PCB by the bonding layer. In some embodiments, the molding material is hydrophilic or hydrophobic.

In some embodiments, the bonding layer chemically couples the molding material to the portion of the substrate (e.g., a portion of the substrate that is a part of a PCB). In some embodiments, the bonding layer chemically couples the thermoplastic to a portion of the heater. In some embodiments, the bonding layer comprises one or more of a covalent bond, hydrogen bond, van der waals forces and ionic bond linking the molding material to a portion of the PCB. In some embodiments, the bonding layer comprises covalent bonds between substituents on the molding material and substituents on the PCB. In some embodiments, the bonding layer is formed at least in part through a reaction of one or more of an epoxy ring, amine, succinic anhydride, methoxyl and/or an ethoxyl moiety with one or more of a carboxyl, amine, epoxy ring, succinic anhydride and/or reactive silanol moiety. In some embodiments, the bonding layer is formed at least in part through a reaction between a succinic anhydride moiety and an amine moiety. In some embodiments, the bonding layer is formed at least in part through a reaction between a succinic anhydride moiety and a secondary amine moiety.

In some embodiments, the silicon-containing linker is a silane coupling linker represented by: 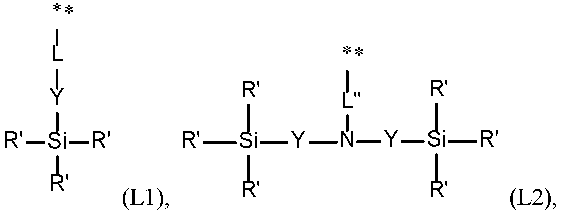 or a polymerized silane coupling linker thereof; wherein ** is a point of attachment to the molding material; each R' is independently selected from the group consisting of hydrogen, halogen, hydroxyl, C1-C8 alkyl, C1-C8 alkoxy, and -O-*, wherein * is a point of attachment to the PCB, and at least one of the R' is -O-* ; Y is a single bond, C1-C8 alkylene, or C1-C8 heteroalkylene containing one or more heteroatoms selected from O and N; and -L-** is selected from the group consisting of: 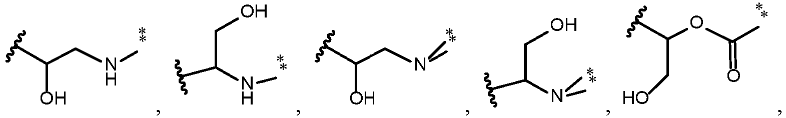 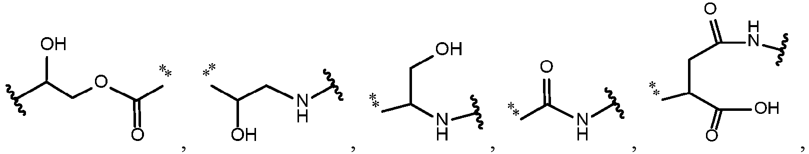 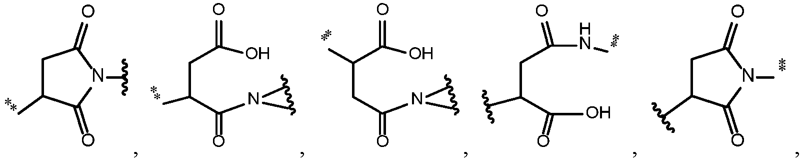 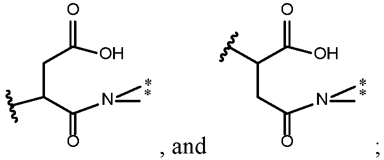 and -L"-* is selected from the group consisting of:

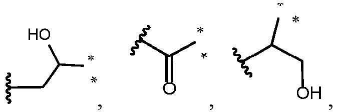

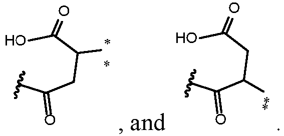

In some embodiments, the silicon-containing linker is a silicon alkoxide linker represented by: 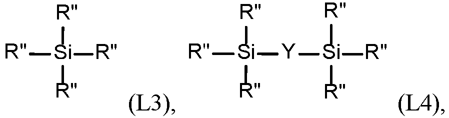 or a polymerized silicon alkoxide linker thereof, wherein R" is independently hydrogen, halogen, hydroxyl, C1-C8 alkyl, C1-C8 alkoxyl, -O-* or -O-** , wherein * is a point of attachment to the substrate, ** is a point of attachment to the thermoplastic material, and at least one of the R" is 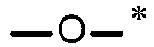 and at least one of the R" is 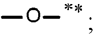 and Y is a single bond, C1-C8 alkylene, or C1-C8 heteroalkylene, wherein the C1-C8 heteroalkylene contains one or more heteroatoms selected from O and N.

In some embodiments, the silicon-containing linker is a siloxane linker selected from the group consisting of: 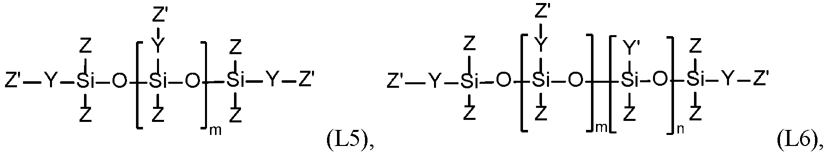 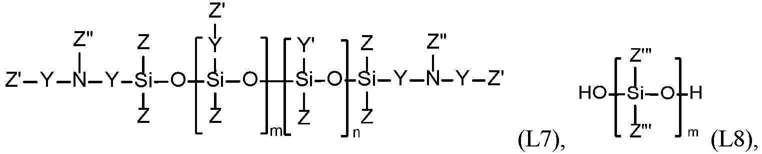 and a polymerized siloxane linker thereof; wherein each Z is independently hydroxyl or 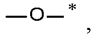 wherein * is a point of attachment to the substrate; each Z' is selected from the group consisting of epoxy, primary and secondary amine, succinic anhydride, and 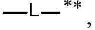 wherein ** is a point of attachment to the thermoplastic material; 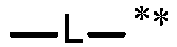 is selected from the group consisting of: 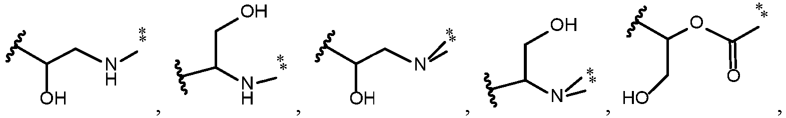 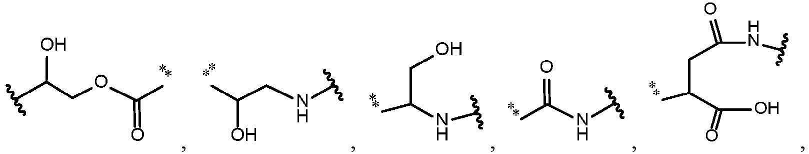 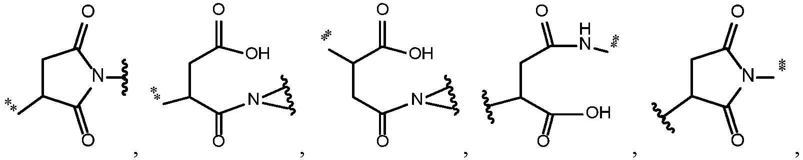 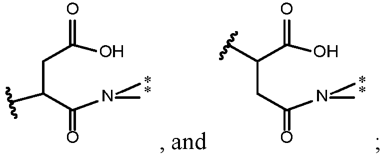 Z" is hydrogen or -L"-**, wherein - L"-** is selected from the group consisting of: 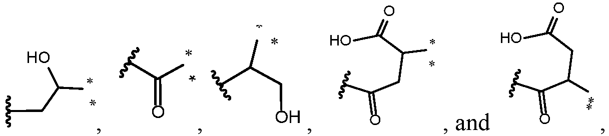 and ** is a point of attachment to the thermoplastic material; at least one of the Z is -O-* , and at least one of the Z' is -L-** or at least one of the Z" is -L"-**; Y is a single bond, C1-C8 alkylene, or C1-C8 heteroalkylene containing one or more heteroatoms selected from O and N; Y' is C1-C8 alkyl or C1-C8 heteroalkyl containing one or more heteroatoms selected from O and N; Z'" is independently hydroxyl, -O-* or -O-** , wherein * is a point of attachment to the substrate, ** is a point of attachment to the thermoplastic materials, at least one of the Z'" is -O-* , and at least one of the Z"' is -O-** ; m is an integer greater than 1; and n is an integer greater than 1.

In some embodiments, the humidification liquid comprises water.

Some embodiments pertain to a respiratory system. In some embodiments, the respiratory system comprises a gas (e.g., air, oxygen, etc.) flow source. In some embodiments, the respiratory system comprises the humidification apparatus as disclosed above or elsewhere herein. In some embodiments, the respiratory system comprises an inspiratory tube. In some embodiments, the respiratory system comprises a patient interface. In some embodiments, the gas flow source is configured to provide high flow therapy. In some embodiments, the system provides flow rates in a range from about 2 L/min to about 150 L/min, or other flow rates as disclosed elsewhere herein.

Some embodiments pertain to a component for use in a respiratory system or medical insufflation system. In some embodiments, the component comprises an electronic component comprising conductive tracks and a substrate, wherein the conductive tracks are located on the substrate. In some embodiments, the component comprises a molding material that is molded over at least a portion of the electronic component. In some embodiments, the component comprises a bonding layer that couples the molding material with at least a portion of the electronic component. In some embodiments, the bonding layer comprises a silicon-containing linker.

In some embodiments, the bonding layer is a separate layer from the molding material and the electronic component. In some embodiments, the bonding layer comprises pendant groups that are covalently bonded to the molding material and coinciding pendant groups that are covalently bonded to the electronic component. In some embodiments, the pendant groups from the substrate (e.g., electronic component) may or may not be covalently linked to the pendant groups of the molding material. In some embodiments, the bonding layer chemically couples the molding material to the portion of the electronic component. In some embodiments, the bonding layer comprises one or more of covalent bonds, hydrogen bonds, van der Waals forces, and/or ionic bond linking the molding material to the portion of the electronic component. In some embodiments, the bonding layer physically couples the molding material and the substrate (e.g., the electronic component).

In some embodiments, the bonding layer is formed through a reaction of one or more of an epoxy ring, amine, succinic anhydride, methoxyl or ethoxyl moiety with one or more of a carboxyl, amine, epoxy ring, succinic anhydride or reactive silanol moiety. In some embodiments, the bonding layer is formed through a reaction between a succinic anhydride moiety and an amine moiety. In some embodiments, the succinic anhydride moiety is provided by the electronic component and the amine moiety is provided by the molding material. In some embodiments, the electronic component and the succinic anhydride moiety is provided by the molding material. In some embodiments, the bonding layer is formed from a reaction between a succinic anhydride moiety and a secondary amine moiety. In some embodiments, the succinic anhydride moiety is provided by the electronic component and the secondary amine moiety is provided by the molding material. In some embodiments, the secondary amine moiety is provided by the electronic component and the succinic anhydride moiety is provided by the molding material.

In some embodiments, the silicon-containing linker is a silane coupling linker represented by: 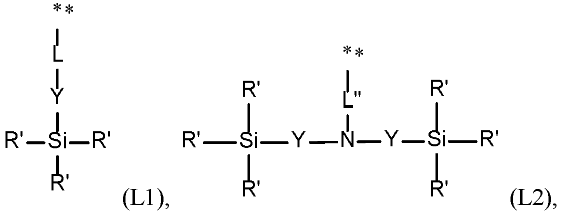 or a polymerized silane coupling linker thereof; wherein ** is a point of attachment to the molding material; each R' is independently selected from the group consisting of hydrogen, halogen, hydroxyl, C1-C8 alkyl, C1-C8 alkoxy, and -O-*, wherein * is a point of attachment to the PCB, and at least one of the R' is -O-* ; Y is a single bond, C1-C8 alkylene, or C1-C8 heteroalkylene containing one or more heteroatoms selected from O and N; and -L-** is selected from the group consisting of: 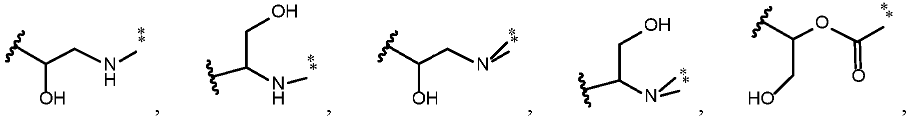 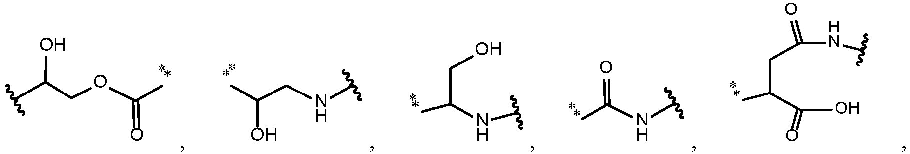 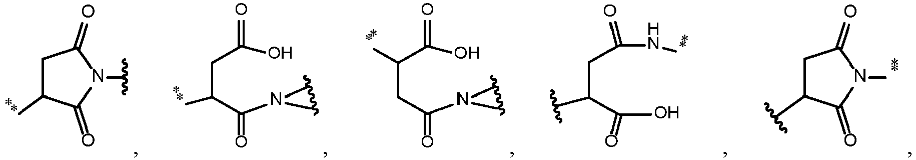 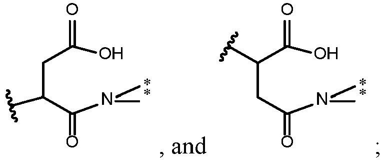 and -L"-** is selected from the group consisting of:

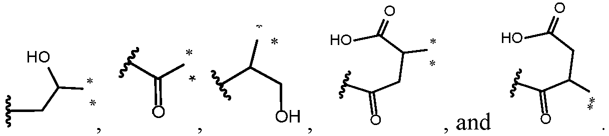

In some embodiments, the silicon-containing linker is a silicon alkoxide linker represented by: 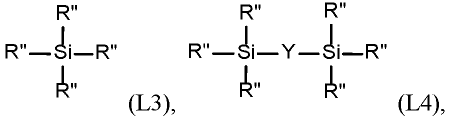 or a polymerized silicon alkoxide linker thereof; wherein R" is independently hydrogen, halogen, hydroxyl, C1-C8 alkyl, C1-C8 alkoxyl, -O-* or -O-**, wherein * is a point of attachment to the substrate, ** is a point of attachment to the thermoplastic material, and at least one of the R" is -O-* and at least one of the R" is -O-**; and Y is a single bond, C1-C8 alkylene, or C1-C8 heteroalkylene, wherein the C1-C8 heteroalkylene contains one or more heteroatoms selected from O and N.

In some embodiments, the silicon-containing linker is a siloxane linker selected from the group consisting of: 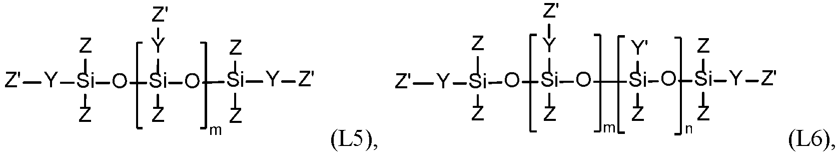 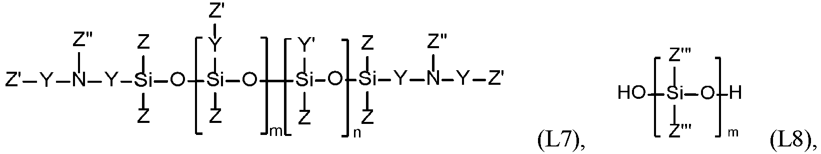 and a polymerized siloxane linker thereof; wherein each Z is independently hydroxyl or -O-* , wherein * is a point of attachment to the substrate; each Z' is selected from the group consisting of epoxy, primary and secondary amine, succinic anhydride, and -L-**, wherein ** is a point of attachment to the thermoplastic material; -L-** is selected from the group consisting of: 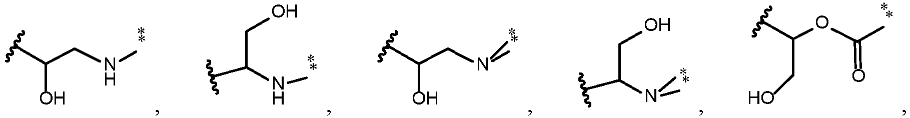 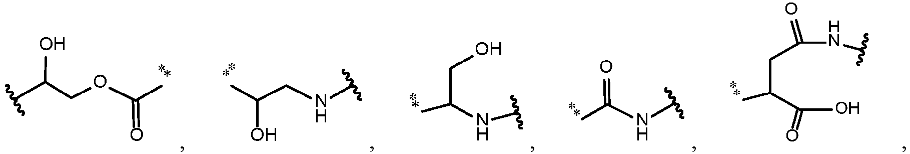 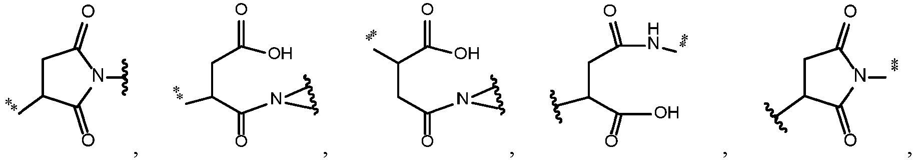 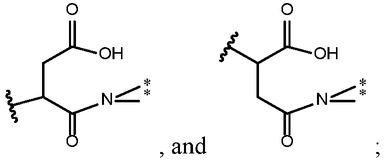 Z" is hydrogen or -L"-**, wherein -L"-** is selected from the group consisting of: 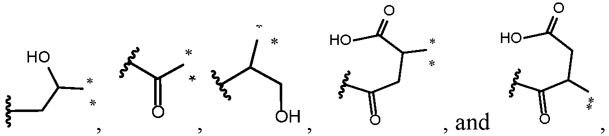 and ** is a point of attachment to the thermoplastic material; at least one of the Z is -O-*, and at least one of the Z' is -L-** or at least one of the Z" is -L"-**; Y is a single bond, C1-C8 alkylene, or C1-C8 heteroalkylene containing one or more heteroatoms selected from O and N; Y' is C1-C8 alkyl or C1-C8 heteroalkyl containing one or more heteroatoms selected from O and N; Z'" is independently hydroxyl, -O-* or -O-**, wherein * is a point of attachment to the substrate, ** is a point of attachment to the thermoplastic materials, at least one of the Z'" is -O-* , and at least one of the Z‴ is -O-**; m is an integer greater than 1; and n is an integer greater than 1.

In some embodiments, the electronic component is a printed circuit board (PCB). In some embodiments, the PCB is rigid or flexible.

In some embodiments, the electronic component comprises silicone rubber, ceramic, metal, glass fiber, fiberglass, sized fiberglass, etched foil, a filled polymer, epoxy resin, phenol formaldehyde resin, paper or polyester resin, a polyester, a polyetherimide, a polyimide, an epoxy, a polyethylene, copper, an ink, or combinations thereof..

In some embodiments, the portion of the electronic component provided with the molding material is exposed to heat and humidity in a respiratory system.

In some embodiments, the molding material comprises a thermoplastic material or a thermosetting material. In some embodiments, a thermoplastic material is used. In some embodiments, the thermoplastic material is selected from the group consisting of ethylene/methacrylic acid copolymer, propylene/methacrylic acid copolymer, ethylene/methacrylic acid/acrylate terpolymer, propylene/methacrylic acid/acrylate terpolymer, ethylene/acrylate copolymer, propylene/acrylate copolymer, succinic anhydride grafted polypropylene, succinic anhydride grafted polyethylene, polyurethane, polyamide, ethylene/butyl acrylate/glycidyl methacrylate terpolymer, propylene/butyl acrylate/glycidyl methacrylate terpolymer, ethylene/glycidyl methacrylate copolymer, propylene/glycidyl methacrylate copolymer, crosslinkable polyethylene, crosslinkable polypropylene, crosslinkable polyolefin, and combinations thereof. In some embodiments, the thermoplastic material comprises an overmolded thermoplastic material. In some embodiments, the component is formed by injection molding the thermoplastic material around the electronic component. In some embodiments, the component is formed by overmolding the thermoplastic material around the electronic component. In some embodiments, the component is formed by insert molding the thermoplastic material around the electronic component. In some embodiments, a thermosetting material is used. In some embodiments, the molding material comprises a thermosetting polymer. In some embodiments, the thermosetting material one or more of silicone rubber, epoxy, and polyurethane.

In some embodiments, the molding material comprises micro-channels and/or structural features on a surface. In some embodiments, the micro-channels and/or structural features are configured to receive, distribute, and/or hold a humidification liquid. In some embodiments, the molding material is hydrophilic or hydrophobic.

In some embodiments, the electronic component comprises one or more sensors. In some embodiments, the one or more sensors comprises one or more of a temperature sensor, a pressure sensor, a flow sensor, a humidity sensor, and/or a fluid level sensor. In some embodiments, the one or more sensors comprises one or more of a temperature sensor, a pressure sensor, a flow sensor, and/or a humidity sensor, wherein the one or more of a pressure sensor, a flow sensor, and/or a humidity sensor are configured to detect parameters of a gas in the gas flow passage. In some embodiments, the PCB comprises a fluid level sensor and/or a liquid sensor. In some embodiments, the molding material covers the sensor(s).

In some embodiments, the component is part of a respiratory system that further comprises one or more of a housing for a filter, a gas conduit, a chamber, an inspiratory tube, a tube connector, a tube joint, a tube elbow, a heating element, a water dosing component, and/or a patient interface component. In some embodiments, the electronic component comprises an electrical contact. In some embodiments, the component is exposed to a higher temperature and/or humidity in a gas flow path when in use relative to an ambient environment. In some embodiments, the component further comprises a humidification chamber.

Some embodiments pertain to a respiratory or medical insufflation apparatus comprising the component. In some embodiments, the respiratory or medical insufflation apparatus further comprises a humidification chamber.

Some embodiments pertain to a heater for a humidification chamber. In some embodiments, the heater comprises a heating component comprising heating tracks and a substrate, the heating tracks being provided on the substrate. In some embodiments, the heater comprises a molding material disposed on at least a portion of the heating component. In some embodiments, the heater comprises a bonding layer coupling at least a portion of the molding material to the heating component. In some embodiments, the heater comprises a bonding layer coupling the molding material to the heating component at a location where the molding material is disposed on the heating component. In some embodiments, the bonding layer comprises a silicon-containing linker.

In some embodiments, the bonding layer is a separate layer from the molding material and the heating component. In some embodiments, the bonding layer comprises pendant groups that are covalently bonded to the molding material and coinciding pendant groups that are covalently bonded to the electronic component. In some embodiments, the bonding layer chemically couples the molding material to the portion of the heating component. In some embodiments, the bonding layer comprises one or more of covalent bonds, hydrogen bonds, van der Waals forces, and/or ionic bond linking the molding material to the portion of the heating component. In some embodiments, the bonding layer is formed through a reaction between one or more of an epoxy ring, amine, succinic anhydride, methoxyl or ethoxyl moiety and one or more of a carboxyl, amine, epoxy ring, succinic anhydride or reactive silanol moiety. In some embodiments, the bonding layer is formed at least in part through a reaction between a succinic anhydride moiety with an amine moiety. In some embodiments, the bonding layer is formed at least in part through a reaction between a succinic anhydride moiety and a secondary amine moiety.

In some embodiments, the silicon-containing linker is a silane coupling linker represented by: 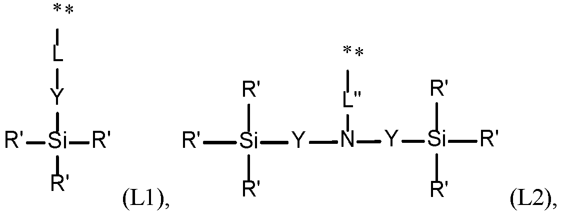 or a polymerized silane coupling linker thereof; wherein ** is a point of attachment to the molding material; each R' is independently selected from the group consisting of hydrogen, halogen, hydroxyl, C1-C8 alkyl, C1-C8 alkoxy, and -O-*, wherein * is a point of attachment to the PCB, and at least one of the R' is -O-* ; Y is a single bond, C1-C8 alkylene, or C1-C8 heteroalkylene containing one or more heteroatoms selected from O and N; and -L-** is selected from the group consisting of: 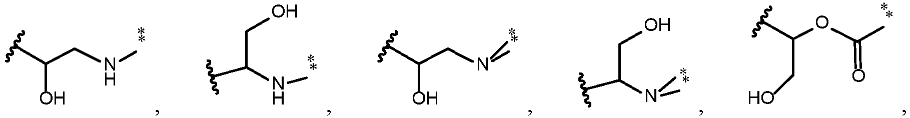 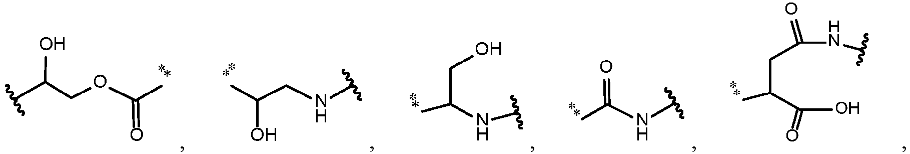 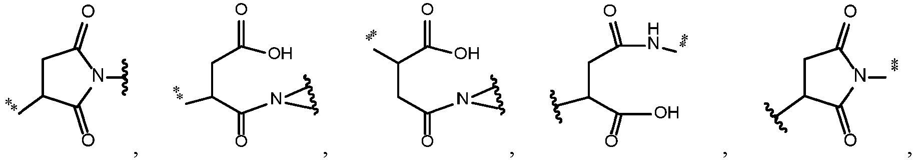 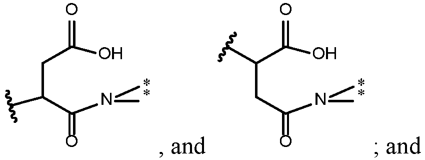 -L"-** is selected from the group consisting of:

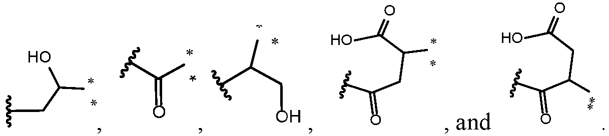

In some embodiments, the silicon-containing linker is a silicon alkoxide linker represented by: 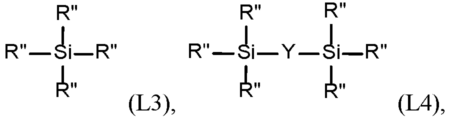 or a polymerized silicon alkoxide linker thereof, wherein R" is independently hydrogen, halogen, hydroxyl, C1-C8 alkyl, C1-C8 alkoxyl, -O-* or -O-**, wherein * is a point of attachment to the substrate, ** is a point of attachment to the thermoplastic material, and at least one of the R" is -O-* and at least one of the R" is -O-** ; and Y is a single bond, C1-C8 alkylene, or C1-C8 heteroalkylene, wherein the C1-C8 heteroalkylene contains one or more heteroatoms selected from O and N.

In some embodiments, the silicon-containing linker is a siloxane linker selected from the group consisting of: 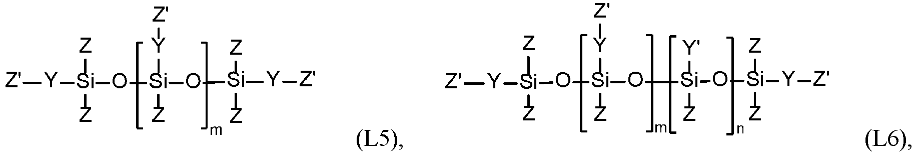 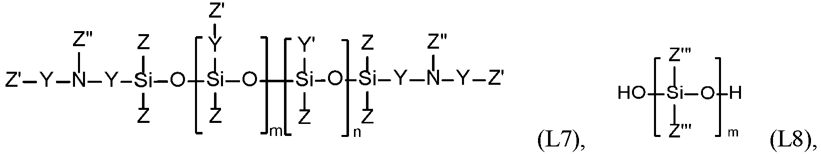 and a polymerized siloxane linker thereof; wherein each Z is independently hydroxyl or -O-* , wherein * is a point of attachment to the substrate; each Z' is selected from the group consisting of epoxy, primary and secondary amine, succinic anhydride, and -L-**, wherein ** is a point of attachment to the thermoplastic material; -L-** is selected from the group consisting of: 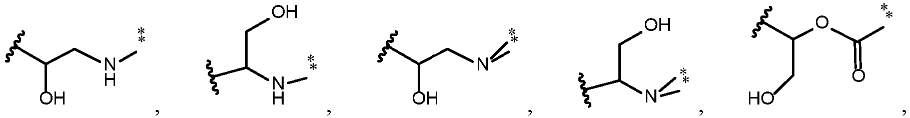 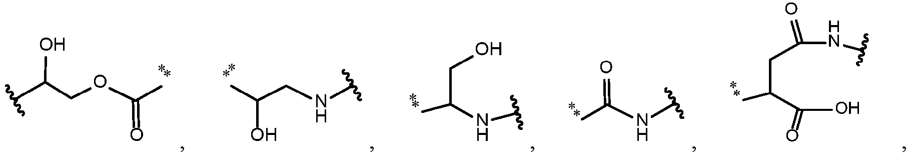 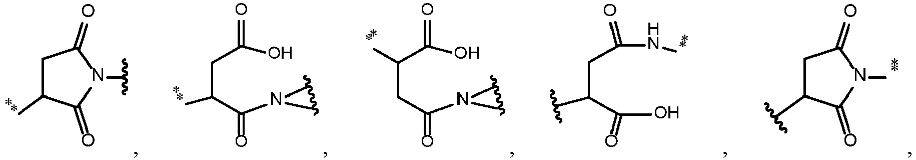 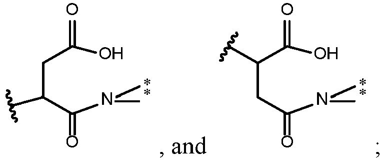 Z" is hydrogen or -L"-**, wherein -L"-** is selected from the group consisting of: 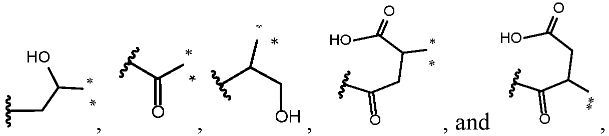 and ** is a point of attachment to the thermoplastic material; at least one of the Z is -O-*, and at least one of the Z' is -L-** or at least one of the Z" is -L"-**; Y is a single bond, C1-C8 alkylene, or C1-C8 heteroalkylene containing one or more heteroatoms selected from O and N; Y' is C1-C8 alkyl or C1-C8 heteroalkyl containing one or more heteroatoms selected from O and N; Z'" is independently hydroxyl, -O-* or -O-**, wherein * is a point of attachment to the substrate, ** is a point of attachment to the thermoplastic materials, at least one of the Z'" is -O-*, and at least one of the Z"' is -O-**; m is an integer greater than 1; and n is an integer greater than 1.

In some embodiments, the heating component is a printed circuit board (PCB). In some embodiments, the PCB is rigid or flexible. In some embodiments, the substrate comprises silicone rubber, ceramic, metal, glass fiber, fiberglass, sized fiberglass, etched foil, a filled polymer, or combinations thereof. In some embodiments, the molding material comprises a thermoplastic material or a thermosetting material. In some embodiments, the thermoplastic material is selected from the group consisting of ethylene/methacrylic acid copolymer, propylene/methacrylic acid copolymer, ethylene/methacrylic acid/acrylate terpolymer, propylene/methacrylic acid/acrylate terpolymer, ethylene/acrylate copolymer, propylene/acrylate copolymer, succinic anhydride grafted polypropylene, succinic anhydride grafted polyethylene, polyurethane, polyamide, ethylene/butyl acrylate/glycidyl methacrylate terpolymer, propylene/butyl acrylate/glycidyl methacrylate terpolymer, ethylene/glycidyl methacrylate copolymer, propylene/glycidyl methacrylate copolymer, crosslinkable polyethylene, crosslinkable polypropylene, crosslinkable polyolefin, and combinations thereof. In some embodiments, the thermoplastic material comprises an injection molded thermoplastic material (e.g., an overmolded thermoplastic material, an insert molded thermoplastic material, etc.). In some embodiments, the heater is formed by injection molding the thermoplastic material around the heating component. In some embodiments, the heater is formed by overmolding the thermoplastic material around the heating component. In some embodiments, the heater is formed by insert molding the thermoplastic material around the heating component. In some embodiments, the thermosetting material comprises one or more of silicone rubber, epoxy, and polyurethane.

In some embodiments, the molding material comprises micro-channels and/or structural features. In some embodiments, the micro-channels or structures are configured to hold a humidification liquid. In some embodiments, the molding material is hydrophilic or hydrophobic.

In some embodiments, the heating tracks are configured to heat the humidification liquid when in thermal communication with the heating component and to evaporate the humidification liquid into a gas flow path. In some embodiments, the heating component comprises an electrical connector.

Some embodiments pertain to a heater for a humidification chamber. In some embodiments, the heater comprises a heating component comprising heating tracks provided on a substrate. In some embodiments, the heater comprises a molding material encapsulating the heating component such that the heating component is separated from an internal area of the humidification chamber by the molding material. In some embodiments, the heater comprises a bonding layer coupling the molding material to the heating component.

In some embodiments, the bonding layer is a separate layer from the molding material and the heating component. In some embodiments, the bonding layer comprises pendant groups that are covalently bonded to the molding material and coinciding pendant groups that are covalently bonded to the electronic component. In some embodiments, the bonding layer comprises a silicon-containing linker layer. In some embodiments, the bonding layer chemically couples the molding material to the electronic component. In some embodiments, the bonding layer comprises one or more of covalent bonds, hydrogen bonds, van der Waals forces, and/or ionic bond linking the molding material and the portion of the heating component. In some embodiments, the bonding layer is formed at least in part through a reaction between one or more of an epoxy ring, amine, succinic anhydride, methoxyl or ethoxyl moiety with one or more of a carboxyl, amine, epoxy ring, succinic anhydride or reactive silanol moiety. An epoxy would be reactive with, at least, an amine or carboxyl. Succinic anhydride would be reactive with, at least, an amine. An amine would be reactive with, at least, a carboxyl, epoxy ring, and succinic anhydride. Ethoxyl and methoxyl would be reactive with a silanol. In some embodiments, the bonding layer is formed at least in part through a reaction between a succinic anhydride moiety and an amine moiety. In some embodiments, the bonding layer is formed at least in part through from a reaction between a succinic anhydride moiety and a secondary amine moiety.

In some embodiments, the silicon-containing linker is a silane coupling linker represented by: 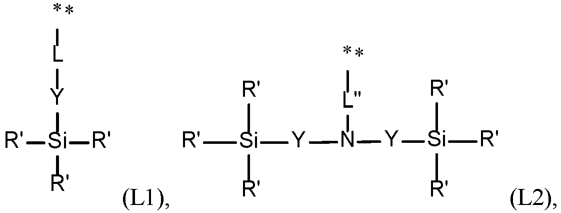 or a polymerized silane coupling linker thereof; wherein ** is a point of attachment to the molding material; each R' is independently selected from the group consisting of hydrogen, halogen, hydroxyl, C1-C8 alkyl, C1-C8 alkoxy, and -O-*, wherein * is a point of attachment to the PCB, and at least one of the R' is -O-* ; Y is a single bond, C1-C8 alkylene, or C1-C8 heteroalkylene containing one or more heteroatoms selected from O and N; and -L-** is selected from the group consisting of: 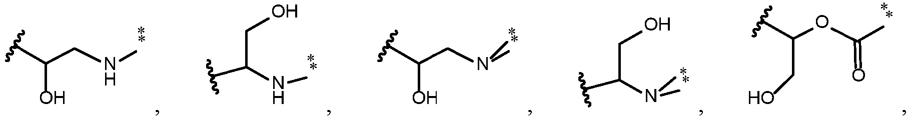 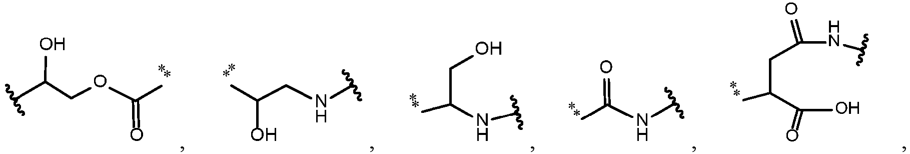 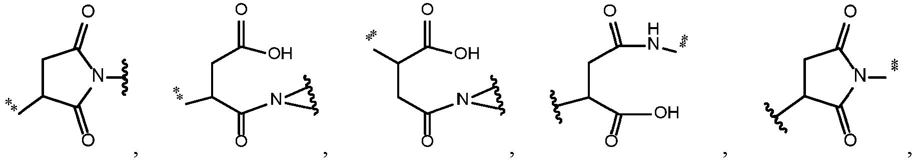 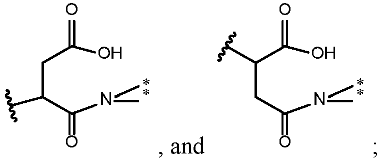 and 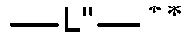 is selected from the group consisting of:

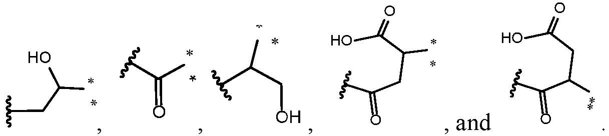

In some embodiments, the silicon-containing linker is a silicon alkoxide linker represented by: 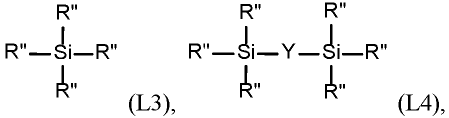 or a polymerized silicon alkoxide linker thereof, wherein R" is independently hydrogen, halogen, hydroxyl, C1-C8 alkyl, C1-C8 alkoxyl, 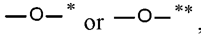 , wherein * is a point of attachment to the substrate, ** is a point of attachment to the thermoplastic material, and at least one of the R" is 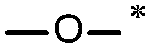 and at least one of the R" is 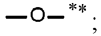 and Y is a single bond, C1-C8 alkylene, or C1-C8 heteroalkylene, wherein the C1-C8 heteroalkylene contains one or more heteroatoms selected from O and N.

In some embodiments, the silicon-containing linker is a siloxane linker selected from the group consisting of: 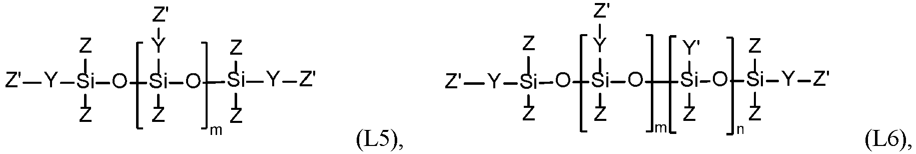 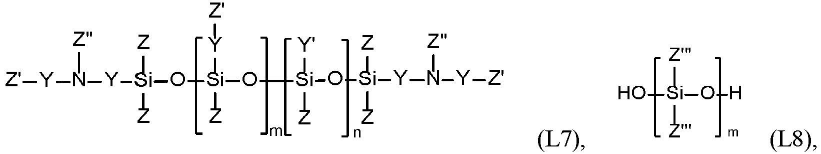 and a polymerized siloxane linker thereof; wherein each Z is independently hydroxyl or -O-* , wherein * is a point of attachment to the substrate; each Z' is selected from the group consisting of epoxy, primary and secondary amine, succinic anhydride, and -L-**, wherein ** is a point of attachment to the thermoplastic material; -L-** is selected from the group consisting of: 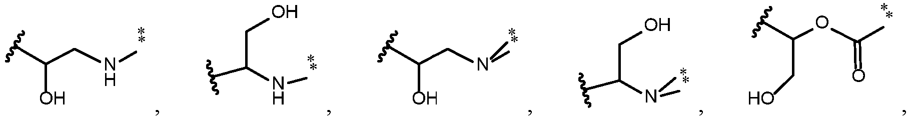 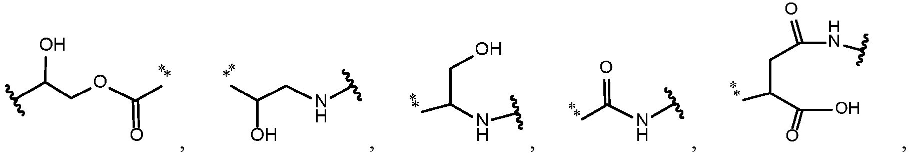 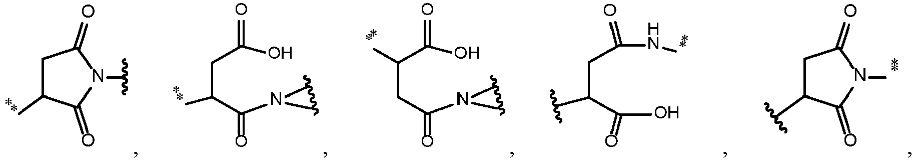 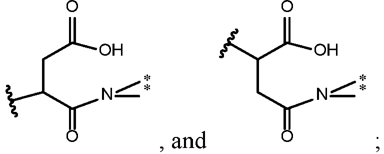 Z" is hydrogen or -L"-**, wherein -L"-** is selected from the group consisting of: 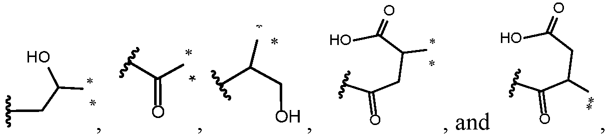 and ** is a point of attachment to the thermoplastic material; at least one of the Z is -O-*, and at least one of the Z' is -L-** or at least one of the Z" is -L"-**; Y is a single bond, C1-C8 alkylene, or C1-C8 heteroalkylene containing one or more heteroatoms selected from O and N; Y' is C1-C8 alkyl or C1-C8 heteroalkyl containing one or more heteroatoms selected from O and N; Z'" is independently hydroxyl, -O-* or -O-**, wherein * is a point of attachment to the substrate, ** is a point of attachment to the thermoplastic materials, at least one of the Z'" is -O-* , and at least one of the Z"' is -O-**; m is an integer greater than 1; and n is an integer greater than 1.

In some embodiments, the heating component comprises a printed circuit board (PCB). In some embodiments, the PCB and/or the substrate of the PCB is rigid or flexible. In some embodiments, the PCB and/or the substrate of the PCB comprises silicone rubber, ceramic, metal, glass fiber, fiberglass, sized fiberglass, etched foil, a filled polymer, epoxy resin, phenol formaldehyde resin, paper or polyester resin, a polyester, a polyetherimide, a polyimide, an epoxy, a polyethylene, copper, an ink, or combinations thereof.

In some embodiments, the molding material comprises a thermoplastic material or a thermosetting material. In some embodiments, the thermoplastic material is selected from the group consisting of ethylene/methacrylic acid copolymer, propylene/methacrylic acid copolymer, ethylene/methacrylic acid/acrylate terpolymer, propylene/methacrylic acid/acrylate terpolymer, ethylene/acrylate copolymer, propylene/acrylate copolymer, succinic anhydride grafted polypropylene, succinic anhydride grafted polyethylene, polyurethane, polyamide, ethylene/butyl acrylate/glycidyl methacrylate terpolymer, propylene/butyl acrylate/glycidyl methacrylate terpolymer, ethylene/glycidyl methacrylate copolymer, propylene/glycidyl methacrylate copolymer, crosslinkable polyethylene, crosslinkable polypropylene, crosslinkable polyolefin, and combinations thereof.

In some embodiments, the heater is formed by injection molding the thermoplastic material around the heating component. In some embodiments, the heater is formed by overmolding the thermoplastic material around the heating component. In some embodiments, the heater is formed by insert molding the thermoplastic material around the heating component. In some embodiments, the thermosetting material comprises one or more of silicone rubber, epoxy, and polyurethane. In some embodiments, the molding material comprises micro-channels and/or structural features. In some embodiments, the micro-channels or structures are configured to hold a humidification liquid. In some embodiments, the molding material is hydrophilic or hydrophobic.

In some embodiments, the heating tracks are configured to heat the humidification liquid when in thermal communication with the heating component and to evaporate the humidification liquid into a gas flow path. In some embodiments, the heating component comprises an electrical connector.

Some embodiments pertain to a humidification chamber comprising an inlet to receive gas from a gas source and an outlet to deliver gases to a patient, wherein the inlet and outlet define a gas flow path. In some embodiments, the humidification chamber includes the heater disclosed above or elsewhere herein. In some embodiments, the heater is configured to heat and humidify the gas when in the gas flow path. In some embodiments, the chamber is configured for use in a respiratory system or medical insufflation system.

Some embodiments pertain to a molded member. In some embodiments, the molded member comprises a thermoplastic material and a substrate. In some embodiments, at least a portion of the substrate is coupled to the thermoplastic material via a silicon-containing linker. In some embodiments, the silicon-containing linker is formed at least in part through a reaction between a succinic anhydride moiety and an amine moiety.

Some embodiments pertain to a method for coupling a thermoplastic material to a substrate. In some embodiments, the method comprises providing a substrate with at least one attached silicon-containing linking agent on the surface. In some embodiments, the method comprises contacting the thermoplastic material to the attached silicon-containing linking agent to form a bond between the thermoplastic material and the substrate. In some embodiments, the bond comprises a reaction product from a reaction between a succinic anhydride moiety and an amine moiety. In some embodiments, the succinic anhydride moiety is provided on the silicon-containing linker. In some embodiments, the amine moiety is provided on the thermoplastic. In some embodiments, the amine moiety is provided on the silicon-containing linker. In some embodiments, the succinic anhydride moiety is provided on the thermoplastic. In some embodiments, the amine moiety comprises a secondary amine moiety. In some embodiments, providing the substrate with at least one attached silicon-containing linking agent on the surface comprises providing the substrate comprising hydroxyl and/or carboxyl on the surface, and reacting a silicon-containing linking agent with the hydroxyl and/or carboxyl on a surface of the substrate. In some embodiments, the reacting the silicon-containing linking agent with the hydroxyl and/or carboxyl on the substrate comprises hydrolyzing the silicon-containing linking agent to form a reactive silanol, and contacting the reactive silanol with the hydroxyl and/or carboxyl on the substrate. In some embodiments, the reacting the silicon-containing linking agent with the hydroxyl and/or carboxyl on the substrate comprises plasma enhanced chemical vapor deposition (PECVD). In some embodiments, the providing the substrate comprising hydroxyl and/or carboxyl on the surface further comprises activating the substrate surface by plasma treatment, corona discharge, ozone treatment, chemical treatment, or flame treatment.

In some embodiments, the silicon-containing linking agent is a silane coupling agent. In some embodiments, the silane coupling agent is represented by the following formula: 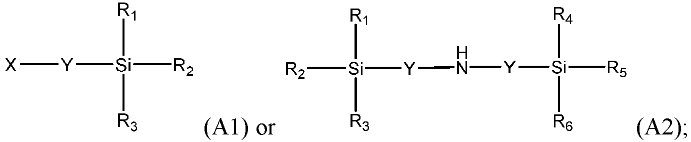 wherein R₁, R₂, R₃, R₄, R₅, and R₆ are each independently selected from the group consisting of hydrogen, halogen, hydroxyl, C1-C8 alkyl, and C1-C8 alkoxy; at least one of R₁, R₂, and R₃ in (A1) or at least one of R₁, R₂, R₃, R₄, R₅, and R₆ in (A2) is hydroxyl or C1-C8 alkoxy; Y is a single bond, C1-C8 alkylene, or C1-C8 heteroalkylene containing one or more heteroatoms selected from O and N; and X is selected from the group consisting of primary and secondary amine, and succinic anhydride.

In some embodiments, the silane coupling agent is selected from the group consisting of 3-aminopropyl trimethoxysilane (APTMS), 3-aminopropyl triethoxysilane (APTES), (3-triethoxysilyl)propylsuccinic anhydride (TEPSA), and bis(3-trimethoxysilylpropyl)amine. In some embodiments, the silicon-containing linking agent is a siloxane.

In some embodiments, the silane coupling agent is represented by the following formula: 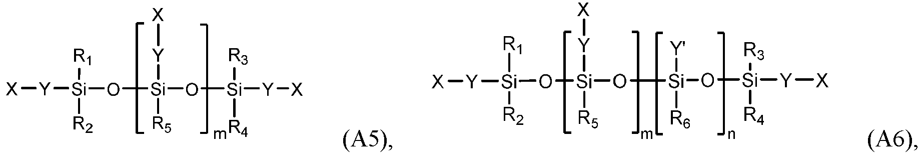 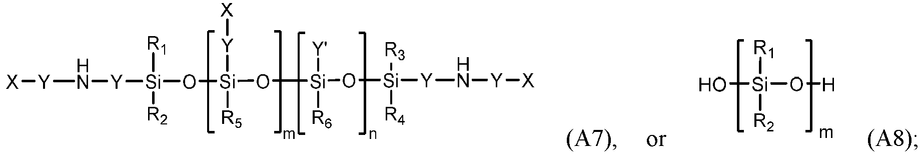 wherein R₁, R₂, R₃, R₄, R₅, and R₆ are each independently selected from the group consisting of hydrogen, halogen, hydroxyl, C1-C8 alkyl, and C1-C8 alkoxy; at least one of R₁, R₂, R₃, R₄, and R₅ in (A5), at least one of R₁, R₂, R₃, R₄, R₅, and R₆ in (A6) and (A7), and at least two of R₁ and R₂ in (A8) are independently hydroxyl or C1-C8 alkoxy; Y is a single bond, C1-C8 alkylene, or C1-C8 heteroalkylene containing one or more heteroatoms selected from O and N; Y' is C1-C8 alkyl or C1-C8 heteroalkyl containing one or more heteroatoms selected from O and N; X is selected from the group consisting of primary amine, secondary amine, and succinic anhydride; m is an integer 1 or greater; and n is an integer 1 or greater.

In some embodiments, the siloxane is selected from the group consisting of aminopropyl silsesquioxane, aminoethyl aminopropyl silsesquioxane, aminopropylsilsesquioxane-methylsilsequioxane, and non-functional siloxane.

In some embodiments, the substrate is part of (e.g., makes up a portion of) a printed circuit board (PCB). In some embodiments, the PCB is rigid. In some embodiments, the PCB is flexible. In some embodiments, the PCB comprises one or more sensor(s). In some embodiments, the one or more sensor(s) is selected from the group consisting of temperature sensor, flow sensor, pressure sensor, humidity sensor, and fluid level sensor. In some embodiments, the PCB comprises a heating element. In some embodiments, the PCB comprises an electrical connector.

Some embodiments pertain to a respiratory apparatus comprising a molded member comprising a thermosetting material and a substrate. In some embodiments, at least a portion of the substrate is coupled to the thermosetting material via a silicon-containing linker. In some embodiments, the silicon-containing linker is represented by the following formula: 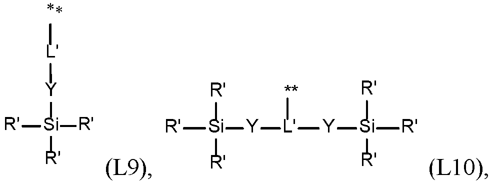 or a polymerized silicon-containing linker thereof; wherein ** is a point of attachment to the thermosetting material; each R' is independently hydrogen, halogen, hydroxyl, C1-C8 alkyl, C1-C8 alkoxy, or -O-* , wherein * is a point of attachment to the substrate, wherein at least one of the R' is -O-*; -L'-** is 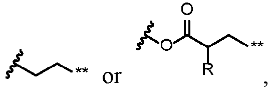 wherein R is H or C1-C8 alkyl; 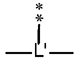 is 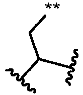 or 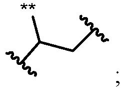 Y is a single bond, C1-C8 alkylene, or C1-C8 heteroalkylene containing one or more heteroatoms selected from O and N.

In some embodiments, the thermosetting material comprises a -Si-H moiety. In some embodiments, the thermosetting material is a silicone rubber. In some embodiments, the molded member is a housing for a filter, a gas conduit, a chamber, an inspiratory tube, a tube connector, a tube joint, a tube elbow, a heating element, or a patient interface component. In some embodiments, the thermosetting material is a molding material on the substrate. In some embodiments, the molded member is formed by injection molding the molding material around the substrate. In some embodiments, the molded member is formed by insert molding the molding material around the substrate. In some embodiments, the molded member is formed by overmolding the molding material around the substrate. In
some embodiments, the molding material comprises micro-channels or structures on a surface.

In some embodiments, the substrate comprises a material that can be activated to form hydroxyl groups on the surface. In some embodiments, the substrate is silicone rubber, ceramic, metal, glass fiber, fiberglass, sized fiberglass, etched foil, filled polymer, epoxy resin, phenol formaldehyde resin, paper or polyester resin, a polyester, a polyetherimide, a polyimide, an epoxy, a polyethylene, copper, an ink, or combinations thereof. In some embodiments, the substrate is part of (e.g., makes up a portion of) a printed circuit board (PCB). In some embodiments, the PCB is rigid. In some embodiments, the PCB is flexible. In some embodiments, the substrate is rigid or flexible. In some embodiments, the PCB comprises one or more sensor(s). In some embodiments, the one or more sensor(s) is selected from the group consisting of temperature sensor, pressure sensor, flow sensor, humidity sensor, and fluid level sensor. In some embodiments, the PCB comprises a heating element. In some embodiments, the PCB comprises an electrical connector.

Some embodiments pertain to a method for coupling a thermosetting material to a substrate. In some embodiments, the method comprises providing the substrate with an attached silicon-containing linking agent on the surface. In some embodiments, the method comprises reacting a silicone resin with the attached silicon-containing linking agent. In some embodiments, the silicone resin comprises a -Si-H moiety. In some embodiments, the method further comprises curing the silicone resin to form a thermosetting material. In some embodiments, the thermosetting material is a silicone rubber. In some embodiments, the attached silicon-containing linking agent comprises a vinyl moiety. In some embodiments, the reacting the silicone resin with the attached silicon-containing linking agent on the substrate comprises reacting the -Si-H moiety in the silicone resin with the vinyl moiety in the attached silicon-containing linking agent. In some embodiments, the providing the substrate with an attached silicon-containing linking agent on the surface further comprises providing the substrate comprising hydroxyl and/or carboxyl on the surface, and reacting a silicon-containing linking agent with the hydroxyl and/or carboxyl on the substrate. In some embodiments, the reacting the silicon-containing linking agent with the hydroxyl and/or carboxyl on the substrate comprises hydrolyzing the silicon-containing linking agent to form a reactive silanol, and contacting the reactive silanol with the hydroxyl and/or carboxyl on the substrate. In some embodiments, the reacting the silicon-containing linking agent with the hydroxyl and/or carboxyl on the substrate comprises plasma enhanced chemical vapor deposition (PECVD). In some embodiments, the providing the substrate comprising hydroxyl and/or carboxyl on the surface further comprises activating the substrate surface by plasma treatment, corona discharge, ozone treatment, chemical treatment, or flame treatment.

In some embodiments, the silicon-containing linking agent is a vinyl functionalized silane coupling agent. In some embodiments, the vinyl functionalized silane coupling agent is represented by the formula: 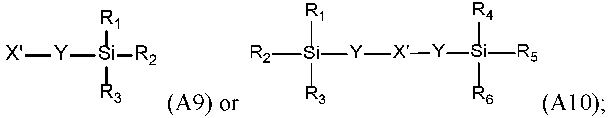 wherein R₁, R₂, and R₃ in (A9) are each independently selected from the group consisting of hydrogen, halogen, hydroxyl, C1-C8 alkyl, and C1-C8 alkoxy, and at least one of R₁, R₂, and R₃ is hydroxyl or C1-C8 alkoxy; R₁, R₂, R₃, R₄, R₅, and R₆ in (A10) are each independently selected from the group consisting of hydrogen, halogen, hydroxyl, C1-C8 alkyl, and C1-C8 alkoxy, and at least one of R₁, R₂, R₃, R₄, R₅, and R₆ is hydroxyl or C1-C8 alkoxy; X' is 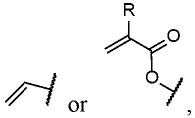 wherein R is H or C1-C8 alkyl; -X'- is 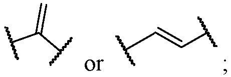 and Y is a single bond, C1-C8 alkylene, C1-C8 heteroalkylene containing one or more heteroatoms selected from N and O.

In some embodiments, the silicon-containing linking agent is gamma-methacryloxypropyltrimethoxysilane (Gamma-MPTS), vinyltrimethylsilane (VTMS) (3-acryloxylpropyl)trimethoxysilane, 1,1-bis(trimethoxysilylmethyl)ethylene, 1,2-bis(methyldiethoxysilyl)ethylene, and bis(3-trimethoxysilylpropyl)fumarate.

In some embodiments, the substrate is ceramic, metal, glass fiber, fiberglass, sized fiberglass, etched foil, or filled polymer. In some embodiments, the substrate is a printed circuit board (PCB). In some embodiments, the PCB is rigid. In some embodiments, the PCB is flexible. In some embodiments, the PCB comprises one or more sensor(s). In some embodiments, the one or more sensor(s) is selected from the group consisting of temperature sensor, flow sensor, pressure sensor, humidity sensor, and fluid level sensor. In some embodiments, the PCB comprises a heating element. In some embodiments, the PCB comprises an electrical connector.

One aspect of the disclosure includes a molded member containing a thermoplastic material and a substrate, wherein at least a portion of the substrate is coupled to the thermoplastic material via a silicon-containing linker. In some embodiments, the silicon-containing linker is covalently bonded to the thermoplastic material. In some embodiments, the silicon-containing linker is covalently bonded to the substrate.

In some embodiments, the silicon-containing linker is a silane coupling linker, a silicon alkoxide linker, or a siloxane linker.

In some embodiments, the silane coupling linker is represented by: 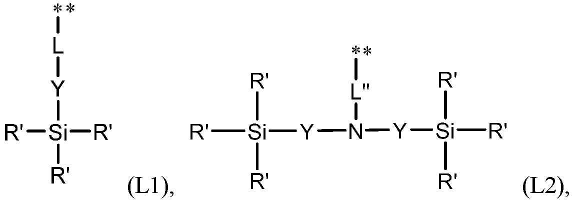 or a polymerized silane coupling linker thereof;
wherein ** is a point of attachment to the thermoplastic material; each R' is independently selected from the group consisting of hydrogen, halogen, hydroxyl, C1-C8 alkyl, C1-C8 alkoxy, and -O-* , wherein * is a point of attachment to the substrate, and at least one of the R' is -O-* ; Y is a single bond, C1-C8 alkylene, or C1-C8 heteroalkylene containing one or more heteroatoms selected from O and N; and -L-** is selected from the group consisting of: 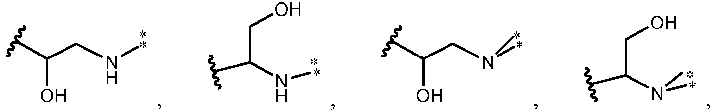 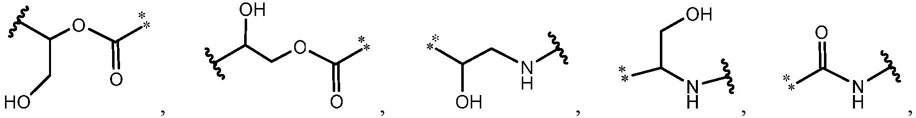 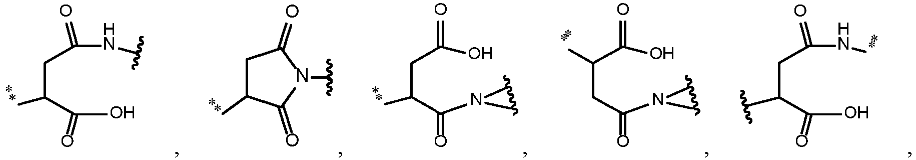 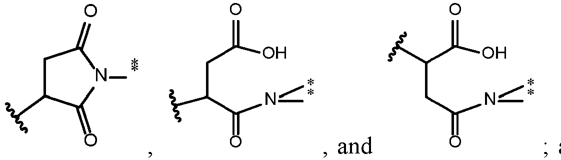 and -L"-** is selected from the group consisting of:

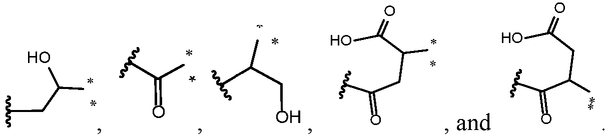

In some embodiments, the silicon alkoxide linker is represented by: 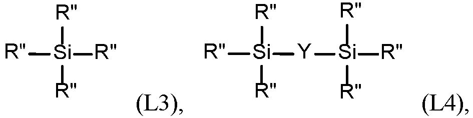 or a polymerized silicon alkoxide linker thereof; wherein R" is independently hydrogen, halogen, hydroxyl, C1-C8 alkyl, C1-C8 alkoxyl, -O-* or -O-** , wherein * is a point of attachment to the substrate, ** is a point of attachment to the thermoplastic material, and at least one of the R" is -O-* and at least one of the R" is -O-**; and Y is a single bond, C1-C8 alkylene, or C1-C8 heteroalkylene, wherein the C1-C8 heteroalkylene contains one or more heteroatoms selected from O and N.

In some embodiments, the siloxane linker is selected from the group consisting of: 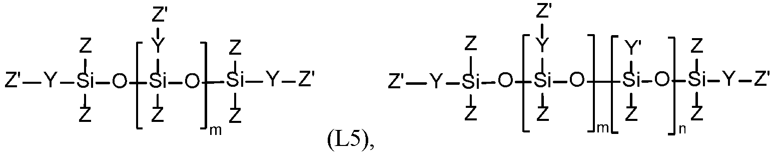 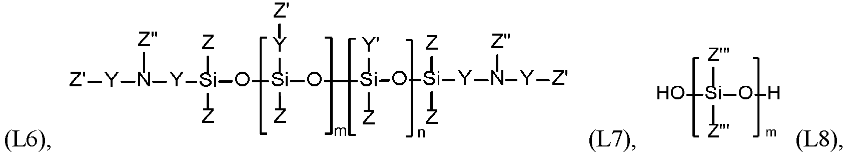 and a polymerized siloxane linker thereof; wherein each Z is independently hydroxyl or -O-*, wherein * is a point of attachment to the substrate; each Z' is selected from the group consisting of epoxy, primary and secondary amine, succinic anhydride, and -L-**, wherein ** is a point of attachment to the thermoplastic material, -L-** is selected from the group consisting of: 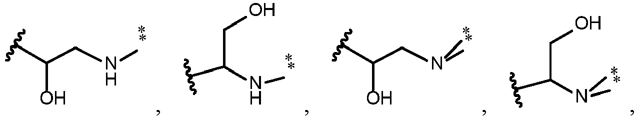 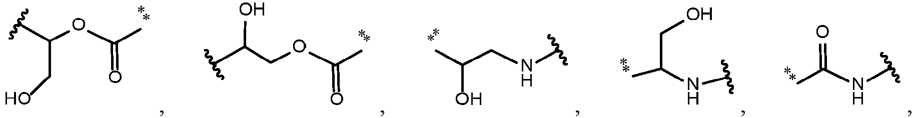 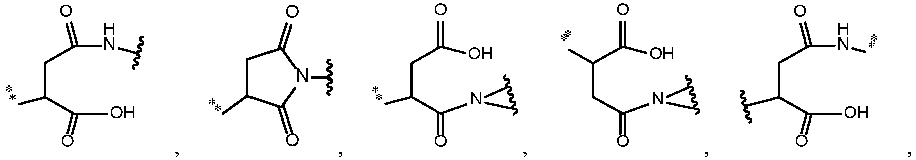 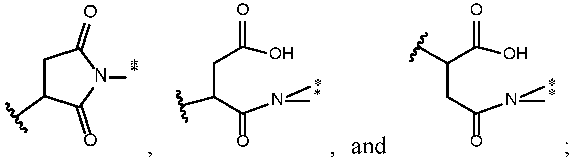 Z" is hydrogen or -L"-**, wherein -L"-** is selected from the group consisting of: 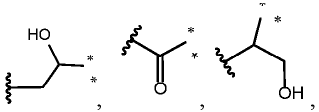 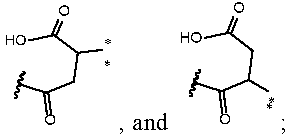 at least one of the Z is -O-* , and at least one of the Z' is -L-** or at least one of the Z" is -L"-**; Y is a single bond, C1-C8 alkylene, or C1-C8 heteroalkylene containing one or more heteroatoms selected from O and N; Y' is C1-C8 alkyl or C1-C8 heteroalkyl containing one or more heteroatoms selected from O and N; Z'" is independently hydroxyl, -O-* or -O-** , wherein * is a point of attachment to the substrate, ** is a point of attachment to the thermoplastic materials, at least one of the Z'" is -O-* , and at least one of the Z‴ is -O-** ; m is an integer greater than 1; and n is an integer greater than 1.

In some embodiments, the thermoplastic material comprises amino, carboxyl, epoxy, succinic anhydride, or silane moiety. In some embodiments, the thermoplastic material is selected from the group consisting of ethylene/methacrylic acid copolymer, propylene/methacrylic acid copolymer, ethylene/methacrylic acid/acrylate terpolymer, propylene/methacrylic acid/acrylate terpolymer, ethylene/acrylate copolymer, propylene/acrylate copolymer, succinic anhydride grafted polypropylene, succinic anhydride grafted polyethylene, polyurethane, polyamide, ethylene/butyl acrylate/glycidyl methacrylate terpolymer, propylene/butyl acrylate/glycidyl methacrylate terpolymer, ethylene/glycidyl methacrylate copolymer, propylene/glycidyl methacrylate copolymer, crosslinkable polyethylene, crosslinkable polypropylene, and crosslinkable polyolefin. The molded member is housing for a filter, a gas conduit, a chamber, an inspiratory tube, a tube connector, a tube joint, a tube elbow, a heating element, a water dosing component, or a patient interface component.

In some embodiments, the thermoplastic material is a molding material on the substrate. In some embodiments, the molded member is formed by injection molding the molding material around the substrate. In some embodiments, the molded member is formed by insert molding the molding material around the substrate. In some embodiments, the molded member is formed by overmolding the molding material around the substrate. In some embodiments, the molding material comprises micro-channels or structures on a surface.

In some embodiments, the thermoplastic material has a shore hardness higher than about 30. In some embodiments, the thermoplastic material is hydrophilic. In other embodiments, the thermoplastic material is hydrophobic.

In some embodiments, the substrate comprises a material that can be activated to form hydroxyl groups on the surface. In some embodiments, the substrate is a printed circuit board (PCB). In some embodiments, the PCB is rigid, and in other embodiments, the PCB is flexible. In some embodiments, the PCB comprises one or more sensor(s). In some embodiments, the one or more sensor(s) is selected from the group consisting of temperature sensor, pressure sensor, flow sensor, humidity sensor, and fluid level sensor. In some embodiments, the PCB comprises a heating element. In some embodiments, the PCB comprises an electrical connector. In some embodiments, the substrate is silicone rubber, ceramic, metal, glass fiber, fiberglass, sized fiberglass, etched foil, filled polymer, epoxy resin, phenol formaldehyde resin, paper or polyester resin, a polyester, a polyetherimide, a polyimide, an epoxy, a polyethylene, copper, an ink, or combinations thereof.

One aspect of the disclosure includes a respiratory apparatus containing the molded member as described above.

One aspect of the disclosure includes a method for coupling a thermoplastic material to a substrate. The method includes providing the substrate with at least one attached silicon-containing linking agent on the surface, and bonding the thermoplastic material to the attached silicon-containing linking agent.

In some embodiments, the thermoplastic material comprises an amino, carboxyl, epoxy, succinic anhydride, or silane moiety. In some embodiments, the thermoplastic material is selected from the group consisting of ethylene/methacrylic acid copolymer, propylene/methacrylic acid copolymer, ethylene/methacrylic acid/acrylate terpolymer, propylene/methacrylic acid/acrylate terpolymer, ethylene/acrylate copolymer, propylene/acrylate copolymer, succinic anhydride grafted polypropylene, succinic anhydride grafted polyethylene, polyurethane, polyamide, ethylene/butyl acrylate/glycidyl methacrylate terpolymer, propylene/butyl acrylate/glycidyl methacrylate terpolymer, ethylene/glycidyl methacrylate copolymer, propylene/glycidyl methacrylate copolymer, crosslinkable polyethylene, crosslinkable polypropylene, and crosslinkable polyolefin.

In some embodiments, the attached silicon-containing linking agent further comprises a moiety selected from the group consisting of primary or secondary amine, succinic anhydride, and epoxy group.

In some embodiments, the step of bonding the thermoplastic material to the substrate further comprises reacting an amino group or carboxyl group in the thermoplastic material with an epoxy in the attached silicon-containing linking agent. In other embodiments, the step of bonding a thermoplastic material to the substrate further comprises reacting an epoxy, carboxyl or succinic anhydride in the thermoplastic material with an amino in the attached silicon-containing linking agent. In other embodiments, the step of bonding a thermoplastic material to the substrate further comprises reacting an amino in the thermoplastic material with a succinic anhydride in the attached silicon-containing linking agent. In other embodiments, the step of bonding a thermoplastic material to the substrate further comprises hydrolyzing a thermoplastic material comprising a silane moiety to form at least one silanol on the surface, and reacting the at least one silanol with a hydroxyl in the attached silicon-containing linking agent.

In some embodiments, the step of providing the substrate with an attached silicon-containing linking agent further comprises providing the substrate comprising hydroxyl and/or carboxyl on the surface, and reacting a silicon-containing linking agent with the hydroxyl and/or carboxyl on the substrate.

In some embodiments, the step of reacting the silicon-containing linking agent with the hydroxyl and/or carboxyl on the substrate comprises hydrolyzing the silicon-containing linking agent to form a reactive silanol, and contacting reactive silanol with the hydroxyl and/or carboxyl on the substrate. In other embodiments, the step of reacting the silicon-containing linking agent with the hydroxyl and/or carboxyl on the substrate further comprises plasma enhanced chemical vapor deposition (PECVD).

In some embodiments, the step of providing the substrate comprising hydroxyl and/or carboxyl on the surface further comprises activating the substrate surface by plasma treatment, corona discharge, ozone treatment, chemical treatment, or flame treatment.

In some embodiments, the silicon-containing linking agent is a silane coupling agent. The silane coupling agent is represented by the following formula: 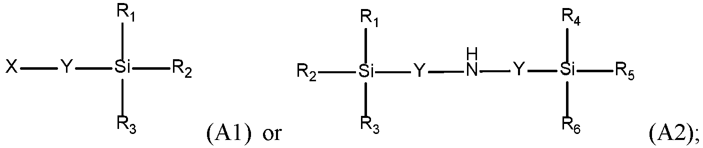 wherein R₁, R₂, R₃, R₄, R₅, and R₆ are each independently selected from the group consisting of hydrogen, halogen, hydroxyl, C1-C8 alkyl, and C1-C8 alkoxy; at least one of R₁, R₂, and R₃ in (A1) or at least one of R₁, R₂, R₃, R₄, R₅, and R₆ in (A2) is hydroxyl or C1-C8 alkoxy; Y is a single bond, C1-C8 alkylene, or C1-C8 heteroalkylene containing one or more heteroatoms selected from O and N; and X is selected from the group consisting of epoxy, primary and secondary amine, and succinic anhydride. In some embodiments, the silane coupling agent is selected from the group consisting of 3-glycidoxypropyltrimethoxysilane (GPTMS), 3-aminopropyl trimethoxysilane (APTMS), 3-aminopropyl triethoxysilane (APTES), (3-triethoxysilyl)propylsuccinic anhydride (TEPSA), and bis(3-trimethoxysilylpropyl)amine.

In some embodiments, the silicon-containing linking agent is a silicon alkoxide. the silicon alkoxide is represented by the following formula: 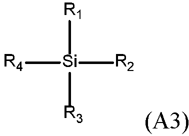 or 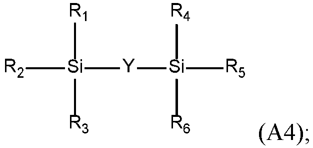 wherein R₁, R₂, R₃, R₄, R₅, and R₆ are each independently selected from the group consisting of hydrogen, halogen, hydroxyl, C1-C8 alkyl, and C1-C8 alkoxy; at least two of R₁, R₂, R₃, and R₄ in (A3) and at least two of R₁, R₂, R₃, R₄, R₅, and R₆ in (A4) are independently hydroxyl or C1-C8 alkoxy; and Y is a single bond, C1-C8 alkylene, or C1-C8 heteroalkylene containing one or more heteroatoms selected from O and N. In some embodiments, the silicon alkoxide is selected from the group consisting of tetramethoxysilane (TMOS), tetraethoxysilane (TEOS), and 1,2-bis(triethoxysilyl)ethane.

In some embodiments, the silicon-containing linking agent is a siloxane. the silane coupling agent is represented by the following formula: 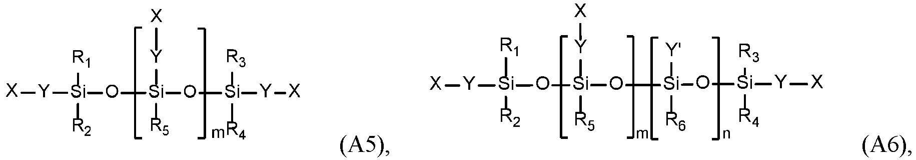 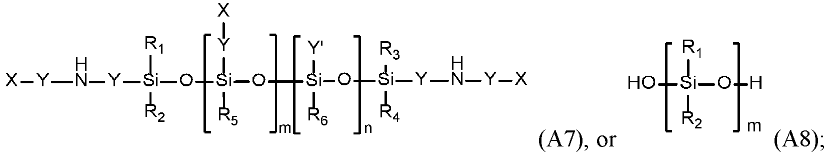 wherein R₁, R₂, R₃, R₄, R₅, and R₆ are each independently selected from the group consisting of hydrogen, halogen, hydroxyl, C1-C8 alkyl, and C1-C8 alkoxy; at least one of R₁, R₂, R₃, R₄, and R₅ in (A5), at least one of R₁, R₂, R₃, R₄, R₅, and R₆ in (A6) and (A7), and at least two of R₁ and R₂ in (A8) are independently hydroxyl or C1-C8 alkoxy; Y is a single bond, C1-C8 alkylene, or C1-C8 heteroalkylene containing one or more heteroatoms selected from O and N; Y' is C1-C8 alkyl or C1-C8 heteroalkyl containing one or more heteroatoms selected from O and N; X is selected from the group consisting of epoxy, primary or secondary amine, succinic anhydride; m is an integer 1 or greater; and n is an integer 1 or greater. In some embodiments, the siloxane is selected from the group consisting of aminopropyl silsesquioxane, aminoethyl aminopropyl silsesquioxane, aminopropylsilsesquioxane-methylsilsequioxane, and non-functional siloxane.

In some embodiments, the substrate is ceramic, metal, glass fiber, fiberglass, sized fiberglass, etched foil, or filled polymer. In other embodiments, the substrate is a printed circuit board (PCB). In some embodiments, the PCB is rigid. In other embodiments, the PCB is flexible. In some embodiments, the PCB comprises one or more sensor. In some embodiments, the one or more sensor is selected from the group consisting of temperature sensor, flow sensor, pressure sensor, humidity sensor, and fluid level sensor. In some embodiments, the PCB comprises a heating element. In some embodiments, the PCB comprises an electrical connector.

One aspect of the disclosure includes a respiratory apparatus containing a molded member, wherein the molded member contains a thermosetting material and a substrate, and wherein at least a portion of the substrate is coupled to the thermosetting material via a silicon-containing linker.

In some embodiments, the silicon-containing linker is covalently bonded to the thermosetting material. In some embodiments, the silicon-containing linker is covalently bonded to the substrate. In some embodiments, the silicon-containing linker is represented by the following formula: 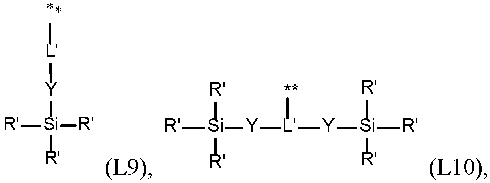 or a polymerized silicon-containing linker thereof; wherein ** is a point of attachment to the thermosetting material; each R' is independently hydrogen, halogen, hydroxyl, C1-C8 alkyl, C1-C8 alkoxy, or -O-* , wherein * is a point of attachment to the substrate, wherein at least one of the R' is -O-* ; -L'-** is 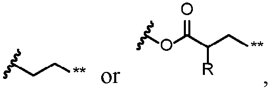 wherein R is H or C1-C8 alkyl; 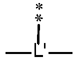 is 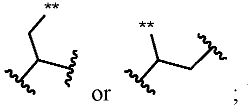 Y is a single bond, C1-C8 alkylene, or C1-C8 heteroalkylene containing one or more heteroatoms selected from O and N.

In some embodiments, the thermosetting material comprises a -Si-H moiety. In some embodiments, the thermosetting material is a silicone rubber

In some embodiments, the molded member is housing for a filter, a gas conduit, a chamber, an inspiratory tube, a tube connector, a tube joint, a tube elbow, a heating element, or a patient interface component. In some embodiments, the thermosetting material is a molding material on the substrate.

In some embodiments, the molded member is formed by injection molding the molding material around the substrate. In some embodiments, the molded member is formed by insert molding the molding material around the substrate. In some embodiments, the molded member is formed by overmolding the molding material around the substrate. In some embodiments, the molding material comprises micro-channels or structures on a surface,

In some embodiments, the substrate comprises a material that can be activated to form hydroxyl groups on the surface. In some embodiments, the substrate is silicone rubber, ceramic, metal, glass fiber, fiberglass, sized fiberglass, etched foil, filled polymer, epoxy resin, phenol formaldehyde resin, paper or polyester resin, a polyester, a polyetherimide, a polyimide, an epoxy, a polyethylene, copper, an ink, or combinations thereof. In some embodiments, the substrate is a printed circuit board (PCB). In some embodiments, the PCB is rigid. In other embodiments, the PCB is flexible. In some embodiments, the PCB comprises one or more sensor. In some embodiments, the one or more sensor is selected from the group consisting of temperature sensor, flow sensor, pressure sensor, humidity sensor, and fluid level sensor. In some embodiments, the PCB comprises a heating element. In some embodiments, the PCB comprises an electrical connector.

One aspect of the disclosure includes a method for coupling a thermosetting material to a substrate. The method includes providing the substrate with an attached silicon-containing linking agent on the surface; and reacting a silicone resin with the attached silicon-containing linking agent.

In some embodiments, the silicone resin comprises a -Si-H moiety.

In some embodiments, the method further comprising curing the silicone resin to form a thermosetting material.

In some embodiments, the thermosetting material is a silicone rubber. In some embodiments, the attached silicon-containing linking agent comprises a vinyl moiety

In some embodiments, the step of reacting the silicone resin with the attached silicon-containing linking agent on the substrate comprises reacting the -Si-H moiety in the silicone resin with the vinyl moiety in the attached silicon-containing linking agent.

In some embodiments, the step of providing the substrate with an attached silicon-containing linking agent on the surface further comprises providing the substrate comprising hydroxyl and/or carboxyl on the surface, and reacting a silicon-containing linking agent with the hydroxyl and/or carboxyl on the substrate.

In some embodiments, the step of reacting the silicon-containing linking agent with the hydroxyl and/or carboxyl on the substrate comprises hydrolyzing the silicon-containing linking agent to form a reactive silanol, and contacting the reactive silanol with the hydroxyl and/or carboxyl on the substrate.

In some embodiments, the step of reacting the silicon-containing linking agent with the hydroxyl and/or carboxyl on the substrate comprises plasma enhanced chemical vapor deposition (PECVD).

In some embodiments, the step of providing the substrate comprising hydroxyl and/or carboxyl on the surface further comprises activating the substrate surface by plasma treatment, corona discharge, ozone treatment, chemical treatment, or flame treatment.

In some embodiments, the silicon-containing linking agent is a vinyl functionalized silane coupling agent. In some embodiments, the vinyl functionalized silane coupling agent is represented by the formula: 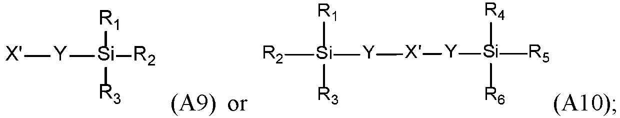 wherein R₁, R₂, and R₃ in (A9) are each independently selected from the group consisting of hydrogen, halogen, hydroxyl, C1-C8 alkyl, and C1-C8 alkoxy, and at least one of R₁, R₂, and R₃ is hydroxyl or C1-C8 alkoxy; R₁, R₂, R₃, R₄, R₅, and R₆ in (A10) are each independently selected from the group consisting of hydrogen, halogen, hydroxyl, C1-C8 alkyl, and C1-C8 alkoxy, and at least one of R₁, R₂, R₃, R₄, R₅, and R₆ is hydroxyl or C1-C8 alkoxy; X' is 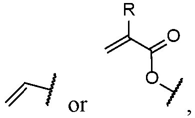 wherein R is H or C1-C8 alkyl; -X'- is 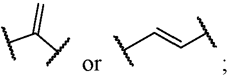 and Y is a single bond, C1-C8 alkylene, C1-C8 heteroalkylene containing one or more heteroatoms selected from N and O. In some embodiments, the silicon-containing linking agent is gamma-methacryloxypropyltrimethoxysilane (Gamma-MPTS), vinyltrimethylsilane (VTMS) (3-acryloxylpropyl)trimethoxysilane, 1,1-bis(trimethoxysilylmethyl)ethylene, 1,2-bis(methyldiethoxysilyl)ethylene, and bis(3-trimethoxysilylpropyl)fumarate.

In some embodiments, the substrate is ceramic, metal, glass fiber, fiberglass, sized fiberglass, etched foil, or filled polymer. In some embodiments, the substrate is a printed circuit board (PCB). In some embodiments, the PCB is rigid. In other embodiments, the PCB is flexible. In some embodiments, the PCB comprises one or more sensor. In some embodiments, the one or more sensor is selected from the group consisting of temperature sensor, flow sensor, pressure sensor, humidity sensor, and fluid level sensor. In some embodiments, the PCB comprises a heating element. In some embodiments, the PCB comprises an electrical connector.

To those skilled in the art to which the disclosure relates, many changes in construction and widely differing embodiments and applications of the disclosure will suggest themselves. The disclosures and the descriptions herein are purely illustrative and are not intended to be in any sense limiting.

### BRIEF DESCRIPTION OF THE DRAWINGS

Various embodiments of the present disclosure will now be described, by way of illustrative example only, with reference to the accompanying drawings. In the drawings, similar elements have like reference numerals.
Figure 1A shows the surface chemical moieties prior to reaction between the thermoplastic material and the silanized substrate.
Figure 1B shows the interface between the bonded thermoplastic material and substrate.
Figure 2A is a perspective view of an exemplified component molded elbow in accordance with one embodiment of the present disclosure.
Figure 2B is a vertical cross-section view of the molded elbow showing the placement of a PCB substrate.
Figure 3A is a perspective view of an exemplified molded elbow in accordance with one embodiment of the present disclosure.
Figure 3B is a vertical cross-section view of the molded elbow showing the placement of a PCB substrate.
Figure 4A is a perspective view of an exemplified chamber molding in accordance with one embodiment of the present disclosure.
Figure 4B is a vertical cross-section view of the molded chamber showing the placement of a PCB substrate.
Figure 4C is a perspective view of an exemplified chamber molding in accordance with another embodiment of the present disclosure.
Figure 4D is a vertical cross-section view of the molded chamber showing the placement of a PCB substrate.
Figure 5A is a perspective view of an exemplified chamber molding in accordance with one embodiment of the present disclosure.
Figure 5B is a vertical cross-section view of the molded chamber showing the placement of a PCB substrate.
Figure 6A is a perspective view of an exemplified chamber molding in accordance with one embodiment of the present disclosure.
Figure 6B is a vertical cross-section view of the molded chamber showing the placement of a PCB substrate.
Figure 7A is a perspective view of an exemplified chamber molding in accordance with one embodiment of the present disclosure.
Figure 7B is a vertical cross-section view of the molded chamber showing the placement of a PCB substrate.
Figure 8A is a perspective view of an exemplified breathing tube connector in accordance with one embodiment of the present disclosure.
Figure 8B is a vertical cross-section view of the breathing tube connector showing the placement of a PCB substrate.
Figure 9A is a perspective view of an exemplified breathing tube connector in accordance with one embodiment of the present disclosure.
Figure 9B is a vertical cross-section view of the breathing tube connector showing the placement of a PCB substrate.
Figure 10A is a perspective view of an exemplified heater for a humidifier showing the configuration of a PCB substrate in accordance with one embodiment of the present disclosure.
Figure 10B is a sectional detail view of the exemplified heater for a humidifier depicted in Figure 10A.
Figure 11 is a perspective view of an exemplified heater for a humidifier showing the configuration of a PCB substrate in accordance with one embodiment of the present disclosure.
Figure 12A is a perspective view of an exemplified heater for a humidifier showing the configuration of a PCB substrate in accordance with one embodiment of the present disclosure.
Figure 12B is a sectional detail view of the exemplified heater for a humidifier depicted in Figure 12A.
Figure 13A is a perspective view of an exemplified heater for a humidifier showing the configuration of a PCB substrate in accordance with one embodiment of the present disclosure.
Figure 13B is a sectional detail view of the exemplified heater for a humidifier depicted in Figure 13A.
Figures 14A-C are diagrams of an exemplary configurations of a respiratory therapy system.
Figure 15A is a perspective view of a printed circuit board heating element in accordance with an embodiment of the present disclosure.
Figure 15B is a top view of a printed circuit board heating element in accordance with an embodiment of the present disclosure.
Figure 15C is a partial top view of a printed circuit board heating element in accordance with an embodiment of the present disclosure.
Figures 15D illustrates a top view of two embodiments of an etched foil heating element in accordance with an embodiment of the present disclosure.
Figure 15E illustrates an embodiment of an etched foil heating element in a rolled configuration.
Figure 16A is a perspective view of an example respiratory humidification system in accordance with one embodiment of the present disclosure.
Figure 16B is a vertical cross-section view showing an gas flow of the humidification system of Figure 16A.
Figure 16C is a vertical cross-section view showing a water flow of the humidification system of Figure 16A.
Figure 16D is a horizontal cross-section view of the respiratory humidification system of Figure 16A.
Figures 16E-16F show the respiratory humidification system installed for use with a flow generation system.
Figure 17A is a diagram illustrating a grid-structured micro-channel water distribution pattern in accordance with an embodiment of the present disclosure.
Figure 17B is a diagram illustrating a radial micro-channel water distribution pattern in accordance with an embodiment of the present disclosure.
Figure 18A is a perspective axially sectioned view of a portion respiratory humidification system including an example of a coupling in accordance with an embodiment of the present disclosure.
Figure 18B is a perspective sectioned side view of the respiratory humidification system of Figure 18A including the example coupling.
Figure 18C is a side view of the humidification system of Figure 18A including the example coupling.
Figure 18D is a perspective assembled axial view of the humidification system of Figure 18A.
Figure 19 is a perspective diagram of a distribution tube coupling wrapped over an edge of a heating surface in accordance with an embodiment of the present disclosure.
Figure 20 is a diagram of a porous media coupling in accordance with an embodiment of the present disclosure.
Figure 21A is a perspective view of a radial coupling in accordance with an embodiment of the present disclosure.
Figure 21B is a perspective sectional view of the radial coupling of Figure 21A.
Figure 22A is a perspective view of a sandwich coupling in accordance with an embodiment of the present disclosure.
Figure 22B is a perspective sectioned view of the sandwich coupling of Figure 22A.
Figure 22C is a sectioned view of the sandwich coupling of Figure 22A attached to a humidification housing in accordance with an embodiment of the present disclosure.
Figure 22D is a sectioned view of the sandwich coupling of Figure 22A attached to a humidification housing that includes a printed circuit board heating element, in accordance with an embodiment of the present disclosure.
Figure 23A is a perspective view of an alternative embodiment of a humidification system in accordance with an embodiment of the present disclosure.
Figure 23B is a cross-section view of the humidification system of Figure 23A.
Figure 23C is a cross-section view showing the top layer of the humidification system of Figure 23A.
Figure 23D is a cross-section view showing the bottom layer of the humidification system of Figure 23A.
Figure 24 is a view of an inline humidification system in accordance with one an embodiment of the present disclosure.
Figure 25 is a view of an embodiment of an insufflation system comprising a humidifier and/or a humidification chamber.
Figures 26A and 26B show peel force data for PP/Scona molded members having bonding layers comprising silicon-containing linkers versus control overmolded devices.
Figures 27A and 27B show peel force data for Elvaloy molded members having bonding layers comprising silicon-containing linkers versus control overmolded devices.
Figures 28A and 28B show peel force data for PEX molded members having bonding layers comprising silicon-containing linkers versus control overmolded devices.
Figure 29A shows peel force data for Desmopan molded members having bonding layers comprising silicon-containing linkers versus control overmolded devices.
Figures 29B-D are scanning electron microscope images. Figure 29B depicts a PCB after a Desmopan molded member having bonding layers comprising silicon-containing linkers was peeled from it. Figures 29C and 29D are control overmolded PCB devices after peel testing.

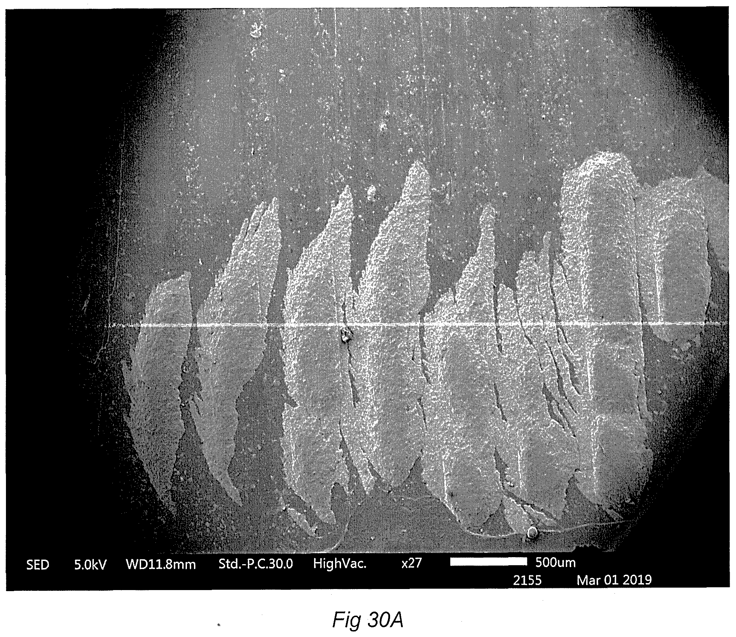

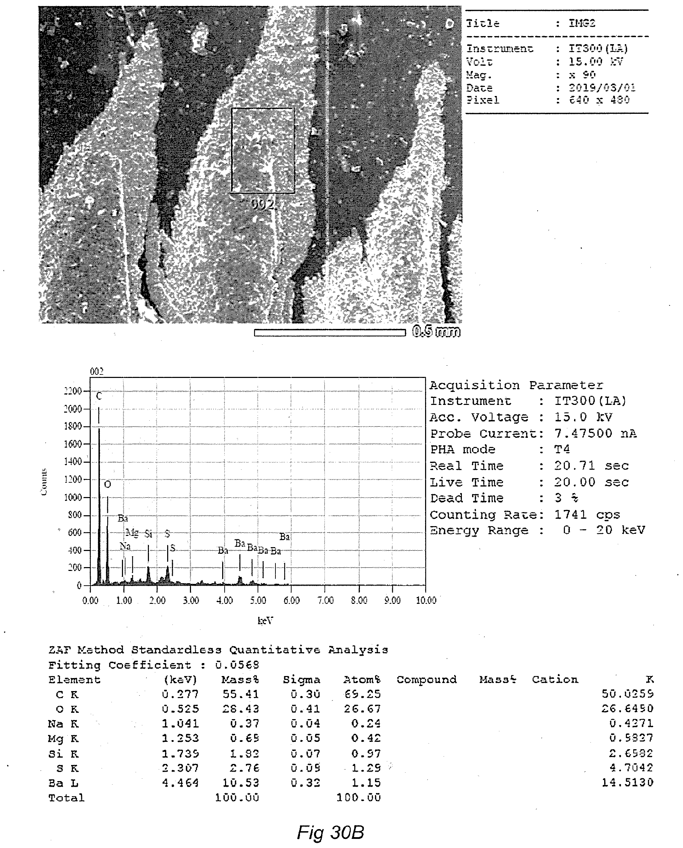

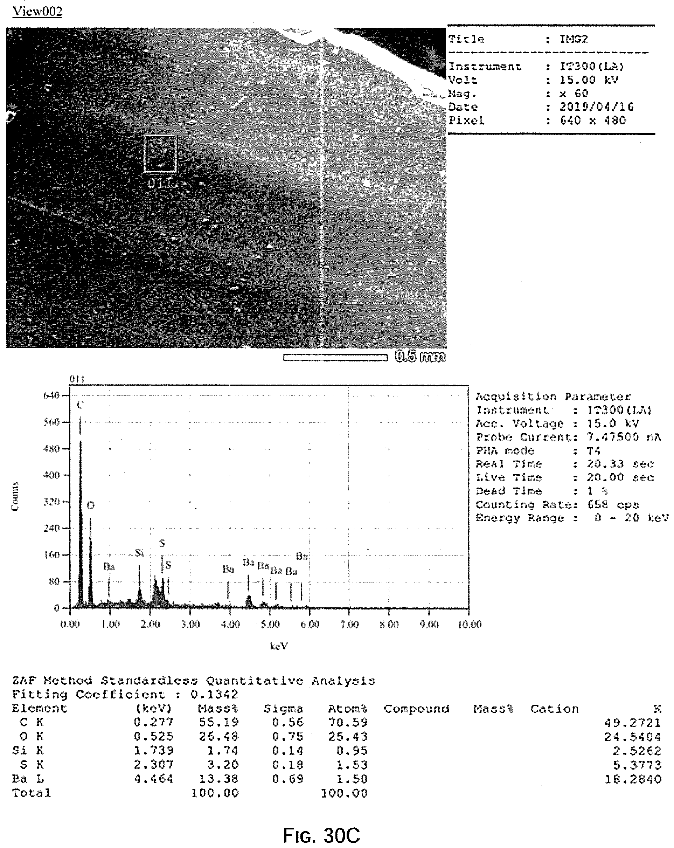

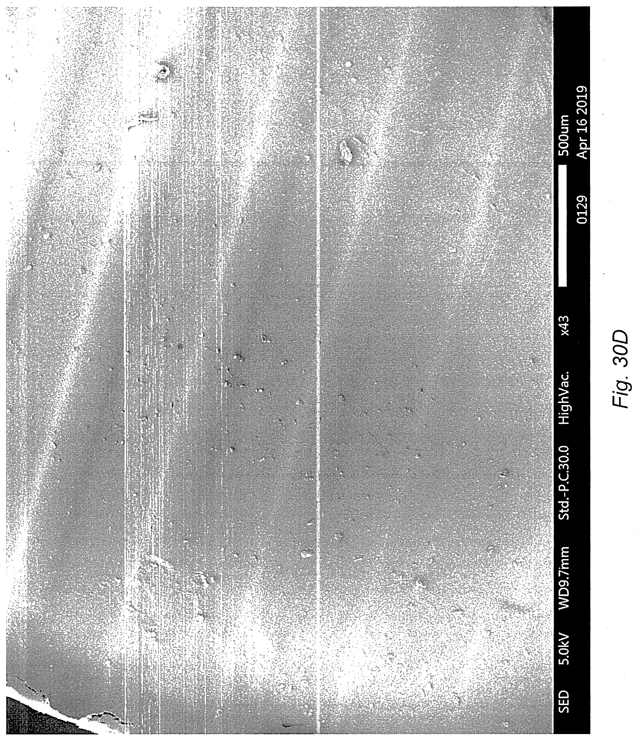

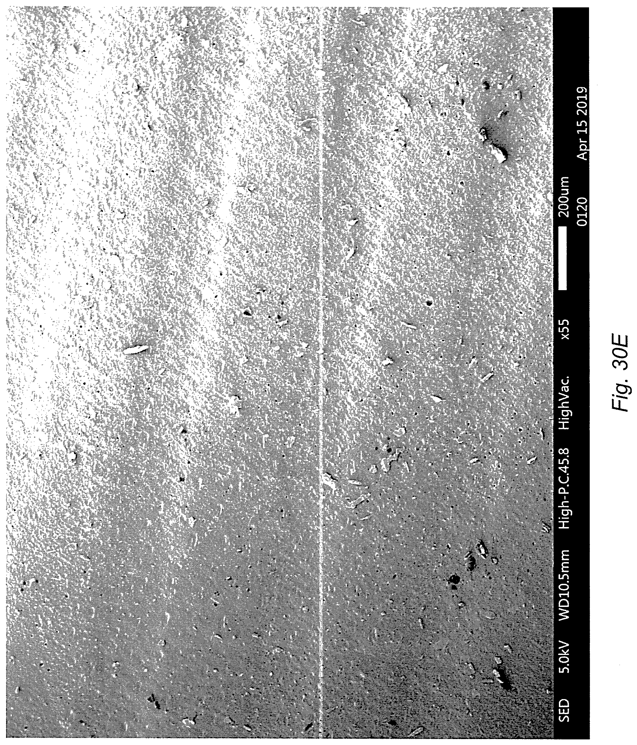

Figures 30A-E are scanning electron microscope images. Figures 30A-30B are images of a Surlyn molding material after it was peeled from a PCB. The molding material was bonded to the PCB via a bonding layer comprising silicon-containing linkers prior to peeling. Figure 30B provides elemental analysis of certain areas of the scanning electron microscope images of the molding material. Figure 30C provides an SEM image of the PCB after the Surlyn molding material was peeled from it, along with elemental analysis of certain areas of the scanning electron microscope images of the PCB. Figures 30D and 30E are images of control overmolded PCB devices after peel testing.
Figures 31A-D are scanning electron microscope images. Figures 31A and 31B depict a PCB after a Nucrel molded member having bonding layers comprising silicon-containing linkers was peeled from it. Figures 31C and 31D are control overmolded PCB devices after peel testing.
Figure 32A shows peel force data for Pebax molded members having bonding layers comprising silicon-containing linkers versus control overmolded devices; and Figures 33 to 52 show various schemes in accordance with aspects of this disclosure.

### DETAILED DESCRIPTION

The following description is merely illustrative in nature and is in no way intended to limit the disclosure, its application, or uses. For purposes of clarity, the same reference numbers will be used in the drawings to identify similar elements. However, for the sake of convenience, certain features present or annotated with reference numerals in some figures of the present disclosure are not shown or annotated with reference numerals in other figures of the present disclosure. Unless the context clearly requires otherwise, these omissions should not be interpreted to mean that features omitted from the drawings of one figure could not be equally incorporated or implemented in the configurations of the disclosed methods, apparatus and systems related to or embodied in other figures. Conversely, unless the context clearly requires otherwise, it should not be assumed that the presence of certain features in some figures of the present disclosure means that the disclosed methods, apparatus and systems related to or embodied in such figures must necessarily include these features.

As used herein, the term "alkyl" refers to a radical of a fully saturated hydrocarbon, including, but not limited to, methyl, ethyl, n-propyl, isopropyl, n-butyl, isobutyl, tert-butyl, n-hexyl, and the like. When the term "alkyl" and a more specific term for alkyl (such as propyl, butyl, etc.) is used without specifying linear or branched, the term is to be interpreted to include linear and branched alkyl.

The term "alkenyl" used herein refers to a monovalent straight or branched chain radical of from two to twenty carbon atoms containing a carbon double bond including, but not limited to, 1-propenyl, 2-propenyl, 2-methyl-1-propenyl, 1-butenyl, 2-butenyl, and the like.

The term "alkoxy" used herein refers to straight or branched chain alkyl radical covalently bonded to the parent molecule through an -O- linkage. Examples of alkoxy groups include, but are not limited to, methoxy, ethoxy, propoxy, isopropoxy, butoxy, n-butoxy, sec-butoxy, t-butoxy and the like. When the term "alkoxy" and a more specific term for alkoxy (such as propoxy, butaoxy, etc.) is used without specifying linear or branched, the term is to be interpreted to include linear and branched alkoxy.

The term "alkylene" used herein refers to a bivalent saturated aliphatic radical regarded as derived from an alkene by opening of the double bond or from an alkane by removal of two hydrogen atom to form a linker, which links together molecular fragments via their terminal carbon atoms. Examples include, but not limited to, methylene (-CH₂-), ethylene (-CH₂CH₂-), propylene (-CH₂CH₂CH₂-), butylene (-(CH₂)₄-), pentylene (-(CH₂)₅-), hexylene (-(CH₂)₆-), and the like.

The term "heteroalkylene" used herein refers to an alkylene group in which one or more of the carbon atoms are independently replaced with the same or different heteroatoms selected from oxygen, sulfur and nitrogen. The heteroalkylene may have 1-4 heteroatoms, 1-3 heteroatoms, 1-2 heteroatoms or 1 heteroatom. Examples of heteroalkylene include, but not limited to, -CH₂-O-, -CH₂-CH₂-O-, -CH₂-CH₂-CH₂-O-, -CH₂-NH-, -CH₂-CH₂-NH-, -CH₂-CH₂-CH₂-NH-, -CH₂-CH₂-NH-CH₂-, -CH₂-CH₂-NH-CH₂-CH₂-, -CH₂-CH₂-O-CH₂-CH₂-, -O-CH₂-CH₂-O-CH₂-CH₂-O-, -O-CH₂-CH₂-O-CH₂-CH₂-, and the like.

The term "epoxy" used herein refers to 3-member cyclic ether.

The term "halo" used herein refers to fluoro, chloro, bromo, or iodo.

The term "amino" or "amine" used herein refers to a primary, secondary or tertiary amine.

The term "glycidyl methacrylate" used herein refers to 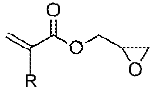 group, wherein R is C1-8 alkyl.

The term "vinyl" used herein refers to -CH=CH-.

The term "succinic anhydride" used herein refers to

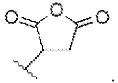

The term "carboxyl" used herein refers to -C(O)OH.

In devices for delivering gases to patients, (e.g., respiratory devices and systems, insufflation devices and systems, etc.), it may be helpful to heat gases or to monitor the humidity level, temperature, and flow rate of gases passing through the gas passage way. Delivering humidified and heated gases to a patient can be beneficial during surgical operation and respiratory therapy. Humidified gases could, for example, increase comfort for patients using the delivery devices and increasing compliance. Thus, various substrates that are configured to include sensors or heating elements may be integrated or embedded in gas delivery device components (e.g., respiratory device components, insufflation device components, etc.), such as connectors, gas flow paths, tubes, or humidifier chambers. The substrates may be integrated with or embedded into the gas delivery device components using injection molding (either high or low pressure). The injection molding may be insert molding or overmolding. For example, the substrate may be insert molded to form a respiratory device component.

While the various substrates can be physically integrated or embedded into components for the respiratory device, liquid ingress into the interface of the substrate and a molding material can occur under the elevated humidity and/or elevated temperature environment. This can cause delamination of the molding material from the substrates. Delamination may lead to failure of the device, leading to leaks that require replacement of often times costly components. Delamination may also lead to a loss of control and/or regulation of the system, which could cause one or more components to overheat and melt. If delamination occurs one or more components of the device could short circuit (e.g., due to water contacting electrical components, water egress out of the humidification system, etc.), causing device failure. If the device fails, patients are inconvenienced or, worse, their safety could be jeopardized. Additionally, partial or complete delamination may make it more difficult to clean and reuse the humidification device or its components. This contamination (e.g., microbial) could lead to increased likelihood of respiratory infection and, in the case of an insufflation device, potential introduction of foreign agents inside the body.

As disclosed elsewhere herein, in some embodiments, a molded member with a robust substrate-molding material interface (i.e. a bonding layer) is provided, which can prevent and/or delay delamination of the molding material from the substrate under an elevated humidity and/or elevated temperature environment. In some embodiments, in order to promote the adhesion between a molding material and a substrate (or a portion thereof) that is integrated with, encapsulated by (e.g. embedded in), or bonded to the molding material, a bonding layer is provided between the molding material and substrate. In some embodiments, the bonding layer attaches the molding material to the substrate via one or more of chemical or physical interactions. In some embodiments, chemical bonding can be formed between the substrate and the molding material. For example, forming covalent bonds (and/or chemical bonding) between the substrate and the molding material may increase the adhesiveness of the two different materials, and slow or prevent delamination due to environmental stress such as heat, pressure, mechanical force, or liquid ingress. A silicon-containing linking agent may be used to chemically (e.g., covalently, etc.) bond two types of materials. The molding material may include thermoplastic materials and thermosetting materials.

In some embodiments, as disclosed elsewhere herein, the bonding layer may be formed at the interface between the surface of the molding material and the substrate. In certain implementations, the substrate and molding material are otherwise unmixed. In other words, the bulk material of the substrate is unchanged as is the bulk material of the molding material. In certain embodiments, the bonding between two layers occurs only at the interface of these materials (e.g., through a bonding layer). In certain implementations, it has been determined that, even with the relatively small amount of interaction between surface functionalities at the interface between the molding material and the substrate (compared to, for example, epoxy resins, paints, etc.), substantially enhanced adhesive properties are obtained.

In some embodiments, as disclosed elsewhere herein, the bonding layer comprises a silicon-containing linker. In some embodiments, the bonding layer is different from conventional adhesive layers in that it may comprise pendant groups from the molding material and pendant groups from the substrate. These pendant groups may interact to provide a strong bonding force between the materials on which they reside. In certain implementations, the layer comprises the silicon containing linker. In certain implementations, as disclosed elsewhere herein, the bonding layer comprises one or more types of chemical interactions. The bonding layer may include one or more of covalent bonding, hydrogen-bonding, bonding through Van der Waals forces, and/or ionic bonding. The bonding layer may comprise chemical bonds, physical bonds, or both. Physical bonds may include chain entanglement, etc.

In some embodiments, the molding material comprises pendant groups (e.g., a silicon-containing linker, a moiety of the molding material, etc.) that are covalently bonded to the molding material. In some embodiments, the substrate comprises pendant groups (and/or functional groups) that are covalently bonded to the substrate. In some embodiments, pendant groups (and/or functional groups) of the molding material interact with the pendant groups (and/or functional groups) of the substrate to provide the bonding layer. In certain implementations, the bonding layer does not comprise a separate adhesive from the pendant groups and/or functional groups of the substrate and/or molding material. In some embodiments, the pendant groups and/or functional groups of the substrate form chemical bonds (e.g., covalent bonds, etc.) with coinciding pendant groups and/or functional groups of the molding material to adhere the substrate and molding material together. In certain implementations, as disclosed elsewhere herein, the chemical bonding does not occur throughout a bulk material (such as throughout the molding member and/or substrate), but instead occurs only at the interface of two materials.

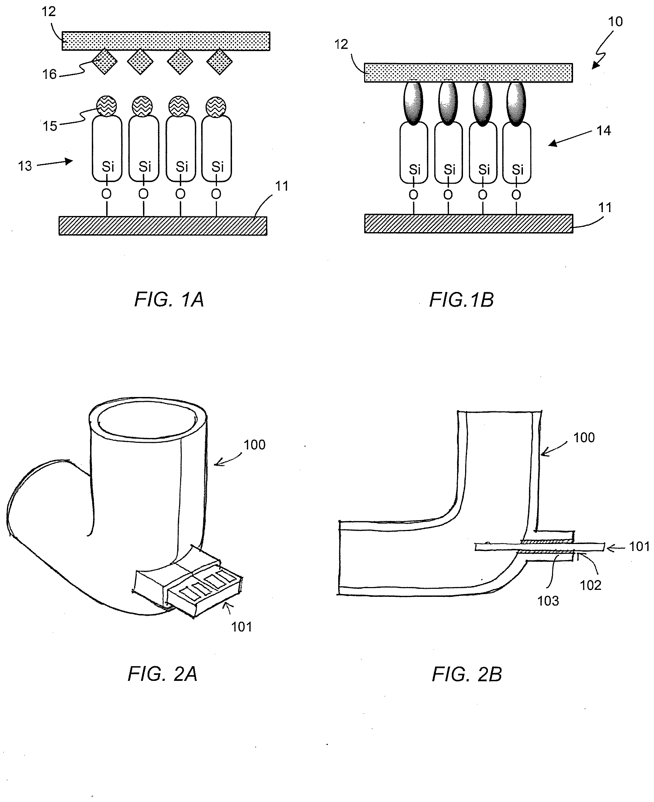

Figs. 1A and 1B illustrate the bonding or coupling between a substrate 11 and a molding material 12. A substrate 11 with attached silicon-containing linking agent 13 on its surface is provided, and a molding material 12 is bonded to said substrate 11. The molding material 12 (or its precursor) contains a moiety 16 that is capable of reacting with the silicon-containing linking agent 13. The bonding occurs when a moiety 15 in the attached silicon-containing linking agent 13 reacts with the moiety 16 in the molding material 12, and forms a silicon-containing linker 14 that bonds the substrate 11 and the molding material 12 together (*see* Figure 1B).

As disclosed herein, a molded member 10 may comprise a substrate 11 and a molding material 12, such as thermoplastic material or thermosetting material, wherein at least a portion of the substrate 11 is coupled to the molding material 12. In some embodiments, the substrate 11 may be embedded in or integrated with a molding material 12. The substrate 11 may be integrated with or embedded within the molding material 12 using injection molding (either high or low pressure). The injection molding may be insert molding or overmolding. In some embodiments, the substrate 11 may be insert molded with molding material 12 to form a molded member 10. In some embodiments, the molding material 12 is an overmolding on or around the substrate 11. At least a portion of the substrate 11 is coupled to the molding material 12 via a silicon-containing linker 14, and the bonding between the silicon-containing linker 14 and the substrate 11 and between the silicon-containing linker 14 and the molding material 12 is covalent.

A molded member may be formed by coupling a molding material 12 to a substrate 11. A substrate with at least one attached silicon-containing linking agent on the surface is provided, and a molding material 12 is bonded to the at least one attached silicon-containing linking agent. In some embodiments, the molded member may be a component of a respiratory apparatus or device. Non-limiting examples of the components include housing for a filter, a gas conduit, a chamber, an inspiratory tube, a tube connector, a tube joint, a tube elbow, a heating element, a patient interface component, and a water dosing component.

In some embodiments, as disclosed elsewhere herein, the disclosed bonding layers are adapted to prevent or delay delamination of a molding material from a substrate (or vice versa) at elevated humidity and/or elevated temperature or due to rapid changes in conditions (e.g., temperature). In some embodiments, an elevated humidity is a humidity higher than that of ambient conditions and/or conditions within the gas source (e.g., a compressed gas cylinder, etc.). In some embodiments, elevated humidity includes gases (e.g., in a gas humidification system) having a relative humidity greater than or equal to about: 40%, 50%, 60%, 70%, 80%, 90%, 99%, 99.9%, 100%, and/or ranges including and/or spanning the aforementioned values. In some embodiments, water dosed onto the surface of the molding material (e.g., liquid water) may contribute to delamination. In some embodiments, elevated temperatures include gases (e.g., in a gas humidification system) having a temperature that is higher than ambient temperature. In some embodiments, the temperature of the gases in the gas delivery system are greater than or equal to about: 15°C, 20°C, 25°C, 30°C, 35°C, 40°C, 45°C, 50°C, 60°C, and/or ranges including and/or spanning these temperatures.

### Silicon-Containing Linker

In embodiments where the molding material 12 is a thermoplastic material, the silicon-containing linker 14 may be a silane coupling linker, a silicon alkoxide linker, or a siloxane linker. In some embodiments, the silicon-containing linker is a silane coupling linker represented by the following formulae: 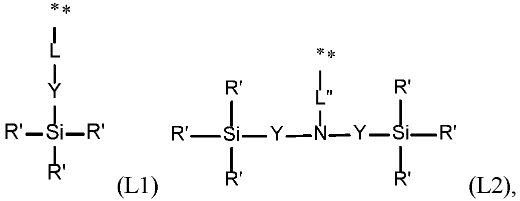 or a polymerized silane coupling linker thereof. In the formulae (L1) and (L2), ** is a point of attachment to the thermoplastic material; each R' is independently selected from the group consisting of hydrogen, halogen, hydroxyl, C1-C8 alkyl, C1-C8 alkoxy, and -O-*, wherein * is a point of attachment to the substrate, and at least one of the R' is -O-* ; Y is a single bond, C1-C8 alkylene, or C1-C8 heteroalkylene containing one or more heteroatoms selected from O and N; -L-** is selected from the group consisting of: 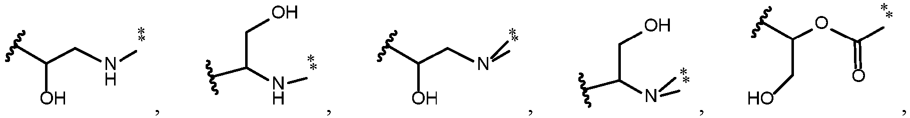 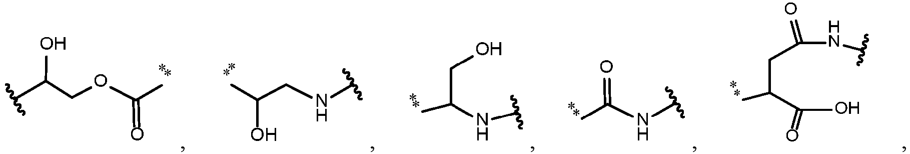 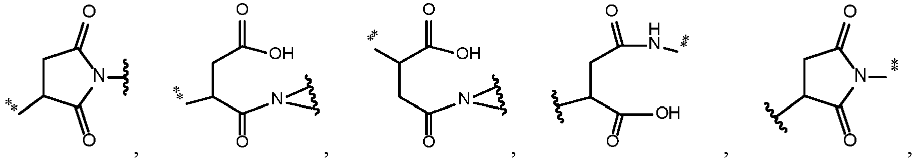 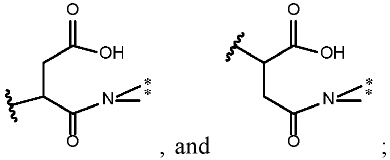 and -L"-** is selected from the group consisting of: 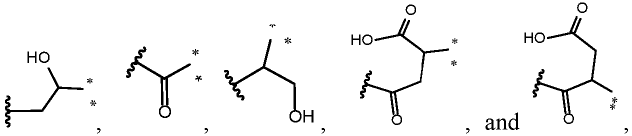 wherein ** is a point of attachment to the thermoplastic materials

In some embodiments, the polymerized silane coupling linker may include: 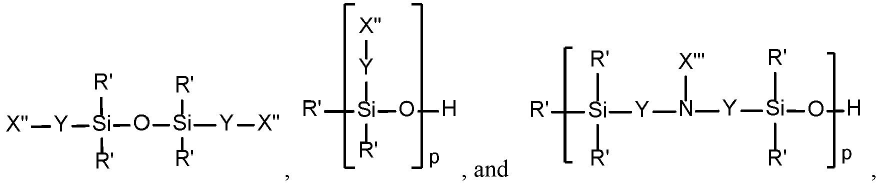 wherein X" is independently X or -L-**, wherein X is selected from the group consisting of epoxy, primary or secondary amine, or succinic anhydride; X‴ is H or -L"-**; at least one of the X" is -L-** ; at least one of the R' is -O-*; at least one of the X‴ is -L"-** ; and p is an integer greater than 1.

In some embodiments, the silicon-containing linker is a silicon alkoxide linker represented by the following formulae: 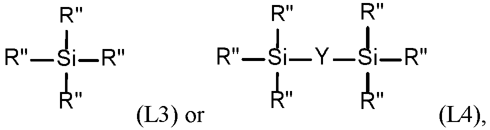 or a polymerized silicon alkoxide linker thereof; wherein R" is independently hydrogen, halogen, hydroxyl, C1-C8 alkyl, C1-C8 alkoxyl, -O-* or -O-**, wherein * is a point of attachment to the substrate, ** is a point of attachment to the thermoplastic material, and at least one of the R" is -O-* and at least one of the R" is -O-**; and Y is a single bond, C1-C8 alkylene, or C1-C8 heteroalkylene, wherein the C1-C8 heteroalkylene contains one or more heteroatoms selected from O and N.

In some embodiments, the polymerized silicon alkoxide linker may include: 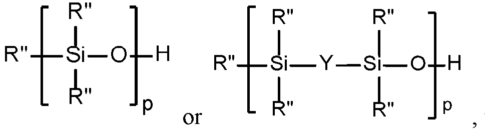 wherein p is an integer greater than 1.

In some embodiments, the silicon-containing linker is a siloxane linker represented by the following formulae: 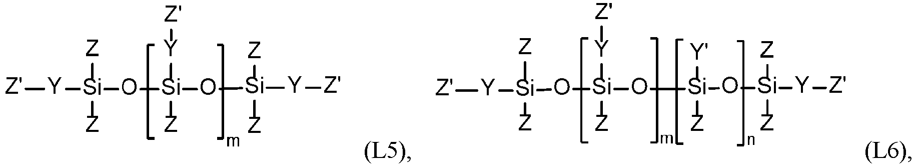 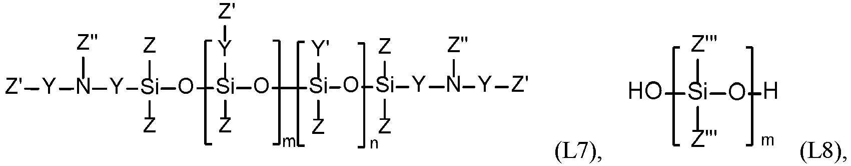 or a polymerized siloxane linker thereof, wherein each Z is independently hydroxyl or -O-*, wherein * is a point of attachment to the substrate; each Z' is selected from the group consisting of epoxy, primary and secondary amine, succinic anhydride, and -L-**, wherein ** is a point of attachment to the thermoplastic material, -L-** is selected from the group consisting of: 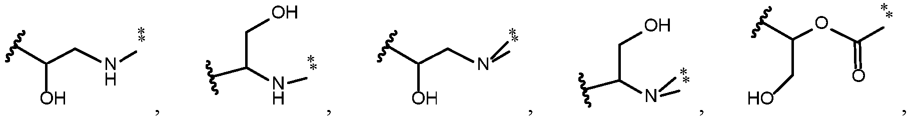 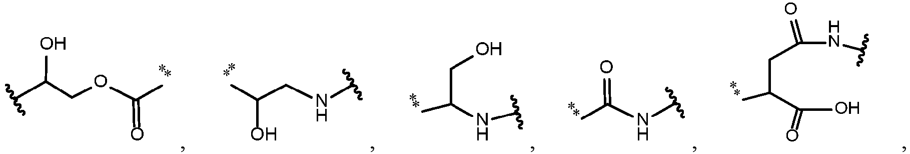 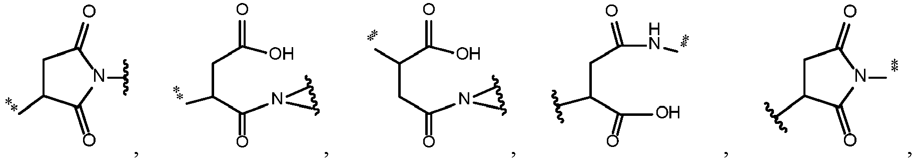 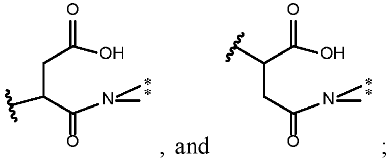 Z" is hydrogen or -L"-**, wherein -L"-**, is selected from the group consisting of: 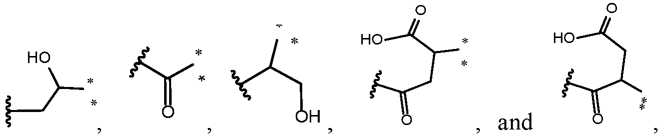 and ** is a point of attachment to the thermoplastic materials; at least one of the Z is -O-*, and at least one of the Z' is -L-** or at least one of the Z" is -L"-**; Y is a single bond, C1-C8 alkylene, or C1-C8 heteroalkylene containing one or more heteroatoms selected from O and N; Y' is C1-C8 alkyl or C1-C8 heteroalkyl containing one or more heteroatoms selected from O and N; Z‴ is independently hydroxyl, -O-* or -O-**, wherein * is a point of attachment to the substrate, ** is a point of attachment to the thermoplastic materials, at least one of the Z‴ is -O-* , and at least one of the Z‴ is -O-** ; m is an integer 1 or greater; and n is an integer 1 or greater. In some embodiments, m and n may independently be an integer from 1 to 200, from 1 to 70, from 1 to 50, from 1 to 30, or from 1 to 10.

In some embodiments, siloxane linkers may further polymerize to form a polymerized siloxane linker (e.g., a polysiloxane linker). Polymerized siloxane may be formed when hydroxyl groups in a siloxane linker structure condense with hydroxyl groups in other siloxane linkers.

In embodiments where the molding material is a thermosetting material, such as a silicone rubber, the silicon-containing linker may be a vinyl functionalized silane coupling linker represented by the following formulae: 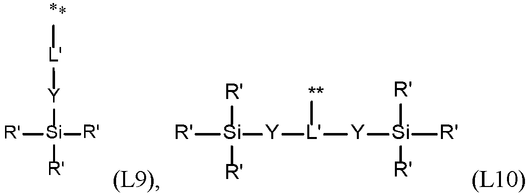 or a polymerized vinyl functionalized silane coupling linker thereof; wherein ** is a point of attachment to the thermosetting material; each R' is independently hydrogen, halogen, hydroxyl, C1-C8 alkyl, C1-C8 alkoxy, or -O-* , wherein * is a point of attachment to the substrate, wherein at least one of the R' is -O-*; -L'-** in (L9) is 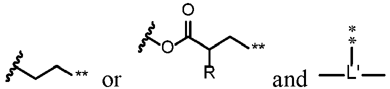 in (L10) is 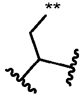 or 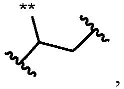 wherein R is H or C1-C8 alkyl; Y is a single bond, C1-C8 alkylene, or C1-C8 heteroalkylene containing one or more heteroatoms selected from O and N.

In some embodiments, the polymerized silane coupling linker may include: 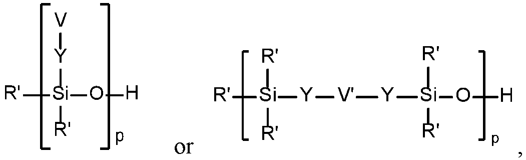 wherein V is independently X' or -L'-**, and V' is independently -X'- or 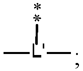 X' is 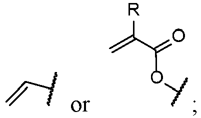 -L'-** is 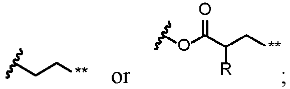 -X'- is 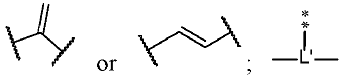 is 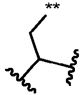 or 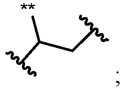 wherein R is H or C1-C8 alkyl; R' is independently hydrogen, halogen, hydroxyl, C1-C8 alkyl, C1-C8 alkoxy, or -O-*, wherein * is a point of attachment to the substrate, wherein at least one of the R' is -O-* ; p is an integer greater than 1.

In some embodiments of the formulae described herein, p may be an integer from 2 to 200, from 2 to 70, from 2 to 50, from 2 to 30, or from 2 to 10. In some embodiments of the formulae described herein, m and n may independently be an integer from 1 to 200, from 1 to 70, from 1 to 50, from 1 to 30, or from 1 to 10. In some embodiments, p is an integer greater than or equal to about: 1, 10, 50, 100, 200, or ranges including and/or spanning the aforementioned values.

In some embodiments, a silicon-containing linker described herein may be in a polymerized form when one or more silanol groups in the structure condense with silanol groups in other structures. In general, polysiloxane can be used to describe siloxane bonds (Si-O-Si) that joined together and become a polymer. For example, two or more molecules of silane coupling linkers or silicon alkoxide linkers may polymerize to form chains or clusters of siloxane linkers, and the chains/clusters of siloxane linkers may further polymerize to form polysiloxane linkers. Two or more siloxane linkers may also further polymerized to form polymerized siloxane linkers, which are also polysiloxane linkers. Thus, the silicon-containing linker as described herein may include any siloxane or polysiloxane that is formed by polymerization of simpler forms of the silicon-containing linker.

In some embodiments, the silicon-containing linker is covalently bonded to the thermoplastic material. In some embodiments, the silicon-containing linkers are covalently bonded to the substrate.

### Silicon-Containing Linking Agents

Particular silicon-containing linking agents may be suitable for covalent bonding with thermoplastic material. The silicon-containing linking agent may be a silane coupling agent, a silicon alkoxide, or a siloxane. In some embodiments, the silane coupling agent is represented by the following formula: 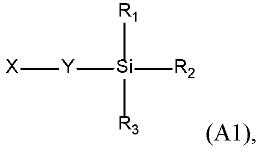 wherein R₁, R₂, and R₃ are each independently selected from the group consisting of hydrogen, halogen, hydroxyl, C1-C8 alkyl, and C1-C8 alkoxy, and at least one of R₁, R₂, and R₃ in (A1) is hydroxyl or C1-C8 alkoxy, Y is a single bond, C1-C8 alkylene, or C1-C8 heteroalkylene containing one or more heteroatoms selected from O and N; and X is selected from the group consisting of epoxy, primary or secondary amine, and succinic anhydride.

In some embodiments, the silane coupling agent is represented by the following formula: 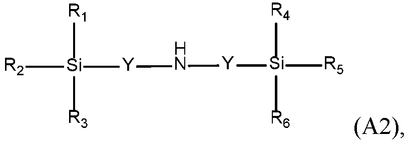 wherein R₁, R₂, R₃, R₄, R₅, and R₆ are each independently selected from the group consisting of hydrogen, halogen, hydroxyl, C1-C8 alkyl, and C1-C8 alkoxy, and at least one of R₁, R₂, R₃, R₄, R₅, and R₆ in (A2) is hydroxyl or C1-C8 alkoxy, and Y is a single bond, C1-C8 alkylene, or C1-C8 heteroalkylene containing one or more heteroatoms selected from O and N.

A fully hydrolyzed silanol has 3 hydroxyl groups in formula (A1), and 6 hydroxyl groups in formula (A2). In some embodiments, as a reactive silanol, formula (A1) may have 1, 2, or 3 hydroxyl group(s), while formula (A2) may have 1, 2, 3, 4, 5, or 6 hydroxyl group(s). In embodiments where any of the R₁, R₂, R₃, R₄, R₅, and R₆ is a hydroxyl group, the silane coupling agent is a reactive silanol. In embodiments where any of the R₁, R₂, R₃, R₄, R₅, and R₆ is a C1-C8 alkoxy group, one or more of C1-C8 alkoxy group(s) can be hydrolyzed to form a hydroxyl group, making the silane coupling agent a reactive silanol.

In some embodiments, a silane coupling agent that has a hydrolyzable group (e.g., alkoxy) can form polysiloxane after the sol-gel process. The sol-gel process involves hydrolysis of silane to form reactive silanol and subsequent condensation of silanol groups. Dipodal silanes have the ability to form six bonds to a substrate while the non-dipodal silanes can only form three bonds to a substrate. Therefore, in some embodiments, dipodal silanes (e.g. formula (L2)) have much higher intrinsic hydrolytic stabilities than non-dipodal silanes (e.g., formula (L1)). As such, in some embodiments, dipodal silanes may used to provide better adhesion to the substrate than non-dipodal silanes, and may also have a higher tolerance to heat and to water ingress.

Molded members with a dipodal silane coupling agent may therefore have a longer shelf life. Commercially available dipodal silanes may not always be available with the desired functional group. However, by using a dipodal silane without a functional group in combination with a non-dipodal silane that does have the desired functional group, an appropriate silicon-containing linking agent may be formed. In such cases, the benefits of dipodal silanes described above are retained. Therefore, in some embodiments, using combination of dipodal silanes with non-dipodal silanes may have an impact on substrate bonding, hydrolytic stability, and mechanical strength of the polysiloxane due to an increase in the crosslink density.

In some embodiments, in the sol-gel process, a silane coupling agent (with the functional group X) can be hydrolyzed and condensed with another functional dipodal silane, forming a polysiloxane with the functional group X.

Examples of silane coupling agents include the following.

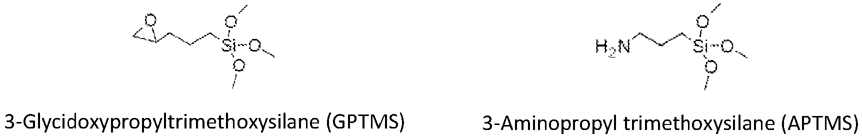

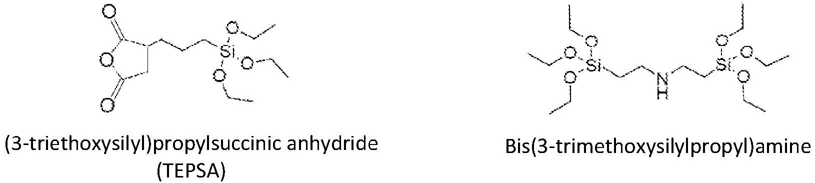

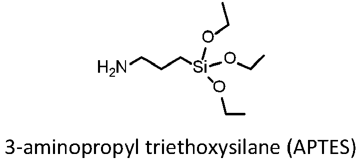

In some embodiments, silicon alkoxide is represented by the following formulae: 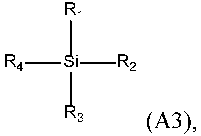 wherein R₁, R₂, R₃, and R₄ are each independently selected from the group consisting of hydrogen, halogen, hydroxyl, C1-C8 alkyl, and C1-C8 alkoxy, and at least two of R₁, R₂, R₃, and R₄ in (A3) is independently hydroxyl or C1-C8 alkoxy.

In some embodiments, silicon alkoxide is represented by the following formulae: 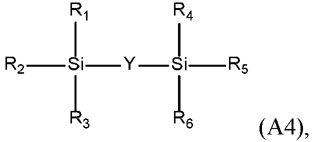 wherein R₁, R₂, R₃, R₄, R₅, and R₆ are each independently selected from the group consisting of hydrogen, halogen, hydroxyl, C1-C8 alkyl, and C1-C8 alkoxy, and at least two of R₁, R₂, R₃, R₄, R₅, and R₆ in (A4) is independently hydroxyl or C1-C8 alkoxy; and Y is a single bond, C1-C8 alkylene, or C1-C8 heteroalkylene containing one or more heteroatoms selected from O and N.

A fully hydrolyzed silanol has 4 hydroxyl groups in formula (A3), and 6 hydroxyl groups in formula (A5). In some embodiments, as a reactive silanol, formula (A3) may have 1, 2, 3, or 4 hydroxyl group(s), while formula (A4) may have 1, 2, 3, 4, 5, or 6 hydroxyl group(s). In embodiments where any of the R₁, R₂, R₃, R₄, R₅, and R₆ is a hydroxyl group, the silicon alkoxide is a reactive silanol. In embodiments where any of the R₁, R₂, R₃, R₄, R₅, and R₆ is a C1-C8 alkoxy group, one or more of C1-C8 alkoxy group(s) can be hydrolyzed to form a hydroxyl group, making the silicon alkoxides a reactive silanol.

A few examples of the silicon alkoxides are provided below:

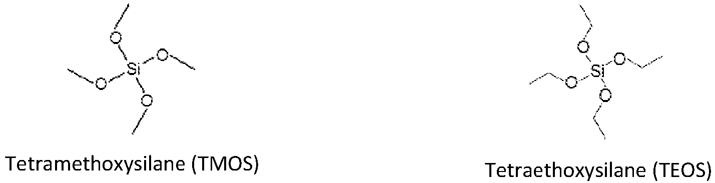

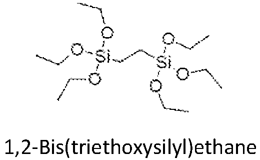

In some embodiments, siloxane is represented by the following formulae: 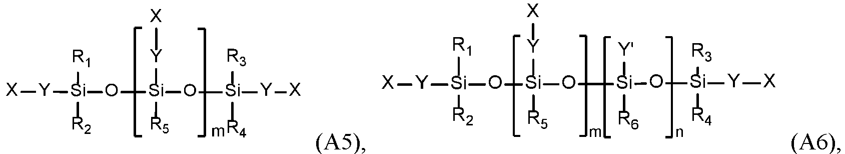 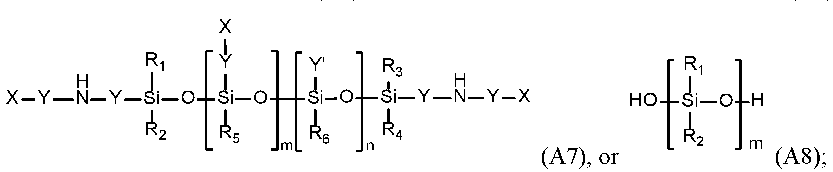 wherein R₁, R₂, R₃, R₄, R₅, and R₆ are each independently selected from the group consisting of hydrogen, halogen, hydroxyl, C1-C8 alkyl, and C1-C8 alkoxy, and at least one of R₁, R₂, R₃, R₄, and R₅ in (A5), at least one of R₁, R₂, R₃, R₄, R₅, and R₆ in (A6) and (A7), and at least two of R₁ and R₂ in (A8) are independently hydroxyl or C1-C8 alkoxy; Y is a single bond, C1-C8 alkylene, or C1-C8 heteroalkylene containing one or more heteroatoms selected from O and N; Y' is C1-C8 alkyl or C1-C8 heteroalkyl containing one or more heteroatoms selected from O and N; X is selected from the group consisting of epoxy, primary or secondary amine, succinic anhydride; m is an integer 1 or greater; and n is an integer 1 or greater. In some embodiments of all the formulae described herein, m and n may independently be an integer from 1 to 200, from 1 to 70, from 1 to 50, from 1 to 30, or from 1 to 10.

Examples of siloxane includes aminopropyl silsesquioxane, aminoethyl aminopropyl silsesquioxane, aminopropylsilsesquioxane-methylsilsesquioxane, and non-functional siloxane.

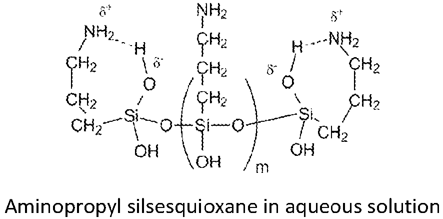

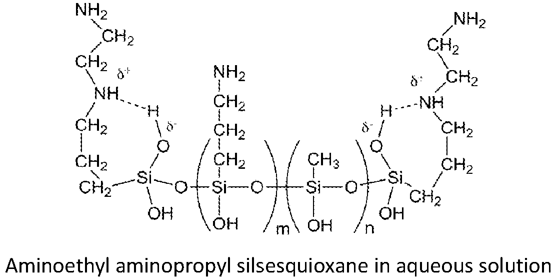

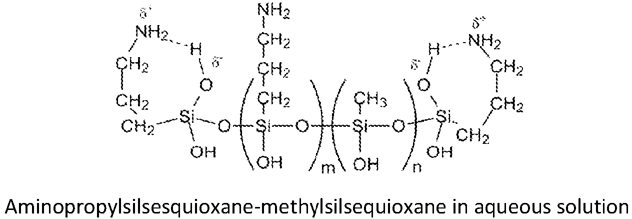

Particular silicon-containing linking agents may be suitable for covalent bonding with thermosetting material, such as silicone rubber. In some embodiments, the silicon-containing linking agent may be a vinyl functionalized silane coupling agent represented by the formulae: 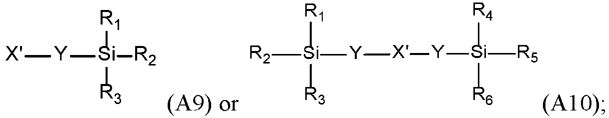 wherein R₁, R₂, and R₃ in (A9) are each independently selected from the group consisting of hydrogen, halogen, hydroxyl, C1-C8 alkyl, and C1-C8 alkoxy, and at least one of R₁, R₂, and R₃ in (A9) is hydroxyl or C1-C8 alkoxy; R₁, R₂, R₃, R₄, R₅, and R₆ in (A10) are each independently selected from the group consisting of hydrogen, halogen, hydroxyl, C1-C8 alkyl, and C1-C8 alkoxy, and at least one of R₁, R₂, R₃, R₄, R₅, and R₆ in (A10) is hydroxyl or C1-C8 alkoxy; X' is 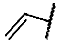 or 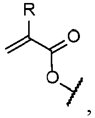 and -X'- is 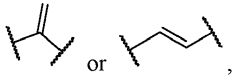 wherein R is H or C1-C8 alkyl; Y is a single bond, C1-C8 alkylene, or C1-C8 heteroalkylene containing one or more heteroatoms selected from O and N.

Example of vinyl-containing silane coupling agents include the following.

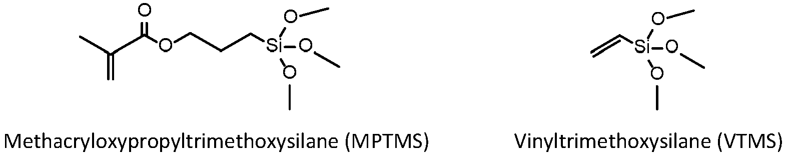

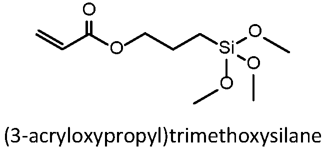

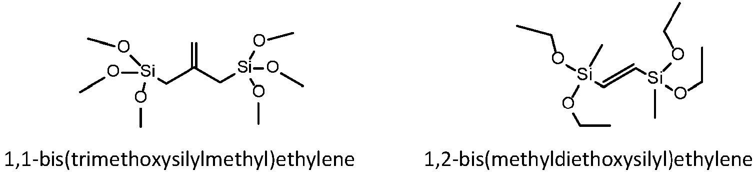

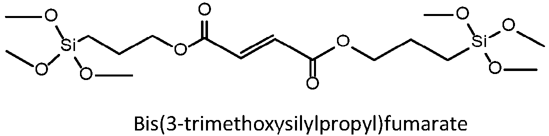

As with the silane coupling agents described above, vinyl functionalized silane coupling agents can also polymerize to form oligomers and polymers (such as siloxanes and/or polysiloxanes). Therefore, polymerized vinyl functionalized silane coupling agents are also within the scope of this application.

### Substrates

Suitable substrates may include materials having a hydroxyl (-OH) containing surface. The substrate may also include materials that can be activated to form hydroxyl or hydroxyl-bearing groups on the surface. The -OH group can react with a silanol derived from or already present in the silicon-containing linking agent and result in anchoring of the silanol derived from the silicon-containing linking agent. The more -OH groups there are on the substrate surface, the more available sites there are for bonding to the silanol derived from or present in the silicon-containing linking agent. A silanol derived from the silicon-containing linking agent with more than one alkoxy group can form more than one bond with the substrate.

In some embodiments, the surface of the substrate has -OH groups or moieties that contain -OH group(s). Some surfaces, for example, such as glass, will react with a silicon-containing linking agent without any pre-treatment. Other surfaces, such as a printed circuit board (PCB), may need pre-treatment or activation before a silanol derived from or present in a silicon-containing linking agent can be attached. The surface of the substrate may be activated through plasma treatment, corona discharge, ozone treatment, flame treatment, or chemical treatment with oxidizing reagent such as chromic acid, etc. These surface treatments promote surface oxidation and hydroxylation in which a broad variety of oxygen moieties are produced. Carbonyl (-C=O), carboxyl (-COOH), hydroxyl (-OH), and hydroperoxides (-OOH) groups are generally found in chemical analysis of the treated substrate.

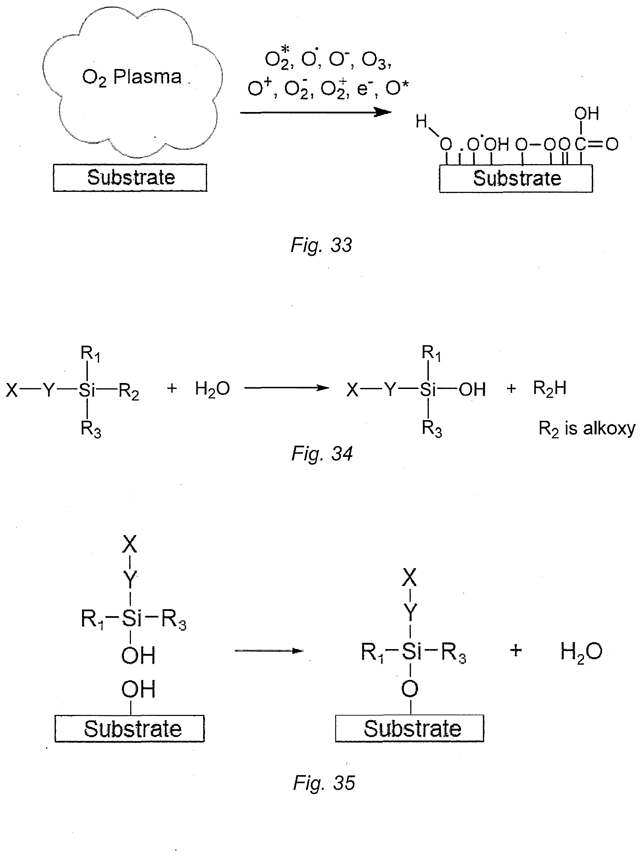

Plasma treatment (i.e., low pressure and atmospheric plasma processes, *see* Figure 33) involves partially ionizing a gas by subjecting a gas to an electric field at high frequency. The derived free electrons are accelerated by the electric field causing the electrons to collide with other atoms in the gas. Consequently, more electrons are released and a mixture of neutral atoms, atomic ions, molecular ions, free radicals, and molecules present in excited and ground states are generated.

Corona discharge is an electrical process involving ionization of air in contact with the substrate. Generally, an electrode with a high potential in air passes an electrical voltage, for example ten kilovolts, to ionize and create plasma around the electrode. The derived ionized gas, positive ions and electrons are passed through an electric field increasing the kinetic energy. The ground electrode then attracts the ion species, completing the circuit and sustaining the current flow to the surface of the substrate.

Ozone treatment uses ultra violet light and ozone to both clean and modify the substrate surface. The process involves ultraviolet irradiation which causes photolysis of the contaminant organic compounds on the surface and generates ions, free radicals, excited molecules, and neutral molecules on the surface.

Flame treatment involves radical reactions due to the combustion of air and a combustible gas. A plasma field is formed when the combustible gas and air are combined and burned to form an intense blue flame. Brief exposure to the energized particles within the flame affects the distribution and density of the electrons on the substrate's surface and polarized surface molecules through oxidation. Water and carbon dioxide are the final reaction products. However, the radical reactions also produce radicals consisting of oxygen species (i.e., -OH, HO₂, H₂O₂, and O₂).

The substrate may be part of a printed circuit board (PCB) and may comprise one or more of ceramic, glass fiber, fiberglass, sized fiberglass, silicone rubber, metal, etched foil, or filled polymer such as glass-reinforced polymer. In some embodiments, a filled polymer can comprise talc, carbon black, carbon fibre, basalt fibre, silica powder, and combinations thereof. In some embodiments, a sized fiberglass comprises fiberglass that has been chemically treated with a sizing agent to allow better bonding with the PCB matrix. In some embodiments, the substrate comprises inorganic materials, organic materials, or both. In some embodiments, the portion of the substrate comprising the linker is organic. In some embodiments, the portion of the substrate comprising the linker is not inorganic. In some embodiments, the substrate does not include inorganic materials.

In some embodiments, the substrate is layered. In some embodiments, the substrate, a layer thereof, and/or an external surface thereof comprises a polymer, e.g. an organic polymer. In some embodiments, the substrate, a layer thereof, and/or a surface thereof comprises one or more of epoxy resin, fiberglass, phenol formaldehyde resin, paper or polyester resin. In some embodiments, the substrate, a layer thereof, and/or a surface of thereof comprises one or more of polyester, polyetherimide or polyimide. In some embodiments, the substrate, a layer thereof, and/or an external surface thereof comprises one or more of epoxy or polyethylene (which may be solvent or water based). In some embodiments, a material (e.g., a conductive material, conductive tracks, etc.) may be provided on the substrate. In some embodiments, the material comprises one or more of copper or aluminum. In some embodiments, the substrate, a layer thereof, and/or an external surface thereof comprises an ink. In some embodiments, the ink is colored and may provide colored text (e.g., white, black, orange, or other colors). In some embodiments, the substrate, a layer thereof, an external surface thereof and/or a material provided on the substrate comprises one or more heat resistant or flame retardant materials.

In some embodiments, the PCB comprises the substrate. In some embodiments, the PCB comprises a substrate and one or more conductive tracks. In some embodiments, the PCB comprises a silk screen ink (e.g., an ink as disclosed elsewhere herein). In some embodiments, the PCB comprises a solder mask (which may be colored). In some embodiments, the solder mask comprises one or more of epoxy or polyethylene (which may be solvent or water based). In some embodiments, the PCB or the conductive tracks comprises a conductor (e.g. one or more of copper or aluminum). In some embodiments, the PCB comprises a heat transfer material (e.g. aluminum for example as a heat sink). In some embodiments, the PCB comprises a bulk material (e.g., one or more of epoxy resin, fiberglass, phenol formaldehyde resin, paper or polyester resin, which may be provided as the substrate). In some embodiments, the PCB comprises a flexible material (e.g., one or more of polyester, polyetherimide or polyimide). In some embodiments, the PCB is arranged as a layered stack so that the layer of flexible material is below the bulk material layer or the conductor, the bulk material layer is under or over the conductor, the conductor or the bulk material is under the solder mask, and the solder mask is under the silk screen ink. In some embodiments, one or more of the layers is optional and any layer may be omitted. For example, in some embodiments, the layered PCB lacks a silk screen, a solder mask, and/or a flexible material. For example, the PCB could be an FR-4 PCB, which is a PCB comprising an FR-4 laminate (e.g., a composite material composed of woven fiberglass cloth with an epoxy resin binder that is flame resistant). In some embodiments, the PCB or a portion thereof is heat resistant or flame retardant.

When the substrate is a portion of a PCB, it may be rigid or flexible. In some embodiments, the substrate can be configured to have one or more sensors on it (e.g., a PCB may comprise a substrate with one or more sensors). For example, the sensor may be a temperature sensor, a pressure sensor, a flow sensor, a humidity sensor, a fluid level sensor. In some embodiments, the substrate may be configured to contain a heating element or an electrical connector.

### Thermoplastic Materials

The thermoplastic material may have at least one moiety that is capable of reacting with the silicon-containing linking agent. For example, the moiety in the thermoplastic material that is capable of reacting with the silicon-containing linking agent may be selected from the group consisting of amino, carboxyl, epoxy, succinic anhydride, and silane. In some embodiments, the thermoplastic material is hydrophilic. In some embodiments, the thermoplastic material is hydrophobic. Useful thermoplastic materials may include, acrylates copolymers, acrylates terpolymers, maleic anhydride grafted polymers, thermoplastic polyurethanes (TPUs), polyamides, crosslinkable polymers. For example, the thermoplastic material may be selected from one of the following: ethylene/methacrylic acid copolymer, propylene/methacrylic acid copolymer, ethylene/methacrylic acid/acrylate terpolymer, propylene/methacrylic acid/acrylate terpolymer, ethylene/acrylate copolymer, propylene/acrylate copolymer, succinic anhydride grafted polypropylene, succinic anhydride grafted polyethylene, polyurethane, polyamide, ethylene/butyl acrylate/glycidyl methacrylate terpolymer, propylene/butyl acrylate/glycidyl methacrylate terpolymer, ethylene/glycidyl methacrylate copolymer, propylene/glycidyl methacrylate copolymer, crosslinkable polyethylene, crosslinkable polypropylene, and crosslinkable polyolefin. In some embodiments, the thermoplastic comprises polypropylene random copolymer; polypropylene functionalized with maleic anhydride; thermoplastic aromatic polyether-based polyurethane; polyether block amide; ethylene/methacrylic acid (E/MAA) copolymer; terpolymer of ethylene, methacrylic acid, and acrylate; trimethoxysilyl modified polyethylene; ethylene-butyl acrylate-glycidyl methacrylate terpolymer; and/or combinations thereof. In some embodiments, the thermoplastic comprises Ranpelen J-560S (polypropylene random copolymer), Scona TPP 9012 (polypropylene functionalized with maleic anhydride); Desmopan 9370A (thermoplastic aromatic polyether-based polyurethane), Pebax MV1074 (polyether block amide), Surlyn 9020 (ethylene/methacrylic acid (E/MAA) copolymer), Nucrel AE (terpolymer of ethylene, methacrylic acid, and acrylate), PEX (trimethoxysilyl modified polyethylene), Elvaloy PTW (ethylene-butyl acrylate-glycidyl methacrylate terpolymer), and/or combinations thereof.

The thermoplastic material may be an overmold on the substrate, or the substrate may be embedded in the thermoplastic material using insert molding. In some embodiments, the thermoplastic material has a shore A hardness higher than about 30, higher than about 35, higher than about 40, higher than about 50, higher than about 60, higher than about 70, higher than about 80, higher than about 90, or higher than about 95. In some embodiments, the thermoplastic material has a shore A hardness greater than or equal to about: 30, 35, 40, 50, 60, 70, 80, 90, 95, or ranges including and/or spanning the aforementioned values. In some embodiments, the thermoplastic material surface (e.g., an injection mold surface, such as an overmold surface, an insert mold surface, etc.) may comprise micro- or nano-sized structure or features. For example, the surface may have micro-channels or micro-structures. In some embodiments, the micro-channels may extend in only a single direction. In some embodiments, the micro-channels may be distributed radially from a single point, or may be interconnected or in a pattern. The thermoplastic surface may be immersed in the gas flow.

### Silanization of the Substrate Surface

Silane coupling agents can be anchored or attached to the substrate surface through wet chemistry treatment or plasma enhanced chemical vapor deposition (PECVD) treatment. The substrate surface that has -OH functional group or other hydroxyl-containing groups, such as carboxyl, can react with silanol moieties to form a covalent bond.

### Wet chemistry

In some embodiments, wet chemistry treatment may be a dip-, spin-, or spray coating process in which a substrate is coated with a solution containing a silanol, either derived from the silicon-containing linking agent (such as silane coupling agents and silicon alkoxides) or already present in the silicon-containing linking agent (such as siloxanes).

As shown in Figure 34, for silane coupling agents and silicon alkoxides, wet chemistry treatment starts with a sol-gel process, which involves hydrolysis of an alkoxy group in the silane coupling agent or silicon alkoxide. This leads to a reactive intermediate that is hydroxyl-silicon species or silanol (containing Si-OH). Thus, a silane solution consists of a small amount of water to hydrolyze the silane. In some embodiments, an organic solvent is used to adjust the viscosity of the reaction solution. In some embodiments, an acid or basic catalyst can be employed in the reaction. An example of the hydrolysis reaction of a silane coupling agent is shown in Figure 34.

Where the silicon containing linking agent is a siloxane, hydrolysis is not required. The siloxane already has hydroxyl groups suitable for covalent bonding with a substrate. Thus, the hydrolysis of a silane coupling agent or a silicon alkoxide forms a reactive silanol, and a siloxane is a reactive silanol.

The reactive silanol can then react with, for example, a hydroxyl group on the substrate surface through condensation reaction. This process would result in the formation of a covalent bond between the reactive silanol and the substrate, such that the reactive silanol forms a silicon containing linker. The reactive silanol is thus attached or anchored to the surface of the substrate. Since the condensation reaction requires an elimination of water, the coated substrate is generally cured at an elevated temperature to accelerate the water removal process. This process is shown below in Figure 35 for a silane coupling agent.

The reactive silanol species can also react with other silanol species to form oligomers through condensation reaction. The two silicon centers are bridged together and results in the formation of siloxane (Si-O-Si bond). When at least one of the R₁ and R₃ contains an alkoxyl group, the alkoxyl group can also be hydrolyzed to -OH, and the -OH moiety in the siloxane can also react with the hydroxyl group on the substrate surface and results in siloxane anchored to the substrate surface.

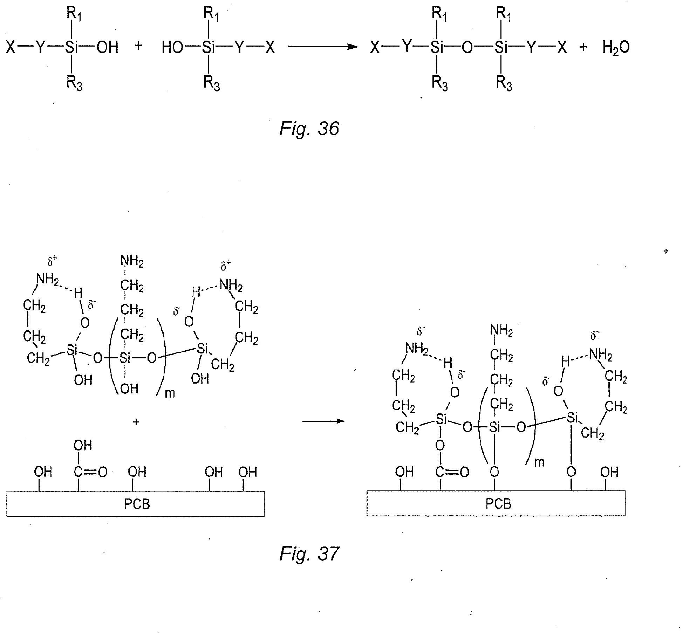

Once the reactive silanol species have formed, a silanol species on one silane coupling agent can also react with a silanol species on another silane coupling agent to form oligomers through condensation reaction. Such reaction is shown in Figure 36 for a silane coupling agent.

As for the siloxane, it can be anchored to the substrate, such as an oxygen plasma treated PCB, via wet chemistry as seen in Figure 37, for example.

### Plasma Enhanced Chemical Vapour Deposition (PECVD)

The silane coupling agent or silicon alkoxide can also be attached to the substrate surface by PECVD. Without being bound to the theory, it is proposed that a silane coupling agent or silicon alkoxide goes through fragmentation caused by collisions with radicals, ions, and free electrons in plasma. The derived silane radicals are then adsorbed onto the substrate surface and form chemical bonds at the reactive sites. The silane radicals may also condense with another silane radicals, polymerize, and form a siloxane network.

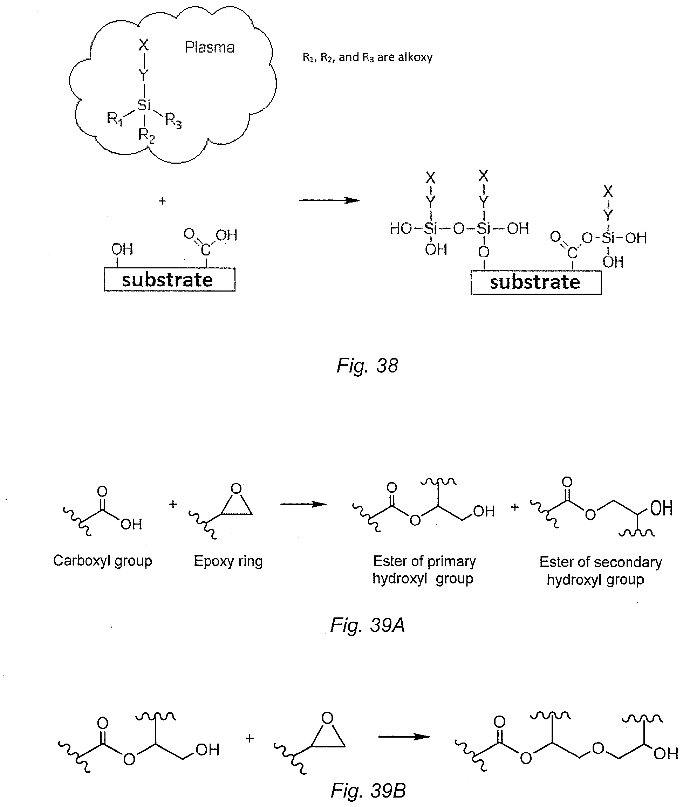

When the silane coupling agent is anchored to the substrate surface through a plasma treatment (*see* Figure 38), the hydrolysis reaction of the silane is not required as the silane coupling agent is activated via the plasma treatment. The activated silane can react with -OH group and carboxyl group (-COOH) on the substrate surface to form covalent bonds. Similarly, siloxane can form during the reaction, and can also attach to the substrate surface as discussed herein. Both low pressure/vacuum plasma treatment and atmospheric plasma treatment may be utilized.

### Bonding Thermoplastic Material to Silanized Substrate

As shown in Figure 38, the silanized substrate has a plurality of pendant functional groups, X, on its surface. The thermoplastic material with a suitable functional group, W, can then be molded onto the silanized substrate. The reaction between X and W would result in covalent bonds that increase the adhesion between the substrate and the thermoplastic material.

In some embodiments, the silicon-containing linking agent may be a silane coupling agent or a siloxane. The silane coupling agent or siloxane will have a functional group X.

In some embodiments, the reactions between the X group in a silane coupling agent or siloxane and the W group in a thermoplastic material are based on epoxy ring opening reactions. This can be achieved by reacting epoxy with a carboxyl or an amine.

As shown in Figure 39A, an epoxy ring which is opened and crosslinked by a carboxyl group produces two different reaction products - an ester of the primary group and an ester of the secondary hydroxyl group.

The ester of primary hydroxyl group can go through an esterification reaction with an epoxy ring, leading to ether formation as shown in Figure 39B.

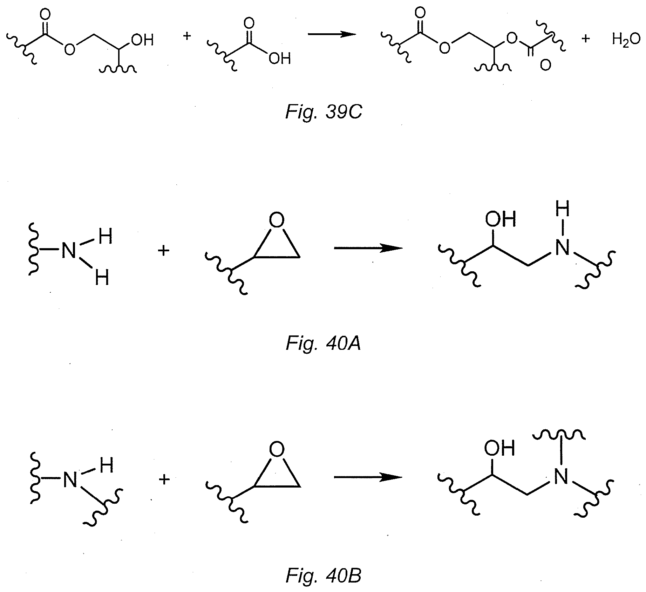

The ester of secondary hydroxyl group can also go through esterification reaction with a carboxyl group, producing a complete ester and water as a byproduct as shown in Figure 39C.

As shown in Figure 40A and Figure 40B, an epoxy ring can be opened and crosslinked by a primary or a secondary amine.

In some embodiments, the reactions between the X group in a silane coupling agent or siloxane and the W group in a thermoplastic material are based on succinic anhydride ring opening reaction. This can be achieved by reacting succinic anhydride with a primary amine.

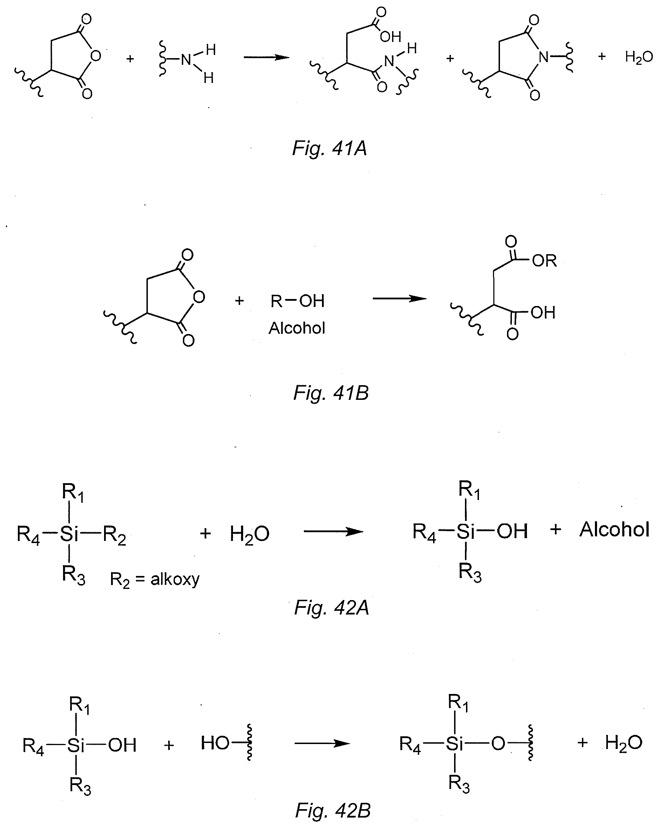

As shown in Figure 41A, a succinic anhydride can be opened and crosslinked by a primary amine forming amide and imide bonds, respectively.

Succinic anhydride ring can react with a hydroxyl group forming an ester bond as shown in Figure 41B.

In some embodiments, the silicon-containing linking agent may be a silicon alkoxide. In this embodiment the silicon alkoxide may be subjected to a sol-gel process. During the sol-gel process the silicon alkoxide may be hydrolyzed to form a reactive silanol, as shown in Figure 42A.

In some embodiments, the reaction between the silanol group derived from a silicon alkoxide and the hydroxyl group in a thermoplastic material is based on a condensation reaction.

As shown in Figure 42B, the silanol group can be reacted with, and thereby crosslinked by, a hydroxyl group in the thermoplastic material.

Surprisingly, the thermoplastic material is able to bond to the substrate and provide desired protective properties. Protective coatings with covalent bonding to a substrate exist, however these protective coatings are paints and resins that contain reactive groups that react with chemical groups on the substrate. The paints and resins benefit from low viscosity, good wetting characteristics and relatively long cure cycles. As the paint dries and the resin cures, the probability of reactive sites on the substrate and in the paint contacting each other is relatively high, and thus covalent bonds can form easily.

By contrast, thermoplastics are high viscosity materials with rapidly increasing viscosity as the thermoplastic material cools after contacting the substrate. Therefore, one may not expect a chemical bond between a reactive group in the thermoplastic and a chemical group on the substrate to form, and good covalent bonding is particularly unexpected. The ability for the molded thermoplastic materials and the substrates disclosed herein to form covalent bonds and/or layers with improved adhesion is unexpected.

### Thermosetting Materials

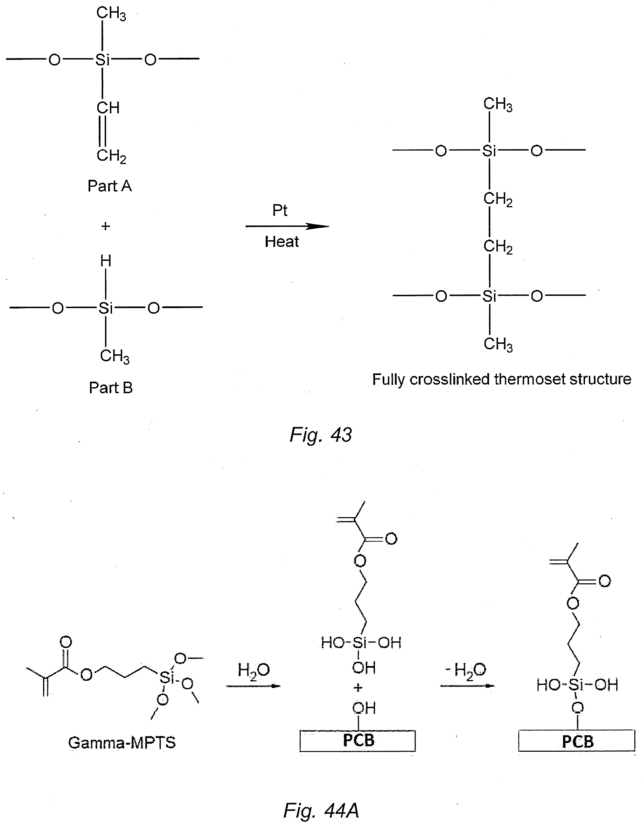

In some embodiments, as disclosed elsewhere herein, a thermosetting material is provided as the molding material. In some embodiments, the thermosetting material is an epoxy, a polyurethane, or a silicone rubber. In some embodiments, the thermosetting material is a liquid silicone rubber (LSR) system which is a two-part platinum-catalyzed silicone elastomer. The Part A component is polydimethyl methylvinyl siloxane, which contains vinyl functional groups and the platinum catalyst. The Part B component is polydimethyl methylhydrogen siloxane, which contains the hydride crosslinker and an inhibitor. In the curing process of LSR, the catalyst promotes addition reaction of the - CH=CH₂ group in the Part A and the ≡SiH in the Part B. The addition reaction of Part A and Part B is shown in Figure 43.

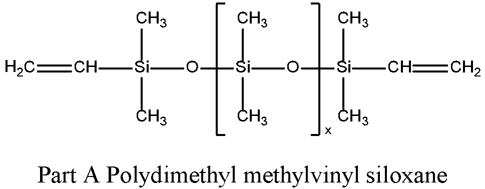

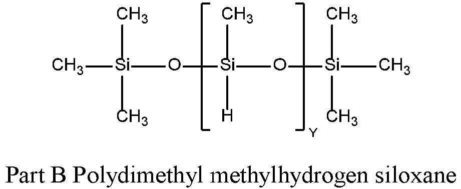

In embodiments where LSR is used as a molding material (or other thermosetting materials as disclosed elsewhere herein), a vinyl functionalized silane such as gamma-methacryloxypropyltrimethoxysilane ("Gamma-MPTS" which is also known as "methacryloxypropyltrimethoxysilane" or "MPTMS"), vinyltrimethoxysilane (VTMS), (3-acryloxypropyl)trimethoxysilane, 1,1-bis(trimethoxysilylmethyl)ethylene, 1,2-bis(methyldiethoxysilyl)ethylene, or bis(3-trimethoxysilylpropyl)fumarate may be used as a silane coupling agent to provide a covalent bond between the PCB and the thermosetting material (e.g., silicone rubber).

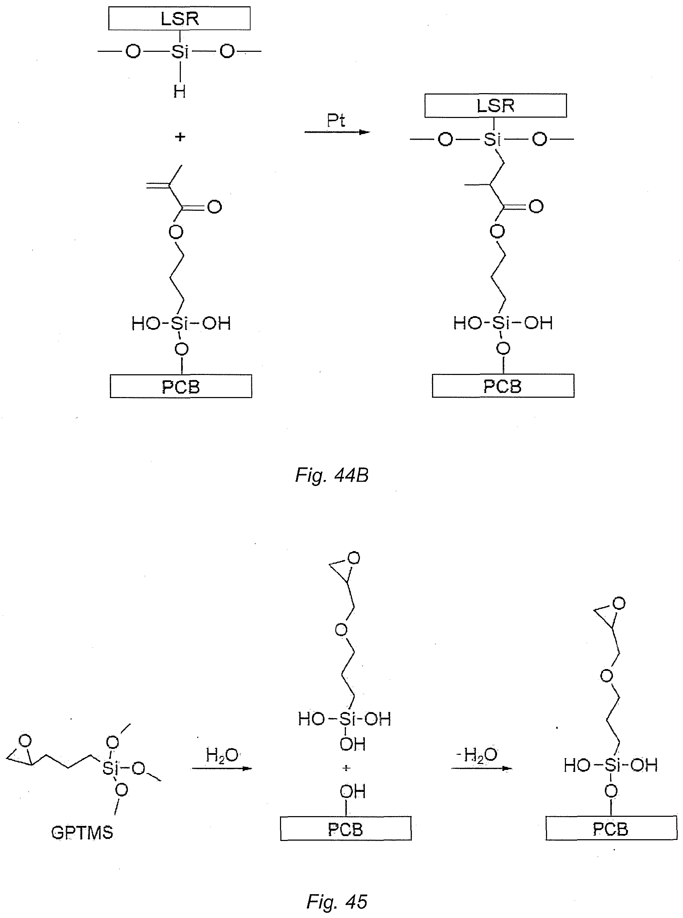

Anchoring vinyl functionalized silane onto the PCB can be done in the same way as shown previously. The vinyl group (-CH=CH₂) in Gamma-MPTS and VTMS can react with platinum catalyst in the Part A and form a crosslink with ≡Si-H in the Part B in the same way as the vinyl group in the Part A of LSR. The chemical reaction of Gamma-MTPS with LSR is shown in Figure 44A and 44B. A vinyl-functionalized silane coupling agent, such as Gamma-MPTS, is first attached to a substrate, then a silicone resin is reacted with the attached vinyl-functionalized silane coupling agent. The silicone resin is then cured to form the silicon rubber.

### Molded Components/Members

Any component that comprises a molding material, such as a thermoplastic material or a thermosetting material, with a substrate embedded in or coupled/bonded to it may benefit from increased adhesion at the interface of these two different materials. The increased adhesion is especially beneficial when the interface between the molding material and the substrates is subject to harsh environments, such as chemical or salt exposure, elevated temperature, and/or humidity. For brevity, when referring to the substrate that is coupled/bonded to the molding material, the bond between the substrate and molding material may be referred to simply as a bonding layer. Wherever a bonding layer is mentioned, that bonding layer may comprise a silicon-containing linker, as disclosed elsewhere herein.

The molded member may be a housing for a filter, a gas conduit, a chamber, an inspiratory tube, a tube connector, a patient interface component (e.g., a respiratory gas interface and/or a surgical gas interface), a tube joint, a tube elbow, a heating element, a water dosing component, or any component for respiratory circuit or device. The molded member may also be a portion of or the entire housing for a filter, a gas conduit, a chamber, a tube connector, a patient interface component, a tube joint, a tube elbow, a heating element, a water dosing component, or any component for insufflation circuit or device. For a more detailed understanding of the disclosure, specifically where the increased adhesion may be applied, reference is made to various components that may be used in a respiratory device, an insufflation device, or other devices providing air and/or gas flow to a patient. The humidifiers and/or humidification chambers described herein may be used, for instance, in any suitable medical/surgical procedure and in any suitable medical humidification system comprising a gas delivery circuit, such as for example invasive ventilation, non-invasive ventilation, high flow delivery, positive pressure delivery (e.g. CPAP), and the like.

Figs. 2A and 2B show an example of a molded member, in this case, a molded elbow 100 that is a component of a respiratory apparatus. A PCB 101 is inserted into and through the wall of the molded elbow 100 by injection molding the elbow around the PCB 101. A portion of the PCB 101 is exposed to the interior of the molded elbow 100 (i.e., not covered by molding material 103), therefore exposed to the gas flow through the molded elbow 100. The PCB 101 can be configured to include one or more sensors, such as a humidity sensor, a temperature sensor, or a flow sensor. The sensor may be a protrusion on the exposed portion of the PCB. In some embodiments, the PCB can also be configured to include an electrical connection to the respiratory apparatus or a component. As shown, the interface 102 may comprise a bonding layer. In some embodiments, this bonding layer comprises a silicon-containing linker. In some embodiments, as in the molded member here, covalent bonds are formed at the interface 102 between the PCB 101 and a molding material 103 of the molded elbow 100.

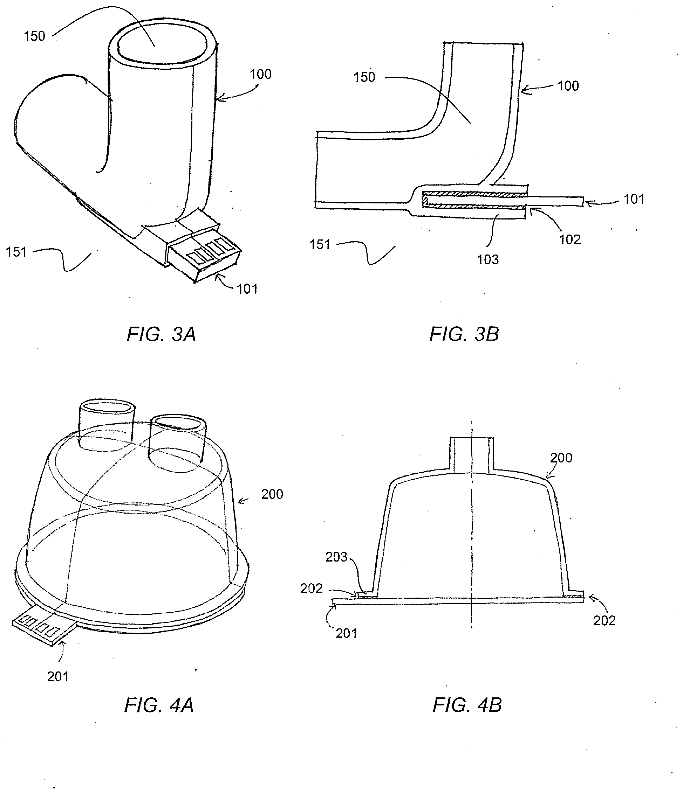

With reference of Figs. 3A and 3B, a portion of the PCB 101 is embedded or encapsulated in a recess in the wall of a molded elbow 100 by injection molding the elbow around the PCB 101. The PCB 101 can be configured to include one or more sensors, such as a humidity sensor, a temperature sensor, or a flow sensor. The PCB 101 can also be configured to include an electrical connection to the respiratory apparatus or a component. As shown, the interface 102 may comprise a bonding layer. In some embodiments, this bonding layer comprises a silicon-containing linker. In some embodiments, as in the molded member here, covalent bonds are formed at the interface 102 between the PCB 101 and the molding material 103 of the molded elbow 100. As shown in Figs. 3A and 3B, in some embodiments, as illustrated with the molded elbow 100, a molded member may comprise an internal area 150 and an external area 151. In some embodiments, though shown as a disconnected piece in Figs. 3A and 3B, as described elsewhere herein, the molded member may be part of a humidifying gas flow system (e.g., gas conduit of a gas flow passage) such that the internal area is substantially isolated from the external area. In some embodiments, the internal area experiences and/or includes a gaseous environment that is different from the gaseous environment of the external area (e.g., the internal area and external area are exposed to one or more of different gases, different ratios of gases, different humidities, different temperatures, etc.). In some embodiments, the molded member is part of a substantially closed system. In some embodiments, features within the internal area are exposed to harsher conditions (e.g., higher temperatures, higher humidities, higher pressures, etc.) than the external area. In some embodiments, the system is not closed.

In some embodiments, as illustrated in Figs. 3A and 3B, the molding material may encapsulate at least a portion of a substrate (e.g., of a PCB). In some embodiments, the PCB (or substrate) may be fully encapsulated, for example any electrically conductive portions (e.g. conductive tracks or electrical contacts) are covered or substantially covered by the molding material. In some embodiments, the PCB may be encapsulated so that the PCB (or substrate) is not directly exposed to a gas flow of a respiratory system or a medical insufflation system (e.g., the environment within the internal area 150 of a molded member of a gas flow passage). In some embodiments, the molding material 103 physically insulates the PCB 101 from the gas flow passage (e.g. the internal area 150). In some embodiments, not shown, the molding material encapsulates the PCB such that the PCB physically insulates the PCB (or a substrate) from the external area of the gas flow passage.

In some embodiments, where a substrate (e.g., of a PCB) is encapsulated (e.g., isolated from humidified and/or heated gases of a humidification apparatus) by the molding material (as shown in Figure 3A and 3B), delamination may be delayed and/or prevented for even longer durations than where part of the substrate of a molded member is directly exposed to the humidification conditions. For example, in some embodiments, encapsulation may prevent liquids, vapors, and gases from humidification apparatus from interacting with the interface between the molding material and the substrate (e.g., the bonding layer). In some embodiments, intersections of the molding material and a substrate (e.g., of a PCB) have higher susceptibility to delamination when the substrate (or PCB) is encapsulated. In some embodiments, where a bonding layer is present, the encapsulation prevents or inhibits access to the bonding layer by water or gases, further inhibiting delamination.

In some embodiments, though isolated from direct contact from the internal area 150, the PCB is nonetheless configured to collect and/or distribute information regarding the internal area 150 of the passage (temperature, humidity, flow rate of gas, etc.). In some embodiments, the PCB is configured to conduct electricity. In some embodiments, as shown, the encapsulated PCB 101 has a portion that is not covered by the molding material (e.g., the portion of the PCB that is external to the gas flow passage) so that contact may be made with the PCB (e.g., with electrical wires/conduits, etc.). In some embodiments, the molding material provides substantially complete or complete physical separation of the PCB from the internal area of the gas flow passage (e.g., so the passage and the PCB are not in gaseous communication). In some embodiments, at least one electrical contact of the PCB is not encapsulated and/or covered by the molding material.

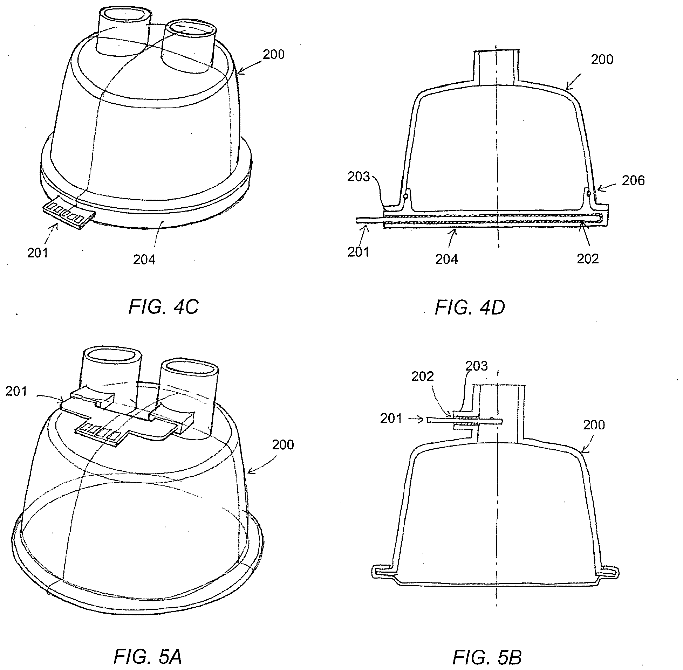

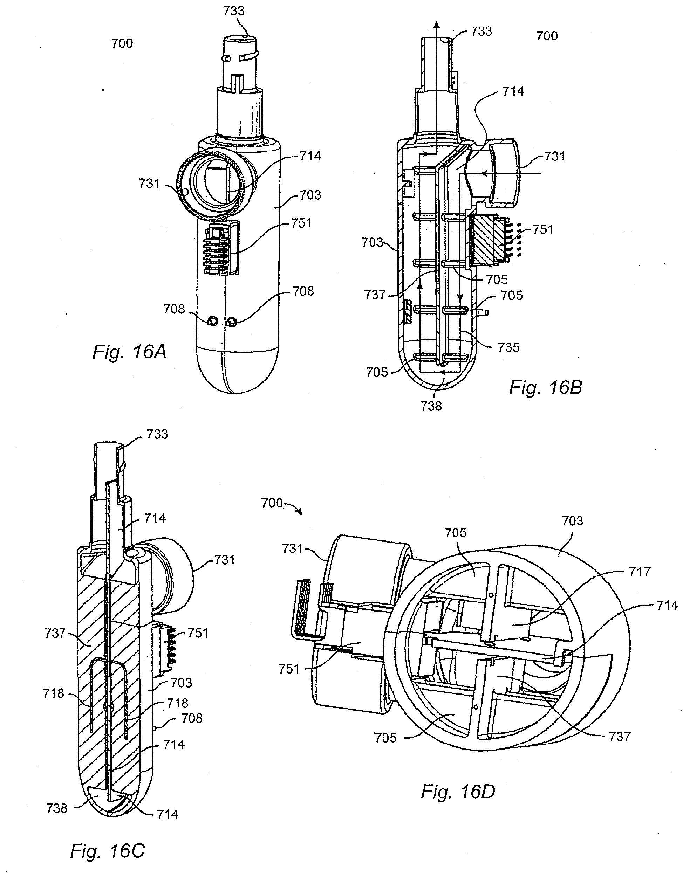

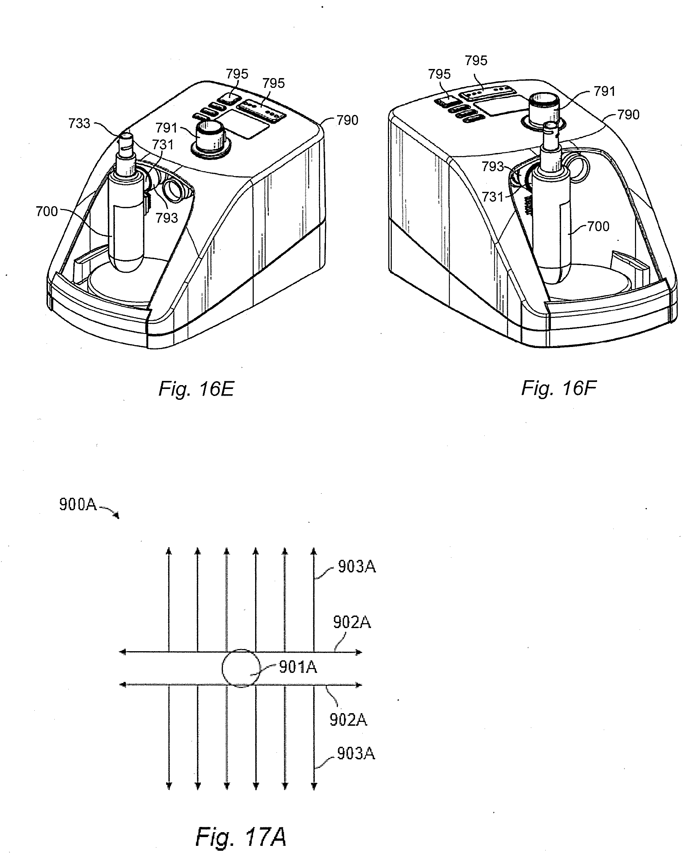

Other embodiments (for example, as shown in, Figures 4C, 4D, 8A, 8B, 9A, and 9B), may comprise a molded member with a molding material that encapsulates a PCB (or substrate) as disclosed above. In some embodiments, the PCB (or substrate) may be fully encapsulated, for example any electrically conductive portions (e.g. conductive tracks or electrical contacts) are covered or substantially covered by the molding material. For example, the electrical contacts may include wires that protrude from the molding material, but the contact themselves are covered by molding material. In some embodiments, as disclosed elsewhere herein, the PCB may be encapsulated so that the PCB (or substrate) is not directly exposed to a gas flow of a respiratory system or a medical insufflation system (e.g., the environment of a gas flow passage). In some embodiments, the molding material physically insulates a PCB from the gas flow passage. In some embodiments, the molding material forms a barrier between the PCB and the gas flow passage. In some embodiments, the molding material encapsulates the PCB such that the PCB physically insulates the PCB from an external area of the gas flow passage. In some embodiments, the molding material forms a barrier between the PCB from an external area of the gas flow passage. In some embodiments, the integrated humidification system of Figure 16A may comprise a partially or completely encapsulated PCB or substrate, as disclosed elsewhere herein.

The molded elbow 100 described in Figs. 2A, 2B, 3A, and 3B may also be a tube or any part of a gas flow path, with a PCB 101 passing through, embedded in, encapsulated in or attached to its wall.

Figs. 4A and 4B show another example of a molded member, in this case, a molded chamber 200 with a PCB 201 attached to it. Covalent bonds are formed at the interface 202 of the molded chamber 200 and the PCB 201. The PCB 201 may be configured to include a heating element for heating a liquid, or more specifically, water. The PCB 201 may extend across the bottom of the chamber, or part way across the base of the chamber. In some embodiments, the PCB 201 may also include one or more sensors, such as a humidity sensor, a temperature sensor, or a flow sensor. In some embodiments, the PCB 201 is configured to be a heater plate, and may further include one or more sensors, such as a humidity sensor, a temperature sensor, or a flow sensor. The portion of the PCB 201 that is outside of the chamber may provide an electrical connection to the respiratory apparatus or other components.

Figs. 4C and 4D shows another example of a molded member, in this case, a molded chamber 200 with a chamber base molding 204 molded over a PCB 201. The PCB 201 is partially embedded in the chamber base molding 204. As shown, the interface 202 may comprise a bonding layer. In some embodiments, this bonding layer comprises a silicon-containing linker. In some embodiments, as in the molded member here, covalent bonds are formed at the interface 202 between the PCB 201 and the molding material 203 of the chamber base molding 204. The chamber base molding 204 can be configured to couple with the molded chamber 200, optionally with an o-ring or seal 206 between the chamber base 204 and the molded chamber 200. The PCB 201 may be configured to include a heating element for heating a liquid, or more specifically, water. The PCB 201 may extend completely across the chamber base molding 204, or part way across the chamber base molding 204. In some embodiments, the PCB 201 may also include one or more sensors, such as a humidity sensor, a temperature sensor, or a flow sensor. In some embodiments, the PCB 201 is configured to be a heater plate, and may further include one or more sensors, such as a humidity sensor, a temperature sensor, or a flow sensor. The portion of the PCB 201 that is outside of the chamber may provide an electrical connection to the respiratory apparatus or other components. In some embodiments, the PCB 201 may be embedded in the molded chamber 200 by injection molding the chamber around the PCB 201.

Figs. 5A and 5B show an example of another molded member, in this case, a molded chamber 200 with PCB 201 integrated into its inlet and outlet. The PCB 201 is inserted into and through the wall of the inlet and the outlet, and a portion of the PCB 201 is exposed (i.e., not covered by molding material) to the interior of the chamber 200 at the inlet and outlet. The other portion of the PCB 201 may be outside of the chamber and configured to provide an electrical connection to a respiratory apparatus or other components. The PCB 201 may be integrated into the molded chamber 200 by injection molding the chamber around the PCB 201. The PCB 201 can contain one or more sensors, such as a humidity sensor, a temperature sensor, a pressure sensor, a flow sensor, or a liquid level sensor. As shown, the interface 202 may comprise a bonding layer. In some embodiments, this bonding layer comprises a silicon-containing linker. In some embodiments, as in the molded member here, covalent bonds are formed at the interface 202 between the PCB 201 and the molding material 203 of the chamber 200.

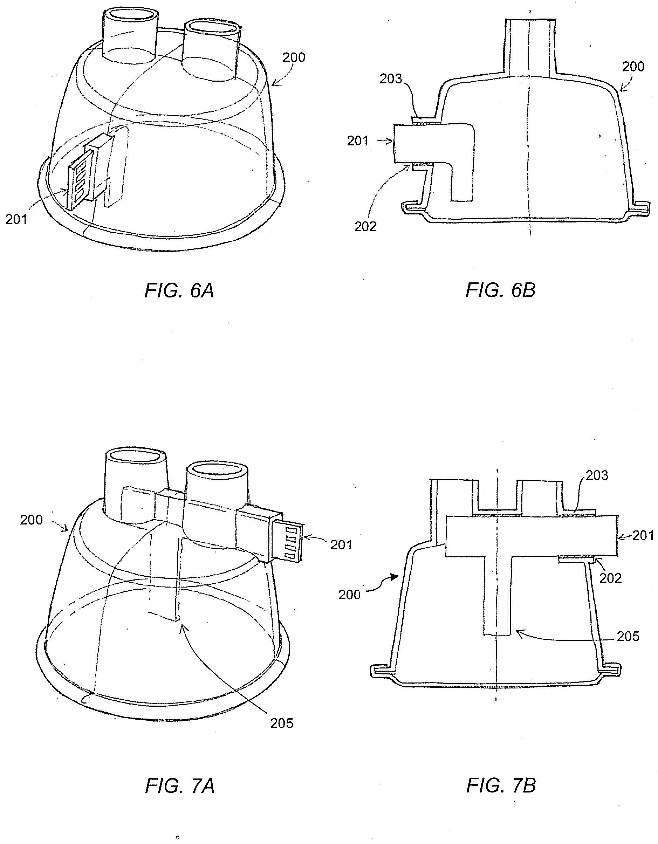

Figs. 6A and 6B show another example of a molded member, in this case, a molded chamber 200 with PCB 201 integrated into the side wall. The PCB 201 may also be integrated into the wall of a molded chamber 200 at other location. The PCB 201 is inserted into and through the wall of the chamber body, and a portion of the PCB 201 is exposed (e.g., not covered by an injection, insert, or overmold material) to the interior of the chamber 200. The other portion of the PCB 201 may be outside of the chamber and configured to provide an electrical connection to a respiratory apparatus or other components. The PCB 201 may be integrated into the molded chamber 200 by injection molding the chamber around the PCB 201. The PCB 201 can be configured to include a humidity sensor, a temperature sensor, a pressure sensor, a flow sensor, or a liquid level sensor. As shown, the interface 202 may comprise a bonding layer. In some embodiments, this bonding layer comprises a silicon-containing linker. In some embodiments, as in the molded member here, covalent bonds are formed at the interface 202 between the PCB 201 and the molding material 203 of the chamber 200.

Figs. 7A and 7B show another example of a molded member, in this case, a molded chamber 200 with PCB 201 integrated into the inlet and the outlet. As shown, in some embodiments, the PCB 201 is inserted into and through the walls of the inlet and the outlet, and a portion of the PCB 201 is exposed (i.e., not covered by molding material) to the interior of the chamber 200. The other portion of the PCB 201 may be outside of the chamber and configured to provide an electrical connection to a respiratory apparatus or other components. The PCB 201 may be integrated into the molded chamber 200 by injection molding the chamber around the PCB 201. The PCB 201 can be configured as a humidity sensor, a temperature sensor, a pressure sensor, a fluid level sensor, or a flow sensor at each of the inlet and the outlet. In some embodiments, the PCB 201 can also be used as a liquid level sensor, for example, having an elongated portion or a protrusion 205 extended to the lower part of the chamber 200. As shown, the interface 202 may comprise bonding layers. In some embodiments, one or more of the bonding layers comprise a silicon-containing linker. In some embodiments, as in the molded member here, covalent bonds are formed at the interface(s) 202 between the PCB 201 and the molding material 203 of the chamber 200.

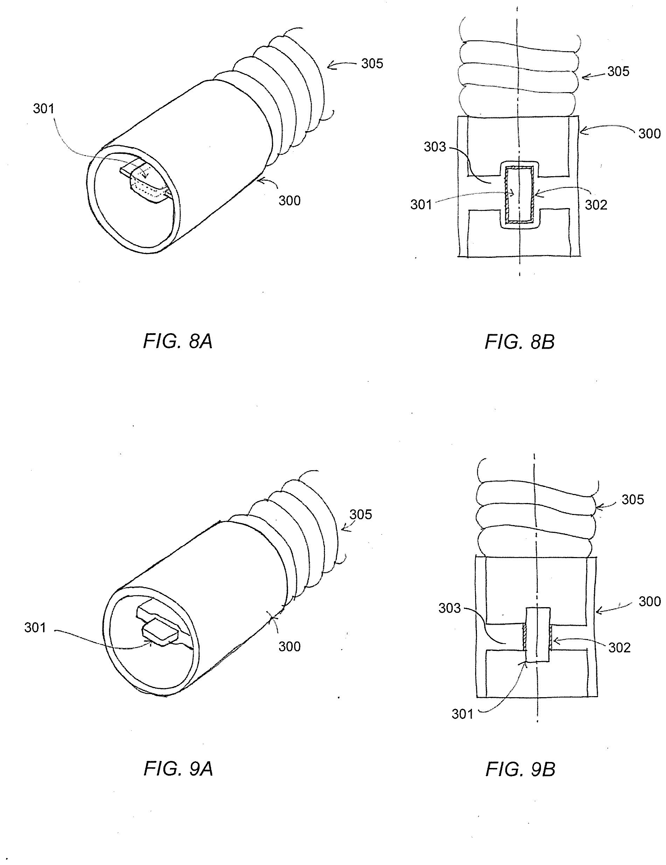

Figs. 8A and 8B show another embodiment of a molded member, in this case, a molded tube connector 300. As shown, in some embodiments, a PCB 301 is encapsulated and positioned within the molded tube connector 300, which can be connected to the respiratory tube 305. In some embodiments, as shown, the PCB 301 is substantially completely covered and protected by molding material 303. As shown, the interface 302 may comprise a bonding layer. In some embodiments, this bonding layer comprises a silicon-containing linker. In some embodiments, as in the molded member here, covalent bonds are formed at the interface 302 between the PCB 301 and the molding material 303 that encapsulates the PCB 301. The PCB 301 may be integrated into the connector 300 by injection molding the connector 300 around the PCB 301. The PCB 301 can also contain a humidity sensor, a temperature sensor, a pressure sensor, or a flow sensor. In some embodiments, the PCB 301 may also contain a tag, such as RFID, EPROM, or a resistor, for allowing identification. In some embodiments, the PCB 301 can also be positioned within an inspiratory tube, an interface tube, or any tubing or connector that is a part of a respiratory device. In some embodiments, the PCB comprises one or more electrical components, such as, a processor, a controller, an identifier, memory component, a sensor(s), etc. In some embodiments, the PCB can receive, transmit and/or process information. Information could be transmitted/received wirelessly.

Figs. 9A and 9B show another embodiment of a molded tube connector 300. A PCB 301 is partially encapsulated and positioned within the molded tube connector 300, which is connected to the respiratory tube 305. A portion of PCB 301 is attached to and bonded to the molding material 303. As shown, the interface 102 may comprise a bonding layer. In some embodiments, this bonding layer comprises a silicon-containing linker. In some embodiments, as in the molded member here, covalent bonds are formed at the interface 302 between the PCB 301 and the molding material 303. The PCB 301 may be integrated into the connector 300 by injection molding the connector 300 around the PCB 301. A portion of the PCB 301 is directly exposed (i.e., not covered by molding material) to the gas flow. The PCB 301 can include a humidity sensor, a temperature sensor, a pressure sensor, or a flow sensor. In some embodiments, the PCB 301 may also contain a tag, such as RFID, EPROM, or a resistor, for allowing identification. In some embodiments, the PCB 301 can be positioned within an inspiratory tube, an interface tube, or any tubing or connector that is a part of a respiratory device.

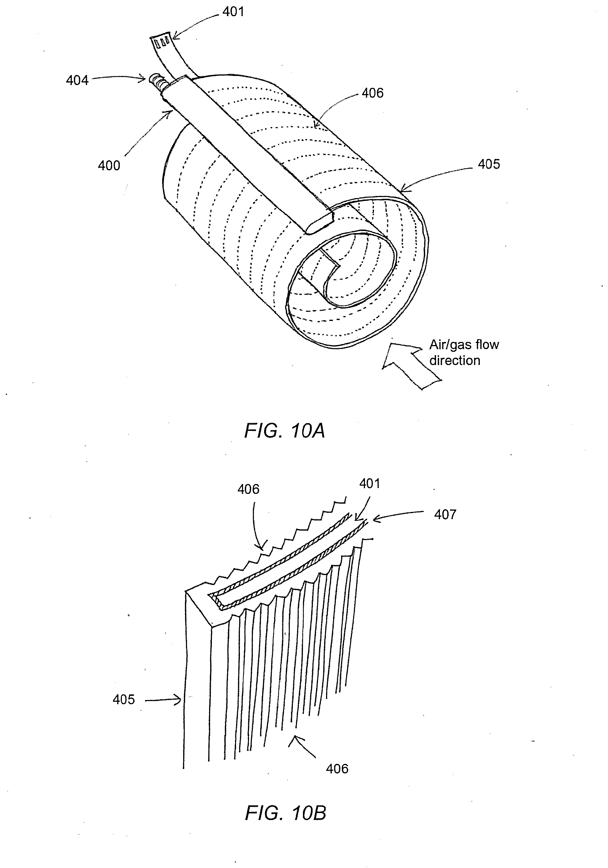

Figure 10A shows an embodiment of a heater 400 for a humidifier, optionally containing a water dosing system. Figure 10B is a cross-sectional view of the portion of the heater 400 comprising a flexible PCB. A molding material 405 is injection molded around a portion of the flexible PCB 401 and adhered to it through a bonding layer (e.g., with silane bonding). In some embodiments, this bonding layer comprises a silicon-containing linker. A portion of the PCB 401 may be configured to include a heating element, and the portion that is not overmolded (or covered by a molding material) serves as a connector to the respiratory apparatus or other components. In some embodiments, as here, covalent bonds are formed at the interface 407 between the PCB 401 and the molding material 405. The flexible heating element can be configured to have a spiral form or shape, which allows air/gas to pass through the spiral while exposed to the heating element. The molding material 405 further includes micro-channels 406 on the surface that is exposed to the air/gas flow. In embodiments with a water dosing system, water may flow onto the heating element via an inlet port 404, and be distributed to the molded flexible heating element along micro-channels 406. The water can then be evaporated from the heated molding surface, and mixed with the air/gas flow. The micro-channels 406 run along the spiral in the embodiments shown in Figure 10A, but can run in any direction or follow other patterns.

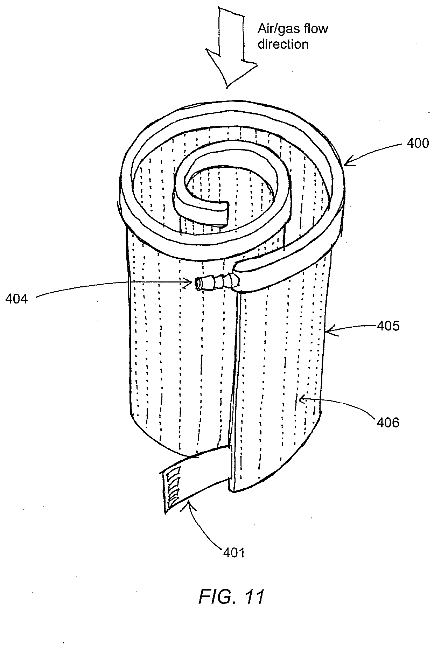

Figure 11 shows another embodiment of a heater 400 for a humidifier, optionally containing a water dosing system 404. Figure 10B also represents a cross-sectional view of the heater shown in Figure 11. A molding material 405 is molded around a portion of the flexible PCB 401 and adhered to it with silane bonding. A portion of the PCB 401 may be configured to contain a heating element, and the portion that is not covered in molding material serves as a connector to the respiratory apparatus or other components. In some embodiments, as here, covalent bonds are formed at the interface 407 between the PCB 401 and the molding material 405. The flexible heating element can be configured to have a spiral form or shape, which allows air/gas to pass through the spiral while exposed to the heating element. The molding material 405 further includes micro-channels 406 on the surface that is exposed to the air/gas flow. In embodiments with a water dosing system, water may flow onto the heating element via an inlet port 404, and distributed to the molded flexible heating element along micro-channels 406. The water can then be evaporated from the heated molding material surface, and mixed with the air/gas flow. The micro-channels 406 run perpendicular to the spiral in the embodiment shown in Figure 11. However, the micro-channels 406 may run in other directions or follow other patterns.

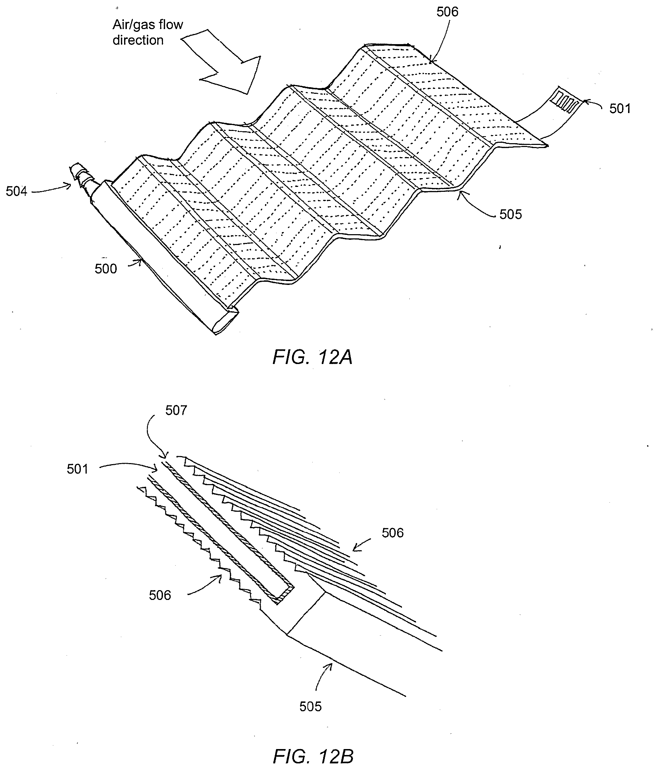

Figure 12A shows one embodiment of a heater 500 for a humidifier, optionally containing a water dosing system 504. Figure 12B is a cross-sectional view of the portion of the heater 500 with PCB 501. A molding material 505 is molded around a portion of the flexible PCB 501 and adhered to it through a bonding layer (e.g., with silane bonding). A portion of the molded PCB 501 may be configured to contain a heating element, and the portion that is not covered in molding material serves as a connector to the respiratory apparatus or other components. In some embodiments, as here, covalent bonds are formed at the interface 507 between the PCB 501 and the molding material 505. The flexible heating element can be formed into a corrugate pattern, and the air/gas can flow past the molding material surface in the same direction of the corrugations while exposed to the heating element. The molding material 505 further includes micro-channels 506 on the surface that is exposed to the air/gas flow. In embodiments with a water dosing system, water may flow onto the heating element via an inlet port 504, and distributed to the molded flexible heating element along micro-channels 506. The water can then be evaporated from the heated molding material surface, and mixed with the air/gas flow. In the embodiment shown in Figure 12A, the micro-channels 506 run in the same direction as the corrugation. In other embodiments, the micro-channels 506 may run in other directions or follow other patterns.

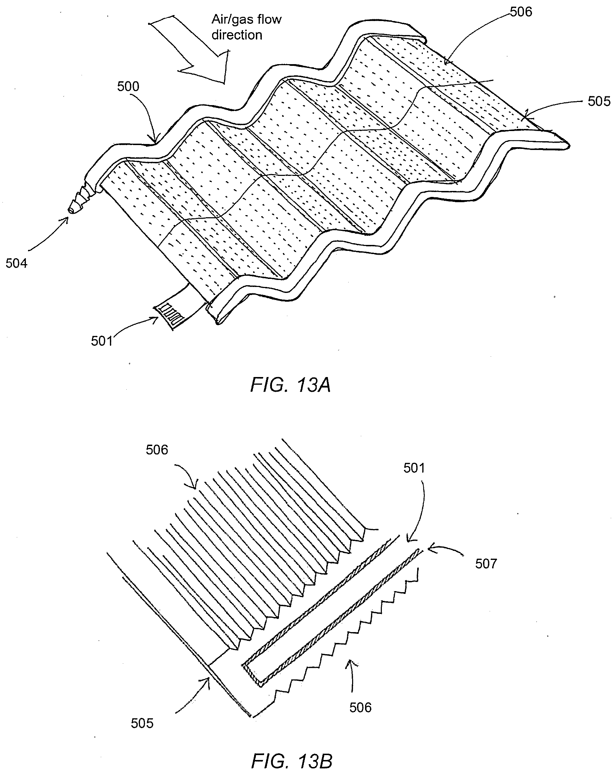

Figure 13A shows yet another embodiment of a heater 500 for humidifier, optionally containing a water dosing system. Figure 13B also represents a cross-sectional view of the heater shown in Figure 13A. A molding material 505 is molded around a portion of the flexible PCB 501 and adhered to it through a bonding layer (e.g., with silane bonding). A portion of the PCB 501 may be configured to contain a heating element, and the portion that is not covered in molding material serves as a connector to the respiratory apparatus or other components. In some embodiments, as here, covalent bonds are formed at the interface between the PCB 501 and the molding material 505. The flexible heating element can be formed into a corrugate pattern, and the air/gas can flow past the molded surface in the same direction of the corrugations while exposed to the heating element. The molding material further includes micro-channels 506 on the surface that is exposed to the air/gas flow. In embodiments with a water dosing system, water may flow onto the heating element via an inlet port 504, and distributed to the molded flexible heating element along micro-channels 506. The water can then be evaporated from the heated molding material surface, and mixed with the air/gas flow. The micro-channels 506 run perpendicular to the corrugation as shown in Figure 13A, but can run in any other direction or follow any pattern.

The molded elbows, tubes, chambers, inlets, outlets, walls, tube connectors, heaters, water dosing elements, and combinations thereof may be included in systems for the humidification of air and gases. These components may comprise a molding material and may be molded members comprising PCBs (or substrates), as disclosed elsewhere herein, bonded together via a bonding layer. Those components, or other molded members as disclosed elsewhere herein, may be a part of a humidification circuit and/or system, which in turn may be a portion or component of a respiratory system, insufflation system, and/or other gas delivery system for providing heated and humidified gases to a patient. In some implementations, an insufflation system may be used to provide heated and/or humidified gases to cavity in a patient (e.g., to the abdomen) before, during, or after surgery. For illustration, in surgery, insufflation gases can be used for a variety of purposes. In open surgery, gas can be insufflated into a body cavity for de-airing, such as for example in cardiac surgery. In laparoscopic surgery, the abdominal wall can be distended using gas to provide room for instrument insertion and tissue dissection. During these surgical procedures, it may be desirable to introduce insufflation gas into a surgical cavity under controlled operating parameters such as a particular flow rate, pressure, temperature, or humidity.

In some embodiments, as disclosed elsewhere herein, the humidification system comprises a heating system comprising one or more molded members as disclosed elsewhere herein. In some embodiments, the heating system comprises a heating surface configured to receive a controlled flow of liquid and provide humidification to gases passing through the humidification system. In some embodiments, as disclosed elsewhere herein, the heating surface comprises a portion of a molded member as disclosed elsewhere herein. In some embodiments, one or more temperature sensors measuring a surface temperature of the heating surface are provided in one or more molded members (as disclosed elsewhere herein) of the system. A temperature sensor (and/or any other sensor disclosed herein, such as flow sensors, humidity sensors, etc.) may comprise a substrate (e.g., a PCB) with one or more sensing regions bonded to a molding material as described elsewhere herein.

In some embodiments, a humidification system as disclosed herein may comprise one or more sensors that may be in electronic communication with one or more of a liquid flow controller providing a controlled flow of liquid; one or more hardware processors providing deterministic control of a humidity level of gases passing through the respiratory system by instructing the liquid flow controller to adjust the controlled flow of liquid received at the heating system and instructing the heating system to adjust the surface temperature of the heating surface, wherein adjusting the surface temperature of the heating surface provides control to produce a known evaporative area; and one or more liquid sensors configured to detect whether the heating surface is wetted in at least one region. In some embodiments, the one or more liquid sensors may be at least two liquid sensors configured to detect whether the heating surface is wetted at two or more regions of the heating surface. The at least two liquid sensors may be two temperature sensors. The one or more liquid sensors may be located at, on, adjacent, or proximal the heating surface and, in some embodiments, may be a portion of the same molded member, as disclosed elsewhere herein. In some embodiments, the liquid may be water.

In some embodiments, the disclosed molded members (as disclosed elsewhere herein), and components or portions thereof, may be configured to receive and/or distribute water onto a heating element that is in fluid communication with a gases channel on an as needed basis as opposed to heating an entire fluid supply at once, or heating an otherwise excess volume of liquid, such as a chamber of liquid. In some embodiments, by measuring an inlet gases flow rate, an inlet gases dew point temperature, and/or a gases channel pressure level, a fluid flow rate of liquid to a heating surface may be determined and controlled to achieve a desired output humidity and temperature level (or outlet dew point temperature) of gases to be delivered to the patient. In some embodiments, where the controller is in electronic communication with one or more sensors, the controller can automatically adjust one or more parameters of the gas, including increasing or decreasing humidity and/or temperature.

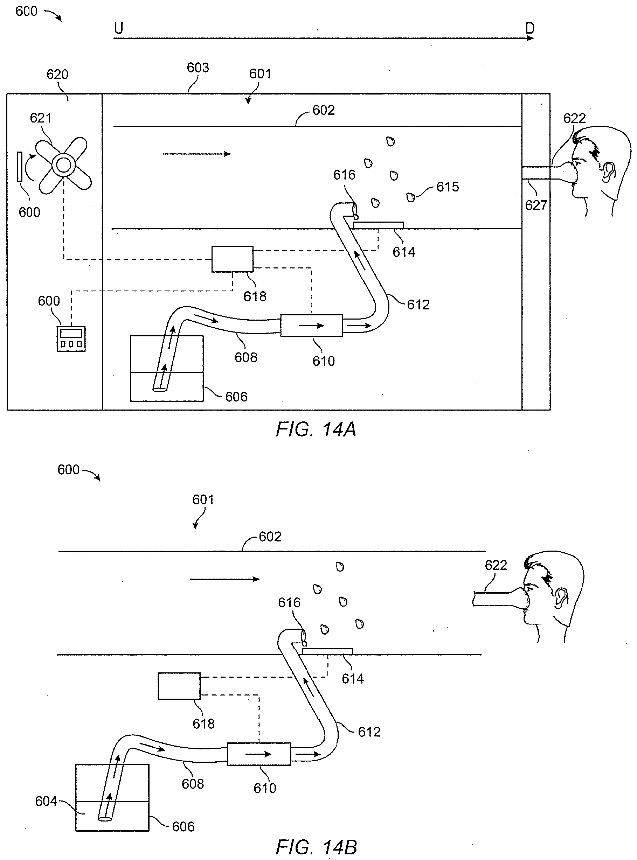

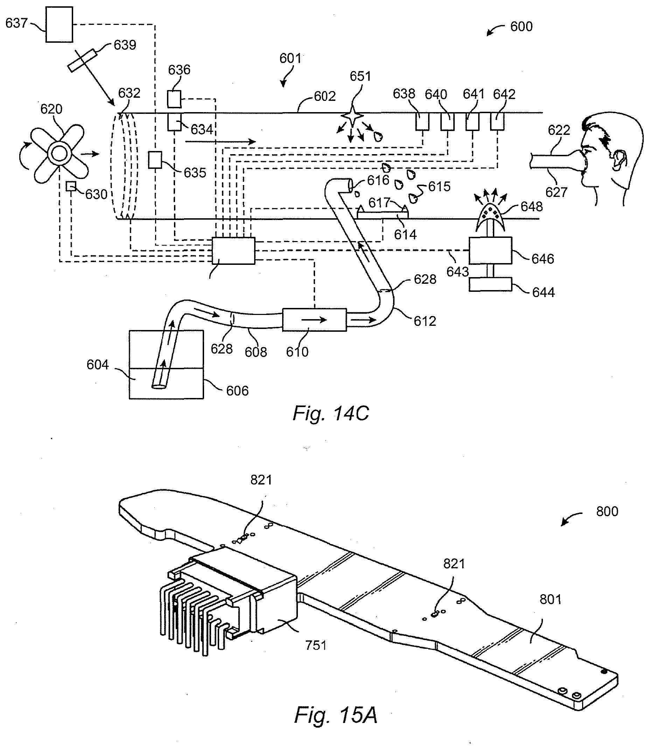

With reference to Figure 14A, a non-limiting exemplary configuration of a respiratory therapy system 600 is shown. In the illustrated configuration, the respiratory therapy system 600 includes a flow generator 620. The flow generator 620 can have, for example, a blower 621 adapted to propel gases through the respiratory therapy system 600. The gases propelled using the blower 621 may, for example, include air received from the environment outside of the respiratory therapy system 600 (for example, 'ambient air' or 'ambient gases') and/or gases (e.g. oxygen, nitrogen, CO₂, etc.) from a gases container in communication with the respiratory therapy system 600 (see for example gases reservoir 637 in Figure 14C). Gases from the flow generator 620 are directed to and/or through a respiratory humidification system 601 adapted to add moisture to the gases. The respiratory humidification system 601 includes a gases channel 602 (which may also be referred to herein as "a breathing tube," or "an inspiratory tube") adapted to receive gases from the flow generator 620 and/or another gases source and channel the gases to an outlet, such as a patient interface 622. As illustrated at the top of Figure 14A (U->D), in use, gases may generally move from the flow generator 620 to the respiratory humidification system 601 (for example, through the gases channel 602), and from the respiratory humidification system 601 to the outlet or patient interface 622 (for example, through the gases channel 602) in a downstream direction. In some embodiments, the patient interface may include a mask, nasal fittings, an esophageal tracheal airway tube, etc. The patient interface may be sealing or non-sealing. As disclosed elsewhere herein, for embodiments providing respiratory gas delivery systems, the patient interface may comprise any suitable interface, including but not limited to a nasal cannula (e.g., comprising a manifold, nasal prongs, etc.), a face mask, a nasal pillows mask, a nasal mask, and/or a tracheostomy interface. In some embodiments, where a portion of a respiratory humidification system comprises a molded member (e.g., a wall, a tube, a chamber, or the like having one or more PCBs) the molding material may be bonded to the substrate of the molded member via a bonding layer.

With further reference to the non-limiting exemplary configuration shown in Figure 14A, the respiratory humidification system 601 includes a fluid reservoir 606 which in use houses a fluid. "Fluid" in this context may refer to liquids or fluent solids suitable for humidifying respiratory gases and may include, for example, water. The fluid may be a water with additives that are more volatile than water. The fluid reservoir 606 is fluidly or otherwise physically linked to a metering arrangement (also referred to as a liquid flow controller or water flow controller herein) 610. The metering arrangement 610 is configured to meter fluid from the fluid reservoir 606 to a humidification housing 615 located in the gases channel 602 or outside of, but in pneumatic communication with, the gases channel 602. The metering arrangement 610 can further include a pump. The pump can be a positive displacement pump, such as, for example, a piezoelectric diaphragm pump, a peristaltic pump, a micro-pump, or a progressive cavity pump. The pump can also be a pressure feed, such as a gravity feed in series with a control valve. The metering arrangement can include a wicking structure that employs capillary action to controllably meter the water to the wicking element and/or to the heating surface.

The metering arrangement 610 can be controlled by a water flow controller. The water flow controller may be a pump in an open-loop configuration. The water flow controller may be a pump or a flow actuator in series with a flow sensor in a closed-loop configuration. The water flow controller may provide a continuous flow of water of equal to or at least about: 0 mL/min, 1 mL/min, 2 mL/min, 5 mL/min, 10 mL/min, or ranges including and/or spanning the aforementioned values (e.g., a range from 2 mL/min to 10 mL/min, a range from 0 mL/min to 5 mL/min, etc.). The water flow controller may provide a flow rate of water at an accuracy of equal to or at least about: ± 1%, ± 2.5%, ± 5%, ± 10%± 15%, or ranges including and/or spanning the aforementioned values.

The water flow controller, including metering system 610, may be configured to ensure that the surface of the heating element 614 is entirely wetted (saturated) (e.g., it may be in electronic communication with the heating element). A fully wetted surface may allow for improved control of the humidity. The wetted surface also means that humidity can be increased more quickly as water travels more quickly over a wet surface than it does over a dry surface.

Any positive displacement pump may be used in the water controller or metering arrangement 610. Positive displacement pumps work by displacing a fixed volume of water and generally yield good accuracy. Any of a variety of positive displacement pumps are suitable, for example, peristaltic, diaphragm, vane, plunger, etc., and a majority of these can be scaled to work at the flow rates contemplated herein. However, piezoelectric micro-pumps (miniature diaphragm pumps using piezoelectric elements as the actuators) and peristaltic pumps (which use rollers to squeeze water through a tube at a constant rate) may be particularly advantageous as many are already commercially available at sizes, prices, operating ranges and powers, etc., that are suitable for the systems described herein. Additionally, a pressure feed, such as a gravity feed, in series with a control valve (see Figure 14D) and/or wicking/capillary action may be used in place of a pump. In some configurations, an electro/magneto-hydrodynamic pump may be used.

When the water flow controller includes a flow sensor, in some configurations, the flow sensor may be a thermal mass meter. The flow sensor may be provided as a PCB comprising a molding material bonded to a substrate (e.g., a PCB) as disclosed elsewhere herein). These sensors work by heating the liquid and measuring either the power required to do so (for example, a heated flow bead) or the temperature gradient introduced, or some variation on this. Alternatively, the flow sensor may be replaced or supplemented with a drip feed (for example, counting drops as is a common method of measuring flow in an IV drip); differential pressure sensors that measure the pressure drop across a restriction to calculate flow; and/or positive displacement sensors that use the same principle as the positive displacement pump to sense flow.

The fluid reservoir 606 may be connected to the metering arrangement 610 via a first fluid conduit 608, as shown. The first conduit 608 can have a non-return valve configured to keep the metering arrangement primed. The first conduit 608 can also have a non-return valve configured to keep the pump primed. The first conduit 608 can also have a safety valve, such as a pressure relief valve, in the conduit leading to the metering arrangement to prevent flow of liquid in case of pump or water controller failure. The respiratory humidification system 601 can also have a flow restriction device positioned between the reservoir 606 and the metering arrangement 610 to prevent gravity driven flow from influencing the water flow path. The flow restriction device can be an elastic protrusion that squeezes or otherwise restricts the flow path. The metering arrangement 610 meters fluid to the humidification housing 615 through a second fluid conduit 612. In some embodiments, the metered fluid can enter the humidification housing 615 through inlets 616 to the humidification housing 615. The first and/or second conduit may comprise or may be a molded member as disclosed elsewhere herein, for example comprising one or more substrates (e.g., having sensors, heating tracks, etc.) bonded to the molding material.

A heating device or heater 614 may be present in (part of a molded member bonded as disclosed elsewhere herein), at, or near the humidification housing 615. The heater 614 may have a wicking element configured to distribute the metered fluid to the heater 614. The wicking element may be part of the molding material (e.g., as disclosed elsewhere herein, bonded to the substrate) formed with a shape coinciding to the mold. In some configurations, the wicking element is configured to wick the metered fluid evenly across the surface of the heating device 614. The heater 614 may be configured to vaporize the metered fluid such that it becomes entrained in the gases flow in use by the respiratory therapy system 600. The heater 614 can be configured to be maintain a heating surface at a temperature range. The temperature of the heating surface through the molding material may be equal to or less than about: 30 °C, 35 °C, 40 °C, 45 °C, 50 °C, 60 °C, 70 °C, 80 °C, 90 °C 99.9 °C, and/or ranges including and/or spanning these temperatures. In some embodiments, molded heating surfaces as disclosed herein can be heated to temperatures over 100 °C for weeks, months, or even years without substantial delamination, unlike conventional heating surface.

The heating surface may include a heating element configured to provide heat to the heating surface. As disclosed elsewhere herein, the heating surface and/or the heating element may be a portion of a PCB that is bonded to a molding material via a bonding layer. The heating element may be a circuit board (e.g., a PCB as disclosed elsewhere herein). The circuit board may be a printed circuit board (for example, as shown and described in reference to Figures 16A-16C below). The circuit board (e.g., PCB) may include resistive elements (e.g., traces or tracking), an etched foil film, a heating coil, or a PTC element, among others. The circuit board may be a flexible circuit board. The flexible circuit board may be made of aluminum-polyimide. The circuit board may have a plurality of resistive tracks. The resistive tracks may be copper. The heating element may be an etched foil (for example, as shown and described in reference to Figures 16D-16E below). The heating element may be a heating wire bonded to a substrate and molded with and coupled to a molding material via a bonding layer as disclosed elsewhere herein. The heating wire may be nichrome. The heating element may be a positive thermal coefficient of resistance (PTC) ceramic. The PTC ceramic may be barium titanate. The heating element may be a thermoelectric device. The thermoelectric device may be a Peltier device.

As disclosed elsewhere herein, the wicking surface may be provided by a molding material on the circuit board, the molding material having micro-channels and the molding material bonded to substrate (e.g., the circuit board) via a bonding layer (e.g., comprising a silicon containing linker). The heating surface temperature may be measured, at least in part, by determining a resistance level or other characteristic of the heating element. The resistance level of the heating element may be used to indicate an average temperature of the heating surface. The heating element may be arranged to deliver a higher power density in a specified region of the heating element as compared to a power density delivered to other regions of the heating element (for example, as explained in reference to Figure 16C). The specified higher density region of the heating element may be located at an outlet of a water supply to the heating surface. The specified higher density region of the heating element may be located at a water pre-heating area on the heating surface.

A component of the respiratory therapy system 600 or of the respiratory humidification system 601 can include a controller 618 that can control the operation of components of the respiratory therapy system 600 or of the respiratory humidification system 601, including but not limited to the flow generator 620, the metering arrangement 610, and/or the heating device 614. The metering arrangement 610 may be configured to meter or allocate fluid to the humidification housing 615 and/or to the heating device 614 at metering rates that raise the moisture content of gases passing through the gases channel 602 such that the gases reach a predetermined, calculated, or estimated humidity level representing a level of gases humidification needed or desired by a patient using the respiratory humidification system 601 while taking care to reduce or eliminate the likelihood of undue moisture accumulation in the gases channel 602. In some embodiments, a controller is provided that may control one or more components in the system. In some embodiments, the controller varies, for example, the metering rate of the metering arrangement 610 based on measured parameters of the system (e.g., a measured flow rate of gases passing through the gases channel 602, a measured humidity of gases upstream of the humidification housing 615 or in a gas flow passage, a measured pressure level corresponding to the pressure level in the gases channel 602, etc.). In some configurations, control of the water flow rate to the heating surface by the controller may be based on a flow rate of the gases in the gases channel. Control of the water flow rate to the heating surface may be based on an evaporation rate of the water from the heating surface. Control of the water flow rate to the heating surface may be based on a temperature of the heating surface wherein the temperature of the heating surface is maintained at a constant temperature. Additional information regarding controllers (including deterministic controllers) can be found in International Application PCT/NZ2015/050128, published as WO2016/036260.

Advantageously, the molded members disclosed herein may increase the lifespan of the system (e.g., a respiratory system and/or insufflation system) by substantially preventing or delaying delamination of one or more molded members of the system. In some aspects, by preventing and/or substantially delaying delamination, the corrosion of sensors, leaking of gas pressure, and/or loss of humidity that results from delamination is prevented or lessened. In some embodiments, delamination is prevented altogether over the lifetime of the device or component thereof. In other instances, the bonding layers disclosed herein (e.g., bonding layers comprising covalent bonds and/or silicon containing linkers) increases the lifetime of the device or component thereof (e.g., through delay of delamination, etc.) by greater than or equal to about: two times, four times, six times, ten times, 20 times, 50 times, 100 times, or ranges including or spanning the aforementioned values.

In some implementations, as shown, the first and second fluid conduits 608, 612 may be configured to communicate fluids to various components of the respiratory humidification system 601. As illustrated in Figure 14A, the first fluid conduit 608 may be configured to fluidly communicate fluid from the fluid reservoir 606 to the metering arrangement 610, and the second fluid conduit 612 may be configured to fluidly communicate fluid from the metering arrangement 610 to the humidification housing 615. In some configurations, the first and/or second fluid conduits 608, 612 are optional. For example, if the fluid reservoir 606 is in direct fluid communication with the metering arrangement 610, the first fluid conduit 608 need not be present. Likewise, if the metering arrangement 610 is in direct fluid communication with the humidification region 615, the second fluid conduit 612 need not be present.

As illustrated in Figure 14C, the first and/or second fluid conduits 608, 612 may additionally comprise one or more filters 628 configured to remove contaminants, impurities, or other undesired materials from the fluid passing from the fluid reservoir 606. In some embodiments, the housing for the filters is or comprises a molded member as disclosed elsewhere herein. In certain implementations, the filters 628 can include any structure configured to do such, including permeable or semipermeable membranes positioned in the fluid flow paths of the first and/or second conduits 608, 612 and/or configured for use in microfiltration, ultrafiltration, or reverse osmosis. The presence of one or more filters 628 in the first and/or second conduits 608, 612 may help to assure a user of the respiratory humidification system 601 that the quality of fluid introduced into the humidification housing 615 is at an acceptable level. If one or more of the filters 628 has been used for too long a period of time, the filters 628 may be replaced. In some embodiments, the first and/or second conduits 608, 612 may also be replaced (e.g., if they become contaminated). However, advantageously, because of the robust nature of the molded members disclosed herein, in some embodiments, purification cycles with disinfecting agents and/or at disinfecting temperatures (e.g., 100 °C or more) may be performed without damaging the structure of the respiratory system or its components. In some implementations, where the filter is replaced, the molded housing for the filter is left intact for continued use. The age of the filters 628 may be indicated to a user through, for example, a chemical color change indicator located in or on the first and/or second conduits 608, 612 or the filters 628 may change in color over time due to prolonged exposure to gases and/or fluids. The filter 218 may serve as a preliminary distributor of the humidification liquid.

As described above, the metering arrangement 610 can serve to meter fluids from the fluid reservoir 606 to the humidification housing 615. The metering arrangement 610 can include, for example, a fluid displacement pump that may actively transfer fluid from the fluid reservoir 606 to the humidification housing 615 along, for example, the first and/or second conduits 608, 612. In certain embodiments, the metering arrangement 610 may run in reverse or act to withdraw fluid from the humidification housing 615. The fluid displacement pump can include, for example, a positive displacement pump, such as a piezoelectric diaphragm pump, a peristaltic pump, a micro-pump, or a progressive cavity pump.

As shown in Figure 14B, the system can be embodied as an in-line humidifier. In this embodiment, the humidification system 601 can be an add on to a respiratory circuit for use with any flow generation system or it can be a stand-alone humidifier using ambient air and relying on normal patient respiration to generate a flow of gases.

In some configurations, not shown, the heating device 614 may be positioned outside of the gas channel 602. For example, the heating device 614 may be present in a separate compartment. In some embodiments, the compartment is or comprises a molded member as disclosed elsewhere herein. The compartment may be physically linked to the gas channel 602 but may be fluidly isolated from the gas channel 602. The compartment 624 may be fluidly isolated from the gas channel 602 through the use of a semipermeable membrane positioned between the compartment and the gas channel 602. In some implementations, the membrane is connected to the system via a housing. In some configurations, the semipermeable membrane may not allow fluid to pass through but may allow vaporized fluids to pass through (and thereby allow vaporized fluids to join gases passing through the gas channel 602). Examples of suitable materials for use with the semipermeable membrane include perfluorinated polymers or polymers with fine pores and include materials such as those used in the tubing described in commonly-owned U.S. Patent 6,769,431, filed May 8, 2001 and titled "Expiratory Limit for a Breathing Circuit," and U.S. Patent Application No. 13/517,925, filed Dec. 22, 2010 and titled "Components for Medical Circuits". In use, fluid may be metered through the outlet 616 to the compartment, vaporized using the heating device 614 (which may additionally be positioned in the compartment), and forced through the semipermeable membrane to join the downstream-moving gases passing through the gas channel 602. Fluidly isolating the outlet 616 from the gas channel 602 may, for example, reduce the likelihood of liquid water being present in the gas channel 602.

It should be understood that the metering arrangement 610 need not necessarily include a pump and may simply include a structure configured to allocate fluid to the humidification housing 615 in predetermined, desired, or regulated amounts. For example, in some embodiments, not shown, the fluid reservoir 606 may be suspended vertically above the gases channel 602 and/or humidification housing 615. The fluid reservoir 606 may be in communication with an electromechanical valve that may, in response to signals generated by the controller 618, partially or fully open or close to control the passage of fluid from the fluid reservoir 606 to the humidification housing 615 through the second fluid conduit 612. In some implementations, the fluid reservoir may comprise one or more sensors (e.g., on one or more PCBs) molded in the reservoir. In certain implementations, the fluid reservoir is or comprises a molded member with one or more sensors in communication with the contents of the reservoir via a bonding layer as disclosed elsewhere herein.

In some configurations, the second fluid conduit 612 may not be present and the fluid reservoir 606 may cooperate with the electromechanical valve to transfer fluids directly to the humidification region 615 (and/or to a location at or near the heating device 614). Fluid flow sensors such as, but not limited to, Micro-Electrical-Mechanical Systems or MEMS sensors, may be used to determine the fluid flow through the electromechanical valve or second fluid conduit 612. In some embodiments, these sensors are PCB sensors, as disclosed elsewhere herein, which may be molded into the system via a bonding layer as disclosed elsewhere herein. Signals from the fluid flow sensor or values derived therefrom may be used to, for example, control the operation of the electromechanical valve via closed loop control. In some implementations, the fluid reservoir 606 may be vertically above the gases channel 602, while in some configurations, the fluid reservoir 606 may be at the same level as the gases channel 602 or lower than the gases channel 602. Other forces may act upon the fluid reservoir 606 to meter fluid in combination with the electromechanical valve. For example, the respiratory humidification system 601 may be configured such that fluids are propelled from the reservoir 606 using the force of gases passing through the respiratory therapy system 600 and/or the respiratory humidification system 601. In some configurations, the gases may act on the fluid in the fluid reservoir 606 directly. In some configurations, the fluid reservoir 606 may be pressurized by fluid filled pouches (filled by, for example, gases from the flow generator 620 or from a separate gas sources) that force fluid from the fluid reservoir 606. The pressure exerted by the pouches may be controlled using a biasing force generated by, for example, a spring or other mechanical arrangement.

In some embodiments, the heating device 614 may be configured to transfer heat to fluids that are metered on to or near the heating device 614 to encourage fluid vaporization and entrainment into the gases flow passing through the gases channel 602. As disclosed elsewhere herein, the particular form of the heating device 614 is not limited and many varieties of heating devices may be envisioned for use with the respiratory humidification system 601 (or an insufflation system, etc.). In some configurations, the heating device 614 may include a molded member (as disclosed elsewhere herein) comprising a heating surface or element that may resistively heat upon the application of electrical energy. The resistive heating plate may be constructed from an electrically conductive metallic material but may also be made from conductive plastics.

The controller 618 can include a microprocessor or some other architecture configured to direct the operation of controllable components of the systems 600, 601. In certain implementations, these microprocessor or other architecture are in communication (e.g., electronic communication) with a sensor or feature that is a portion of a molded member (as disclosed elsewhere herein e.g., a sensor on a PCB that is connected to a component of the system and coupled to a molding material via a bonding layer). In some configurations, one controller 618 may control the operation of every controllable component of the respiratory therapy system 600 and/or respiratory humidification system 601 (or other humidification system), including but not limited to the metering arrangement 610, the heating device 614, and/or the flow generator 620. The controller 618 may be physically present in, on, or near a component of the respiratory therapy system 600, including but not limited to the flow generator 620, the respiratory humidification system 601, the housing 603, and/or the gas channel 602. In some configurations, the controller 618 may be physically separate from the respiratory therapy system 600. For example, the controller 618 could be located on a remote computer, tablet, mobile phone, smartwatch, or another device, and the controller 618 may remotely direct the operation of the controllable components of the respiratory therapy system 600. In some configurations, multiple controllers may be used to control the controllable components of the respiratory therapy system 600 and/or respiratory humidification system 601. The multiple controllers may each be directed to exclusive control of one or more controllable components of one or both of the systems 600, 601. In some configurations, the control of one or more controllable components of one or both of the systems 600, 601 may be handled by multiple controllers. The multiple controllers may be configured to communicate with one another.

As shown in Figure 14C, a flow sensor may be present. Flow signals representative of the flow rate of gases passing through the gases channel 602 may be generated by a gases flow sensor 634 (see Figure 14C) positioned in the gases channel 602. A signal generated by the gases flow sensor 634 may be processed and converted to a gases flow rate value. The flow sensor may be a portion of a molded member as disclosed elsewhere herein. A moisture signal representative of the relative or absolute humidity of the gases upstream of the humidification housing 615, or of ambient gases outside of the respiratory therapy system 600 may be generated by a humidity sensor 636 (for example, as illustrated in Figure 14C) positioned upstream of the humidification housing 615 or outside of the respiratory therapy system 600. A signal generated by the humidity sensor 636 may be processed and converted to a moisture value..

Various sensor modules may also be positioned in the gases channel 602 downstream of the humidification housing 615. As demonstrated in Figure 14C, the sensor modules may include, for example, a flow sensor 638, a humidity sensor 640 (for example, including absolute and/or relative humidity sensors), a temperature sensor 641, and/or a pressure sensor 642. Each of these sensors, individually or collectively, may be bonded via a bonding layer to a molding material providing a component of the system. For example, the molded member (as disclosed elsewhere herein) may be a portion of a wall of the channel 602. The molded member may be provided with one or more sensors. One or more of these sensors may be used by the controller 618 to facilitate control of components of the respiratory therapy system 600 and/or respiratory humidification system 601, including control of the operation of the gases flow generator 620 (including, for example, a motor speed of a blower 621), of the heat output of the heating device 614, of the metering rate of the metering arrangement 610, and/or of some other component.

Also, as demonstrated in Figure 14C, a gas concentration sensor 635 (which may be bonded to a component of the system via a bonding layer) may be positioned in the gases channel 602. The gas concentration sensor 635 may be configured to sense the concentration of one or more gases in the gases stream. The gas concentration sensor 635 can include an ultrasonic sensor adapted to sense, for example, oxygen. The gas sensed may include, for example, oxygen, nitric oxide, carbon dioxide, and/or heliox introduced to the gases channel 602 from a gases reservoir 637 through a gases concentration adjustment valve 639. The gas concentration sensor 635 may use a gas concentration signal generated by the gas concentration sensor 635 to control the gas concentration adjustment valve 639 (for example, via closed-loop control) based on a predetermined desired gas concentration (for example, entered by a user through the user interface 605).

In some configurations, and as demonstrated in Figure 14C, a fluid sensor 617 may be in communication with the humidification housing 615 and/or the heating device 614 as a safety measure to help avoid burning the patient with overheated gases. Illustratively, the fluid sensor 617 may be configured to generate a signal upon detection of the presence of fluid in the humidification housing 615 and/or in or on the heating device 614. The controller 618 may use the signal emitted by the fluid sensor 617 to control the operation of the metering arrangement 610 and/or the operation of the heating device 614. For example, the metering rate of the metering arrangement 610 and/or the heat output of the heating device 614 may be set to a function of the signal generated by the fluid sensor 617. The metering rate of the metering arrangement 610 may be increased if the signal does not indicate the presence of fluids in or near the humidification housing 615, or on a modular area of the heating device, since the heating devices is intended to be covered with a film of humidification fluid. Likewise, the heat output of the heating device 614 may be reduced or set to zero if the signal does not indicate the presence of fluids in or near the humidification housing 615, or on a modular area of the heating device, so as to avoid heating the gases to an unsafe temperature. The fluid sensor 617 may be thus used to aid in the control of the metering arrangement 610 and/or the heating device 614 if it is determined that fluids are not present in the humidification region 615 and/or on the heating surface of heating device 614 when they would otherwise be expected to be present in such locations (for example, if the metering arrangement 610 is attempting to meter fluids at a positive rate). In some configurations, the respiratory therapy system 600 or a component thereof (including the respiratory humidification system 601) may be configured to generate an alarm or convey a message to a user (for example, through the user interface 605) upon such a determination to let the user know that the situation should be corrected (for example, by refilling the fluid reservoir 606).

In some configurations, the fluid sensor 617 may include a capacitive fluid sensor. If a heating surface of heating device 614 is present, the capacitive fluid sensor may, for example, include a pair of conductive sense electrodes positioned on opposing sides of the heating surface. If the conductive sense electrodes are connected in a circuit and a voltage is applied, the capacitance of the circuit will vary depending on the presence or absence of water. The capacitance of the circuit may be measured using, for example, a standard AC measuring circuit. Many other sensing systems, including ultrasonic or optical level sensing systems, may also be used to determine the presence of fluid. The capacitive fluid sensor, or a portion thereof, may be molded into place and coupled to a molding material via a bonding layer.

Various sensor modules may be utilized by the controller 618 to control various components of the respiratory therapy system 600 and/or the respiratory humidification system 601. The sensor modules can include one or more sensors for detecting various characteristics of gases in the gases channel 602 or elsewhere in, around, or near the respiratory therapy system 600 (including in or near the gases inlet 623, the gases outlet 627, the patient interface 622, or at, upstream and/or downstream of the humidification housing 615), including pressure, gases flow rate, temperature, absolute humidity, relative humidity, enthalpy, gas composition, oxygen concentration, carbon dioxide concentration, ambient temperature, and/or ambient humidity. One or more of these sensors and/or sensor modules may be used, for example, to facilitate the control of the flow generator 620 (including control of the pressure and/or flow rate of gases propelled downstream by the flow generator 620), control of the heat output of the heating device 614 (including control of the temperature of the heating device), and/or control of the metering rate of the metering arrangement 610 (including control of power and/or current applied to the metering arrangement 610). These sensors may be molded into a molded member (as disclosed elsewhere herein). In some configurations, respiratory activity of a patient using the respiratory therapy system 600 and/or respiratory humidification system 601 may be determined, estimated or calculated using one or more of the sensors or sensing modules described above or elsewhere in this disclosure. The controller 618 may control various components of the respiratory therapy system 600 and/or the respiratory humidification system 601 such that the components operate based on a determined respiratory activity or respiratory state.

In some configurations, the flow generator 620 may, for example, include a source or container of compressed gas (for example, air, oxygen, etc.). If a container is used, the container may include a valve that can be adjusted to control the flow of gases leaving the container. In some configurations, the flow generator 620 may use such a source of compressed gases and/or another gases source in lieu of the blower 621. In some configurations, the flow generator 620 may use such a source of compressed gases and/or another gases source together with the blower 621. The blower 621 can include a motorized blower or a bellows arrangement or some other structure adapted to generate a gases flow. In some configurations, the flow generator 620 may draw in atmospheric gases through the gases inlet 623. In some configurations, the flow generator 620 may be adapted to both draw in atmospheric gases through the gases inlet 623 and accept other gases (for example, oxygen, nitric oxide, carbon dioxide, etc.) through the same gas inlet 623 or through a different gas inlet (not shown). In some configurations and as demonstrated in Figure 14B, the flow generator 620 may not be present and the respiratory therapy system 600 may be configured such that only unpressurized ambient air is humidified and channeled to the outlet/patient interface 622.

In some configurations and as demonstrated in Figure 14C, the respiratory therapy system 600 and/or respiratory humidification system 601 may comprise an electromagnetic radiation emitter 651 (positioned in, for example, the gas channel 602). The emitter 651 may comprise an ultraviolet light source (e.g., UV LED), a microwave emitter, or some other radiation emitter configured to sterilize the gas flow path. Means for sterilizing the passage through which gases pass through the respiratory therapy system 600 and/or respiratory humidification system 601 can reduce concerns of patient infection through the introduction of undesired pathogens. This emitter may be provided as a molded member (e.g., emitter could be a portion of a PCB that is bonded to a molding material of the system via a bonding layer).

In some configurations and as demonstrated in Figure 14C, the respiratory therapy system 600 and/or the respiratory humidification system 601 can include a gases heating region 632. The gases heating region 632 can pre-heat gases passing through the gases channel 602 before the gases reach the humidification housing 615. If the gases are pre-heated before they are humidified, the efficiency of the humidification may be improved. The gases heating region 632 can include, for example, one or more resistive heating wires or heating elements on PCBs present in, on, around, or near the inner and/or outer walls of the gases channel 602. The gases heating region 632 may be controlled by and in electrical communication with the controller 618, which may control the heat output of the gas heating region 632 using sensor signals in a manner, for example, similar to the control of the heat output of the heating device 614 as described elsewhere in this disclosure. The controller 618 can control the temperature and/or the heat output of the gases heating region 632 such that gases arrive at the gases outlet 627, the patient interface 622, or at the patient at a temperature of between approximately 31 °C to approximately 43 °C. In some cases, if the gases heating region 632 is distal from the gases outlet 627 or the patient interface 622, the gases heating region 632 may heat the gases to a temperature higher than between approximately 37°C to approximately 43°C such that the gases arrive at the gases outlet 627, the patient interface 622, or the patient at the desired temperature (due to temperature loss as the gases pass along, for example, the gases channel 602). In some embodiments, the heating device of the gases heating region 632 or a portion thereof may be molded to a molding material of the system via a bonding layer.

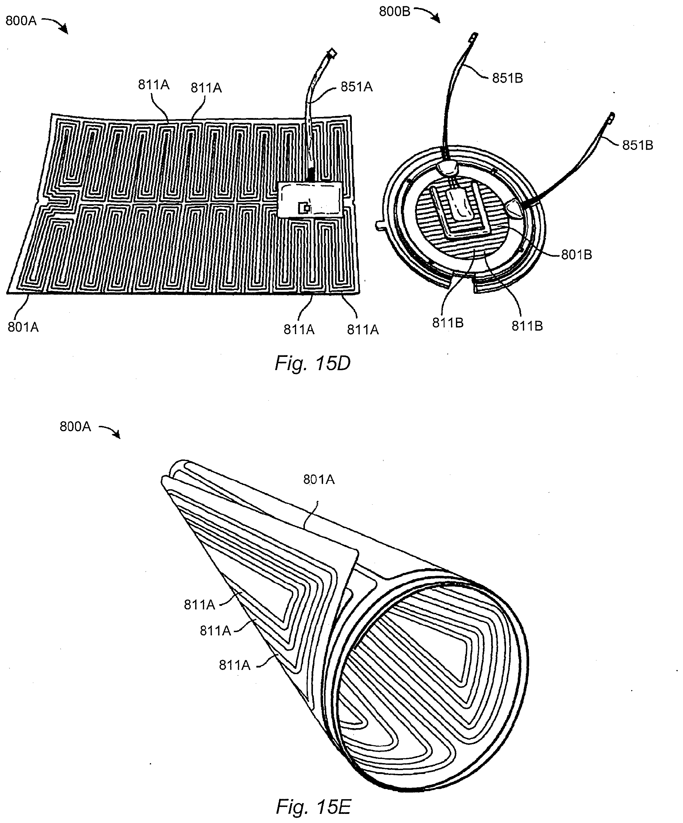

The gases heating region 632 may include a gases pre-heater which may include a gases heating element. The gases heating element may be a printed circuit board (as shown in Figures 15A-C). As disclosed elsewhere herein, the PCB may be a part of a molded member and may be molded with a molding material via a bonding layer. The PCB may have resistive elements. The gases heating element may be an etched foil film (see for example, Figures 15D and 15E). The gases heating element may be a heating coil. The gases heating element may be a PTC ceramic. The respiratory humidification system 600 may have a temperature sensor. The temperature sensor may be positioned in the gases channel downstream of the gases pre-heater. A temperature sensor may be positioned in the gases channel upstream of the gases pre-heater, either instead of or in addition to the temperature sensor positioned downstream of the gases pre-heater. A characterization of the gases heating element may be used to determine a temperature of the gases. Control of a power level delivered to the gases heating element may be based on information provided by a temperature sensor positioned in the gases channel downstream of the gases pre-heater. Control of the power level delivered to the gases heating element may be based on information provided by a gases flow sensor and by a temperature sensor positioned in the gases channel upstream of the gases pre-heater. A desired downstream temperature of the gases may be determined based on an evaporation rate of the water from the heating surface. As disclosed elsewhere herein, the heating surface may include a heating element configured to provide heat to the heating surface. The heating element may include a plurality of resistive tracks. The heating element may be a printed circuit board. The printed circuit board may have resistive elements. The heating element may be an etched foil film (see for example, Figures 15D and 15E). In a similar vein, the system may also comprise pre-heating the water flow. This could be done by either heating the water source, heating the water feed line, or having a special zone on the heater-plate (e.g., the water wicks over the water pre-heater before reaching the evaporative region, or the initial region has a higher power density). Each of these heat sources may be a portion of a molded member of the humidification system (e.g., part of a PCB bonded to a molding material via a bonding layer).

The inlet conditioning and testing can include an inlet sub system at or near the gases supply location including one or more inlet sensors configured to measure inlet gases ambient humidity, inlet gases flow, inlet gases temperature, and a pressure level of the gases channel. An inlet gases heater may also be provided at or near the gases supply location to pre-heat the gases to a desired (predetermined) temperature as they enter and pass through the gases channel such that the gases arrive at the humidification location at a desired temperature. By separately pre-heating the gases, the energy delivered to the heating element in the humidification region can be used to vaporize the humidification fluid, thereby separating the functions of heating the gases (by supplying sensible heat from the gases pre-heater) and humidifying the gases (by providing latent heat from the heating element) in the gases channel. Advantageously, such a separation of functions permits the heating element to be operated at lower power levels corresponding to lower temperature levels which results in safer and more efficient operation of the respiratory humidification system. Moreover, the temperature of the heated gases can be altered quickly, such that the system becomes more responsive to changes than a system which heats an entire fluid reservoir, or a significant volume in excess to that required. A liquid flow controller can include a humidification fluid flow control sub-system that monitors and controls the rate at which fluid is metered to the humidification region and, more specifically, to the heating element. A fluid flow sensor measures the flow of the humidification fluid and provides the measurement to a fluid flow controller. In some embodiments, the controller compares the measured fluid flow rate with the desired fluid flow rate (which may be predefined, estimated, or deterministically derived), and adjusts the power level to the metering arrangement accordingly. In some embodiments, the humidification fluid is pre heated before being delivered to the heating element for vaporization to reduce the amount of latent heat required by the heating element to vaporize the humidification fluid. Various modes of pre-heating the humidification fluid can be used, including heating the fluid reservoir, heating the fluid feed line, or having a special fluid pre-heating zone on the heating element before reaching an evaporation region. In accordance with certain embodiments, a check valve is disposed within the fluid feed line prior to the metering arrangement to prevent back-flow of the humidification fluid. In some embodiments, a safety valve is disposed within the fluid feed line prior to the metering arrangement to release pressure in the line due to pump failure, along with other possible causes. A heater-plate controller can include a heated surface sub-system that monitors and controls the temperature of the heating element. A heating surface includes an area over which humidification fluid is distributed and vaporized by heat energy provided by the heating surface. A wicking element is provided over at least a portion of the heating surface. The wicking element is configured to receive and distribute a layer of the humidification fluid, the layer having a thickness, over the one or more portions of the heating surface that delivers the heat to vaporize the fluid. The wicking element can include paper, fabric, micro fiber, or microstructures, including microfluidic channels (e.g., that are molded and bonded via a bonding layer onto the heating surface). The heating surface can include a heating plate, a resistive heating plate, or a circuit board having resistive tracks, to name a few. In some embodiments, the heating surface is a circuit board that is molded with a molding material, for example a thermoplastic material, as disclosed elsewhere herein. In some embodiments, multiple heating surfaces, or zones, may be used. Each heating surface may be maintained at the same or at different temperature levels. A heating surface temperature sensor is in thermal contact with the heating surface and in communication with a heating surface temperature controller. A surface heater, also in communication with the surface temperature controller, is configured to control the temperature of the heating surface, or multiple heating surfaces or zones, depending on the configuration of the heating surface.

Figure 16A is a perspective view of an example integrated humidification system 700 in accordance with one embodiment of the present disclosure. Figure 16B is a vertical cross-section view showing an air flow of the humidification system 700. Figure 16C is a vertical cross-section view showing a water flow of the humidification system 700. Figure 16D is a horizontal cross-section view of the humidification system 700. In some configurations, the humidification system 700 can be a stand-alone humidifier using ambient air and relying on normal patient respiration to generate a flow of gases. In some configurations, the humidification system 700 can be an add on to a respiratory circuit for use with any flow generation system, for instance with a ventilator. Figures 16E-16F show the humidification system 700 installed for use with a flow generation system in an integrated system.

As shown in Figure 16A, the humidification system 700 includes a housing 703, a gases inlet 731, a gases outlet 733. The gases inlet 731 is configured to receive gases into the humidification system 700. In some configurations, the gases inlet 731 is adapted to connect with a gases inlet tube, flow generation system, or other gases sources. The gases outlet 733 is configured to deliver humidified gases out of the humidification system 700 and to a patient. In some configurations, the gases outlet 733 is adapted to connect to a gases outlet tube, for example, a respiratory tube connected to a patient interface (for example, a cannula). The humidification system 700 also includes, one or more water inlets 708 configured to allow water received from a water flow controller into the humidification system 700. In some embodiments, the humidification system 700 includes an inlet and an outlet for water. In some embodiments, the humidification system 700 only includes inlets, as all water input into the system is evaporated to humidify the gas. The humidification system 700 also includes an electrical connector 751 for supplying power to the system and for communicating with various components thereof. The humidification system may also serve to provide power to the heated respiratory tube and in-built sensors, such that the design acts as a conduit for downstream system components that require power or communications. Figure 16B is a vertical cross-section view showing an air flow of the humidification housing of Figure 16A. As shown in Figure 16B the housing 703 defines a gases flow path 738. In this configuration, the gases enter the humidification system 700 at the gases inlet 731 and are directed downward by an interior wall 737. An opening 738 at the bottom of the interior wall 737 allows the gases to pass to the other side of the interior wall 737 where the gases are directed upwards and out of the humidification system 700 at the gases outlet 733. The housing 703 may include internal baffles 705. Along the flow path 735 the gases are humidified by vaporized water evaporating off of the heating element 714. The heating element 714 can be partially seen through the gases inlet 731 in Figure 16A and a cross-sectional view of the heating element 714 is visible in Figures 16C and 16D. An example configuration of the heating element 714 is described as heating element 800 in reference to Figures 15A-15C. In some embodiments, any portion of the housing, inlets, outlets, etc., of the respiratory humidification system comprise molded members that can include one or more PCBs.

Figure 16C is a vertical cross-section view showing a water flow of the humidification housing of Figure 16A. In the illustrated configuration, water entering at the inlets 708 is distributed through channels 718 so as to contact the heating element 714. In the illustrated configuration, the channels 718 are partially located within the interior wall 737. As shown, a majority or substantially all of the heating element 714 is encapsulated (e.g., enveloped, enclosed-in, etc.) in a molding material, which can be bonded to the substrate of the heating device via a bonding layer. Figure 16D is a horizontal cross-section view of the humidification housing of Figure 16A. As shown in Figure 16D, the heating element 714 may divide the housing 703 in a first direction and the internal wall 737 may divide the housing 703 in a second direction, orthogonal to the first direction. Thus, the heating element 714 is immersed in the flow path. In some configurations this configuration effectively doubles the surface area, provides a dramatic increase in power efficiency, makes the surface temperature reading more accurate, and allows the housing 703 to be kept relatively cool (and therefore, safe). In the embodiment of Figure 16D, baffles 705 are included in the air flow path.

Figures 16E-16F show the humidification system 700 installed for use with an embodiment of flow generation system 790. The flow generation system 790 can include a gases inlet 791 for connecting to an external gases source and a gases outlet 793 that can be adapted to connect to the gases inlet 731 of the humidification system 700. In the illustrated configuration, the flow generation system 790 includes a plurality of input controls 795. In some configurations, the flow generation system 790 can be an Airvo available from Fisher & Paykel Healthcare of Auckland, NZ. In some embodiments, one or more components of the flow generation system (e.g., a flow tube, etc.) comprise sensors molded with and coupled to a molding material via a bonding layer into a molded member (as disclosed elsewhere herein) of the system.

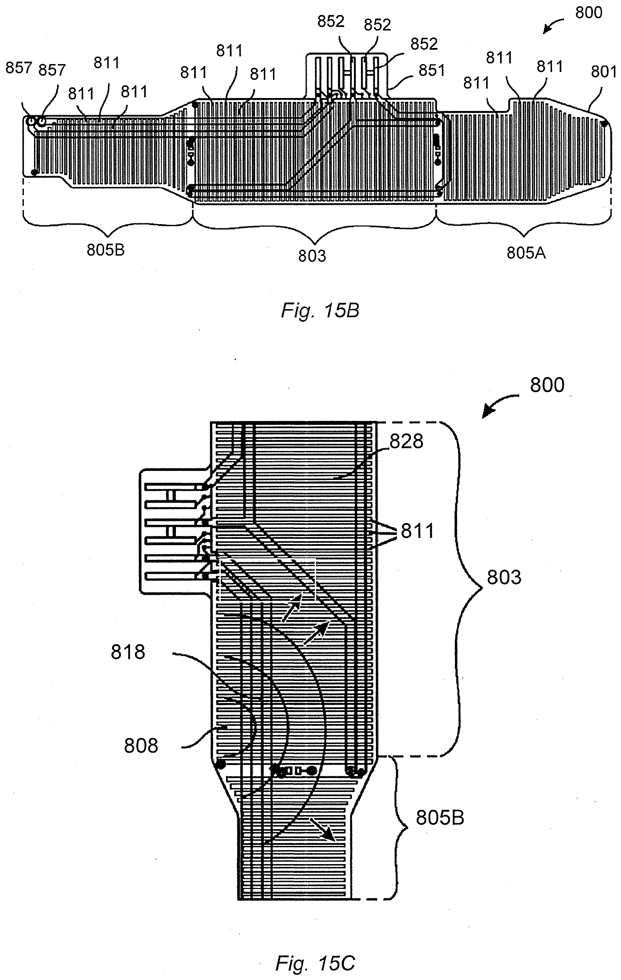

Figure 15A is a perspective view of an embodiment of a heating element 800 in accordance with an embodiment of the present disclosure. Figure 15B is a top view of the heating element 800. Figure 15C is a partial top view of the heating element 800. In some configurations, as disclosed elsewhere herein, the printed circuit board heating element 800 may be used as the heating device 814 of the humidification system 700 described above in reference to Figures 16A-16F, or as any other heating device described herein (for example, the heating device 614 of Figures 14A-14C).

As disclosed elsewhere herein, the heating element 800 may include a printed circuit board 801 for providing heating. The printed circuit board 801 may have a plurality of resistive tracks 811. The resistive tracks 811 may be copper and/or aluminum. An outer surface of the heating element 800 may include a wicking surface. The wicking surface may be provided by a molding material (and bonded via a bonding layer) on the printed circuit board 801. The molding material may have micro-channels in it (the micro-channels are described in greater detail below). The molding material may be a thermoplastic material as disclosed elsewhere herein and/or may be a thermosetting material. The heating element 800 may have modular zones. For example, in the illustrated embodiment, the resistive tracks 811 are divided into three modular zones, 803, 805A, 805B. In some configurations, the modular zones 805A and 805B are connected in series. In some configurations, the heating element 800 may have a first zone configured to pre-heat the water and a second zone configured to vaporize the water as will be described in reference to Figure 15C. A single zone could be wet, and that single zone could be powered. This gives flexibility in the controller. Alternatively, the entire heater surface can be powered, and the entire heater surface can be kept wet rather than operating the isolated zones.

As shown in Figure 15B, the heating element 800 may include electrical contacts 857 (for either power transfer or communication) that can be used to power additional components of a respiratory humidification system. While the remainder of the heating element may be covered with a molding material (e.g.,encapsulated by it), these electrical components may span a wall or surface of the molding material and may be connected to an electrical conduit, etc., outside a humidification chamber of the humidification system, for example. In some embodiments, electrical contacts 857 may provide power to a heated breathing tube (HBT). As another example, the electrical contacts 857 may be used to power or communicate with additional sensors (e.g., temperature sensors, pressure sensors, or other sensors as described herein, which may be molded and bonded to the molding material via a bonding layer).

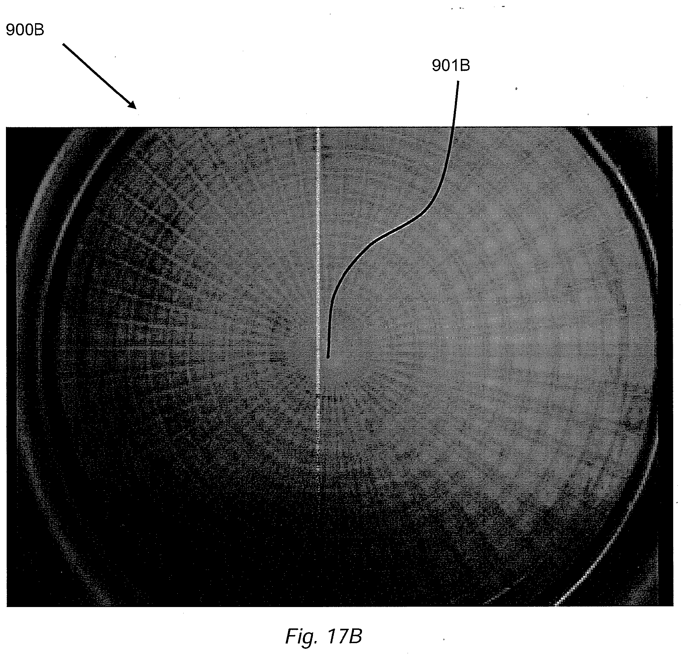

In some embodiments, the micro-channels may provide a wicking surface. The wicking surface may work synergistically with the pre-heating of the gas to allow the heating surface to be maintained at a relatively low temperature. This is because lower temperatures require larger surface areas to generate the requisite vapor flux, and larger areas require more efficient mechanisms for spreading the liquid so as to recruit more of the heated surface for evaporation. In some configurations, micro-channels may be small scale (for example, micro-scale) grooves formed on a surface. The surface may be either flat or curved. In some configurations, the micro-channels may be highly ordered. In some configurations, the micro-channels are arranged in a pattern (see, for example, Figure 17A, showing one example of a grid structured pattern, and Figure 17B, showing one example of a radial pattern; these examples are non-limiting and other patterns are possible). In some configurations, the purpose of the micro-channels is to spread a liquid across a surface thereby increasing its surface area for a given volume. In some configurations, the micro-channels have a substantially uniform cross-sectional profile along their length. For example, the micro-channels may have a circular or semi-circular, elliptic or semi-elliptic, rectangular, triangular (V-shaped), or trapezoidal cross-sections. In some configurations, the micro-channels may include rounded edges and/or corners. In some configurations, the micro-channels may have a variable cross-sectional profile that changes over the length of the micro-channel. For example, a micro-channel can become deeper and/or wider along its length. The micro-channels may be "open" micro-channels, which include at least one side open to the environment. For example, a micro-channel may be a V-shaped groove formed into a surface, and liquid in or on the micro-channel may be open to the environment at at least the open side of the V. Such micro-channels may facilitate evaporation of the liquid as the open side of the micro-channel provides a place for the evaporated liquid to go. For example, an open side of an open micro-channel may open into a gas passageway. Liquid on or in the micro-channel may evaporate, and the evaporated liquid may be entrained in the gases flowing through the gas passage way. In some configurations, the micro-channels may have a depth (which can also be considered a height) less than or equal to about: 1 µm, 10 µm, 20 µm, 30 µm, 50 µm, 100 µm, 200 µm, 500 µm, 1000 µm, or ranges including and/or spanning the aforementioned values. In some configurations, the micro-channels may have a width less than or equal to about: 1 µm, 10 µm, 20 µm, 50 µm, 100 µm, 200 µm, 500 µm, 1000 µm, or ranges including and/or spanning the aforementioned values. In some configurations, the tilt of the side walls of a micro-channel can range from 0-45 degrees. As used herein, the tilt of the sidewalls is measured between the wall and vertical (in other words, between the wall and an axis extending normal to the surface in which the micro-channel is formed). That is, a wall tilt of 0 degrees represents a totally vertical wall. For example, if the side walls of a micro-channel include a 0 degree wall tilt, the micro-channel may be substantially square shaped, and the top of the square may be open. As another example, if the side walls of a micro-groove include a 45 degree wall tilt, the micro-channel may be substantially V-shaped if the tilted side wall directly intersect, or substantially trapezoidally shaped if the side walls intersect a horizontal flat bottom surface of the micro-channel, and the top of the micro-channel may be open. In some configurations, the tilt of the side walls of a micro-channel can range from 5-20 degrees. The micro-channels can spread the liquid by wicking (capillary action) or, in some situations, by gravitational flow of the liquid through the channels. In some configurations, the micro-channels may be defined by protrusions extending above a surface, where the micro-channel is formed by the space between the protrusions.

In some configurations, the heating element 800 includes one or more sensors for measuring the temperature of the surface of the heating element 800. The one or more sensors may be thermistors 821. In some configurations, the heating surface temperature may be calculated at least in part, by determining a resistance level or other characteristic of the heating element 800. The resistance level of the heating element may be used to indicate an average temperature of the heating surface. The heating element may be arranged to deliver a higher power density in a specified region of the heating element as compared to a power density delivered to other regions of the heating element. The specified higher density region of the heating element may be located at an outlet of a water supply to the heating surface. The specified higher density region of the heating element may be located at a water pre-heating area on the heating surface. The respiratory humidification system may include a temperature sensor at the outlet location of the gases channel, which may act as a safety check.

The resistive tracks 811 and/or sensors, for example, the thermistors 821 may be electrically connected to electrical contacts 852 positioned on a contact region 851 of the printed circuit board 801 (e.g., that may span from the molding-material-encapsulated portion of the PCB to an external region of the chamber (e.g., humidification chamber). The contact region 851 can be positioned so as to mate with the electrical connector 751 of the humidification system 700.

In some configurations, the heating element 800 is configured to provide some "pre-heating" to the water. This can be accomplished, in some configurations, simply by increasing the track (and therefore power) density at the area(s) where the water is introduced. This zone would have the power density increased by the extra amount required to heat the water within a small area. For example, as shown in Figure 15C, if water is introduced to the heating element 800 at location 808 and the surface of the heating element is configured to wick the water across the heating element 800 in the direction of the arrows, the heating element 800 can include a greater density of resistive tracking 811 at locations at and around location 818 (in other words, near location 808 where the water is introduced) and a lower density of resistive tracking 811 at locations around location 828 (in other words, distanced from location 818). In some embodiments, the air and/or the water is pre-heated with a heating element. Another option for providing pre-heating for the water is to include a heater in the water supply line (i.e., between the pump/flow sensor and coupling to the surface), this could be a PTC (positive temperature coefficient) element, or a heating coil, or any other heater, in thermal contact with the water flow, which heats the water to the same temperature as the surface of the heating element 800. While the heating element 800 has been described above in reference to heating water, a similar heating element 800 may also be used to heat gas, for example, as a gas pre-heater.

Figure 15D illustrates a top view of two alternative embodiments of heating element 800A, 800B in accordance with an embodiment of the present disclosure. The heating elements 800A, 800B may include an etched foil film 801A, 801B. The etched foil film 801A, 801B may include a plurality of resistive tracking 811A, 811B. The heating elements 800A, 800B may each also include electrical connections 851A, 851B. These electrical connections may be located external to the over-molded portion of the heating element. For example, as disclosed elsewhere herein, the heating portion of a PCB may be inside a chamber of the system where it is encapsulated by a molding material and fused thereto via a bonding layer, while the electrical conduit is external to that chamber. In some embodiments, circular embodiment 800B may provide a bottom or top surface of a chamber humidification system (as shown in, for example, Figure 4A). Figure 15E illustrates an embodiment of the heating element 800A in a rolled configuration.

In some configurations, a humidification system includes various components, for example, a distribution and/or wicking system, to deliver the humidification fluid to the heating element. In some configurations, it is preferred to deliver water to the heating element surface across the entire surface, in other words, to saturate it. In some embodiments, the distribution/wicking system is able to sustain a flow rate. In some configurations, it is not enough to have water distribution over the surface, if that distributor cannot wick the water fast enough to keep the heating element saturated. In some configurations, the distributor is capable of sustaining a liquid flow rate of between about 0 mL per minute to about 5 mL per minute, between about 1 mL per minute to about 4 mL per minute, and about 2 mL per minute to about 3 mL per minute.

A distribution and/or wicking system may include two parts: the wicking surface, which distributes the water across the surface, and the coupling, which connects the water supply to the surface at one or more points. The coupling can also do some of the water distribution (e.g., by coupling the water over a region or line rather than at a point). The technologies that can be used for both coupling and wicking include, but are not limited to: fabrics/papers (for example, Kimberly-Clark Hydroknit); micro-channels; hydrophilic coatings (for example, Lotus Leaf Coatings HydroPhil); capillary/contact wicks (custom designs) and/or porous polymers (for example, Porex fibres). In some configurations, a wicking surface can be a micro-channeled surface, which can include parallel channels in only one direction; a small set of distribution channels connected to a larger number of main channels; and/or channels distributed radially from a single point, among other possible configurations. A wicking surface may also be an absorptive fabric or paper, a super-hydrophilic coated surface, or, a thin porous media.

Figure 17A is a diagram illustrating an embodiment of a grid-structured micro-channel water distribution pattern 900A in accordance with an embodiment of the present disclosure. The distribution pattern 900A includes a water input area 901A, first micro-channels 902A, and second micro-channels 903A. The first micro-channels can serve as distribution channels which distribute the water to the second micro-channels. The second micro-channels distribute the water across the surface. The grid-structured micro-channel water distribution pattern 900A can be applied to the surface of the heating element 800 (e.g., as a molding material that is molded and bonded via a bonding layer to a PCB as a substrate). The grid-structured micro-channel water distribution pattern 900A is one example of a wicking element as described herein. In some configurations, the first micro-channels 902A move the water in a first direction and the second micro-channels 903A move the water in a second direction orthogonal to the first direction. However, the grid-structured micro-channel water distribution pattern can be modified to include first micro-channels oriented at other positions relative the second micro-channels. In some configurations, the grid-structured micro-channel water distribution pattern includes only first micro-channels or only second micro-channels. In general, grid-structured micro-channel water distribution pattern is a system where the micro-channels distribute the water: the water is supplied to several distribution channels, which splits off onto many channels that wick across the bulk of the surface.

Figure 17B illustrates a radial micro-channel water distribution pattern 900B in accordance with an embodiment of the present disclosure. Figure 17B is a still image taken from a video showing the radial micro-channels wicking a fluorescent dye. The fluorescent dye was dropped onto the center point 901B and wicked outwards by the channels. The radial micro-channel water distribution pattern 900B includes micro-channels that spread radially from a center point 901B where the water is introduced. In some configurations, to keep the channel density the same the micro-channels may split as they radiate from the center point 901B. The radial micro-channel water distribution pattern 900B may also include circumferentially extending micro-channels.

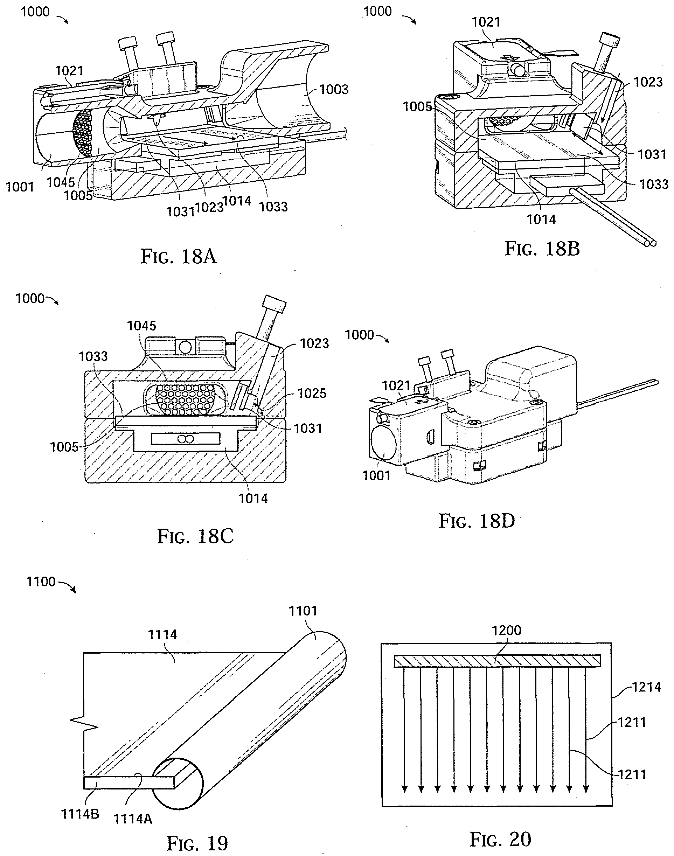

Figure 18A is a perspective axially sectioned view of an embodiment of a respiratory humidification system 1000 including a glass slide coupling 1031 in accordance with an embodiment of the present disclosure. Figure 18B is a perspective sectioned side view of the respiratory humidification system 1000 of Figure 18A. Figure 18C is a side view of the respiratory humidification system 1000 of Figure 18A. Figure 18D is a perspective assembled axial view of the respiratory humidification system 1000 of Figure 18A. The glass slide coupling 1031 may be considered a contact-angle/capillary line distributor. In the illustrated embodiment, the respiratory humidification system 1000 includes a gas inlet 1001 and a gas outlet 1003 with a gas flow channel 1005 extending there between. As gases move from the inlet 1001 to the outlet 1003 they are humidified in the flow channel 1005. The respiratory humidification system 1000 also includes a micro pump 1021 adapted to supply water from a water source into the system. The water is delivered from the micro pump 1021 into the flow channel 1005 via water pipe inlet 1021. The respiratory humidification system further includes a glass slide coupling 1031, which is held at an acute angle 1025 (see Figure 18C) against the surface 1033 of the heating element 1014. The surface 1033 includes micro-channels extending in the direction of the arrows and perpendicular to the glass slide 1031. The water supply tube 1023 is placed at the intersection of the glass slide coupling 1031 and surface 1033. Because of the acute angle 1025 (see Figure 18C) between the glass slide coupling 1031 and surface 1033, the water is wicked along the intersection, and then wicked across the surface 1033 by the micro-channels. Notably, the coupling 1031 only exposes the heater element 1014 on one side; however, in some configurations the design could be modified to expose the heater element 1014 on both sides. The respiratory humidification system 1000 may also include a honey-comb gas diffuser 1045 in the gas flow path 1005. The heating element described herein may be a portion of a PCB that is molded with a molding material to provide a molded member comprising a bonding layer.

Figure 19 is a perspective diagram of a distribution tube coupling 1100 wrapped over an edge of a heating element 1114 in accordance with an embodiment of the present disclosure. This drawing shows a tube 1101 being used as a coupling or distributor. The tube 1101 clips over the heating element 1114, and then water is pumped into the tube 1101. As the tube 1114 fills, water is drawn across the heating element 1114. Notably, the tube coupling 1100 can distribute water onto both the top surface 1114a and the bottom surface 1114b of a heating element 1114. In some embodiments, the tube comprises a molded member as disclosed elsewhere herein.

Figure 20 is a diagram of a porous media coupling 1200 in accordance with an embodiment of the present disclosure. The coupling 1200 is shown as a hashed strip extending along the surface of a heating element 1214. The coupling may be, for instance, a piece of fabric. The water is dosed onto the fabric to allow the water to be distributed along the µ-channels. In some configurations, the coupling 1200 may be a thin, porous media, such as a porous or sintered polymer. In some embodiments, the polymer or other material may be molded on the heating element and attached to it via a bonding layer as disclosed elsewhere herein.

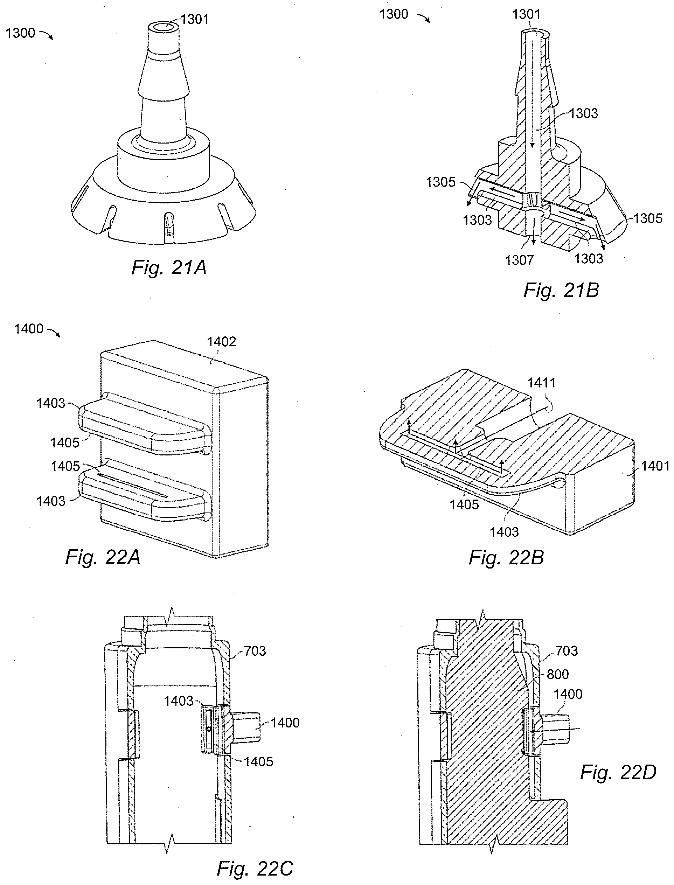

Figure 21A is a perspective view of a radial coupling 1300 in accordance with an embodiment of the present disclosure. Figure 21B is a perspective sectional view of the radial coupling 1300 of Figure 21A. The radial coupling 1300 may be considered a cavity/face coupling. In general, the coupling 1300 pushes water against the surface of a heating element. In some configurations, the coupling 1300 is configured to work with surfaces that are sufficiently hydrophilic or absorptive. In some configurations, the coupling 1300 is adapted so that, when there are multiple outlets, the outlets are balanced, e.g., that the water doesn't simply favor one path and flow entirely in that direction. In some embodiments, the coupling 1300 receives a supply of water at an inlet 1301 and supplies it radially at the center of a heating element and to both sides. As shown in Figure 21B, water flows down from the inlet 1301 through a series of channels 1303 to the heating element (not shown). The coupling 1300 can include multiple outlets 1305. In some configurations, the coupling 1300 also delivers water through a central channel 1307 that extends through a hole in the heating element to a similar system on the other side. In Figure 21B arrows are added to illustrate the flow of water.

Figure 22A is a perspective view of a sandwich coupling 1400 in accordance with an embodiment of the present disclosure. Figure 22B is a schematic perspective sectioned view of the sandwich coupling 1400 of Figure 22A. The coupling 1400 includes a body 1401 with one or more protruding sections 1403. A water outlet 1405 may be positioned on one of or each inward facing surface of the protruding sections 1403. As shown in Figure 22B, the coupling 1400 includes a water inlet 1411 and internal channels that deliver water to the water outlet 1405. Arrows have been added to Figure 22B to show the flow of water. A heating element (as shown in Figures 22C and 22D) may be positioned between the protruding sections 1403 and receive water from the outlets 1405. As disclosed elsewhere herein, the heating element may be coupled (e.g. fixed) to a molding material (e.g., a portion of the sandwich coupling) via a bonding layer. Figure 22C is a sectioned view of the sandwich coupling 1400 of Figure 22A attached to a humidification housing 703 in accordance with an embodiment of the present disclosure. Figure 22D is a schematic sectioned view of the sandwich coupling 1400 of Figure 22A attached to a humidification housing 703 that includes a printed circuit board heating element 800, in accordance with an embodiment of the present disclosure. The housing 703 may be similar to the housing 703 of the humidification system 700 described in reference to Figures 16A-16D and the heating element 800 may be similar to the heating element 800 described in reference to Figures 15A-15C.

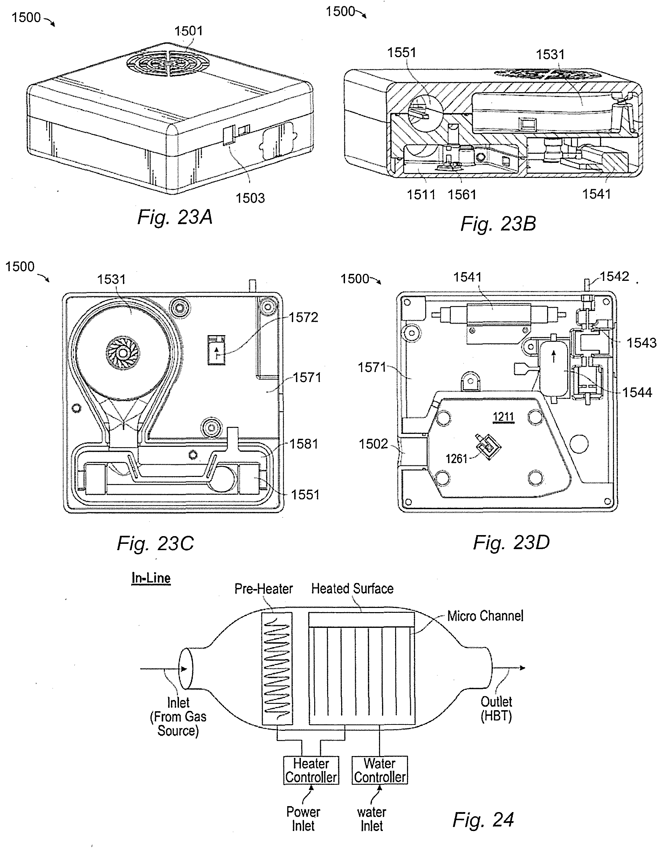

Figure 23A is a perspective view of an alternative embodiment of a humidification system 1500 in accordance with an embodiment of the present disclosure. Figure 23B is a cross-section view of the humidification system 1500 of Figure 23A. As shown in Figure 23B, the humidification system 1500 includes a top layer and a bottom layer. Figure 23C is a cross-section view showing the top layer of the humidification system 1500 of Figure 23A. Figure 23D is a cross-section view showing the bottom layer 1500 of the humidification system of Figure 23A. The humidification system 1500 may include a gas inlet 1501 and a gas outlet 1502. The humidification system can include a blower 1531 configured to move gas from the gas inlet 1501 to the gas outlet 1502. The inlet 1501 and the outlet 1502 may be connected by a channel. A flow sensing device 1551 and a gas sensing device 1581 may be located within the channel. A portion of the flow sensing device and gas sensing device may be over-molded and bonded via a bonding layer to a molding material as disclosed elsewhere herein. The humidification system 1500 includes power/communication connectors 1503.

The humidification system 1500 can include a heater surface cavity 1511 configured to receive a heating element as described elsewhere herein. As described elsewhere herein, the heating element may be molded with a molding material via a bonding layer. The heating surface cavity also includes a water dosing section 1561 which may be configured with a coupling to apply water to the heating element. The water dosing section 1561 may be in fluid communication with a liquid flow module 1541, a water inlet 1542, a check valve 1543, and micro pump 1544. The humidification system 1500 may also include an electronics cavity 1571 accessible via a port 1572.

Figure 24 is a view of an inline humidification system in accordance with one an embodiment of the present disclosure. The inline humidification system of Figure 24 includes a pre-heater and a heater (the heater represented by the heated surface) in a gas passageway between an inlet and an outlet. A heater controller is connected to both the pre-heater and the heater. The pre-heater heats the gas before the gas reaches the heater. The heater is also connected to a water controller which dispenses water onto the heater surface. The amount of water applied by the water controller and the amount of heat applied by the heater controller may be deterministically controlled according to the principles described herein to vaporize the water and humidify the gas. The outlet of the system may be connected to a heated breathing tube (HBT), i.e. an inspiratory or delivery tube. The necessary power and sensing systems for the HBT may be provided integrally by the humidification system, or provided separately or externally. The advantages of including the humidification system as part of the delivery tube is simplicity, reduction in cost, and quality control by ensuring that it is replaced as necessary.

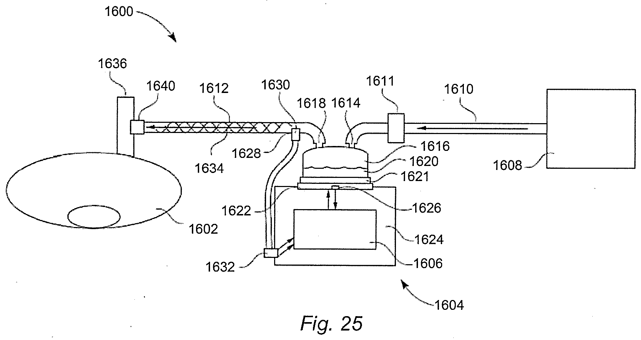

Reference is now made to Figure 25, which is a view of a medical insufflation humidification system including a humidifier and/or a humidification chamber constructed and operative in accordance with an embodiment. Figure 25 illustrates an insufflation humidification system 1600 for delivering a temperature- and humidity-controlled gases flow to a patient 1602, the insufflation system 1600 having a humidification apparatus or humidifier 1604 incorporating a humidifier control system 1606. The humidifier 1604 is connected to a gas source 1608 through an inlet conduit 1610. The humidifier 1604 delivers humidified gas to the patient 1602 through a patient or gas delivery conduit 1612. The conduits 1610, 1612 can be made of flexible plastic tubing. The conduits 1610, 1612 comprise part of a breathing circuit being a gas flow path for delivering gas from the gas source 1608 to the patient. The gas source 1608 could be integral to the system 1600 and comprise a flow generator including a blower for example (i.e. the gas source 1608 and the humidifier 1604 may be combined to form a single apparatus or the two separate components may be modular and configured to couple together without the need for separate conduit(s) therebetween, as is known in the art). Alternatively, or additionally the gas source could be separate but connected to the system 1600 and comprises a hospital compressed gas source for example.

The humidifier 1604 can receive gas from the gas source 1608 through the gas supply or inlet conduit 1610. The gas can be filtered through a filter 1611 and delivered to the humidifier 1604 through a humidifier inlet 1614. The gas is humidified as it passes through a humidifying chamber 1616, and the gas flows out through a humidifier outlet 1618 and into the patient conduit 1612. The gas then moves through the patient conduit 1612 to the patient 1602 via a connector 1640 (e.g. Luer connector) and the patient interface 1636. The patient interface 1636 may be, for example, but not limited to, a trocar or cannula for laparoscopic surgery or a diffuser for open surgery. Patient conduit 1612 comprises a gas delivery conduit comprising part of a breathing circuit comprising one or more conduits or tubes forming a gas flow path between the flow generator and the patient, including gas supply conduit 1610.

The humidifier chamber 1616 may be removably engageable with a body 1624 of the humidifier 1604. The humidification chamber 1616 may comprise a side wall, and a base/bottom. The humidification chamber 1616 may further comprise a top, an openable lid, or may be topless (e.g. open topped, in which case, the humidification chamber 1616 may be received in a sealable cavity of the humidifier body 1624). In some embodiments, the top, base and side walls may together define any one of a substantially circular chamber, substantially square chamber, substantially rectangular chamber, or other shapes (triangular, etc.) when the chamber is viewed from the top. For rounded (e.g., circular) chambers, the side wall may be arcuate in shape and may define other arcuate shapes. For example, the chamber may be an elliptical chamber when viewed from the top, or any other chamber having an arcuate but not necessarily perfectly circular shape when viewed from above. The chamber may be symmetrical about at least one axis when viewed from the top. Preferably the chamber is symmetrical about 2 axes, that is, a horizontal and a vertical axis when viewed from above.

The base 1621 of the humidification chamber 1616 may have a heat conductive (e.g. metal) region or may be entirely heat conductive, and may be adapted to hold a volume or reservoir of water 1620, which can be heated by a heater plate 1622. The heater plate 1622 may include a heating element configured to provide heat to the heating surface. As disclosed elsewhere herein, the heating plate and/or the heating element may be a portion of a molded member that is bonded to a molding material (e.g., the chamber base) via a bonding layer. The heater plate 1622 may be in thermal contact with the heat conductive base 1621 of the humidification chamber 1616. Providing power to the heater plate 1622 can cause heat to flow from the heater plate 1622 to the water 1620 through the heat conductive base 1621. As the water 1620 within the humidification chamber 1616 is heated it can evaporate and the evaporated water can mix with gases flowing through the humidification chamber 1616 from the gas source 1608, optionally via the filter 1611. Accordingly, the humidified gases leave the humidification chamber 1616 via outlet 1618 and are passed to the patient 1602 via the patient conduit 1612, the connector 1640, the patient interface 1636 and into the surgical site to, for example, insufflate the surgical site and/or expand a body cavity.

The humidifier 1604 includes a humidifier control system 1606 configured to control a temperature and/or humidity of the gases being delivered to the patient 1602. The humidifier control system 1606 can be configured to regulate an amount of humidity supplied to the gases by controlling an electrical power supplied to the heater plate 1622. The humidifier control system 1606 can control operation of the humidification system 1604 in accordance with instructions set in software and in response to system inputs. System inputs can include a heater plate sensor 1626, a chamber outlet temperature sensor 1628, and a chamber outlet flow sensor 1630. For example, the humidifier control system 1606 can receive temperature information from the heater plate sensor 1626 which it can use as an input to a control module used to control the power or temperature set point of the heater plate 1622. The humidifier control system 1606 can be provided with inputs of temperature and/or flow rates of the gases. For example, the chamber outlet temperature sensor 1628 can be provided to indicate to the humidifier control system 1606 the temperature of the humidified gases as they leave the outlet 1618 of the humidification chamber 1616. The temperature of the gases exiting the chamber can be measured using any suitable temperature sensor 1628, such as a wire-based or PCB-based temperature sensor. The chamber outlet flow sensor 1630 can be provided to indicate to the humidifier control system 1606 the flow rate of the humidified gases. The flow rate of the gases through the chamber 1616 can be measured using any suitable flow sensor 1630, such as a hot wire anemometer, or a thermistor configured for use as a flow sensor. In some embodiments, the temperature sensor 1628 and flow sensor 1630 are in or otherwise provided to the same sensor housing. The temperature sensor 1628 and flow sensor 1630 can be connected to the humidifier 1604 via connector 1632. Additional sensors may be incorporated into the insufflation system 1600, for example, for sensing parameters at the patient end of the patient conduit 1612. Alternatively the sensors may be wirelessly coupled to the controller. Alternatively the connector may be internal, that is, the sensors are coupled to the controller by integrated wires rather than an external cable. The sensors (such as flow sensors, humidity sensors, temperature sensors, etc.), as disclosed elsewhere herein may be a substrate (e.g., a PCB) with one or more sensing regions over which a molding material is bonded via a bonding layer as described elsewhere herein.

The humidifier control system 1606 can be in communication with the heater plate 1622 such that the humidifier control system 1606 can control a power delivered to the heater plate 1622 and/or control a temperature set point of the heater plate 1622. As described further herein, the humidifier control system 1606 can determine an amount of power to deliver to the heater plate 1622, or a heater plate set point, based at least in part on any one or more of a flow condition, an operation mode, a flow reading, an outlet temperature reading, a heater plate sensor reading.

The insufflation system 1600 can include a conduit heating wire 1634 configured to provide heat to the gases traveling along the patient or gas delivery conduit 1612. In some embodiments, the heating wire is a resistive track on a PCB. In some embodiments, the PCB is coupled to a molding material via a bonding layer as disclosed elsewhere herein. Gases leaving the outlet 1618 of the humidification chamber 1616 can have a high relative humidity (e.g., about 100%). As the gases travel along the patient conduit 1612 there is a chance that water vapor may condense on the conduit wall, reducing the water content of the gases. In some embodiments, to reduce condensation of the gases within the conduit, the conduit heating wire 1634 can be provided in the gas flow path along the entire or part of the patient conduit. In some embodiments, the conduit heating wire 1634 can be embedded within the wall of the patient conduit. In some embodiments, the conduit heating wire 1634 can be external to the patient conduit along the entire or part of the patient conduit. In some embodiments, the wire may be positioned at least partly within a wall of the patient conduit. Power can be supplied to the conduit heating wire 1634 from the humidifier 1604 and can be controlled through the humidifier control system 1606. In some embodiments, the heating wire 1634 is configured to maintain the temperature of the gas flowing through the patient conduit 1612. In some embodiments, the conduit heating wire 1634 can be configured to provide additional heating of the gases to elevate the gases temperature to maintain the humidity generated by the heated water bath in the humidifier 1604. The conduit heating wire may be provided on a substrate and part of a molded member.

The gas delivery conduit may be single walled or comprise a dual conduit configuration that includes an outer conduit and an inner conduit. The inner conduit may carry the gases and include the heating wire 1634. The outer conduit may provide insulation of the inner conduit. The outer conduit and inner conduit may be co-axial and there may be an air gap between the outer conduit and the inner conduit.

A further filter or filter assembly (not shown) may be optionally provided and disposed in use between the humidifier 1604 and the patient interface 1636 so as to allow re-use of the inlet conduit 1610 and/or an inspiratory conduit, and in some instances re-use of the humidification chamber 1616. The filter assembly can comprise a filter medium for filtering the gases exiting the outlet 1618 of the humidification chamber 1616. The filter assembly may also comprise a housing and a heating element. The filter medium can be positioned inside the housing so that the humidified gases flowing through the housing are filtered and particles removed. The heating element can be positioned in the gases flow path between the inlet and the outlet of the housing but spaced apart from the filter medium and the housing. In some embodiments, the housing for the filter and/or the heating element is or comprises a molded member as disclosed elsewhere herein. The heating element can be configured to heat the filter medium to reduce condensation and prevent the filter becoming clogged. Such filters are described in, for example, WO2018/106127.

The connector 1640 can be a Luer connector comprising a body having an interior region defining a gases flow passageway allowing insufflation gases to flow through. The body can comprise a first end that removably connects to a fitting of the patient interface 1636 and a second end that attaches, preferably permanently, to the tubing of the patient conduit 1612. It will be appreciated that the Luer connector 1640 of Figure 25 can be a high flow Luer connector providing particular sealing and retention features with less resistance to gases flow than traditional Luer connectors of the art. Embodiments of such high flow Luer connectors are described, for example, in WO2018097738.

In some embodiments, the humidifier chamber 1616 of the humidifier 1604 can be configured to provide a controlled gases flow pattern for the insufflation gases. A humidification chamber according to embodiments described herein, including as shown in Figures 4A to 7B offer high humidification levels and strong bonding between the molded material and the heating surface. The embodiments herein are particularly adapted for use in respiratory systems such as CPAP or high flow respiratory gas systems, for example a high flow system for use in anaesthesia and sedation procedures. Respiratory systems in which the chamber may be particularly useful are CPAP, BiPAP, high flow therapy, varying high flow therapy, low flow air, low flow O2 delivery, bubble CPAP, apnoeic high flow (i.e. high flow to anesthetized patients), invasive ventilation and non-invasive ventilation. Further, a chamber as described herein may be useful in systems other than respiratory systems.

High flow therapy as discussed herein is intended to be given its typical ordinary meaning as understood by a person of skill in the art which generally refers to a respiratory assistance system delivering a targeted flow of humidified respiratory gases via an intentionally unsealed patient interface with flow rates generally intended to meet or exceed inspiratory flow of a patient. Typical patient interfaces include, but are not limited to, a nasal or tracheal patient interface. Typical flow rates for adults often range from, but are not limited to, about fifteen liters per minute to about seventy liters per minute or greater. Typical flow rates for pediatric patients (such as neonates, infants and children) often range from, but are not limited to, about one liter per minute per kilogram of patient weight to about three liters per minute per kilogram of patient weight or greater. High flow therapy can also optionally include gas mixture compositions including supplemental oxygen and/or administration of therapeutic medicaments. High flow therapy is often referred to as nasal high flow (NHF), humidified high flow nasal cannula (HHFNC), high flow nasal oxygen (HFNO), high flow therapy (HFT), or tracheal high flow (THF), among other common names. In some configurations, a flow rate of gases supplied or provided to an interface via a system or from a flow source, comprises flows of at least about: 5, 10, 20, 30, 40, 50, 60, 70, 80, 90, 100, 110, 120, 130, 140, 150 liters/minute (LPM), or more, and useful ranges may be selected to be any of these values (for example, about 20 LPM to about 90 LPM, about 40 LPM to about 70 LPM, about 40 LPM to about 80 LPM, about 50 LPM to about 80 LPM, about 60 LPM to about 80 LPM, about 70 LPM to about 100 LPM, about 70 LPM to about 80 LPM).

High flow therapy may be used as a means to promote gas exchange and/or respiratory support through the delivery of oxygen and/or other gases, and through the removal of CO2 from the patient's airways. High flow of delivered gases may be particularly useful prior to, during or after a medical procedure. When used prior to a medical procedure, high gas flow can pre-load the patient with oxygen so that their blood oxygen saturation level and volume of oxygen in the lungs is higher to provide an oxygen buffer while the patient is in an apnoeic phase during the medical procedure. A continuous supply of oxygen is essential to sustain healthy respiratory function during medical procedures (such as during anaesthesia) where respiratory function might be compromised (e.g. diminishes or stops). When this supply is compromised, hypoxia and/or hypercapnia can occur. During medical procedures such as anaesthesia and/or general anaesthesia where the patient is unconscious, the patient is monitored to detect when this happens. If oxygen supply and/or CO2 removal is compromised, the clinician stops the medical procedure and facilitates oxygen supply and/or CO2 removal. This can be achieved for example by manually ventilating the patient through an anaesthetic bag and mask, or by providing a high flow of gases to the patient's airway using a high flow therapy system. These high flow systems may include molded members as disclosed herein. Further advantages of high gas flow can include that the high gas flow increases pressure in the airways of the patient, thereby providing pressure support that opens airways, the trachea, lungs/alveolar and bronchioles. The opening of these structures enhances oxygenation, and to some extent assists in removal of CO2. The increased pressure can also keep structures such as the larynx from blocking the view of the vocal chords during intubation. When humidified, the high gas flow can also prevent airways from drying out, mitigating mucociliary damage, and reducing risk of laryngospasms and risks associated with airway drying such as nose bleeding, aspiration (as a result of nose bleeding), and airway obstruction, swelling and bleeding. Another advantage of high gas flow is that the flow can clear smoke created during surgery in the air passages. For example, smoke can be created by lasers and/or cauterizing devices.

A pressure relief or regulating device is particularly desirable for use in a respiratory system such as a high flow system comprising an unsealed patient interface, to provide an upper pressure limit for the system. Most importantly, the upper pressure limit may be configured to provide a patient safety limit, or may be configured to prevent damage to tubes, fluid connections, or other components. A pressure relief or regulating device may be used in a CPAP (continuous positive airway pressure), BiPAP (bilevel positive airway pressure) and/or Bubble CPAP systems to regulate the pressure provided to the patient. An exemplary CPAP apparatus is described in WO 2011/056080.

The gas delivery systems as disclosed herein may comprise one or more of the following features. The system may include a flow source, such as an in-wall source of oxygen, an oxygen tank, a blower, a flow therapy apparatus, or any other source of oxygen (e.g. compressed oxygen) or other gas (e.g. compressed air). The system may also comprise an additive gas source, comprising one or more other gases that can be combined with the flow source. The flow source can provide a pressurised high gas flow that can be delivered to a patient via a delivery conduit, and patient interface (such as a nasal cannula). A humidifier is also provided, which can humidify the gas and, in some embodiments, adjust the temperature of the gas. The humidifier comprises a humidification chamber as disclosed above. One or more sensors, such as flow, oxygen, pressure, humidity, temperature or other sensors can be placed throughout the system (on a PCB in the humidifier) and/or at, on or near the patient. As disclosed elsewhere herein, portions of the blower, the valves, the sensors, and/or the heating elements may be attached and/or integral to the system.

In some embodiments, a controller may be provided in the gas delivery system. In some embodiments, the controller may be in communication with a flow source, an additive gas source, a humidifier and sensors. In some embodiments, the controller may operate the flow source to provide the desired flow of gas. In some embodiments, it can control the flow, pressure, composition (where more than one gas is being provided), volume and/or other parameters of gas provided by the flow source based on feedback from sensors. In some embodiments, controller can also control any other suitable parameters of the flow source to meet oxygenation requirements. In some embodiments, controller can control the humidifier (e.g., based on feedback from the sensors). In some embodiments, using input from the sensors, the controller can determine oxygenation requirements and control parameters of the flow source and/or humidifier as required.

In some embodiments, as noted above, the humidified gas can be delivered to the patient via a delivery conduit and the patient interface or 'interface' (as described elsewhere herein), such as a cannula, mask, nasal interface, oral device or combination thereof. In some embodiments, the high gas flow (optionally humidified) can be delivered to the patient for surgical uses, e.g. surgical insufflation. In these embodiments, the 'interface' is a surgical interface and could be a surgical cannula, trocar, or other suitable interface. The patient interface can be substantially sealed, partially sealed or substantially unsealed. A nasal interface as used herein is a device such as a cannula, a nasal mask, nasal pillows, or other type of nasal device or combinations thereof. A nasal interface can also be used in combination with a mask or oral device (such as a tube inserted into the mouth) and/or a mask or oral device (such as a tube inserted into the mouth) that can be detached and/or attached to the nasal interface. A nasal cannula is a nasal interface that includes one or more prongs that are configured to be inserted into a patient's nasal passages. A mask refers to an interface that covers a patient's nasal passages and/or mouth and can also include devices in which portions of the mask that cover the patient's mouth are removable, or other patient interfaces such as laryngeal mask airway or endotracheal tube. A mask also refers to a nasal interface that includes nasal pillows that create a substantial seal with the patient's nostrils. The controller controls the system to provide the required oxygenation.

In some embodiments of a system, a gas flow generator in the form of a motor/impeller arrangement is provided along with a humidifier. In some embodiments, the humidifier comprises a humidification chamber as disclosed above. In some embodiments, a controller is configured or programmed to control the components of the apparatus, including: operating the flow generator to create a flow of gas (gas flow) for delivery to a patient, operating the humidifier to humidify and/or heat the generated gas flow, receive user input from the user interface for reconfiguration and/or user-defined operation of the apparatus, and output information (for example on the display) to the user. The user could be a patient, healthcare professional, or anyone else interested in using the apparatus. In some embodiments, the controller adjusts one or more parameters of the humidifier based on information collected from a sensor on a PCB (e.g., a PCB that is part of a molded member that may be in the humidifier).

In some embodiments, a breathing assistance apparatus may be a standalone humidifier apparatus comprising a main housing and a humidifier. An exemplary standalone humidifier apparatus is described in WO 2015/038013.

A patient breathing conduit may be connected to a gas flow output or patient outlet port in the housing of a breathing assistance apparatus, and may be connected to a patient interface such as a nasal cannula with a manifold and nasal prongs. Additionally, or alternatively, the patient breathing conduit could be connected to a face mask (or other respiratory system patient interface). Additionally, or alternatively, the patient breathing conduit could be connected to a nasal pillows mask, and/or a nasal mask, and/or a tracheostomy interface, or any other suitable type of patient interface. The humidified gas flow generated by the respiratory system (e.g., a breathing assistance apparatus) may be delivered to the patient via a patient breathing conduit and through the patient interface. The patient breathing conduit can have a heater wire to heat gas flow passing through to the patient. As disclosed elsewhere herein, the heating wire may be located on a substrate that is molded and bonded via the substrate to a molding material (e.g., as part of a PCB). In some embodiments, the heat output of the heater wire is controlled by a controller. The patient breathing conduit and/or patient interface can be considered part of the breathing assistance apparatus, or alternatively peripheral to it. The breathing assistance apparatus, breathing conduit, and patient interface may together form a breathing assistance system or, in some configurations, a flow therapy system.

In some embodiments, the controller controls the flow generator to generate a gas flow of the desired flow rate, controls one or more valves to control the mix of air and oxygen or other alternative gas, and controls the humidifier to humidify the gas flow and/or heat the gas flow to an appropriate level. The gas flow may be directed out through the patient breathing conduit and patient interface to the patient. The controller can also control a heating element in the humidifier (which together comprise may comprise a molded member as described elsewhere herein) and/or the heating element in the patient breathing conduit (which also may together comprise a molded member as described elsewhere herein) to humidify and/or heat the gas to a desired temperature that achieves a desired level of therapy and/or comfort for the patient. In some embodiments, the controller can be set, programmed with, or can determine, a suitable target temperature of the gas flow.

Operation sensors, such as flow, temperature, humidity, and/or pressure sensors, can be placed in various locations in the gas delivery apparatus (e.g., respiratory apparatus, insufflation apparatus, etc.) and/or the patient breathing conduit and/or other patient interface(e.g., surgical cannula, etc.). These sensors may be, as described elsewhere herein, molded and bonded to a molding material via a bonding layer formed between the sensor (and/or a substrate on which sensing features of the sensor reside) and the molding material. As disclosed elsewhere herein, in some embodiments, output from the sensors can be received by a controller, to assist it to operate the gas delivery apparatus in a manner that provides targeted therapy. In some configurations, providing targeted therapy includes meeting or exceeding a patient's inspiratory flow. The apparatus may have a transmitter and/or receiver to enable the controller to receive signals from the sensors and/or to control the various components of the gas delivery apparatus, including but not limited to the flow generator, humidifier, and heater wire, or accessories or peripherals associated with the breathing assistance apparatus. Each of these features may be molded with a molding material and bonded to the molding material via a bonding layer. Additionally, or alternatively, the transmitter and/or receiver may deliver data to a remote server or enable remote control of the apparatus.

In some embodiments, as disclosed elsewhere herein, a humidification chamber according to embodiments described herein may be adapted for use in a respiratory assistance system for delivery of heated and humidified gases to a patient can include a respiratory patient interface configured to deliver a flow of respiratory gases received from a gases source, an inspiratory conduit configured to be in fluid communication with the patient interface and the gases source via a humidifier. In some embodiments, as disclosed elsewhere herein, a humidification chamber (and/or the molded members that are described throughout this disclosure) may be adapted for use in a gas delivery system that provides gases for surgical procedures that are not for respiration (e.g., for delivery of heated and humidified gases to a patient during insufflation) and can include a patient interface (e.g., a cannula) configured to deliver a flow of gases received from a gases source, a conduit configured to be in fluid communication with the patient interface and the gases source via a humidifier. The humidifier can include a humidification chamber, in accordance with the above disclosure, having at least one wall that defines the chamber such that the chamber can hold a liquid, a chamber inlet, a chamber outlet, and a gases flow path between the chamber inlet and the chamber outlet. The chamber inlet can be configured to be in fluid communication with the gases source and the chamber outlet can be configured to be in fluid communication with the inspiratory conduit. The humidification chamber can hold a volume of liquid (such as water). The humidifier can include a heater plate (e.g., that is over-molded by a molding material of the chamber and fixed via a bonding layer). The heater plate may be configured to heat the volume of liquid and the flow of respiratory gases in the gases flow path within the humidification chamber so as to heat and humidify the flow of respiratory gases. In some embodiments, the humidifier can also include a controller having one or more hardware processors configured to control the amount of power delivered to the heater plate.

In some embodiments, the humidifier and methods of use described herein can be used to provide non-invasive therapies and/or invasive therapies. The humidifier can be operated in invasive mode, non-invasive mode, high flow mode, or other modes. In some embodiments, the humidifier can operate with various patient interfaces such as, for example, an endotracheal tube (ET tube), full face mask, nasal mask, nasal cannula, nasal pillows, sealed prongs, or any other interface. Other desired humidity levels may be possible and other types of therapy systems may be used. The chamber outlet temperature set point can be adjusted according to the therapies provided and the desired humidity levels.

In some embodiments, a respiratory assistance system includes a humidifier, a gas source, a patient interface, and an inspiratory conduit configured to transport respiratory gases from the humidifier to the patient interface. In some embodiments, the gas source and the humidifier may be in separate housings, or optionally may be co-located, within the same housing, and/or included in a single apparatus. In some embodiments, the humidifier comprises a humidification chamber as disclosed elsewhere herein. In some embodiments, the respiratory assistance system includes an optional expiratory conduit configured to transport gases from the patient interface to the gas source and an optional wye-piece configured to connect the inspiratory conduit and the expiratory conduit to the patient interface. In some embodiments, the respiratory assistance system may not include the expiratory conduit or may include an exhalation port.

In some embodiments, the gas source includes a ventilator, which may include a blower or alternatively a turbine. The gas source may also include other mechanisms to deliver or push a flow of respiratory gases to the humidifier, such as a valve arrangement or a pump. In some embodiments, the gas source is an example room or ambient air entraining ventilator. In some embodiments, the gas source may include an inlet through which ambient air is drawn into the gas source, for example, by the ventilator. In some embodiments, the gas source may optionally include a controller configured to control the operation of the ventilator, the humidifier, or other components of the respiratory assistance system. In some embodiments, the gas source may optionally include a user interface that can provide information regarding user input to the controller. In some embodiments, the controller can control the operation of the ventilator based on information provided by the user interface and/or based on other information, for example but not limited to, feedback from the ventilator or humidifier, such as from a sensor associated with the ventilator or humidifier. As disclosed elsewhere herein, such sensors may be molded and bonded to a molding material via a bonding layer. In some embodiments, instead of drawing ambient air, the inlet can be connected to a supply of dry gas, for example, a gas canister or tank. These types of ventilators can be referred to non-entraining ventilators and may be controlled by one or more valves such as proportional valves. The valve or valves may be controlled by a controller.

In some embodiments, as disclosed elsewhere herein, humidifier may include a humidification chamber and a heater plate. The humidification chamber may be configured to hold a volume of water or other suitable liquid, and may comprise features in accordance with those of the humidification chambers disclosed above. The heater plate may be configured to heat the volume of water and respiratory gases within the humidification chamber, which may increase the temperature of the respiratory gases and may create vapor from the volume of water that is taken up by the respiratory gases. In some embodiments, as disclosed elsewhere herein, the heater plate may comprise a PCB molded with a molding material, the molding material and PCB being connected via a bonding layer. The humidification chamber may include a chamber inlet and a chamber outlet. The inspiratory conduit (or insufflation conduit, as the case may be) may be configured to be connected to the chamber outlet, such that heated and humidified respiratory gases (or insufflation gases, etc.) may be transported by the inspiratory conduit from the humidification chamber to the patient interface and then delivered to a patient. Gases exhaled by the patient into the patient interface may optionally be returned by the expiratory conduit to the gas source. The respiratory assistance system may not include the expiratory conduit and thus gases exhaled by the patient into the patient interface may be vented to the atmosphere, such as directly, or optionally through an exhalation port.

In some embodiments, the humidifier may include a controller that can control, for example but not limited to, the operation of the heater plate. When the humidifier and the gas source form an integrated device, the controller may be the same hardware processor or separate processors. In some embodiments, the humidifier may also include a user interface for providing and/or receiving information regarding user input to the controller. In some embodiments, the humidifier may further include an inlet temperature sensor. In some embodiments, the inlet temperature sensor may be configured to detect the temperature of gases entering the humidifier. In some embodiments, the inlet temperature sensor may measure a characteristic of the ambient air near the location of the inlet temperature sensor, such as a temperature of the ambient air. In some embodiments, the inlet temperature sensor can also be a temperature sensor located at or near the chamber inlet. In some embodiments, the temperature sensor at the chamber inlet can optionally measure both temperature and flow rate of the air coming in from the gas source. This measurement can provide an indication of ambient conditions. Additionally and/or alternatively, the respiratory assistance system may include more than one sensor located at or near the chamber inlet. In some embodiments, the inlet sensors can include a temperature sensor and a separate flow sensor. In some embodiments, the one or more inlet sensors can be located at any location from the gas source 1902 to the humidification chamber. In some embodiments, the one or more outlet sensors and the one or more inlet sensors may be bonded with a molding material of the humidification chamber. In some embodiments, for example, the sensors may be provided on a PCB (or other substrate), which may be molded with a molding material. In some embodiments, the molding material is affixed to the substrate (or some portion of the sensor) via a bonding layer as disclosed elsewhere herein. In some embodiments, the controller may receive information regarding a characteristic of the ambient air near the location of the inlet temperature sensor from the inlet temperature sensor. The controller may be configured to control the operation of the heater plate based on information provided by the user interface, based on information provided by the inlet temperature sensor, and/or based on other information, for example but not limited to, feedback from the heater plate, such as from a temperature sensor located at or near the heater plate. As disclosed elsewhere herein, in some embodiments, where a PCB-based heating element is used, the temperature sensor may be on the same PCB as the heating element. In some embodiments, the controller may be configured to determine an amount of power, or a power duty cycle, to provide to the heater plate such that the heater plate delivers a desired amount of heat to respiratory gases and the volume of water within the humidification chamber.

In some embodiments, the respiratory assistance system (or other gas delivery system, such as an insufflation system) may include one or more outlet sensors that are associated with the chamber outlet location. In some embodiments, the one or more outlet sensors may also be located at or near the chamber outlet. In some embodiments, the outlet sensors can include two sensors: a temperature sensor and a flow sensor. In some embodiments, the temperature sensor can be a thermistor (such as a heated thermistor). In some embodiments, the thermistor can also be used as a flow sensor. Accordingly, there may be a single sensor at or near the chamber outlet. Other types of temperature sensors and flow sensors that can work in a respiratory assistance system may also be used. In some embodiments, the outlet sensor(s) may be located at the chamber outlet, at the inspiratory conduit (or patient interface conduit) near the connection between the chamber outlet and the inspiratory conduit, or at another suitable location downstream of the humidification chamber. In some embodiments, the controller may receive information from the outlet sensor(s) regarding a characteristic of respiratory gases flowing past the location of the outlet sensor. In some embodiments, the controller may be configured to control the operation of the heater plate based on information provided by the outlet sensor(s), instead of or in addition to other sources of information as previously described. An outlet sensor may be integrated into the heater base (e.g., molded) or may be disposed on a cartridge that is removably attachable to a vertical portion of a heater base. In some embodiments, the sensors may be insertable into the inlet port and outlet port as the chamber is positioned in an operative position on the heater base. In some embodiments, the chamber inlet and outlet may include openings that correspond to the inlet temperature sensor and outlet sensor to receive the sensors. The sensor openings in the chamber may include polymer covers that are configured to cover the sensor tip as the sensors are inserted into the gases path such that the sensors do not need to sterilized, since the sensors are not actually in contact with the gases. The sensors may be bonded to a molding material of a chamber, wall, and/or conduit, as disclosed elsewhere herein, and may be affixed to the molding material by one or more bonding layers.

As disclosed elsewhere herein, respiratory gases (or surgical gases for insufflation or other surgical uses) flowing through the inspiratory conduit may lose heat through the walls of the inspiratory conduit, which may reduce the temperature of the respiratory gases and may cause condensation to form within the inspiratory conduit (or patient interface conduit). In some embodiments, the inspiratory conduit (or patient interface conduit) may include a conduit heater configured to heat the conduit to reduce or prevent this loss of heat. In some embodiments, this conduit heater may be, as disclosed elsewhere herein, linked to a molding material of the conduit via a bonding layer. In some embodiments, the controller may be configured to control the operation of the conduit heater based on one or several sources of information as previously described. In some embodiments, the controller may be configured to determine an amount of power, or a power duty cycle, to provide to the conduit heater such that the conduit heater delivers a desired amount of heat to the conduit. The conduit heater may be disposed into the wall of the conduit or may be disposed within the lumen of the conduit.

In some embodiments, the respiratory assistance system may include one or more conduit sensors located within the inspiratory conduit (or interface conduit). In some embodiments, the conduit sensor(s) may be located at the conduit near the connection between the conduit and the wye-piece (where present), at the connection between the inspiratory conduit and the patient interface if the conduit is connected directly to the patient interface, or at the wye-piece or the patient interface. The conduit sensor(s) may measure a characteristic of gases flowing past the location of the conduit sensor, such as a temperature of the gases. The conduit sensor can include a temperature sensor. In some embodiments, the conduit sensor can also include a separate flow sensor. In some embodiments, the conduit sensor can include an integral flow and temperature sensor that is capable of measuring both the temperature and flow rate such as described herein. In some embodiments, the controller may receive information regarding a characteristic of gases flowing past the location of the conduit sensor from the conduit sensor. In some embodiments, the controller may determine the flow rate of gases flowing past the conduit sensor. In some embodiments, the controller may be configured to control the operation of the conduit heater, and/or the operation of the heater plate, based on information received from the conduit sensor, instead of or in addition to other sources of information as previously described. In some embodiments, the conduit sensor may be integrated into the conduit and extend into the gases pathway defined by the conduit. Further, the conduit sensor's wires may be integrated into the wall of the conduit or extend along the conduit. As disclosed elsewhere herein, the sensors may be part of a PCB that is bonded with a molding material by one or more bonding layers.

In some embodiments, gases may also lose heat through the walls of the patient interface, the wye-piece, and/or any other system component that may connect the patient interface to the inspiratory conduit (or patient interface conduit). One or more of the patient interface, the wye-piece, and any other system component that may connect the patient interface to the conduit may include an associated heater and/or an associated sensor. In some embodiments, the heater and/or associated sensor is provided on a PCB that is bonded to a molding material by a bonding layer as disclosed elsewhere herein. In some embodiments, the controller may receive information from such an associated sensor regarding a characteristic of respiratory gases flowing past the location of the sensor. In some embodiments, the controller may use information received from such an associated sensor to control the operation of the respective associated heater. In some embodiments, the heater and/or sensors may be bonded to a molding material by one or more bonding layers to form a molded member and the molded member may be coupled to a chamber, wall and/or conduit as disclosed elsewhere herein.

In some embodiments, one or more of the patient interface, the wye-piece, and any other system component that may connect the patient interface to the conduit may not include an associated heater and/or an associated sensor.

In some embodiments, the humidifier may be used in any respiratory assistance system to deliver heated and humidified respiratory gases to the patient for multiple types of respiratory therapies, including but not limited to invasive ventilation therapy, non-invasive ventilation therapy, high flow therapy, BiPaP therapy, Continuous Positive Airway Pressure therapy, or other respiratory assistance therapy. The humidity conditions of the respiratory gases provided to the humidifier by the gas source may vary. For example, the type of the gas source used in the respiratory assistance system may depend on the type of respiratory therapy, respiratory system configurations, location of use (such as home or hospital), or availability of different gas supplies. Gases from different supplies may have different characteristics, including temperature and humidity. Ambient air, in particular, ambient air in tropical weather and/or during summer time can have a higher humidity than gas obtained from a compressed gas tank or bottle. It may be beneficial to adjust the operating parameters of the respiratory assistance system using a control system such that the patient receives comfortable care, for example, with reduced and/or minimized rain out in the inspiratory tube and/or patient interface while still receiving adequately humidified gases in spite of different supply gas characteristics. The control system may be able to automatically adjust operating parameters based on an inference of whether the supply gas is dry or ambient. The operating parameters may include certain temperature set points. Additionally or alternatively, the operating parameters may be a dew point, humidity output of the humidifier, or other suitable parameter. Additionally, high flow therapy may generate a flushing effect in the nasopharynx such that the anatomical dead space of the upper airways is flushed by the high incoming gas flows. This creates a reservoir of fresh gas available for each and every breath, while minimising re-breathing of carbon dioxide, nitrogen, etc.

The patient interface may be a non-sealing interface to prevent barotrauma (e.g. tissue damage to the lungs or other organs of the respiratory system due to difference in pressure relative to the atmosphere). The patient interface may be a nasal cannula with a manifold and nasal prongs, and/or a face mask, and/or a nasal pillows mask, and/or a nasal mask, and/or a tracheostomy interface, or any other suitable type of patient interface.

There have been described and illustrated herein several embodiments of a humidification chamber. While particular embodiments have been described, it is not intended that the disclosure be limited thereto, as it is intended that the disclosure be as broad in scope as the art will allow and that the specification be read likewise. Thus, while particular types of inlet configurations/arrangements, outlet configurations/arrangements, heater plate, heating elements, number, size, shape, for the humidification chambers have been disclosed, it will be appreciated that any suitable combination of these features may be used to provide a humidification chamber. In addition, while particular types of configurations/arrangements, heater plate, heating elements, number, size, shape, for the humidification chambers etc. have been disclosed, it will be understood that other types can be used.

In any of the embodiments described herein, the shape of the heater for a humidifier is not limited. The flexible heating element can be formed into any shape, including flat, folded, or curved, according to the need for a particular location and/or conditions. The micro-channels may also be surface structures that can influence or direct the flow of gas/air and water/liquid. The directions of the channels and structures are not limited, and can be designed according to any particular needs. The patterns and layout of the channels and structures are also not limited.

As disclosed elsewhere herein, in some implementations, the bonding layers as disclosed herein prevent or delay delamination of thermoplastic from substrates. In some embodiments, the lifetime of a molded material as disclosed herein (relative to a conventional molded material not comprising a silicon-containing linker) is improved and/or lengthened by equal to or greater than about: 1.25 times, 1.5 times, 1.75 times, 2 times, 5 times, 10 times, 20 times, 40 times, or ranges including and/or spanning the aforementioned values. Peel strength can also be used as a measure to demonstrate the improved properties of embodiments of the molded materials disclosed herein relative to conventional molded materials (that lack bonding layers comprising a silicon-containing linker). In some embodiments, using a Instron peel strength apparatus and a 90° peel angle at 50 mm/min, a molded member, as disclosed herein, has a peel strength that is improved (relative to a coinciding conventional plasma treated control not comprising a silicon-containing linker and/or a control that is not plasma treated and lacks a silicon-containing linker) by equal to or greater than about: 2.5N/mm, 5N/mm, 10N/mm, 20N/mm, 40N/mm, 60N/mm, 80N/mm, 100N/mm, 150N/mm, or ranges including and/or spanning the aforementioned values. In some embodiments, using these peel testing parameters, a molded member as disclosed herein has a peel strength equal to or greater than about: 2.5N/mm, 5N/mm, 10N/mm, 20N/mm, 40N/mm, 60N/mm, 70N/mm, 100N/mm, 150N/mm, 200N/mm, 250N/mm, or ranges including and/or spanning the aforementioned values. In some embodiments, using these peel testing parameters, a molded material as disclosed herein will undergo cohesive failure in addition to or instead of adhesive failure.

For brevity, in several embodiments disclosed herein, the molding member may be disclosed as being one of overmolded or insert molded (and/or as being prepared by either of these methods) member. It should be understood that when one of these terms is used, injection molding more generally is also envisioned because injection molding may be overmolding or insert molding. Thus, even where an embodiment is disclosed as being overmolded, those embodiments may more generally be injection molded. Also, where an embodiment is disclosed as being overmolded, those embodiments may be insert molded. Also for brevity, in several embodiments disclosed herein, reference is made to a respiratory device when discussing, for example, housings for filters, gas conduits, gas flow chambers, chambers, tube connectors, tube joints, tube elbows, heating elements, PCBs, water dosing components, humidification chambers, etc. However, it should be understood that the description of any one of these parts, components, and combinations thereof is also applicable to apparatuses and components that deliver surgical gases (e.g., insufflation apparatuses, etc.). Thus, even where an embodiment is disclosed in the context of a respiratory apparatus, those embodiments may also be used for an insufflation device. Likewise, where an embodiment is disclosed in the context of a insufflation apparatus, those embodiments may also be used for an respiratory device.

### EXAMPLES

### Example 1 (Figure 45): silanization of epoxy-containing silane via wet chemistry

A PCB substrate was subjected to oxygen plasma treatment using a low pressure plasma chamber to activate the surface. As the surface oxidized, the reactive species, such as -OH and -COOH were generated on the surface. An epoxy ring-containing silane coupling agent, 3-glycidoxypropyltrimethoxysilane (GPTMS), was anchored to the PCB surface by wet chemistry process.

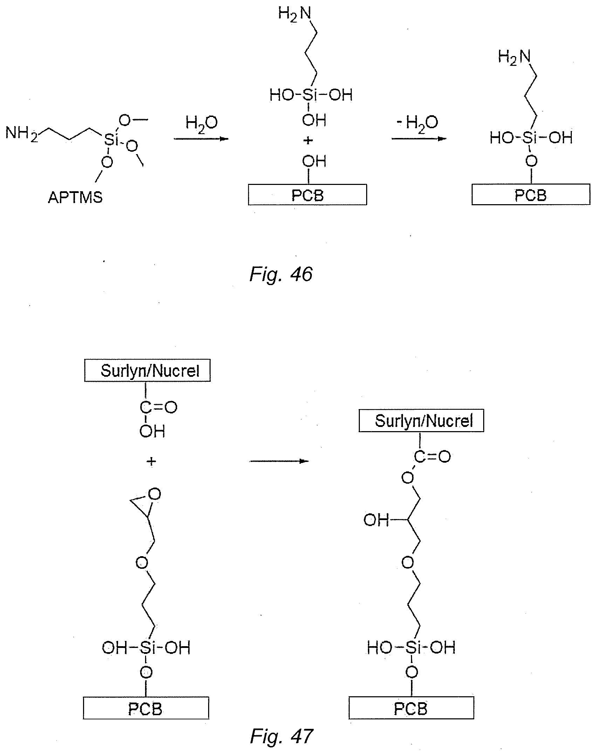

### Example 2 (Figure 46): silanization of amine-containing silane via wet chemistry

An amine-containing silane coupling agent, 3-aminopropyl trimethoxysilane (APTMS), was anchored to the PCB surface by wet chemistry, following activation of PCB surface using oxygen plasma treatment.

### Example 3 (Figure 47): bonding thermoplastic material to the PCB

Thermoplastic polymers, Surlyn^{®} 9020 ionomer and Nucrel^{®} AE, were overmolded onto the PCB substrate. Surlyn^{®} 9020 is an advanced ethylene/methacrylic acid (E/MMA) copolymer in which the MMA groups have been partially neutralized with zinc ions. Nucrel^{®} AE is a terpolymer of ethylene, methacrylic acid, and acrylate. The epoxy ring on the GPTMS on the PCB surface can be opened and crosslinked by the carboxyl group in Surlyn^{®} and Nucrel^{®}.

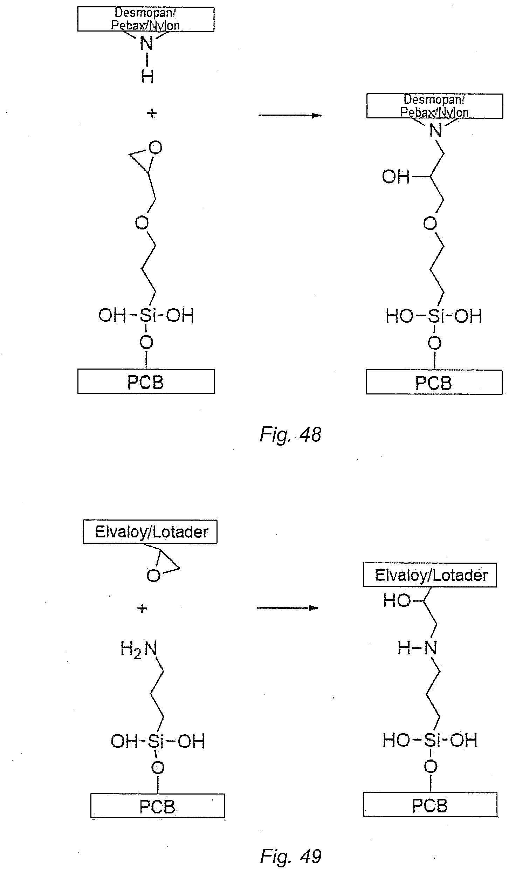

### Example 4 (Figure 48): bonding thermoplastic material to the PCB

Three amine-containing thermoplastic materials were bonded to the silane-modified PCB surface. Desmopan^{®} 9370A, which is a polyurethane, Pebax^{®} MV 1074, which is a block copolymer consisting of polyamide and polyether, and Nylon Ultramid^{®} B3S, which is a polyamide 6, are thermoplastics that contain secondary amine groups. The amine group in Desmopan^{®}, Pebax^{®} and Nylon reacted with the epoxy group on the GPTMS to form a covalent bond.

### Example 5 (Figure 49): bonding thermoplastic material to the PCB

Elvaloy^{®} PTW is an ethylene terpolymer consists of ethylene/butyl acrylate/glycidyl methacrylate terpolymer (E/BA/GMA) and Lotader^{®} AX 8840 is a random copolymer of ethylene and glycidyl methacrylate. The epoxy ring in Elvaloy^{®} and Lotader^{®} can be opened and crosslinked with the primary amine from the APTMS.

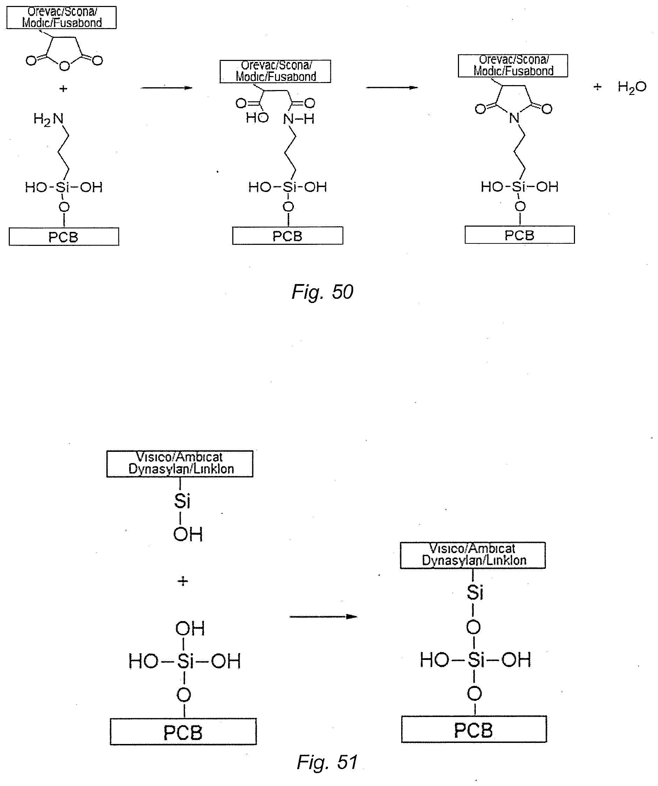

### Example 6 (Figure 50): bonding thermoplastic material to the PCB

Orevac^{®} 18732, Scona^{®} TPPP 9012 PA, Modic^{™} P563, and Fusabond^{®} P613 are propylene functionalized with succinic anhydride. The succinic anhydride in Orevac^{®}, Scona^{®}, Modic^{™} P563, and Fusabond^{®} P613 can be opened and crosslinked with the primary amine from the APTMS.

The combinations of silane coupling agent and the molding material are summarized in the Table 1.

**Table 1.**

| **PCB** | | **Molding material** | |
|---|---|---|---|
| **Silane coupling agent** | **Functional group** | **Thermoplastic** | **Functional group** |
| GPTMS | Epoxy ring | Surlyn^{®} 9020 ionomer | Carboxyl group |
| | | Nucrel^{®} AE | Carboxyl group |
| | | Desmopan^{®} 9370A | Secondary amine |
| | | Pebax^{®} MV 1074 | Secondary amine |
| | | Nylon Ultramid^{®} B3S | Secondary amine |
| APTMS | Primary amine | Elvaloy^{®} PTW | Epoxy ring |
| | | Lotader^{®} AX 8840 | Epoxy ring |
| | | Surlyn^{®} 9020 | Carboxyl group |
| | | ionomer | |
| | | Nucrel^{®} AE | Carboxyl group |
| | | Orevac^{®} 18732 | Succinic anhydride |
| | | Scona^{®} TPPP 9012 PA | Succinic anhydride |
| | | Modic^{™} P563 | Succinic anhydride |
| | | Fusabond^{®} P613 | Succinic anhydride |
| TEPSA | Succinic anhydride | Desmopan^{®} 9370A | Secondary amine |
| | | Pebax^{®} MV 1074 | Secondary amine |
| | | Nylon Ultramid^{®} B3S | Secondary amine |

### Example 7 (Figure 51): bonding thermoplastic material to the PCB

Visico^{™}/Ambicat^{™} system and Dynasylan^{®}, which are crosslinkable polyethylene (PEX), and Linklon^{™}, which is a silane crosslinkable polyolefin based resin, are built on silane copolymer technology. They can crosslink with the PCB modified with tetramethoxysilane (TMOS) or tetraethoxysilane (TEOS). The summary of the combinations of silicon alkoxides and PEX are listed in Table 2.

**Table 2.**

| **PCB** | | **Molding material** | |
|---|---|---|---|
| **Silicon alkoxide** | **Functional group** | **Thermoplastic** | **Functional group** |
| TMOS | Methoxyl | Visico^{™}/Ambicat^{™} Linklon^{™} Dynasylan^{®} | Reactive Silanol |
| TEOS | Ethoxyl | | |

### Example 8: Preparation and testing of molded members

### General silane preparation procedures

The following outlines general methods of preparing reactive coupling silanols from silanes and functionalizing a substrate (e.g., a PCB) with those reactive silanols. As disclosed below, these reactive coupling silanols can be used to prepare substrates functionalized with silane coupling linkers.

Generic ingredients include a silane, deionized water, 5% acetic acid, and methanol or ethanol. In general, for coating a PCB, a 2% silane solution in methanol was prepared. Alternatively, silanes can be prepared based on the intended concentration of linker on the PCB coating, adjusted based on the molecular weight and density of the silane. Water was used to achieve hydrolysis of the silane. A calculation was used to determine a minimum water requirement for the preparation of the reactive silanol for functionalization of each silane to a PCB substrate. The hydrolysis of certain silanes may be slow at neutral pH values and such silanes may benefit from using an acid catalyst to accelerate hydrolysis. Aminosilanes are naturally alkaline and do not require a catalyst to catalyse hydrolysis. The procedures for preparing the reactive silanol was as follows: add specified amount of water, add silane, add enough 5% acetic acid to alter the pH to 4-5 (where a catalyst is desired), and add methanol to achieve a desired amount of volume and concentration. For example, to prepare a 50 mL of solution of silanol from a trialkoxy silane, 3 equivalents of water and 1 equivalent silane was added to a 50 mL volumetric flask. Acetic acid was added to achieve a pH of 4-5. Then, the solution was then diluted to 50 mL using methanol.

### Epoxide-Based Silanes

### (3-glycidoxypropyl)trimethoxysilane, [GPTMS] - Product code: SIG5840.0

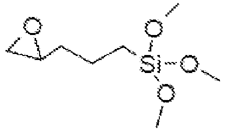

Molecular weight - 236.34 g/mol; Density - 1.07 g/mL; 2% GPTMS = 1 mL in 50 mL solution. 1 mL GPTMS = 1.07 g/mL x 1 = 1.07 g; 1.07 g GPTMS/ 236.34 g/mol = 0.00453 mol; GPTMS:H₂O = 1:3 (trimethoxysilane); minimum amount of water is 0.00453 x 3 = 0.0136 x 18.015 g/mol = 0.245 g.

To prepare a solution for functionalization of a substrate, 0.245 mL of water (e.g., 0.245 g water) was added to a 50 mL volumetric flask. To the water was added, 1 mL of GPTMS. The solution was acidified to pH 4-5 with 5% acetic acid and the resulting solution was diluted to 50 mL with methanol.

### Amine-Based Silanes

### Aminopropylsilsesquioxane in aqueous solution, 25% (WSA-9911) - Product code: WSA-9911

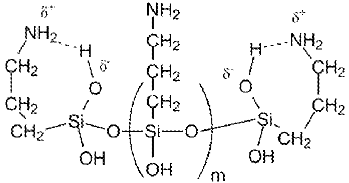

A 4 mL of a 25% concentration of WSA-9911 aqueous solution (e.g., 1 mL of WSA-9911) was added to a 50 mL flask which was diluted to 50 mL with methanol.

### Bis(3-triethoxysilylpopyl)amine, 95% (SIB1824.5) - Product code: SIB1824.5

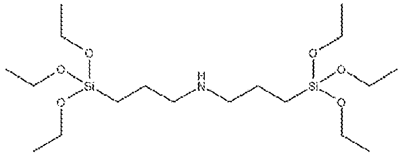

Molecular weight SIB1824.5 - 425.71 g/mol; Density - 0.97 g/mL; 2% SIB1824.5 = 1 mL in 50 mL solution; 1 mL SIB1824.5 x 0.97 g/mL = 0.97 g; 0.97 g of SIB1824.5/ 425.71 g/mol = 0.00228 mol; SIB1824.5:H₂O = 1:6 (dipodal); minimum amount of water is 0.00228 x 6 = 0.0137 x 18.015 g/mol = 0.247 g.

To prepare a solution for functionalization of a substrate, 0.247 mL of water was added to a 50 mL volumetric flask. To the water was added, 1 mL of SIB1824.5. The solution was acidified to pH 4-5 with 5% acetic acid and the resulting solution was diluted to 50 mL with methanol.

### Bis(3-trimethoxysilylpropyl)amine, 96% (SIB1833.0) - Product code: SIB1833.0

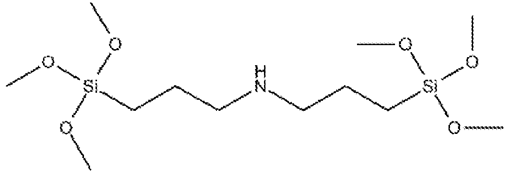

Molecular weight - 341.56 g/mol; Density - 1.04 g/mL; 2% SIB1833.0 = 1 mL in 50 mL solution; 1 mL SIB 1833.0 x 1.04 g/mL = 1.04g; 1.04 g of SIB1833.0/ 341.56 g/mol = 0.00304 mol; SIB1833.0:H2O = 1:6 (dipodal); minimum amount of water is 0.00304 x 6 = 0.0182 x 18.015 g/mol = 0.328 g.

To prepare a solution for functionalization of a substrate, 0.328 mL of water was added to a 50 mL volumetric flask. To the water was added, 1 mL of SIB1833.0. The resulting solution was diluted to 50 mL with methanol.

### Succinic Anhydride Silanes

### (3-triethoxysilyl)propylsuccinic anhydride, 95% (TEPSA) - Product code: SIT8192.6

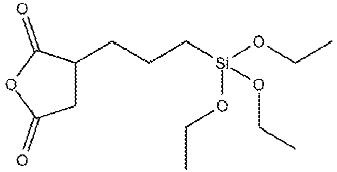

Molecular weight - 304.41 g/mol; Density - 1.07 g/mL; 2% TEPSA = 1 mL in 50 mL solution; 1 mL TEPSA x 1.07 g/mL = 1.07 g; 1.07 g of TEPSA/ 304.41 g/mol = 0.00351 mol; TEPSA:H₂O = 1:3 (trialkoxysilane); minimum amount of water is 0.00351 x 3 = 0.0105 x 18.015 g/mol = 0.190 g.

To prepare a solution for functionalization of a substrate, 0.190 mL of water was added to a 50 mL volumetric flask. To the water was added, 1 mL of TEPSA. The solution was acidified to pH 4-5 with 5% acetic acid and the resulting solution was diluted to 50 mL with methanol.

### TEOS/ TMOS

### Tetraethoxysilane, 98% (TEOS) - Product code: SIT7110.0

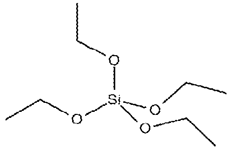

Molecular weight - 208.33 g/mol; Density - 0.9335 g/mL; 2% TEOS = 1 mL in 50 mL solution; 1 mL TEOS x 0.9335 g/mL = 0.9335 g; 0.9335 g of TEOS/ 208.33 g/mol = 0.00448 mol; TEOS:H₂O = 1:4 (tetraalkoxysilane); minimum amount of water is 0.00448 x 4 = 0.0179 x 18.015 g/mL = 0.323 g.

To prepare a solution for functionalization of a substrate, 0.323 mL of water was added to a 50 mL volumetric flask. To the water was added, 1 mL of TEOS. The solution was acidified to pH 4-5 with 5% acetic acid and the resulting solution was diluted to 50 mL with methanol.

### Tetramethoxysilane, 98% (TMOS) - Product code: SIT7510.0

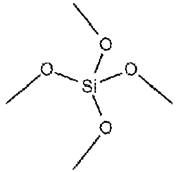

Molecular weight - 152.22 g/mol; Density - 1.032 g/mL; 2% TMOS = 1 mL in 50 mL solution; 1 mL TMOS x 1.032 g/mL = 1.032 g; 1.032 g of TMOS/ 152.22 g/mol = 0.00678 mol; TMOS:H₂O = 1:4 (tetralkoxysilane); minimum amount of water is 0.00678 x 4 = 0.0271 x 18.015 g/mL = 0.488 g.

To prepare a solution for functionalization of a substrate, 0.488 mL of water was added to a 50 mL volumetric flask. To the water was added, 1 mL of TMOS. The solution was acidified to pH 4-5 with 5% acetic acid and the resulting solution was diluted to 50 mL with methanol.

### Plasma Treatment of Substrate

For preparing plasma treated substrates (e.g., PCBs), the following general procedures were performed. The PCB (or other substrate) is plasma treated using an Plasmaetch PE-25 instrument. The following conditions were used: Oxygen pressure: 20 PSI from the oxygen tank regulator; treatment time: 15 mins; power level: high; oxygen flow rate: ~zero; Vacuum pressure: 110 mtorr.

### Functionalization of Substrate with Silanols

To prepare a substrate coated with silane, the following procedures were used. The plasma treated PCBs are dipped in the silane solution. At that time, the silane coated PCBs are processed to remove excess silanes. The PCBs are then cured.

### Overmolding of the Substrate

The following procedures were performed using an Arburg Thermoplastic Moulder 375. A mold is placed over the substrate. A molding cycle is then performed wherein the molding material is distributed into the mold over a portion of the PCB. Depending on the sample, the PCB may remain connected to power until injection phase is over (an attempt to prevent premature solidification and to allow ample time for any bonding reaction to occur).

### Peel Testing

The following procedures were performed using an Instron device. A jig was attached to the base of the Instron device. This jig holds the PCB and overmold in the same place relative to a jaw clamp on the cross member. The test sample was secured onto the jig. The jaw clamp was attached to a 'peel tab' of the molding material of the molded member. A pull was performed at 50 mm/min until failure. The maximum peel force was recorded. A peel test at 90° was performed, noting the initial force required to initial peeling. Each molded member (e.g., the molding material on the PCB) tested was 11 mm wide at the location where peel test was initiated. The initial peel force was measured as N/mm (calculated by dividing the initial peel force by 11 mm). This test therefore was performed to determine the peak initial force required to start peeling the overmold which is always perpendicular to the PCB surface.

### Example 9: Results Using Amine / Succinic Anhydride-Based Bonding Layers

### Sample Preparation

Two embodiments of molded members were prepared using the procedures as described generally in Example 8. Briefly, for the first experimental sample, a thermoplastic polypropylene/Scona molding material (having a succinic anhydride functionality) was overmolded on a functionalized PCB having a primary amine silane coupling agent (e.g., functionalized with WSA-9911) to form a molded member having bonding layer comprising a silicon containing linker. For the second experimental sample, a thermoplastic polypropylene/Scona molding material (having a succinic anhydride functionality) was overmolded on a functionalized PCB having a secondary amine silane coupling agent (e.g., functionalized with SIB 1824.5) to form a molded member having bonding layer comprising a silicon containing linker.

In addition to the first and second experimental samples, control samples were prepared. The first control sample was prepared by overmolding polypropylene/Scona on a PCB that had not been plasma treated or treated with a silane coupling agent. The second control sample was prepared by overmolding polypropylene/Scona on a PCB that had been plasma treated, but that had not been treated with a silane coupling agent.

The PCB used in each experiment disclosed herein was FR-4 IT158TC. This is a medium Tg (>150°C by DSC) multifunctional filled epoxy PCB with high thermal reliability.

### Sample Testing

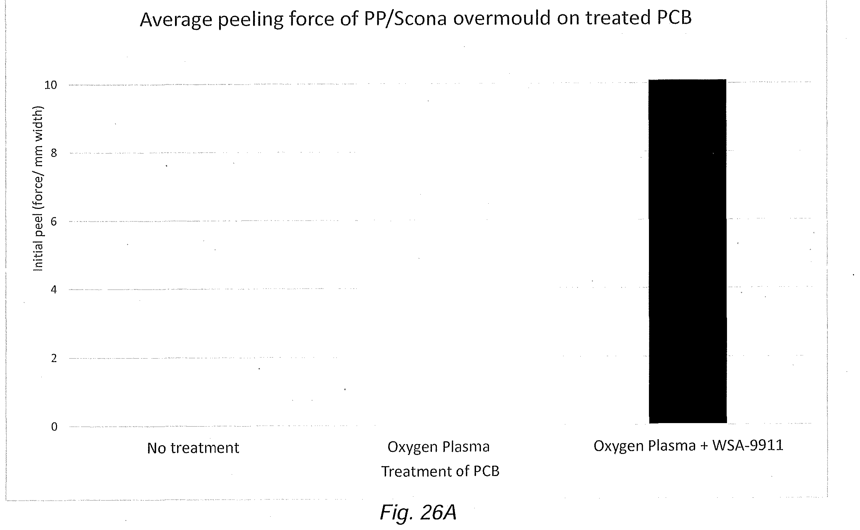

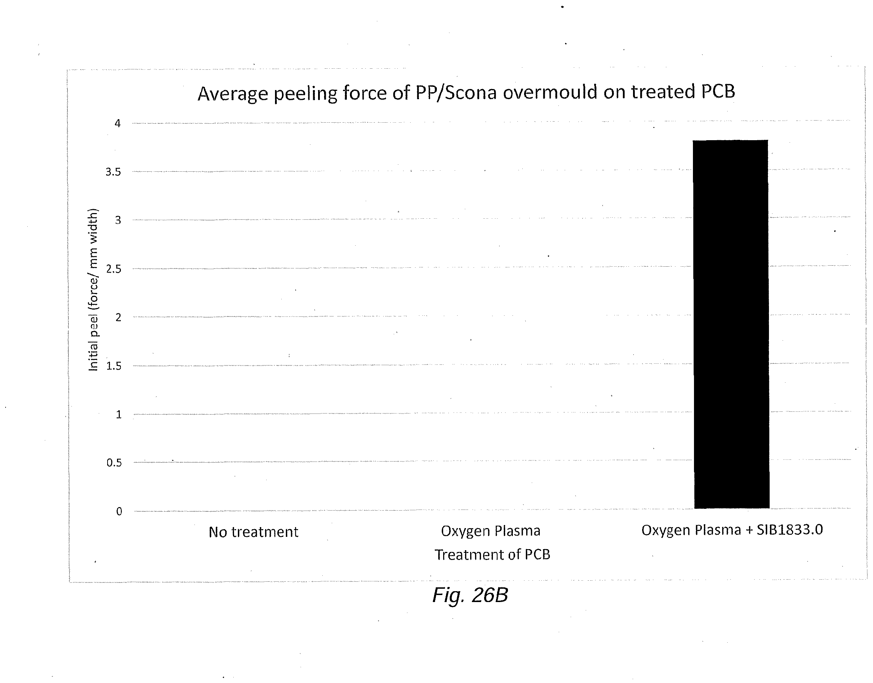

Peel testing was performed in triplicate using the procedures described generally in Example 8. Table 3 below and Figure 26A show the results for the WSA-9911 experimental sample versus controls. Table 4 below and Figure 26B show the results for the SIB1833.0 experimental sample versus controls. Figures 26A and 26B provide bar graphs showing a comparison of the average initial peel force for the experimental versus control samples.

**Table 3. Peel data for WSA-9911/Scona experimental samples versus controls that were either untreated or only subject to plasma treatment. Peel force is expressed as Force / mm width (N/mm).**

| PCB treatment | Overmold Material | Sample 1 | Sample 2 | Sample 3 | Average (N/mm) |
|---|---|---|---|---|---|
| None | Polypropylene/ Scona | 0 | 0 | 0 | 0 |
| Oxygen Plasma | Polypropylene/ Scona | 0 | 0 | 0 | 0 |
| Oxygen Plasma + WSA-9911 | Polypropylene/ Scona | 12.3 | 11.1 | 6.9 | 10.1 |

**Table 4. Peel data for SIB1833.0/Scona experimental samples versus controls that were either untreated or only subject to plasma treatment. Peel force is expressed as Force / mm width (N/mm).**

| PCB treatment | Overmold Material | Sample 1 | Sample 2 | Sample 3 | Average (N/mm) |
|---|---|---|---|---|---|
| None | Polypropylene/ Scona | 0 | 0 | 0 | 0 |
| Oxygen Plasma | Polypropylene/ Scona | 0 | 0 | 0 | 0 |
| Oxygen Plasma + SIB1833.0 | Polypropylene/ Scona | 4.01 | 2.04 | 5.36 | 3.80 |

### Results and Discussion

### WSA-9911

As shown in Table 3 and Figure 26A, for the controls, there was no adhesion after overmolding for both non-treated and plasma treated PCBs prepared without a silane coupling agent. It appeared that polypropylene, even with 5% Scona, did not provide any intermolecular attractions with the non-treated and plasma treated PCBs, resulting in zero peeling force during the peel test. The WSA-9911 treated PCB boards had a greater average peeling force of 10.1N/mm based on the three experimental samples. Thus, the silane treated sample had an adhesion that was, on average about 10N/mm higher than the non-silane treated samples. This result suggests that the silane coupling agent treatment provided improved and/or increased chemical interactions that enhanced the coupling of the WSA-9911 surface treated PCB boards with the PP/Scona overmold. Such chemical interactions could include the formation of covalent bonds between the primary amine of the silane with the maleic anhydride of the scona (as shown below).

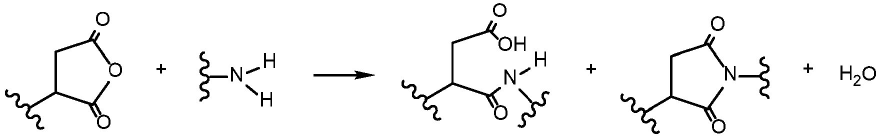

Other types of bonding and forces could have also contributed to the increase in the peeling forces observed. Scona is typically not used as a molding material. Scona is normally used in composite materials in which the succinic anhydride reacts with a functional group in the reinforcing material, for example, cellulose based fiber. However, it was surprisingly found that it provided good bonding with a bonding layer comprising a silicon-containing linker. Scona also has beneficial material properties, such as, a high heat distortion temperature (also known as heat deflection temperature) and is impermeable to water (which would help prevent or inhibit delamination).

### SIB1824.5

As shown in Table 4 and Figure 26B, and as mentioned above, for the controls, there was no adhesion after overmolding for both non-treated and plasma treated PCBs prepared without a silane coupling agent. It appeared that polypropylene even with 5% Scona did not provide any intermolecular attractions with the non-treated and plasma treated PCBs, resulting in zero peeling force during the peel test. The SIB1833.0 treated PCB boards had a greater average peeling force of 3.80N/mm based on the three experimental samples. It is believed that (as shown above) this initial peel force can be improved even further (up to or at least about 10 N greater than that for the non-silane treated materials) through additional fine-tuning of the experimental overmolding conditions and peel testing conditions. Thus, the silane treated sample had an adhesion that was, on average about 4N/mm higher than the non-silane treated samples. This suggests that the silane coupling agent treatment provided improved and/or increased chemical interactions that enhanced the coupling of the SIB1833.0 surface treated PCB boards with the PP/Scona overmold. Such chemical interactions could include the formation of covalent bonds between the secondary amine of the silane with the succinic anhydride of the scona. Other types of bonding and forces could have also contributed to the increase in the peeling forces observed.

### Example 10: Results Using Amine / Epoxide-Based Bonding Layers

### Sample Preparation

Two embodiments of molded members were prepared using the procedures as described generally in Example 8. Briefly, for the first experimental sample, a thermoplastic Elvaloy PTW (ethylene-butyl acrylate-glycidyl methacrylate terpolymer) molding material (having an epoxy functionality) was overmolded on a functionalized PCB having a primary amine silane coupling agent (e.g., functionalized with WSA-9911) to form a molded member having bonding layer comprising a silicon containing linker. For the second experimental sample, a thermoplastic Elvaloy PTW (ethylene-butyl acrylate-glycidyl methacrylate terpolymer) molding material (having an epoxy functionality) was overmolded on a functionalized PCB having a secondary amine silane coupling agent (e.g., functionalized with SIB 1833.0) to form a molded member having bonding layer comprising a silicon containing linker.

In addition to the first and second experimental samples, control samples were prepared. The first control sample was prepared by overmolding Elvaloy PTW on a PCB that had not been plasma treated or treated with a silane coupling agent. The second control sample was prepared by overmolding Elvaloy PTW on a PCB that had been plasma treated, but that had not been treated with a silane coupling agent. The PCB was identical to that used in Example 9.

### Sample Testing

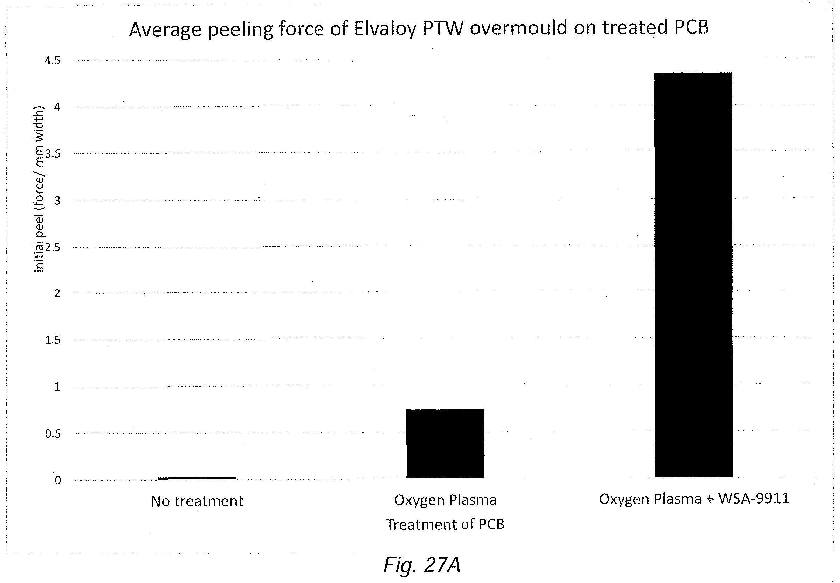

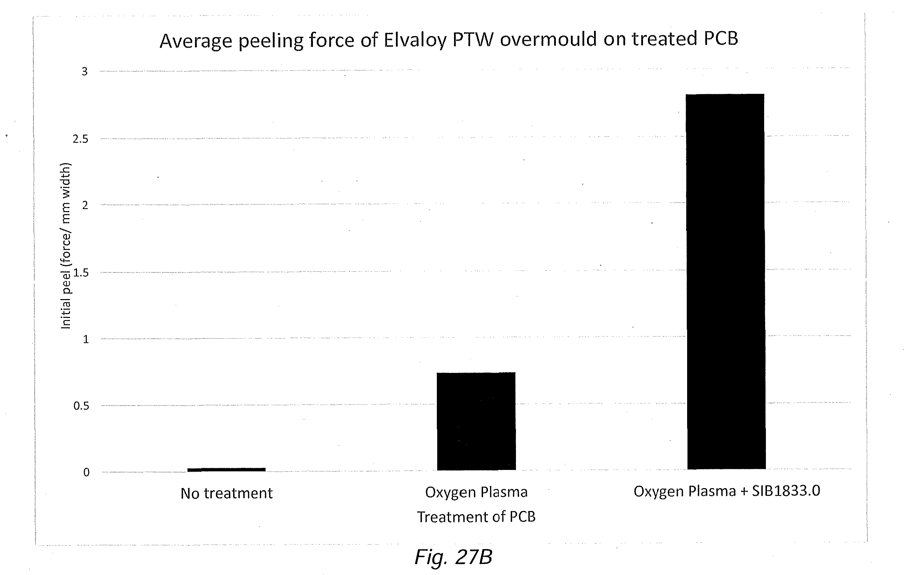

Peel testing was performed in triplicate using the procedures described generally in Example 8. Table 5 below and Figure 27A provide the results for the WSA-9911 experimental sample versus controls. Table 6 below and Figure 27B show the results for the SIB1833.0 experimental sample versus controls. Figures 27A and 27B provide bar graphs showing a comparison of the average initial peel force for the experimental versus control samples.

**Table 5. Peel data for WSA-9911/Elvaloy PTW experimental samples versus controls that were either untreated or only subject to plasma treatment. Peel force is expressed as Force / mm width (N/mm).**

| PCB treatment | Overmold Material | Sample 1 | Sample 2 | Sample 3 | Average |
|---|---|---|---|---|---|
| None | Elvaloy PTW | 0.03 | 0.03 | 0.03 | 0.03 |
| Oxygen Plasma | Elvaloy PTW | 0.79 | 0.66 | 0.77 | 0.74 |
| Oxygen Plasma + WSA-9911 | Elvaloy PTW | 4.77 | 4.23 | 4.02 | 4.34 |

**Table 6. Peel data for SIB1833.0/Elvaloy PTW experimental samples versus controls that were either untreated or only subject to plasma treatment. Peel force is expressed as Force / mm width (N/mm).**

| PCB treatment | Overmold Material | Sample 1 | Sample 2 | Sample 3 | Average (N/mm) |
|---|---|---|---|---|---|
| None | Elvaloy PTW | 0.03 | 0.03 | 0.03 | 0.03 |
| Oxygen Plasma | Elvaloy PTW | 0.79 | 0.66 | 0.77 | 0.74 |
| Oxygen Plasma + SIB1833.0 | Elvaloy PTW | 2.73 | 2.60 | 3.10 | 2.81 |

### Results and Discussion

### WSA-9911

As shown in Table 5 and Figure 27A, the peeling forces between the overmold and non-treated / plasma treated-only PCB is lower than the silane treated sample. The initial peeling force for the experimental samples was, on average, about 3.6N/mm higher than for the plasma treated / non-silane treated controls and about 4.3N/mm higher than the non-treated controls. It is believed that (as shown above) this initial peel force can be improved even further (up to or at least about 10 N/mm greater than that for the non-silane treated materials) through additional fine-tuning of the experimental overmolding conditions and peel testing conditions. The observed improved adhesion could be due to increased and/or improved chemical interactions between the silane layer and the thermoplastic/PCB in the silane-treated sample. This result suggests that the silane coupling agent treatment provided improved and/or increased chemical interactions that enhanced the coupling of the WSA-9911 surface treated PCB boards with the Elvaloy overmold. Such chemical interactions could include the formation of covalent bonds between the primary amine of the silane with the epoxy of the Elvaloy (as shown in figure 49).

Other types of bonding and forces could have also contributed to the increase in the peeling forces observed.

### SIB1833

As shown in Table 6 and Figure 27B, and as mentioned above, the peeling forces between the overmold and non-treated / plasma treated-only PCB is lower than the silane treated sample. The initial peeling force for the experimental samples was, on average, about 2.1N/mm higher than for the plasma treated / non-silane treated controls and about 2.8N/mm higher than the non-treated controls. It is believed that (as shown above) this initial peel force can be improved even further (up to or at least about 10 N/mm greater than that for the non-silane treated materials) through additional fine-tuning of the experimental overmolding conditions and peel testing conditions. The higher peel force for the silane treated sample suggests that the silane treatment provided improved and/or increased chemical interactions that enhanced the coupling of the SIB1833.0 surface treated PCB boards and Elvaloy thermoplastic. Such chemical interactions could include the formation of covalent bonds between the secondary amine of the silane with the epoxy of the Elvaloy. Other types of bonding and forces could have also contributed to the increase in the peeling forces observed.

### Example 11: Results Using Silanol-Based Bonding Layers

### Sample Preparation

Two embodiments of molded members were prepared using the procedures as described generally in Example 8. Briefly, for the first experimental sample, a PEX thermoplastic (trimethoxysilyl modified polyethylene) molding material (having an silanol functionality) was overmolded on a functionalized PCB having a TEOS-based reactive silanol silane coupling agent to form a molded member having bonding layer comprising a silicon-containing linker. For the second experimental sample, a PEX thermoplastic (trimethoxysilyl modified polyethylene) molding material (having an silanol functionality) was overmolded on a functionalized PCB having a TMOS-based reactive silanol silane coupling agent to form a molded member having bonding layer comprising a silicon-containing linker.

In addition to the first and second experimental samples, control samples were prepared. The first control sample was prepared by overmolding PEX on a PCB that had not been plasma treated or treated with a silane coupling agent. The second control sample was prepared by overmolding PEX on a PCB that had been plasma treated, but that had not been treated with a silane coupling agent. The PCB was identical to that used in Example 9.

### Sample Testing

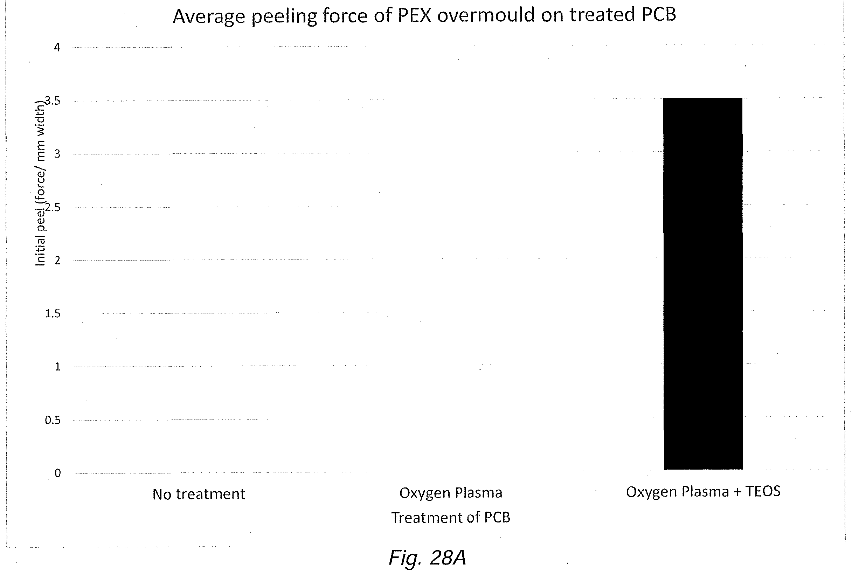

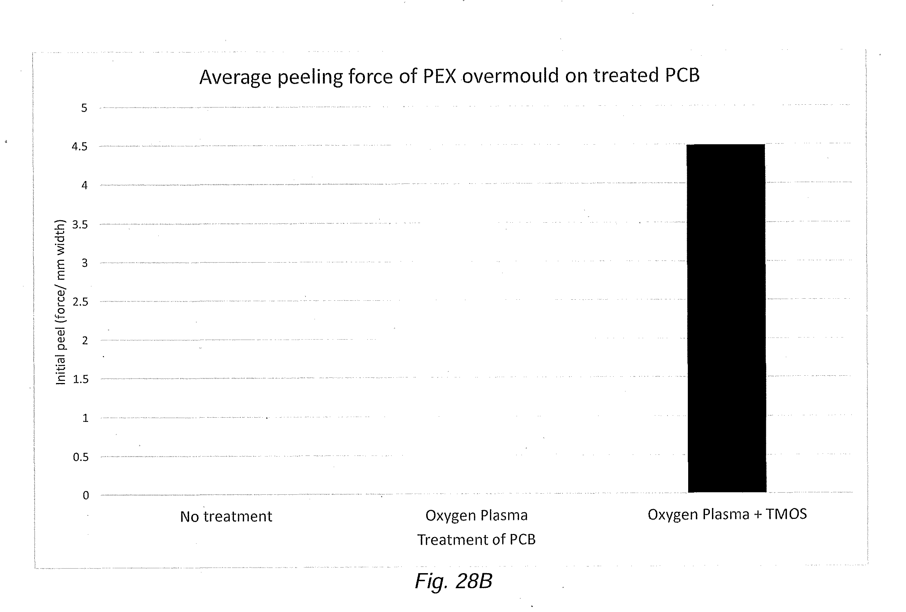

Peel testing was performed in triplicate using the procedures described generally in Example 8. Table 7 below and Figure 28A provide the results for the TEOS experimental sample versus controls. Table 8 below and Figure 28B show the results for the TMOS experimental sample versus controls. Figures 28A and 28B provide bar graphs showing a comparison of the average initial peel force for the experimental versus control samples.

**Table 7. Peel data for TEOS/PEX experimental samples versus controls that were either untreated or only subject to plasma treatment. Peel force is expressed as Force / mm width (N/mm).**

| PCB treatment | Overmold Material | Sample 1 | Sample 2 | Sample 3 | Average (N/mm) |
|---|---|---|---|---|---|
| None | PEX | 0.00 | 0.00 | 0.00 | 0.00 |
| Oxygen Plasma | PEX | 0.00 | 0.00 | 0.00 | 0.00 |
| Oxygen Plasma + TEOS | PEX | 5.73 | 3.91 | 0.86 | 3.50 |

**Table 8. Peel data for TMOS/PEX experimental samples versus controls that were either untreated or only subject to plasma treatment. Peel force is expressed as Force / mm width (N/mm).**

| PCB treatment | Overmold Material | Sample 1 | Sample 2 | Sample 3 | Average (N/mm) |
|---|---|---|---|---|---|
| None | PEX | 0.00 | 0.00 | 0.00 | 0.00 |
| Oxygen Plasma | PEX | 0.00 | 0.00 | 0.00 | 0.00 |
| Oxygen Plasma + TMOS | PEX | 6.52 | 5.14 | 1.82 | 4.49 |

### Results and Discussion

### TEOS

As shown in Table 7 and Figure 28A, the peeling forces between the overmold and non-treated / plasma treated-only PCB is lower than the silane treated sample. No adhesion was observed in the initial peel tests between the non-treated and plasma treated PCBs and the overmold after overmolding. The initial peeling force for the experimental samples was, on average, about 3.5N/mm higher than for the non-silane treated controls. It is believed that (as shown above) this initial peel force can be improved even further (up to about 10 N/mm greater than that for the non-silane treated materials) through additional fine-tuning of the experimental overmolding conditions and peel testing conditions. This improved adhesion could be due to increased and/or improved chemical interactions between the silane layer and the thermoplastic/PCB in the silane-treated sample. This result suggests that the silane coupling agent treatment provided improved and/or increased chemical interactions that enhanced the coupling of the TEOS surface treated PCB boards with the PEX overmold. Such chemical interactions could include the crosslinking of hydroxyl groups of the TEOS with the side chains of the PEX (as shown in figure 51).

Other types of bonding and forces could have also contributed to the increase in the peeling forces observed.

### TMOS

As shown in Table 8 and Figure 28B, and as mentioned above, the peeling forces between the overmold and non-treated / plasma treated-only PCB is lower than the silane treated sample. No adhesion was observed in the initial peel tests between the nontreated and plasma treated PCBs and the overmold after overmolding. The initial peeling force for the experimental samples was, on average, about 4.5N/mm higher than for the non-silane treated controls. It is believed that (as shown above) this initial peel force can be improved even further (up to or at least about 10 N/mm greater than that for the non-silane treated materials) through additional fine-tuning of the experimental overmolding conditions and peel testing conditions. The higher peel force for the silane treated sample suggests that the silane coupling agent treatment provided improved and/or increased chemical interactions that enhanced the coupling of the TMOS surface treated PCB boards with the PEX overmold. Such chemical interactions could include the crosslinking of hydroxyl groups of the TMOS with the side chains of the PEX Other types of bonding and forces could have also contributed to the increase in the peeling forces observed.

### Example 12: Additional Results Using Amines and Succinic Anhydrides or Epoxides

### Sample Preparation

Two embodiments of molded members were prepared using the procedures as described generally in Example 8. Briefly, for the first experimental sample, a Desmopan 9370A thermoplastic (thermoplastic aromatic polyether-based polyurethane) molding material (having an secondary amine functionality) was overmolded on a functionalized PCB having a succinic anhydride-based reactive silane coupling agent (TEPSA) to form a molded member having bonding layer comprising a silicon-containing linker. For the second experimental sample, a Desmopan 9370A thermoplastic molding material was overmolded on a functionalized PCB having an epoxy ring (epoxide)-based reactive silane coupling agent GPTMS to form a molded member having bonding layer comprising a silicon-containing linker.

In addition to the first and second experimental samples, control samples were prepared. The first control sample was prepared by overmolding Desmopan 9370A on a PCB that had not been plasma treated or treated with a silane coupling agent. The second control sample was prepared by overmolding Desmopan 9370A on a PCB that had been plasma treated, but that had not been treated with a silane coupling agent. The PCB was identical to that used in Example 9.

### Sample Testing

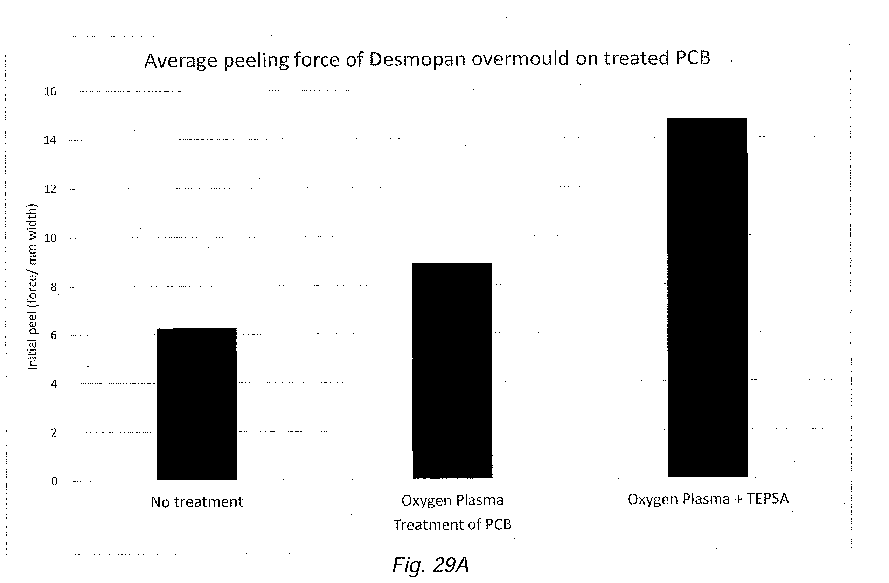

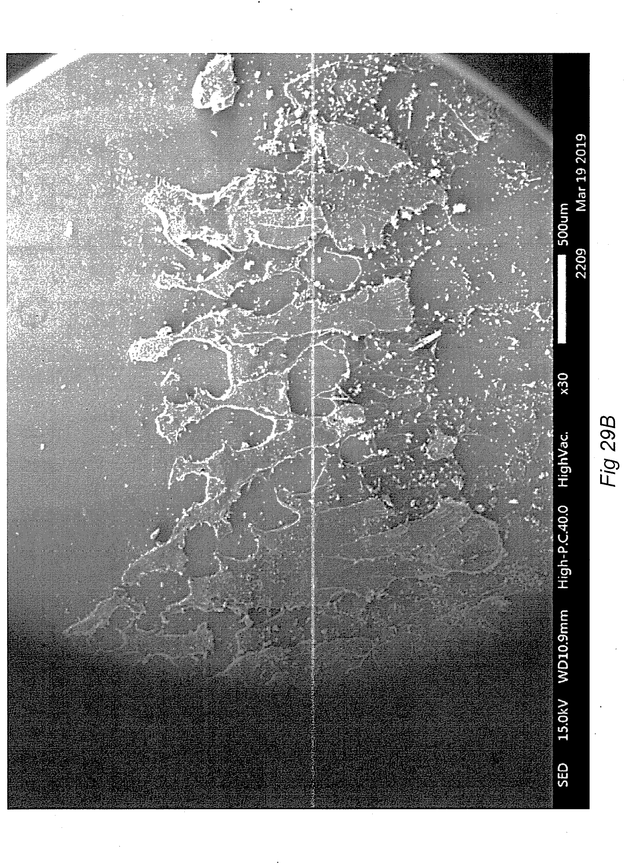

Peel testing was performed in triplicate using the procedures described generally in Example 8. Table 9 below and Figure 29A provide the results for the succinic anhydride experimental sample versus controls. Figure 29B shows the SEM results for the epoxide experimental sample.

**Table 9. Peel data for TEPSA/Desmopan 9370A experimental samples versus controls that were either untreated or only subject to plasma treatment. Peel force is expressed as Force / mm width (N/mm).**

| PCB treatment | Overmold Material | Sample 1 | Sample 2 | Sample 3 | Average (N/mm) |
|---|---|---|---|---|---|
| None | Desmopan 9370A | 6.03 | 5.64 | 7.08 | 6.25 |
| Oxygen Plasma | Desmopan 9370A | 9.09 | 8.81 | 8.76 | 8.88 |
| Oxygen Plasma + TEPSA | Desmopan 9370A | 13.8 | 15.6 | 14.8 | 14.8 |

### Results and Discussion

### TEPSA

As shown in Table 9 and Figure 29A, the peeling forces between the overmold and non-treated / plasma treated-only PCB is lower than the silane treated sample. The initial peeling force for the experimental samples was, on average, about 5N/mm higher than for the plasma treated / non-silane treated controls, and about 8.6N/mm higher than for non-treated controls. It is believed that (as shown above) this initial peel force can be improved even further (up to about 10 N/mm greater than that for the non-silane treated materials) through additional fine-tuning of the experimental overmolding conditions and peel testing conditions. The observed improved adhesion could be due to increased and/or improved chemical interactions between the silane layer and the thermoplastic/PCB in the silane-treated sample. This result suggests that the silane coupling agent treatment provided improved and/or increased chemical interactions that enhanced the coupling of the TEPSA surface treated PCB boards with the Desmopan overmold. Other types of bonding and forces could have also contributed to the increase in the peeling forces observed.

### GPTMS

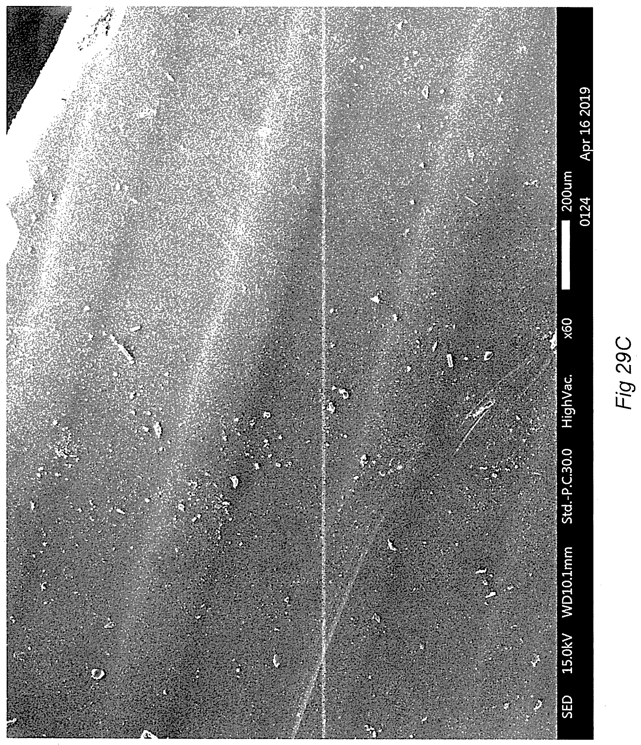

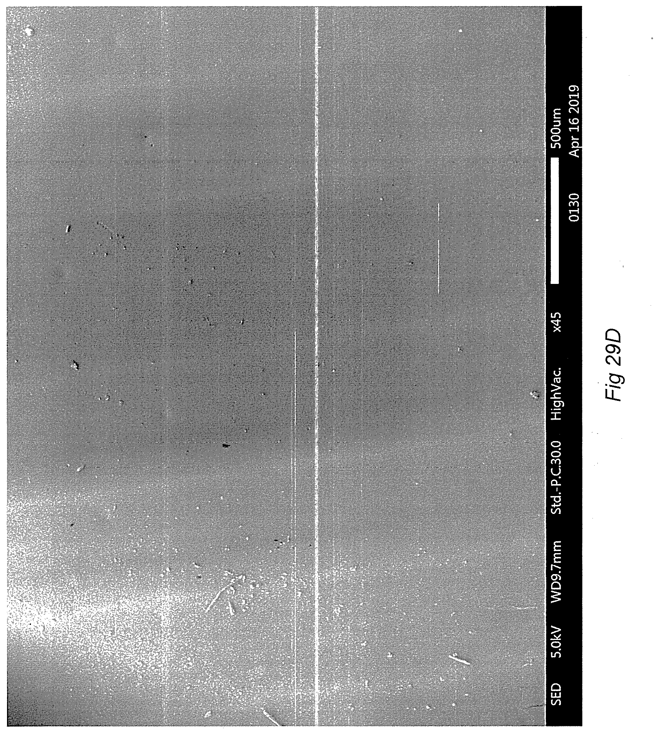

It was observed during testing that the GPTMS sample had a lower peel force compared to plasma-only treated samples but higher than the non-treated samples. It is believed that the sample preparation was not optimized and therefore the peel force data here was not reliable. For instance, as shown in Figure 29B, it was observed in scanning electron microscope (SEM) data that, for the experimental sample, Desmopan was adhered so strongly that portions of it actually tore away from the bulk thermoplastic overmolded material during peel testing and residue of the Desmopan remained adhered to the PCB after the peel test. This same effect was not observed in either of the control samples, which are shown in Figure 29C (plasma-only) and Figure 29D (non-treated).

This thin layer of Desmopan suggests that the thermoplastic elastomer material itself failed prior to the failure of the bonding layer. The tearing appears to be a cohesive failure (e.g., failure of the bulk material to withstand the peel test rather than the layers adhered via the bonding layer). When examining the silane PCB treated sample, it suggests that certain regions of the Desmopan are adhered strongly to the PCB by the silane layer, which in turn suggests that the interactions between the silane layer and the Desmopan/PCB are stronger than the internal cohesive forces of the Desmopan material itself such that parts of Desmopan break off and/or tear and remain adhered to the PCB during the peel test. This testing additionally suggests that the silane treatment provided certain chemical interactions that enhanced the coupling of the GPTMS surface treated PCB boards with the Desmopan. Such chemical interactions could include covalent bonding resulting from the epoxy of GPTMS with the secondary amine of Desmopan. Additionally, a combination of other chemical interactions and forces may have contributed to a cohesive failure. In conclusion, the adhesion in the bonding layer appeared to be so strong that it caused strain and ripping of the bulk material of the Desmopan instead of the bonding layer. This result indicates that the layers may resist delamination under humidification conditions, where the molded member is not subject to any peel forces.

As noted above, it was observed under the SEM, both non-treated and plasma treated PCBs left no Desmopan residue adhered to the surface which identifies as an adhesive failure. The adhesive failure of the plasma treated sample suggests that only weak bonding was present, therefore no residues were left on the surface under the SEM image. Thus, the bonding layer with the silicon-containing linker improved the adhesion of the layers relative to control samples without such a bonding layer. These results also may be indicative of a peel test that has not been optimized for this particular combination. In this case, it is believed that the SEM provides a more accurate representation of the improved adhesion of the silane layer. It is believed that (as shown above for other amine/epoxide samples) this initial peel force can be improved (up to about 100 N greater than that for the non-silane treated materials) through additional fine-tuning of the experimental overmolding conditions and peel testing conditions.

### Example 13: Results Using Epoxides and Carboxyl Groups

### Sample Preparation

An embodiment of molded members was prepared using the procedures as described generally in Example 8. Briefly, Surlyn 9020 (ethylene/methacrylic acid (E/MAA) copolymer) thermoplastic molding material was overmolded on a functionalized PCB having an epoxy ring (epoxide)-based reactive silane coupling agent (GPTMS) to form a molded member having bonding layer comprising a silicon-containing linker.

In addition to experimental sample, control samples were prepared. The first control sample was prepared by overmolding Surlyn 9020 on a PCB that had not been plasma treated or treated with a silane coupling agent. The second control sample was prepared by overmolding Surlyn 9020 on a PCB that had been plasma treated, but that had not been treated with a silane coupling agent.

### Sample Testing

Peel testing was performed in triplicate using the procedures described generally in Example 8. Figures 30A-30E show ESM data for the testing.

### Results and Discussion

### GPTMS

It was observed that the GPTMS sample had a higher peel for than the non-treated sample but a lower peel force compared to plasma-only treated samples. It is believed that the sample preparation was not optimized and therefore the peel force data here was not reliable. However, as shown in Figure 30A and Figure 30B, it was observed in scanning electron microscope (SEM) data that residues of the PCB mask was torn away from the PCB during the peel testing and adhered to the Surlyn after the peel test. The elemental analysis in Figure 30B supports this by indicating the chemical constituents of the PCB mask that remained adhered to the Surlyn, as shown in Figure 30B.

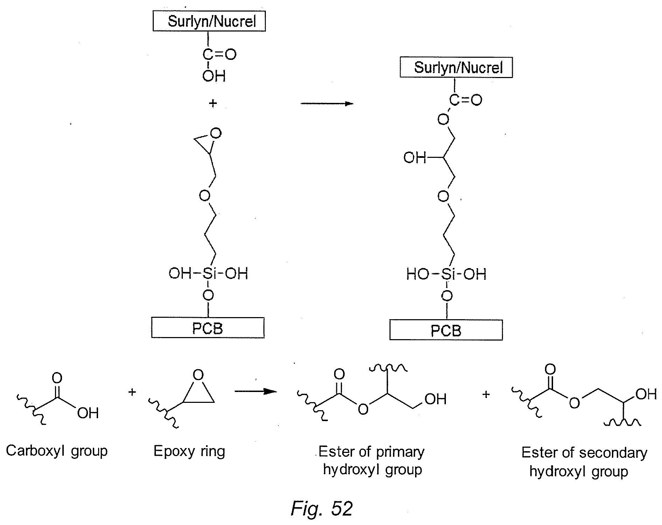

As shown in Figure 30A, this tearing left a layer of PCB on the Surlyn molding material and suggests that the substrate layer failed prior to the failure of the bonding layer. It is also believed that the silane layer contributed to increased adhesion between Surlyn/PCB in at least certain areas of the sample. The cohesive failure of the silane PCB treated sample suggests that certain regions of the Surlyn are adhered strongly to the PCB by the silane layer, which in turn suggests that the interactions between the silane layer and the Surlyn/PCB are stronger than the cohesive forces of the PCB such that parts of PCB break off and/or tear and remain adhere to the PCB during the peel test. This testing additionally suggests that the silane treatment provided certain chemical interactions that enhanced the coupling of the GPTMS surface treated PCB boards with the Surlyn. Such chemical interactions could include covalent bonding resulting from the epoxy of GPTMS with the carboxy group of Surlyn (as shown in figure 52).

Additionally, the combination of chemical interactions and forces may have contributed to a cohesive failure. As noted above, it was observed under the SEM, both non-treated and plasma treated PCBs left no Surlyn residue adhered to the surface which identifies as an adhesive failure. The adhesive failure of the plasma treated sample suggests that only weak bonding was present, therefore no residues were left on the surface under the SEM image. These results also suggest that the peel test for this particular combination was not optimized. In this case, it is believed that the SEM would provide a more accurate representation of the improved adhesion of the silane layer. It is believed that (as shown above for other amine/epoxide samples) this initial peel force can be improved (up to about 10 N/mm greater than that for the non-silane treated materials) through additional fine-tuning of the experimental overmolding conditions and peel testing conditions.

### Example 14: Additional Results Using Amines and Carboxyl Groups

### Sample Preparation

An embodiments of a molded member was prepared using the procedures as described generally in Example 8. Briefly, for the experimental sample, a Nucrel^{®} AE (is a terpolymer of ethylene, methacrylic acid, and acrylate thermoplastic) molding material (having an carboxyl functionality) was overmolded on a functionalized PCB having either a SIB 1824.5, a secondary amine-based silane coupling agent, to form a molded member having bonding layer comprising a silicon-containing linker. In addition to the experimental sample, control samples were prepared. The first control sample was prepared by overmolding Nucrel^{®} AE on a PCB that had not been plasma treated or treated with a silane coupling agent. The second control sample was prepared by overmolding Nucrel^{®} AE on a PCB that had been plasma treated, but that had not been treated with a silane coupling agent. The PCB was identical to that used in Example 9.

### Sample Testing

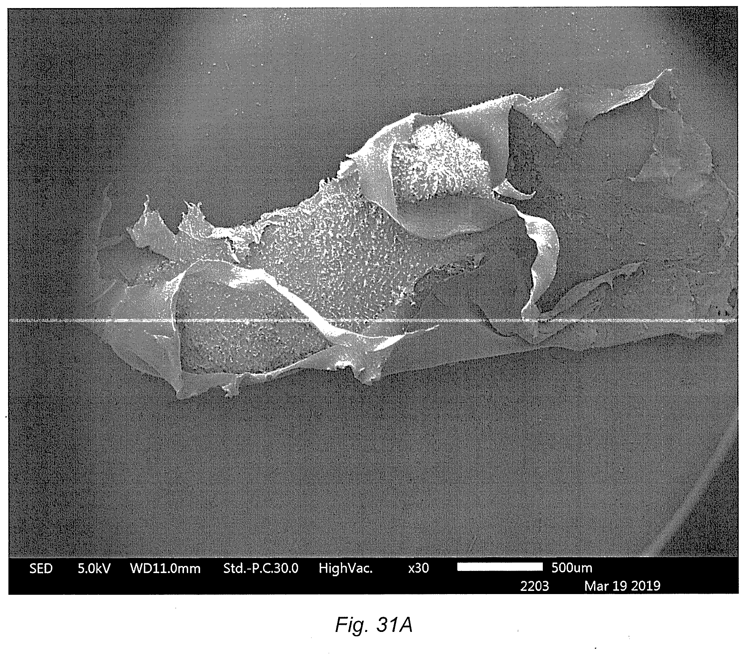

Peel testing was performed in triplicate using the procedures described generally in Example 8. Figures 31A-31D provide SEM results for the secondary amine/carboxyl experimental sample versus controls.

### Results and Discussion

### SIB1824.5

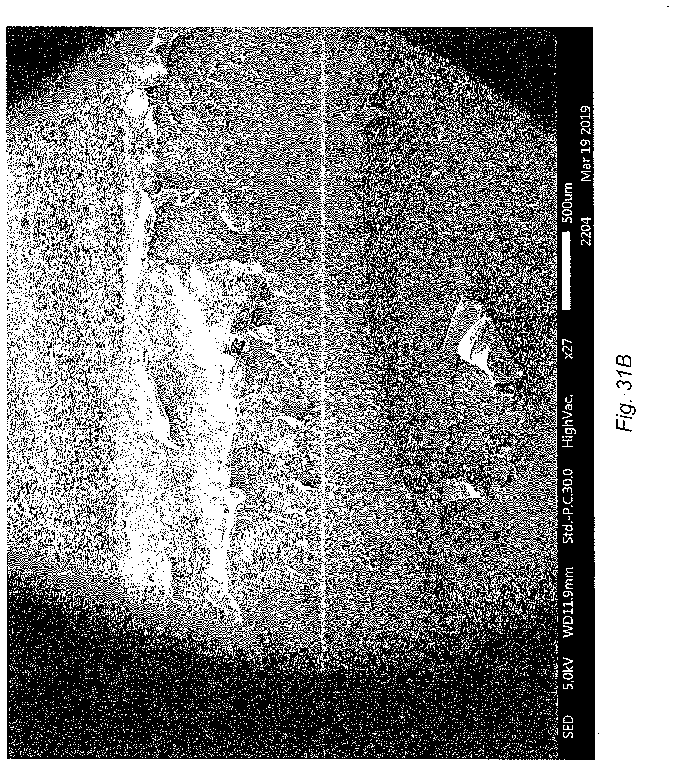

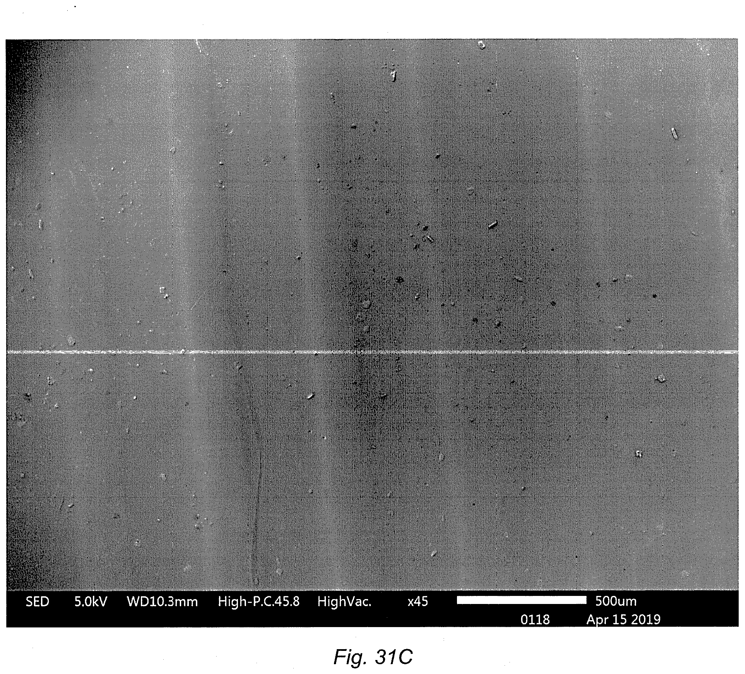

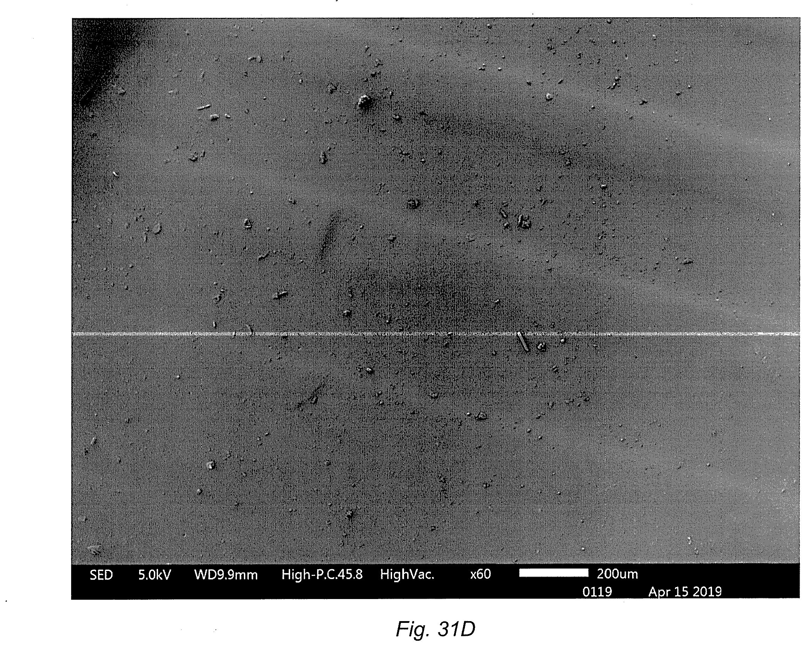

It was observed that the SIB1824.5 sample had a higher peel force than the nontreated sample but a lower peel force compared to plasma-only treated samples. It is believed that the sample preparation was not optimized and therefore the peel force data here was not reliable. However, as shown in Figure 31A and Figure 31B, it was observed in SEM data that Nurcrel AE tore away from the bulk thermoplastic overmold during the peel testing and remained adhered to the PCB after the peel test. This same effect was not observed in either of the control samples, which are shown in Figure 31C (plasma-only) and Figure 31D (non-treated). Figure 31A shows the plastic deformation after the peel test, meaning that this is a cohesive failure. Figure 31B provides evidence that the material deformation could be attributed to chemical interactions between the silane layer and Nucrel/PCB. These chemical interactions appear stronger than the intramolecular forces within the Nucrel bulk material in that the peel test caused parts of Nucrel to break apart, thereby indicating a cohesive failure of the bulk polymer (unlike the images of the non-treated and plasma treated PCBs shows no Nucrel material adhered to the surface).

Figures 31A and 31B suggest that the thermoplastic elastomer layer failed prior to the failure of the bonding layer. The cohesive failure of the silane PCB treated sample suggests that certain regions of the Nucrel are adhered strongly to the PCB by the silane layer, which in turn suggests that the interactions between the silane layer and the Nucrel are stronger than the cohesive forces within the Nucrel itself such that parts of Nucrel break off and/or tear and remain adhered to the PCB after the peel test. This testing additionally suggests that the silane treatment provided certain chemical interactions that enhanced the coupling of the SIB 1824.5 surface treated PCB boards with the Nucrel. Such chemical interactions could include covalent bonding resulting from the secondary amine of the SIB 1824.5 with the carboxy group of Nucrel.

Additionally, the combination of chemical interactions and forces may have contributed to a cohesive failure (e.g., the failure of the bulk material to stay together). Thus, it appears that the cohesive forces in the bulk material are weaker than the adhesive forces between the thermoplastic and bonding layers such that the thermoplastic is torn off. As noted above, it was observed under the SEM, both non-treated and plasma treated PCBs left no Nucrel residue adhered to the surface which identifies as an adhesive failure. The adhesive failure of the plasma treated sample suggests that only weak bonding was present, therefore no residues were left on the surface under the SEM image. These results also suggest that the peel test for this particular combination was not optimized. In this case, it is believed that the SEM would provide a more accurate representation of the improved adhesion of the silane layer. It is believed that (as shown above for other samples) this initial peel force can be improved (up to about 10 N/mm greater than that for the non-silane treated materials) through additional fine-tuning of the experimental overmolding conditions and peel testing conditions.

### Example 15: Results Using Amine / Succinic Anhydride-Based Bonding Layers

### Sample Preparation

Embodiments of molded members were prepared using the procedures as described generally in Example 8. Briefly, for the experimental sample, a thermoplastic Pebax MV 1074 (a block copolymer consisting of polyamide and polyether) molding material (having a secondary amine functionality) was overmolded on a functionalized PCB having a succinic anhydride coupling agent (e.g., functionalized with TEPSA) to form a molded member having bonding layer comprising a silicon containing linker. In addition to the experimental sample, control samples were prepared. The first control sample was prepared by overmolding Pebax MV 1074 on a PCB that had not been plasma treated or treated with a silane coupling agent. The second control sample was prepared by overmolding Pebax MV 1074 on a PCB that had been plasma treated, but that had not been treated with a silane coupling agent. The PCB was identical to that used in Example 9.

### Sample Testing

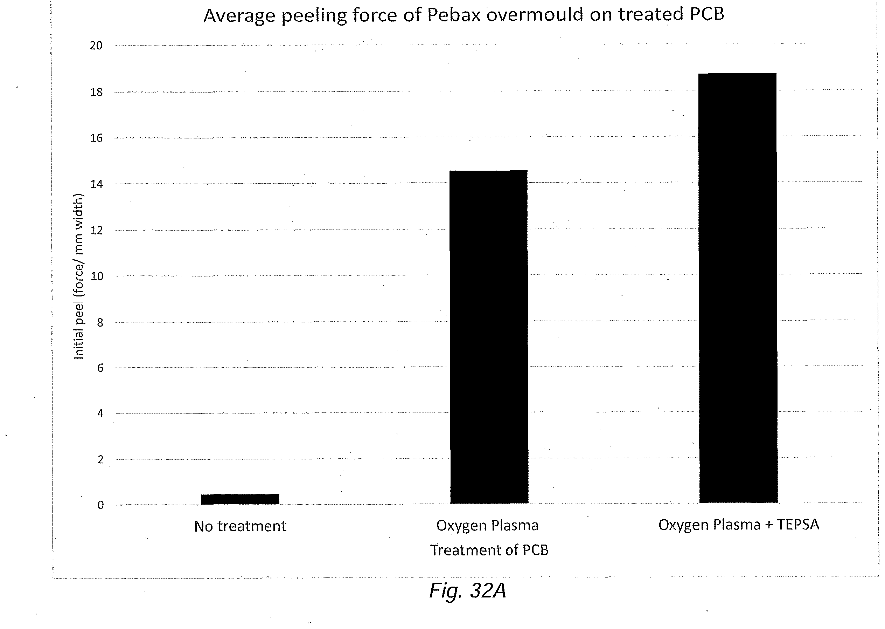

Peel testing was performed in triplicate using the procedures described generally in Example 8. Table 10 below shows the results for the experimental sample versus controls. Figure 32A provides a bar graphs showing a comparison of the average initial peel force for the experimental versus control samples.

**Table 10. Peel data for TEPSA/Pebax MV1074 experimental samples versus controls that were either untreated or only subject to plasma treatment. Peel force is expressed as Force / mm width (N/mm).**

| PCB treatment | Overmould Material | Sample 1 | Sample 2 | Sample 3 | Average (N/mm) |
|---|---|---|---|---|---|
| None | Pebax MV1074 | 0.50 | 0.29 | 0.62 | 0.47 |
| Oxygen Plasma | Pebax MV1074 | 9.99 | 13.89 | 19.75 | 14.54 |
| Oxygen Plasma + TEPSA | Pebax MV1074 | 18.01 | 19.01 | 19.16 | 18.72 |

### Results and Discussion

### TEPSA

As shown in Table 10 and Figure 32A, the peeling forces between the overmold and non-treated / plasma treated-only PCB is lower than the silane treated sample. The initial peeling force for the experimental samples was, on average, about 4.2N/mm higher than for the plasma treated / non-silane treated controls and about 18.2N/mm higher than the non-treated controls. It is believed that (as shown above) this initial peel force can be improved even further (up to or at least about 10 N/mm greater than that for the non-silane treated materials) through additional fine-tuning of the experimental overmolding conditions and peel testing conditions. The observed improved adhesion could be due to increased and/or improved chemical interactions between the silane layer and the thermoplastic/PCB in the silane-treated sample. This result suggests that the silane coupling agent treatment provided improved and/or increased chemical interactions that enhanced the coupling of the TEPSA surface treated PCB boards with the Pebax overmold. Such chemical interactions could include the formation of covalent bonds between the succinic anhydride of the silane with the secondary amine of the overmolding material. Other types of bonding and forces could have also contributed to the increase in the peeling forces observed.

### Example 16: Prophetic Example for additional peel testing

The following is a prophetic example of results that would be expected based on the experiments provided elsewhere herein. Molded members are prepared using optimized conditions. Briefly, for the first experimental sample, a Desmopan 9370A thermoplastic molding material (having a secondary amine) is overmolded on a functionalized PCB having an epoxy-based reactive silane coupling agent (GPTMS) to form a molded member having bonding layer comprising a silicon-containing linker. For a second experimental sample, a Surlyn 9020 thermoplastic molding material (having a carboxyl group) was overmolded on a functionalized PCB having an epoxy ring (epoxide)-based reactive silane coupling agent (GPTMS) to form a molded member having bonding layer comprising a silicon-containing linker. For a third experimental sample, a Nucrel AE thermoplastic molding material (having a carboxyl group) was overmolded on a functionalized PCB having secondary amine (SIB1824.5) reactive silane coupling agent to form a molded member having bonding layer comprising a silicon-containing linker. In addition to the experimental samples above, coinciding control samples are prepared. The first set of control samples are prepared by overmolding the relevant thermoplastics on a PCB that has not been plasma treated. The second set of control samples are prepared by overmolding the relevant thermoplastics on a PCB that has been plasma treated, but that has not been treated with a silane coupling agent.

### Sample Testing and Results

Peel testing is performed in triplicate using the procedures described generally in Example 8. In each case, the peeling forces between the overmold and nontreated / plasma treated-only PCB is lower than the silane treated sample. The initial peeling force for the Desmopan experimental samples is, on average, about 5N/mm to about 10N/mm higher than for the plasma treated / non-silane treated controls and about 5 N/mm to about 15N/mm higher than the non-treated controls. The initial peeling force for the Surlyn experimental samples is, on average, about 5N/mm to about 10N/mm higher than for the plasma treated / non-silane treated controls and about 5N/mm to about 30N/mm higher than the non-treated controls. The initial peeling force for the Nucrel experimental samples is, on average, about 5N/mm to about 10N/mm higher than for the plasma treated / non-silane treated controls and about 5N/mm to about 20N/mm higher than the non-treated controls. Without being bound to any particular mechanism, it is believed that the improved adhesion is due to increased and/or improved chemical interactions between the silane layer and the thermoplastic/PCB in the silane-treated sample. This result suggests that the silane coupling agent treatment provided improved and/or increased chemical interactions that enhanced the coupling of the surface treated PCB boards with the overmolds. Other types of bonding and forces could have also contributed to the increase in the peeling forces observed.

### Example 17: Accelerated Wear Testing

The following is a prophetic example of results that would be expected based on the experiments provided elsewhere herein. An accelerated wear test is performed on experimental examples as disclosed in Examples 9-16 above. In addition to these experimental samples, control samples with coinciding molding materials and PCBs are prepared. The first set of control samples (Cont 1) is prepared by overmolding a molding material (coinciding to the molding material of an experimental sample) on a PCB that has not been plasma treated or treated with a silane coupling agent. The second control sample (Cont 2) is prepared by overmolding a molding material (coinciding to the molding material of an experimental sample) on a PCB that has been plasma treated, but that has not been treated with a silane coupling agent.

Each experimental sample and control sample is placed in a high humidity chamber (99% humidity) at elevated temperature (60°C). A unit of time under these conditions is equivalent to about 5 times the unit of time experienced under normal operating conditions (e.g., 1 day under accelerated wear conditions is equivalent to 5 days under normal operating conditions). After each week (equivalent to 5 weeks under normal conditions), the samples are removed from the accelerated wear chamber and examined for any signs of delamination. Table 15 below shows the expected results of testing:

**Table 15.**

| PCB | | Molding material | | Results (Days (under normal operating conditions) to Delamination) | | |
|---|---|---|---|---|---|---|
| Silane | Functional | Thermoplastic | Functional | Expt | Cont 1 | Cont 2 |
| coupling agent | group | | group | | | |
| GPTMS or cyclic epoxy | Epoxy ring | Surlyn^{®} 9020 ionomer | Carboxyl group | > 9 | < 1 | ≤9 |
| | | Nucrel^{®} AE | Carboxyl group | > 9 | < 1 | ≤9 |
| | | Desmopan^{®} 9370A | Secondary amine | > 20 | < 7 | ≤ 20 |
| | | Pebax^{®} MV 1074 | Secondary amine | > 16 | < 6 | ≤ 16 |
| APTMS/ WSA-9911 | Primary amine | Elvaloy^{®} PTW | Epoxy ring | > 10 | < 1 | ≤ 10 |
| | | Surlyn^{®} 9020 ionomer | Carboxyl group | > 10 | < 1 | ≤ 10 |
| | | Nucrel^{®} AE | Carboxyl group | > 8 | < 1 | ≤8 |
| | | Scona^{®} TPPP 9012 PA | Succinic anhydride | > 16 | < 1 | ≤ 16 |
| SIB1833.0 | Secondary amine | Elvaloy^{®} PTW | Epoxy ring | > 13 | < 1 | ≤ 13 |
| | | Surlyn^{®} 9020 ionomer | Carboxyl group | > 11 | < 1 | ≤ 11 |
| | | Nucrel^{®} AE | Carboxyl group | > 10 | < 1 | ≤ 10 |
| | | Scona^{®} TPPP 9012 PA | Succinic anhydride | > 14 | < 1 | ≤ 14 |
| TEPSA | Succinic anhydride | Desmopan^{®} 9370A | Secondary amine | > 17 | < 7 | ≤ 17 |
| | | Pebax^{®} MV 1074 | Secondary amine | > 15 | <6 | ≤ 15 |
| TMOS | Methoxyl | PEX | Reactive Silanol | > 3 | < 1 | ≤3 |
| TEOS/ | Ethoxyl | | | > 5 | < 1 | ≤5 |
| Nonfunctional dipodal (SIB1817.0) | | | | | | |

It is surprisingly observed that, for some experimental samples, the overmolding material itself appears to begin to degrade before the bonding layer between the molding material and the PCB. The invention is defined in the following claims.

## Claims

1. A respiratory humidification apparatus (601; 700; 1000; 1500; 1604) comprising:
a gas flow passage (602; 718; 1005; 1610) having an internal area, the gas flow passage (602; 718; 1005; 1610) having an inlet (623; 731; 1001; 1501; 1614) configured to receive gases into the internal area of the gas flow passage (602; 718; 1005; 1610) and an outlet (622; 733; 1003; 1502; 1618) configured to allow the passage of gases out of the internal area of the gas flow passage (602; 718; 1005; 1610);
a heater (400; 500; 614; 714; 800; 800A; 800B; 1014; 1622) having a heating surface (1033), the heater (400; 500; 614; 714; 800; 800A; 800B; 1014; 1622) disposed between the inlet (623; 731; 1001; 1501; 1614) and the outlet (622; 733; 1003; 1502; 1618) of the gas flow passage (602; 718; 1005; 1610) and configured to heat a humidification liquid received by the heating surface (1033) to humidify gases flowing through the gas flow passage (602; 718; 1005; 1610), the heater (400; 500; 614; 714; 800; 800A; 800B; 1014; 1622) comprising:
a printed circuit board (PCB) (401; 501; 801) with heating tracks (811; 811A; 811B), the PCB (401; 501; 801) having at least one electrical contact (852; 857; 851A; 851B);
a molding material (405; 505) disposed over at least a portion of the PCB (401; 501; 801); and
a bonding layer comprising a silicon-containing linker (14), the bonding layer coupling at least a portion of the molding material (405; 505) to the PCB (401; 501; 801);
wherein the respiratory humidification apparatus (601; 700; 1000; 1500; 1604) is configured to provide humidified gases to a patient.

2. The respiratory humidification apparatus of claim 1, wherein the PCB (401; 501; 801) spans a portion of the gas flow passage (602; 718; 1005; 1610) from the internal area to an area that is external to the gas flow passage (602; 718; 1005; 1610); wherein a portion of the PCB (401; 501; 801) is exposed in the external area; and wherein a portion of the PCB (401; 501; 801) is in thermal communication with the internal area of the gas flow passage (602; 718; 1005; 1610).

3. The respiratory humidification apparatus of claim 1 or 2, wherein the PCB (401; 501; 801) comprises one or more electrical components configured to receive, transmit, and/or process information about conditions of the respiratory humidification apparatus (601; 700; 1000; 1500; 1604) and/or gas flow passage (602; 718; 1005; 1610).

4. The respiratory humidification apparatus of any one of claims 1 to 3, wherein the PCB (401; 501; 801) comprises one or more temperature sensors (641; 821) in thermal communication with the internal area of the gas flow passage (602; 718; 1005; 1610),
and/or wherein the PCB (401; 501; 801) comprises two or more temperature sensors (641; 821) and wherein at least one of the temperature sensors (641; 821) is configured to measure a temperature of the heating surface (1033),
and/or the PCB (401; 501; 801) comprises a temperature sensor (641; 821) configured to detect whether the heating surface (1033) is wetted by the humidification liquid in at least one region,
wherein, optionally, at least one temperature sensor (641; 821) is located at, on, adjacent, or proximal to the heating surface (1033).

5. The respiratory humidification apparatus of any one of the preceding claims, wherein the PCB (401; 501; 801) comprises one or more of a pressure sensor (642), a flow sensor (634, 638), and/or a humidity sensor (636, 640), wherein the one or more of a pressure sensor (642), a flow sensor (634, 638), and/or a humidity sensor (636, 640) are configured to detect parameters of a gas in the gas flow passage (602; 718; 1005; 1610),
and/or wherein the PCB (401; 501; 801) comprises a fluid level sensor and/or a liquid sensor.

6. The respiratory humidification apparatus of any one of claims 3 to 5, wherein the molding material (405; 505) covers one or more of the electrical components and/or sensors of the PCB (401; 501; 801); and wherein the molding material (405; 505) separates the one or more of the electrical components and/or sensors from direct contact to the internal area of the gas flow passage (602; 718; 1005; 1610),
wherein, optionally, the molding material (405; 505) is configured to allow monitoring of the internal area of the gas flow passage (602; 718; 1005; 1610) by the electrical components and/or sensors of the PCB (401; 501; 801) through the molding material (405; 505).

7. The respiratory humidification apparatus of any one of the preceding claims, wherein the heating tracks (811; 811A; 811B) of the PCB (401; 501; 801) are in thermal communication with the internal area of the gas flow passage (602; 718; 1005; 1610),
wherein, optionally, the heating tracks (811; 811A; 811B) of the PCB (401; 501; 801) are covered by the molding material (405; 505) and are in thermal communication with the internal area of the gas flow passage (602; 718; 1005; 1610) through the molding material (405; 505), and
wherein, optionally, the heating surface (1033) comprises a portion of the molding material (405; 505) covering the heating tracks (811; 811A; 811B).

8. The respiratory humidification apparatus of any one of the preceding claims, wherein the respiratory humidification apparatus (601; 700; 1000; 1500; 1604) comprises a humidification liquid inlet (616; 708; 1021; 1542) for delivering the humidification liquid from a reservoir (606) to the heater (400; 500; 614; 714; 800; 800A; 800B; 1014; 1622),
and/or the respiratory humidification apparatus (601; 700; 1000; 1500; 1604) comprises a humidification liquid pre-heater,
and/or the respiratory humidification apparatus (601; 700; 1000; 1500; 1604) comprises a humidification liquid flow controller (610), wherein, optionally, the liquid flow controller (610) comprises a metering system and/or a pump.

9. The respiratory humidification apparatus of any one of the preceding claims, wherein the respiratory humidification apparatus (601; 700; 1000; 1500; 1604) comprises a gas pre-heater.

10. The respiratory humidification apparatus of any one of the preceding claims, wherein the molding material (405; 505) comprises a thermoplastic material or a thermosetting material.

11. The respiratory humidification apparatus of any one of the preceding claims, wherein the molding material (405; 505) encapsulates at least a portion of the PCB (401; 501; 801),
and/or the at least one electrical contact (852; 857; 851A; 851B) of the PCB (401; 501; 801) is not encapsulated and/or covered by the molding material (405; 505),
wherein, optionally, the portion of the PCB (401; 501; 801) encapsulated by the molding material (405; 505) comprises a portion of the heating tracks (811; 811A; 811B), and
wherein, optionally, the molding material (405; 505) provides a barrier between the PCB (401; 501; 801) and the gas flow passage (602; 718; 1005; 1610).

12. The respiratory humidification apparatus of any one of the preceding claims, wherein a portion of the molding material (405; 505) comprises micro-channels (406; 506; 902A, 903A) and/or surface structures,
wherein, optionally, the micro-channels (406; 506; 902A, 903A) and/or surface structures are configured to receive, distribute, and/or hold the humidification liquid so it can be heated by the heating tracks (811; 811A; 811B).

13. The respiratory humidification apparatus of any one of the preceding claims, wherein a portion of the molding material (405; 505) is configured to receive the humidification liquid and retain it so that it can be heated by the heating tracks (811; 811A; 811B),
wherein, optionally, the portion of molding material (405; 505) that is configured to receive the humidification liquid is coupled to the PCB (401; 501; 801) by the bonding layer.

14. The respiratory humidification apparatus of any one of the preceding claims, wherein the molding material (405; 505) is hydrophilic or hydrophobic.

15. The respiratory humidification apparatus of any one of the preceding claims, wherein the bonding layer chemically couples the molding material (405; 505) to the portion of the PCB (401; 501; 801).

## Patentansprüche

1. Atembefeuchtungseinrichtung (601; 700; 1000; 1500; 1604), umfassend:
einen Gasströmungskanal (602; 718; 1005; 1610) mit einem Innenbereich, wobei der Gasströmungskanal (602; 718; 1005; 1610) einen Einlass (623; 731; 1001; 1501; 1614) aufweist, der so konfiguriert ist, dass er Gase in den Innenbereich des Gasströmungskanals (602; 718; 1005; 1610) aufnimmt, und einen Auslass (622; 733; 1003; 1502; 1618), der so konfiguriert ist, dass er den Durchgang von Gasen aus dem Innenbereich des Gasströmungskanals (602; 718; 1005; 1610) ermöglicht;
eine Heizvorrichtung (400; 500; 614; 714; 800; 800A; 800B; 1014; 1622) mit einer Heizfläche (1033), wobei die Heizvorrichtung (400; 500; 614; 714; 800; 800A; 800B; 1014; 1622) zwischen dem Einlass (623; 731; 1001; 1501; 1614) und dem Auslass (622; 733; 1003; 1502; 1618) des Gasströmungskanals (602; 718; 1005; 1610) angeordnet ist und so konfiguriert ist, dass sie eine von der Heizfläche (1033) aufgenommene Befeuchtungsflüssigkeit erhitzt, um Gase zu befeuchten, die durch den Gasströmungskanal (602; 718; 1005; 1610) strömen, wobei die Heizvorrichtung (400; 500; 614; 714; 800; 800A; 800B; 1014; 1622) umfasst:
eine Leiterplatte (PCB) (401; 501; 801) mit Heizbahnen (811; 811A; 811B), wobei die PCB (401; 501; 801) mindestens einen elektrischen Kontakt (852; 857; 851A; 851B) aufweist;
ein Formmaterial (405; 505), das über mindestens einem Abschnitt der PCB (401; 501; 801) angeordnet ist; und
eine Verbindungsschicht, die einen siliziumhaltigen Linker (14) umfasst, wobei die Verbindungsschicht mindestens einen Abschnitt des Formmaterials (405; 505) mit der PCB (401; 501; 801) koppelt;
wobei die Atembefeuchtungseinrichtung (601; 700; 1000; 1500; 1604) so konfiguriert ist, dass sie einem Patienten befeuchtete Gase bereitstellt.

2. Atembefeuchtungseinrichtung nach Anspruch 1, wobei sich die PCB (401; 501; 801) über einen Abschnitt des Gasströmungskanals (602; 718; 1005; 1610) von dem Innenbereich zu einem Bereich außerhalb des Gasströmungskanals (602; 718; 1005; 1610) erstreckt; wobei ein Abschnitt der PCB (401; 501; 801) dem Außenbereich ausgesetzt ist; und wobei ein Abschnitt der PCB (401; 501; 801) in thermischer Verbindung mit dem Innenbereich des Gasströmungskanals (602; 718; 1005; 1610) steht.

3. Atembefeuchtungseinrichtung nach Anspruch 1 oder 2, wobei die PCB (401; 501; 801) eine oder mehrere elektrische Komponenten umfasst, die so konfiguriert sind, dass sie Informationen über Zustände der Atembefeuchtungseinrichtung (601; 700; 1000; 1500; 1604) und/oder des Gasströmungskanals (602; 718; 1005; 1610) empfangen, übertragen und/oder verarbeiten.

4. Atembefeuchtungseinrichtung nach einem der Ansprüche 1 bis 3, wobei die PCB (401; 501; 801) einen oder mehrere Temperatursensoren (641; 821) umfasst, die in thermischer Verbindung mit dem Innenbereich des Gasströmungskanals (602; 718; 1005; 1610) stehen,
und/oder wobei die PCB (401; 501; 801) zwei oder mehr Temperatursensoren (641; 821) umfasst und wobei mindestens einer der Temperatursensoren (641; 821) so konfiguriert ist, dass er eine Temperatur der Heizfläche (1033) misst,
und/oder die PCB (401; 501; 801) einen Temperatursensor (641; 821) umfasst, der so konfiguriert ist, dass er detektiert, ob die Heizfläche (1033) in mindestens einem Bereich von der Befeuchtungsflüssigkeit benetzt ist,
wobei optional mindestens ein Temperatursensor (641; 821) an, auf, neben oder in der Nähe der Heizfläche (1033) angeordnet ist.

5. Atembefeuchtungseinrichtung nach einem der vorstehenden Ansprüche, wobei die PCB (401; 501; 801) einen oder mehrere von einem Drucksensor (642), einem Strömungssensor (634, 638) und/oder einem Feuchtigkeitssensor (636, 640) umfasst, wobei der eine oder die mehreren von einem Drucksensor (642), einem Strömungssensor (634, 638) und/oder einem Feuchtigkeitssensor (636, 640) so konfiguriert ist/sind, dass er/sie Parameter eines Gases in dem Gasströmungskanal (602; 718; 1005; 1610) detektiert/detektieren,
und/oder wobei die PCB (401; 501; 801) einen Fluidstandssensor und/oder einen Flüssigkeitssensor umfasst.

6. Atembefeuchtungseinrichtung nach einem der Ansprüche 3 bis 5, wobei das Formmaterial (405; 505) einen oder mehrere von den elektrischen Komponenten und/oder Sensoren der PCB (401; 501; 801) abdeckt; und wobei das Formmaterial (405; 505) die eine oder mehreren von den elektrischen Komponenten und/oder Sensoren von einem direkten Kontakt mit dem Innenbereich des Gasströmungskanals (602; 718; 1005; 1610) trennt,
wobei das Formmaterial (405; 505) optional so konfiguriert ist, dass es eine Überwachung des Innenbereichs des Gasströmungskanals (602; 718; 1005; 1610) durch die elektrischen Komponenten und/oder Sensoren der PCB (401; 501; 801) durch das Formmaterial (405; 505) ermöglicht.

7. Atembefeuchtungseinrichtung nach einem der vorstehenden Ansprüche, wobei die Heizbahnen (811; 811A; 811B) der PCB (401; 501; 801) in thermischer Verbindung mit dem Innenbereich des Gasströmungskanals (602; 718; 1005; 1610) stehen,
wobei optional die Heizbahnen (811; 811A; 811B) der PCB (401; 501; 801) durch das Formmaterial (405; 505) abgedeckt sind und über das Formmaterial (405; 505) in thermischer Verbindung mit dem Innenbereich des Gasströmungskanals (602; 718; 1005; 1610) stehen, und
wobei die Heizfläche (1033) optional einen Abschnitt des Formmaterials (405; 505) umfasst, der die Heizbahnen (811; 811A; 811B) abdeckt.

8. Atembefeuchtungseinrichtung nach einem der vorstehenden Ansprüche, wobei die Atembefeuchtungseinrichtung (601; 700; 1000; 1500; 1604) einen Befeuchtungsflüssigkeitseinlass (616; 708; 1021; 1542) zum Zuführen der Befeuchtungsflüssigkeit aus einem Vorratsbehälter (606) zu der Heizvorrichtung (400; 500; 614; 714; 800; 800A; 800B; 1014; 1622) umfasst,
und/oder die Atembefeuchtungseinrichtung (601; 700; 1000; 1500; 1604) eine Befeuchtungsflüssigkeitsvorheizvorrichtung umfasst,
und/oder die Atembefeuchtungseinrichtung (601; 700; 1000; 1500; 1604) eine Befeuchtungsflüssigkeitsströmungsregler (610) umfasst, wobei der Flüssigkeitsströmungsregler (610) optional ein Dosiersystem und/oder eine Pumpe umfasst.

9. Atembefeuchtungseinrichtung nach einem der vorstehenden Ansprüche, wobei die Atembefeuchtungseinrichtung (601; 700; 1000; 1500; 1604) eine Gasvorheizvorrichtung umfasst.

10. Atembefeuchtungseinrichtung nach einem der vorstehenden Ansprüche, wobei das Formmaterial (405; 505) ein thermoplastisches Material oder ein thermisch aushärtbares Material umfasst.

11. Atembefeuchtungseinrichtung nach einem der vorstehenden Ansprüche, wobei das Formmaterial (405; 505) mindestens einen Abschnitt der PCB (401; 501; 801) ummantelt,
und/oder der mindestens eine elektrische Kontakt (852; 857; 851A; 851B) der PCB (401; 501; 801) nicht von dem Formmaterial (405; 505) ummantelt und/oder bedeckt ist, wobei optional der mit dem Formmaterial (405; 505) ummantelte Abschnitt der PCB (401; 501; 801) einen Abschnitt der Heizbahnen (811; 811A; 811B) umfasst, und
wobei das Formmaterial (405; 505) optional eine Barriere zwischen der PCB (401; 501; 801) und dem Gasströmungskanal (602; 718; 1005; 1610) bildet.

12. Atembefeuchtungseinrichtung nach einem der vorstehenden Ansprüche, wobei ein Abschnitt des Formmaterials (405; 505) Mikrokanäle (406; 506; 902A, 903A) und/oder Oberflächenstrukturen umfasst,
wobei die Mikrokanäle (406; 506; 902A, 903A) und/oder Oberflächenstrukturen optional so konfiguriert sind, dass sie die Befeuchtungsflüssigkeit aufnehmen, verteilen und/oder halten, damit sie durch die Heizbahnen (811; 811A; 811B) erhitzt werden kann.

13. Atembefeuchtungseinrichtung nach einem der vorstehenden Ansprüche, wobei ein Abschnitt des Formmaterials (405; 505) so konfiguriert ist, dass er die Befeuchtungsflüssigkeit aufnimmt und zurückhält, so dass sie durch die Heizbahnen (811; 811A; 811B) erhitzt werden kann,
wobei optional der Abschnitt des Formmaterials (405; 505), der so konfiguriert ist, dass er die Befeuchtungsflüssigkeit aufnimmt, durch die Verbindungsschicht mit der PCB (401; 501; 801) gekoppelt ist.

14. Atembefeuchtungseinrichtung nach einem der vorstehenden Ansprüche, wobei das Formmaterial (405; 505) hydrophil oder hydrophob ist.

15. Atembefeuchtungseinrichtung nach einem der vorstehenden Ansprüche, wobei die Verbindungsschicht das Formmaterial (405; 505) chemisch mit dem Abschnitt der PCB (401; 501; 801) koppelt.

## Revendications

1. Appareil d'humidification respiratoire (601 ; 700 ; 1000 ; 1500 ; 1604) comprenant :
un passage de flux de gaz (602 ; 718 ; 1005 ; 1610) présentant une zone interne, le passage de flux de gaz (602 ; 718 ; 1005 ; 1610) présentant une entrée (623 ; 731 ; 1001 ; 1501 ; 1614) configurée pour recevoir des gaz dans la zone interne du passage de flux de gaz (602 ; 718 ; 1005 ; 1610) et une sortie (622 ; 733 ; 1003 ; 1502 ; 1618) configurée pour permettre le passage des gaz hors de la zone interne du passage de flux de gaz (602 ; 718 ; 1005 ; 1610) ;
un réchauffeur (400 ; 500 ; 614 ; 714 ; 800 ; 800A ; 800B ; 1014 ; 1622) présentant une surface chauffante (1033), le réchauffeur (400 ; 500 ; 614 ; 714 ; 800 ; 800A ; 800B ; 1014 ; 1622) étant disposé entre l'entrée (623 ; 731 ; 1001 ; 1501 ; 1614) et la sortie (622 ; 733 ; 1003 ; 1502 ; 1618) du passage de flux de gaz (602 ; 718 ; 1005 ; 1610) et configuré pour chauffer un liquide d'humidification reçu par la surface chauffante (1033) pour humidifier des gaz circulant à travers le passage de flux de gaz (602 ; 718 ; 1005 ; 1610), le réchauffeur (400 ; 500 ; 614 ; 714 ; 800 ; 800A ; 800B ; 1014 ; 1622) comprenant :
une carte de circuit imprimé (PCB) (401 ; 501 ; 801) avec des pistes chauffantes (811 ; 811A ; 811B), la PCB (401 ; 501 ; 801) présentant au moins un contact électrique (852 ; 857 ; 851A ; 851B) ;
un matériau de moulage (405 ; 505) disposé au-dessus d'au moins une partie de la PCB (401 ; 501 ; 801) ; et
une couche de liaison comprenant un liant contenant du silicium (14), la couche de liaison couplant au moins une partie du matériau de moulage (405 ; 505) à la PCB (401 ; 501 ; 801) ;
dans lequel l'appareil d'humidification respiratoire (601 ; 700 ; 1000 ; 1500 ; 1604) est configuré pour fournir des gaz humidifiés à un patient.

2. Appareil d'humidification respiratoire selon la revendication 1, dans lequel la PCB (401 ; 501 ; 801) recouvre une partie du passage de flux de gaz (602 ; 718 ; 1005 ; 1610) de la zone interne à une zone externe au passage de flux de gaz (602 ; 718 ; 1005 ; 1610) ; dans lequel une partie de la PCB (401 ; 501 ; 801) est exposée dans la zone externe ; et dans lequel une partie de la PCB (401 ; 501 ; 801) est en communication thermique avec la zone interne du passage de flux de gaz (602 ; 718 ; 1005 ; 1610).

3. Appareil d'humidification respiratoire selon la revendication 1 ou 2, dans lequel la PCB (401 ; 501 ; 801) comprend un ou plusieurs composants électriques configurés pour recevoir, transmettre, et/ou traiter des informations relatives aux conditions de l'appareil d'humidification respiratoire (601 ; 700 ; 1000 ; 1500 ; 1604) et/ou du passage de flux de gaz (602 ; 718 ; 1005 ; 1610).

4. Appareil d'humidification respiratoire selon l'une quelconque des revendications 1 à 3, dans lequel la PCB (401 ; 501 ; 801) comprend un ou plusieurs capteurs de température (641 ; 821) en communication thermique avec la zone interne du passage de flux de gaz (602 ; 718 ; 1005 ; 1610),
et/ou dans lequel la PCB (401 ; 501 ; 801) comprend deux ou plusieurs capteurs de température (641 ; 821) et dans lequel au moins un des capteurs de température (641 ; 821) est configuré pour mesurer une température de la surface chauffante (1033),
et/ou la PCB (401 ; 501 ; 801) comprend un capteur de température (641 ; 821) configuré pour détecter si la surface chauffante (1033) est mouillée par le liquide d'humidification dans au moins une région,
dans lequel, facultativement, au moins un capteur de température (641 ; 821) est situé au niveau de, sur, de manière adjacente à, ou proximal par rapport à la surface chauffante (1033).

5. Appareil d'humidification respiratoire selon l'une quelconque des revendications précédentes, dans lequel la PCB (401 ; 501 ; 801) comprend un ou plusieurs d'un capteur de pression (642), d'un capteur de flux (634, 638), et/ou d'un capteur d'humidité (636, 640), dans lequel le un ou les plusieurs d'un capteur de pression (642), d'un capteur de flux (634, 638), et/ou d'un capteur d'humidité (636, 640) sont configurés pour détecter des paramètres d'un gaz dans le passage de flux de gaz (602 ; 718 ; 1005 ; 1610),
et/ou dans lequel la PCB (401 ; 501 ; 801) comprend un capteur de niveau de fluide et/ou un capteur de liquide.

6. Appareil d'humidification respiratoire selon l'une quelconque des revendications 3 à 5, dans lequel le matériau de moulage (405 ; 505) couvre un ou plusieurs des composants électriques et/ou des capteurs de la PCB (401 ; 501 ; 801) ; et dans lequel le matériau de moulage (405 ; 505) sépare le un ou les plusieurs des composants électriques et/ou capteurs d'un contact direct avec la zone interne du passage de flux de gaz (602 ; 718 ; 1005 ; 1610),
dans lequel, facultativement, le matériau de moulage (405 ; 505) est configuré pour permettre la surveillance de la zone interne du passage de flux de gaz (602 ; 718 ; 1005 ; 1610) par les composants électriques et/ou les capteurs de la PCB (401 ; 501 ; 801) à travers le matériau de moulage (405 ; 505).

7. Appareil d'humidification respiratoire selon l'une quelconque des revendications précédentes, dans lequel les pistes chauffantes (811 ; 811A ; 811B) de la PCB (401 ; 501 ; 801) sont en communication thermique avec la zone interne du passage de flux de gaz (602 ; 718 ; 1005 ; 1610),
dans lequel, facultativement, les pistes chauffantes (811 ; 811A ; 811B) de la PCB (401 ; 501 ; 801) sont couvertes par le matériau de moulage (405 ; 505) et sont en communication thermique avec la zone interne du passage de flux de gaz (602 ; 718 ; 1005 ; 1610) à travers le matériau de moulage (405 ; 505), et
dans lequel, facultativement, la surface chauffante (1033) comprend une partie du matériau de moulage (405 ; 505) couvrant les pistes chauffantes (811 ; 811A ; 811B).

8. Appareil d'humidification respiratoire selon l'une quelconque des revendications précédentes, dans lequel l'appareil d'humidification respiratoire (601 ; 700 ; 1000 ; 1500 ; 1604) comprend une entrée de liquide d'humidification (616 ; 708 ; 1021 ; 1542) pour la fourniture du liquide d'humidification d'un réservoir (606) vers le réchauffeur (400 ; 500 ; 614 ; 714 ; 800 ; 800A ; 800B ; 1014 ; 1622),
et/ou l'appareil d'humidification respiratoire (601; 700 ; 1000 ; 1500 ; 1604) comprend un préchauffeur de liquide d'humidification,
et/ou l'appareil d'humidification respiratoire (601; 700 ; 1000 ; 1500 ; 1604) comprend un contrôleur de flux de liquide d'humidification (610), dans lequel, facultativement, le contrôleur de flux de liquide (610) comprend un système de mesure et/ou une pompe.

9. Appareil d'humidification respiratoire selon l'une quelconque des revendications précédentes, dans lequel l'appareil d'humidification respiratoire (601 ; 700 ; 1000 ; 1500 ; 1604) comprend un préchauffeur de gaz.

10. Appareil d'humidification respiratoire selon l'une quelconque des revendications précédentes, dans lequel le matériau de moulage (405 ; 505) comprend un matériau thermoplastique ou un matériau thermodurcissable.

11. Appareil d'humidification respiratoire selon l'une quelconque des revendications précédentes, dans lequel le matériau de moulage (405 ; 505) encapsule au moins une partie de la PCB (401 ; 501 ; 801),
et/ou le au moins un contact électrique (852 ; 857 ; 851A ; 851B) de la PCB (401 ; 501 ; 801) n'est pas encapsulé et/ou couvert par le matériau de moulage (405 ; 505),
dans lequel, facultativement, la partie de la PCB (401 ; 501 ; 801) encapsulée par le matériau de moulage (405 ; 505) comprend une partie des pistes chauffantes (811 ; 811A ; 811B), et
dans lequel, facultativement, le matériau de moulage (405 ; 505) fournit une barrière entre la PCB (401 ; 501 ; 801) et le passage de flux de gaz (602 ; 718 ; 1005 ; 1610).

12. Appareil d'humidification respiratoire selon l'une quelconque des revendications précédentes, dans lequel une partie du matériau de moulage (405 ; 505) comprend des micro-canaux (406 ; 506 ; 902A ; 903A) et/ou des structures de surface,
dans lequel, facultativement, les micro-canaux (406 ; 506 ; 902A ; 903A) et/ou les structures de surface sont configuré(e)s pour recevoir, distribuer et/ou retenir le liquide d'humidification de telle manière qu'il puisse être chauffé par les pistes chauffantes (811 ; 811A ; 811B).

13. Appareil d'humidification respiratoire selon l'une quelconque des revendications précédentes, dans lequel une partie du matériau de moulage (405 ; 505) est configurée pour recevoir le liquide d'humidification et le retenir de telle manière qu'il puisse être chauffé par les pistes chauffantes (811 ; 811A ; 811B),
dans lequel, facultativement, la partie du matériau de moulage (405 ; 505) configurée pour recevoir le liquide d'humidification est couplée à la PCB (401 ; 501 ; 801) par la couche de liaison.

14. Appareil d'humidification respiratoire selon l'une quelconque des revendications précédentes, dans lequel le matériau de moulage (405 ; 505) est hydrophile ou hydrophobe.

15. Appareil d'humidification respiratoire selon l'une quelconque des revendications précédentes, dans lequel la couche de liaison couple chimiquement le matériau de moulage (405 ; 505) à la partie de la PCB (401 ; 501 ; 801).
